# EUROPEAN PATENT APPLICATION

(11) **EP 1 903 056 A2**
(43) Date of publication of application: **26.03.2008**
(21) Application number: 07023757.3
(22) Date of filing: 10.12.2003
(51) Int. Cl.: C07K 14/47, A61K 38/00

(54) **HLA-A1, -A2 -A3, -A24, -B7, and -B44 tumor associated antigen peptides and compositions**

(30) Priority: 10.12.2002 US 432017 P
(62) Divisional of application: 03812851.8
(71) Applicant: IDM Pharma, Inc., San Diego, CA 92121 (US)
(72) Inventor: Keogh, Elissa A., San Diego, CA 92122 (US); Southwood, Scott, Santee, CA 92071 (US); Fikes, John D., San Diego, CA 92122 (US); Sette, Alessandro, La Jolla, CA 92037 (US)
(74) Representative: Cripps, Joanna Elizabeth

(57) **Abstract**

A peptide or composition comprising at least one epitope or analog from CEA, HER2/neu, MAGE2, MAGE3, or p53.

## Description

### BACKGROUND OF THE INVENTION

### Field of the Invention

This invention relates to the field of biology. In a particular embodiment, it relates to peptides, polynucleotides, and compositions useful to monitor or elicit an immune response to selected tumor-associated antigens.

### Related Art

The field of immunotherapy is yielding new approaches for the treatment of cancer, including the development of improved cancer vaccines (Krul, K.G., Decision Resources, 10.1-10.25 (1998)). While vaccines provide a mechanism of directing immune responses towards the tumor cells, there are a number of mechanisms by which tumor cells circumvent immunological processes (Pardoll, D. M., Nature Medicine (Vaccine Supplement), 4:525-531 (1998)). Recent advances indicate that the efficacy of peptide vaccines may be increased when combined with approaches which enhance the stimulation of immune responses, such as the use of Interleukin-2 or autologous dendritic cells (DC) (Abbas et al., eds., Cellular and Molecular Immunology, 3rd Edition, W. B. Saunders Company, pub. (1997)).

In a Phase I study, Murphy, *et al.,* demonstrated that Human Leukocyte Antigen (HLA)-A2-binding peptides corresponding to sequences present in prostate specific antigen (PSA) stimulated specific cytotoxic T-cell lymphocyte (CTL) responses in patients with prostate cancer (Murphy et al., The Prostate 29:371-380 (1996)). Recently, Rosenberg, *et al.,* evaluated the safety and mechanism of action of a synthetic HLA-A2 binding peptide derived from the melanoma associated antigen, gp100, as a cancer vaccine to treat patients with metastatic melanoma (Rosenberg et al., Nature Med., 4:321-327 (1998)). Based on immunological assays, 91% of patients were successfully immunized with the synthetic peptide. In addition, 42% (13/31) of patients who received the peptide vaccine in combination with IL-2 treatment, demonstrated objective cancer responses. Finally, Nestle, *et al.,* reported the vaccination of 16 melanoma patients with peptide- or tumor lysate-pulsed DC (Nestle et al., Nature Med4:328-332 (1998)). Peptide-pulsed DC induced immune responses in (11/12) patients immunized with a vaccine comprised of 1-2 peptides. Objective responses were evident in 5/16 (3 peptide-pulsed, 2 tumor-lysate pulsed) evaluated patients in this study. These Phase I safety studies provided evidence that HLA-A2 binding peptides of known tumor-associated antigens demonstrate the expected mechanism of action. These vaccines were generally safe and well tolerated. Vaccine molecules related to four cancer antigens, CEA, HER2/neu, MAGE2, and, MAGE3 have been disclosed. (Kawashima et al., Human Immunology, 59:1-14(1998))

Preclinical studies have shown that vaccine-pulsed DC mediate anti-tumor effects through the stimulation of antigen-specific CTL (Mandelboim et al., Nature Med., 1: 1179-1183 (1995); Celluzzi et al., J Exp Med 183:283-287 (1996); Zitvogel et al., J Exp Med 183:87-97 (1996); Mayordomo et al., Nature Med 1:1297-1302 (1995)). CTL directly lyse tumor cells and also secrete an array of cytoldnes such as interferon gamma (IFNγ), tumor necrosis factor (TNF) and granulocyte-macrophage colony stimulating factor (GM-CSF), that further amplify the immune reactivity against the tumor cells. CTL recognize tumor associated antigens (TAA) in the form of a complex composed of 8-11 amino acid residue peptide epitopes, bound to Major Histocompatibility Complex (MHC) molecules (Schwartz, B. D., The human major histocompatibility complex HLA in basic & clinical immunology Stites et al., eds., Lange Medical Publication: Los Altos, pp. 52-64, 4th ed.). Peptide epitopes are generated through intracellular processing of proteins. The processed peptides bind to newly synthesized MHC molecules and the epitope-MHC complexes are expressed on the cell surface. These epitope-MHC complexes are recognized by the T cell receptor of the CTL. This recognition event is required for the activation of CTL as well as induction of the effector functions such as lysis of the target tumor cell.

MHC molecules are highly polymorphic proteins that regulate T cell responses (Schwartz; B. D., The human major histocompatibility complex HLA in basic & clinical immunology Stites et al., eds., Lange Medical Publication: Los Altos, pp. 52-64, 4th ed.). The species-specific MHC homologues that display CTL epitopes in humans are termed HLA. HLA class I molecules can be divided into several families or "supertypes" based upon their ability to bind similar repertoires of peptides. Vaccines which bind to HLA supertypes such as A2, A3, and B7, will afford broad, non-ethnically biased population coverage. As seen in Table 11, population coverage is 84-90% for various ethnicities, with an average coverage of the sample ethnicities at 87%.

One of the main factors contributing to the dynamic interplay between host and disease is the immune response mounted against the pathogen, infected cell, or malignant cell. In many conditions such immune responses control the disease. Several animal model systems and prospective studies of natural infection in humans suggest that immune responses against a pathogen can control the pathogen, prevent progression to severe disease and/or eliminate the pathogen. A common theme is the requirement for a multispecific T cell response, and that narrowly focused responses appear to be less effective.

In the cancer setting there are several findings that indicate that immune responses can impact neoplastic growth:

First, the demonstration in many different animal models, that anti-tumor T cells, restricted by MHC class I, can prevent or treat tumors.

Second, encouraging results have come from immunotherapy trials.

Third, observations made in the course of natural disease correlated the type and composition of T cell infiltrate within tumors with positive clinical outcomes (Coulie PG, et al. Antitumor immunity at work in a melanoma patient In Advances in Cancer Research, 213-242, 1999).

Finally, tumors commonly have the ability to mutate, thereby changing their immunological recognition. For example, the presence of monospecific CTL was also correlated with control of tumor growth, until antigen loss emerged (Riker A, et al., Immune selection after antigen-specific immunotherapy of melanoma Surgery, Aug: 126(2):112-20, 1999; Marchand M, et al., Tumor regressions observed in patients with metastatic melanoma treated with an antigenic peptide encoded by gene MAGE-3 and presented by HLA-A1 Int. J. Cancer 80(2):219-30, Jan. 18, 1999). Similarly, loss of beta 2 microglobulin was detected in 5/13 lines established from melanoma patients after receiving immunotherapy at the NCI (Restifo NP, et al., Loss of functional Beta2 - microglobulin in metastatic melanomas from five patients receiving immunotherapy Journal of the National Cancer Institute, Vol. 88 (2), 100-108, Jan. 1996). It has long been recognized that HLA class I is frequently altered in various tumor types. This has led to a hypothesis that this phenomenon might reflect immune pressure exerted on the tumor by means of class I restricted CTL. The extent and degree of alteration in HLA class I expression appears to be reflective of past immune pressures, and may also have prognostic value (van Duinen SG, et al., Level of HLA antigens in locoregional metastases and clinical course of the disease in patients with melanoma Cancer Research 48, 1019-1025, Feb. 1988; Möller P, et al., Influence of major histocompatibility complex class I and II antigens on survival in colorectal carcinoma Cancer Research 51, 729-736, Jan. 1991). Taken together, these observations provide a rationale for immunotherapy of cancer and infectious disease, and suggest that effective strategies need to account for the complex series of pathological changes associated with disease.

The frequency of alterations in class I expression is the subject of numerous studies (Algarra I, et al., The HLA crossroad in tumor immunology Human Immunology 61, 65-73, 2000). Rees and Mian estimate allelic loss to occur overall in 3-20% of tumors, and allelic deletion to occur in 15-50% of tumors. It should be noted that each cell carries two separate sets of class I genes, each gene carrying one HLA-A and one HLA-B locus. Thus, fully heterozygous individuals carry two different HLA-A molecules and two different HLA-B molecules. Accordingly, the actual frequency of losses for any specific allele could be as little as one quarter of the overall frequency. They also note that, in general, a gradient of expression exists between normal cells, primary tumors and tumor metastasis. In a study from Natali and coworkers (Natali PG, et al., Selective changes in expression of HLA class I polymorphic determinants in human solid tumors PNAS USA 86:6719-6723, September 1989), solid tumors were investigated for total HLA expression, using W6/32 antibody, and for allele-specific expression of the A2 antigen, as evaluated by use of the BB7.2 antibody. Tumor samples were derived from primary cancers or metastasis, for 13 different tumor types, and scored as negative if less than 20%, reduced if in the 30-80% range, and normal above 80%. All tumors, both primary and metastatic, were HLA positive with W6/32. In terms of A2 expression, a reduction was noted in 16.1 % of the cases, and A2 was scored as undetectable in 39.4 % of the cases. Garrido and coworkers (Garrido F, et al., Natural history of HLA expression during tumour development Immunol Today 14(10):491-99, 1993) emphasize that HLA changes appear to occur at a particular step in the progression from benign to most aggressive. Jiminez *et al* (Jiminez P, et al., Microsatellite instability analysis in tumors with different mechanisms for total loss of HLA expression. Cancer Immunol Immunother 48:684-90, 2000) have analyzed 118 different tumors (68 colorectal, 34 laryngeal and 16 melanomas). The frequencies reported for total loss of HLA expression were 11% for colon, 18% for melanoma and 13 % for larynx. Thus, HLA class I expression is altered in a significant fraction of the tumor types, possibly as a reflection of immune pressure, or simply a reflection of the accumulation of pathological changes and alterations in diseased cells.

A majority of the tumors express HLA class I, with a general tendency for the more severe alterations to be found in later stage and less differentiated tumors. This pattern is encouraging in the context of immunotherapy, especially considering that: 1) the relatively low sensitivity of immunohistochemical techniques might underestimate HLA expression in tumors; 2) class I expression can be induced in tumor cells as a result of local inflammation and lymphokine release; and, 3) class I negative cells are sensitive to lysis by NK cells.

Recent evidence has shown that certain patients infected with a pathogen, whom are initially treated with a therapeutic regimen to reduce pathogen load, have been able to maintain decreased pathogen load when removed from the therapeutic regimen, i.e., during a "drug holiday" (Rosenberg, E., et al., Immune control of HIV-1 after early treatment of acute infection Nature 407:523-26, Sept. 28, 2000) As appreciated by those skilled in the art, many therapeutic regimens for both pathogens and cancer have numerous, often severe, side effects. During the drug holiday, the patient's immune system is keeping the disease in check.

Various approaches have, or are, being employed as cancer vaccines. Table 1 overviews the major cancer vaccine approaches and the various advantages and disadvantages of each.

Currently there are a number of unmet needs in the area of cancer treatment. This is evidenced by the side effects associated with existing therapies employed for cancer treatment and the fact that less than 50% of patients are cured by current therapies. Therefore, an opportunity exists for a product with the ability to either increase response rates, duration of response, overall survival, disease free survival or quality of life.

### SUMMARY OF THE INVENTION

In some embodiments, the invention is directed to an isolated peptide comprising or consisting of one or more HLA-A1, -A3, -A24, -B7, and/or B44 epitopes and/or HLA-A1, -A2, -A3, -A24, -B7, and/or B44 analogs. The peptide may comprise mutiple epitopes and/or analogs, and may comprise additional amino acids, including other CTL epitopes, HTL epitopes, linkers, spacers, carriers, etc.

In further embodiments, the invention is directed to polynucleotides encoding such peptides.

In further embodiments, the invention is directed to a composition comprising one or more of the above peptides and/or polynucleotides and one or more additional components. Additional components include diluents, excipients, CTL epitopes, HTL epitopes, carriers, liposomes, HLA heavy chains, β2-microglobulin, strepavidin, antigen-presenting cells, adjuvants, etc.

In further embodiments, the invention is directed to prophylactic, therapeutic, diagnostic, and prognostic methods using the peptides, polynucleotides, and compositions of the invention.

### BRIEF DESCRIPTION OF THE DRAWINGS/FIGURES

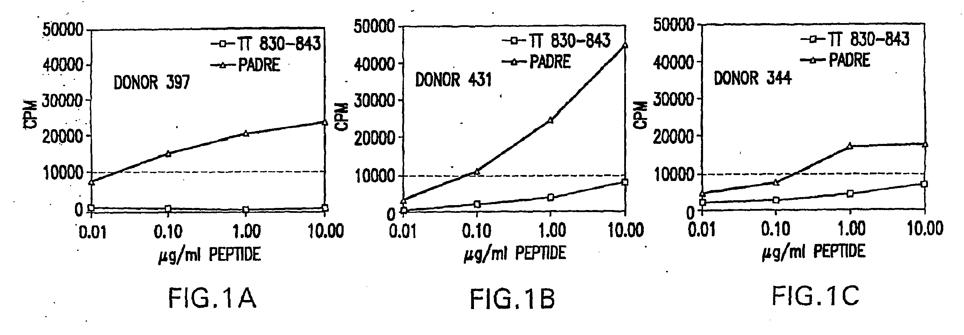

Figure 1 depicts that PADRE promotes antigen specific T cell responses from human PBMC. In Figure 1, PBMC from three healthy donors (donors 431, 397, and 344) were stimulated *in vitro.* In brief, Ficoll-Paque (Pharmacia LKB) purified PBMC were plated at 4 x 10⁶ cells/well in a 24-well tissue culture plate (Costar). The peptides were added at a final concentration of 10 µg/ml and incubated at 37°C for 4 days. Recombinant interleukin-2 was added at a final concentration of 10 ng/ml and the cultures were fed every three days with fresh media and cytokine. Two additional stimulations of the T cells with antigen were performed on approximately days 14 and 28. The T cells (3 x 10⁵ cells/well) were restimulated with 10 µg/ml peptide using irradiated (7500 rads) autologous PBMC cells. T cell proliferative responses were determined using a ³H-thymidine incorporation assay.

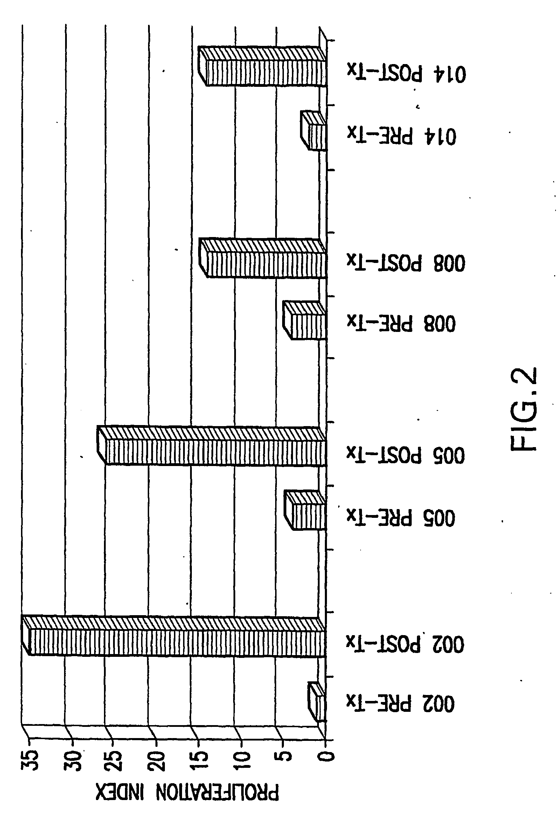

Figure 2 depicts that PADRE-specific proliferative responses are induced via peptide vaccination. In Figure 2, two weeks after vaccination, PBMC of 4 out of 12 cervical cancer patients (002, 005, 008, and 014) displayed proliferation when stimulated *in vitro* with 5 µg/ml PADRE peptide (4/12= 33% responding patients, 95% interval 10-65%) (Tx = treatment). The proliferation index of multiple wells was calculated as the mean cpm from experimental wells divided by the mean cpm from control wells. PADRE-specific responses were considered positive when the proliferation index exceeded 5. The variation between replicates was always less than 25% (Ressing et al., "Detection of T helper responses, but not of himan papillomavirus-specific cytotoxic T lymphicyte responses, after peptide vaccination of captients with cervical carcinoma," J. Immuother 23(2):255-66 (Mar.-Apr. 2000)).

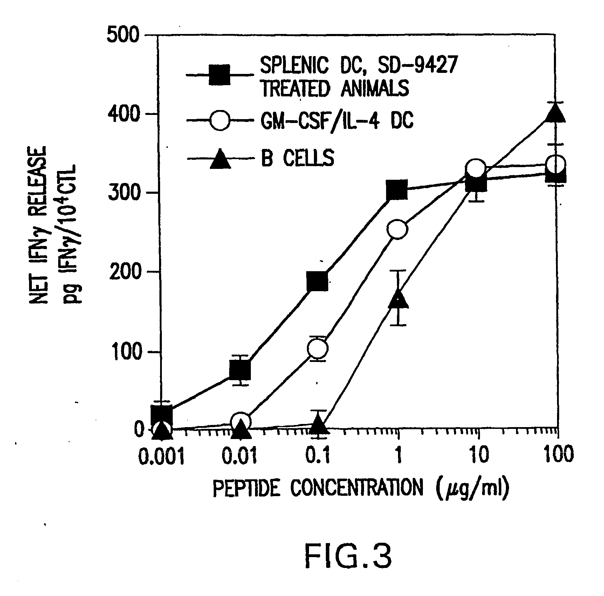

Figure 3 depicts that splenic DC from ProGP -treated mice present HBV-derived CTL epitopes to a CTL line. In Figure 3, Splenic DC from ProGP-treated HLA-A2.1/K^{b}-H-2^{bxs} transgenic mice (33 µg/animal, QD, SC for 7 days) were enriched using an anti-CD11c antibody (Miltenyi Biotec). B cells were isolated from normal spleen by magnetic separation after treating cells with biotinylated anti-CD19 antibody and Strepavidin-coupled beads (Miltenyi Biotec). DC were also generated from bone marrow cells by culture with GM-CSF/IL-4. DC or B cells, (1 x 10⁵ cells) were incubated with 1 x 10⁴ CTL line 1168 and varying concentrations of the HBV Pol 455 peptide in Opti-MEM I medium containing 3 µg/ml β2-microglobulin (Scripps Laboratories). Cells were added to 96-flat bottom well ELISA plates that were pre-coated with an anti-IFNγ capture antibody. After incubation for 18-20 hr at 37 °C, in situ production of IFNγ by stimulated line 1168 was measured using a sandwich ELISA. Data shown is from one experiment. Similar results have been obtained in additional experiments. Studies were performed at Epimmune Inc., San Diego, CA.

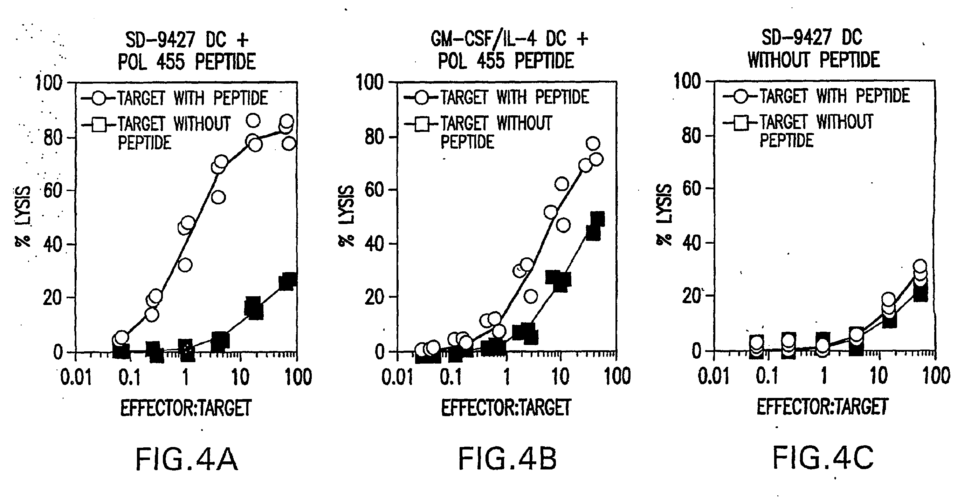

Figure 4 depicts that splenic DC from ProGP-treated mice induce CTL responses *in vivo.* In Figure 4, Splenic DC from ProGP treated HLA-A2.1 transgenic mice (33 µg/mouse, QD, SC for 7 days) were pulsed *in vitro* with HBV Pol 455 peptide (10⁶ cell per ml peptide at 10 µg/ml) in Opti-MEM I medium (Gibco Life Sciences) containing 3µg/ml β2-microglobulin (Scripps Laboratories). After peptide pulsing for 3 hr at room temperature, DC were washed twice and 10⁶ cells were injected IV into groups of three transgenic mice. Epitope-pulsed GM-CSF/IL-4 expanded DC and "mock-pulsed" ProGP derived DC were also tested for comparison. Seven days after receiving the primary immunization with DC, animals were boosted with the same DC populations. At fourteen days after the primary immunization, spleen cells from immunized animals were restimulated twice *in vitro* in the presence of the Pol 455 peptide. CTL activity following restimulations was measured using a standard ⁵¹Cr release assay in which the lysis of ⁵¹Cr-labeled HLA-A2.1-transfected Jurkat target cells was measured in the presence (circle symbols) or absence of peptide (square symbols). The data points shown in Panels A-C represent a composite of lytic activity from a triplicate set of cultures. Panel A, splenic DC from ProGP (SD-9427) treated animals pulsed with the HBV Pol 455 peptide. Panel B, GM-CSF/IL-4 expanded DC pulsed with HBV Pol 455 peptide. Panel C, mock-pulsed DC from ProGP treated animals. Studies were performed at Epimmune Inc., San Diego, CA.

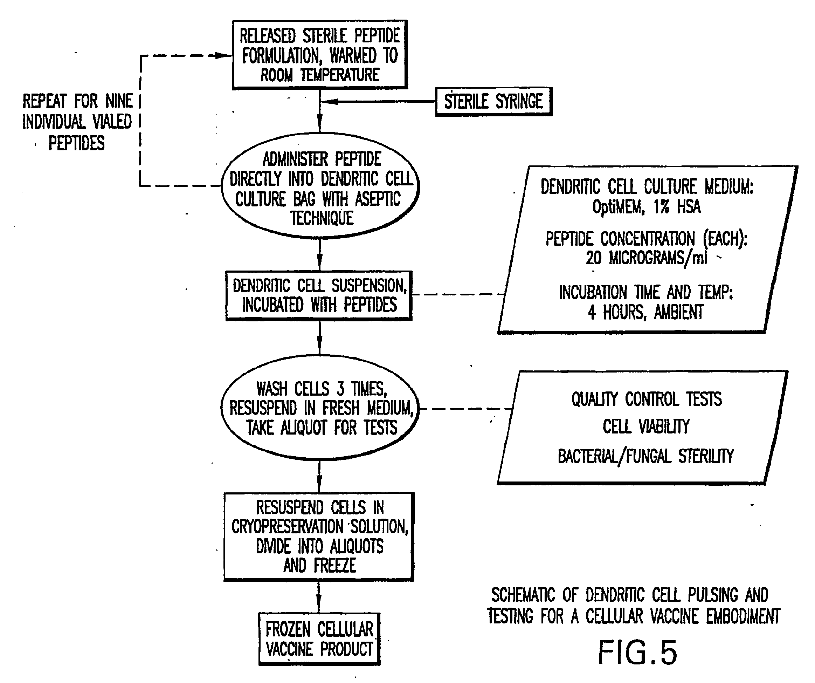

Figure 5 presents a schematic of a dendritic cell pulsing and testing procedure.

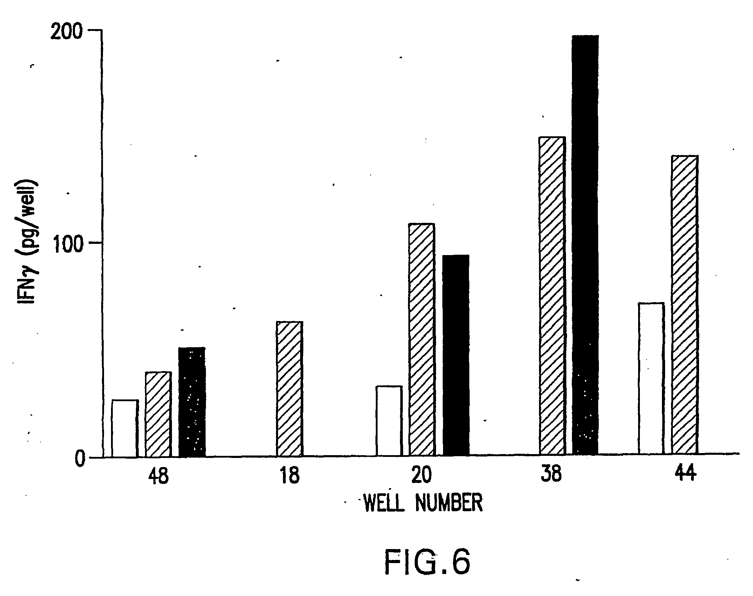

Figure 6 shows that CEA.241K10-specific CTLs recognize analog and wildtype peptide-pulsed targets. Individual cultures were tested against EHM without peptide (open bar), EHM pulsed with CEA.241K10 (hatched bar) and with EHM pulsed with CEA.241 (solid bar). A positive response was 50pg/well above background and twice background. Well number, 48, is negative and is included only for comparison.

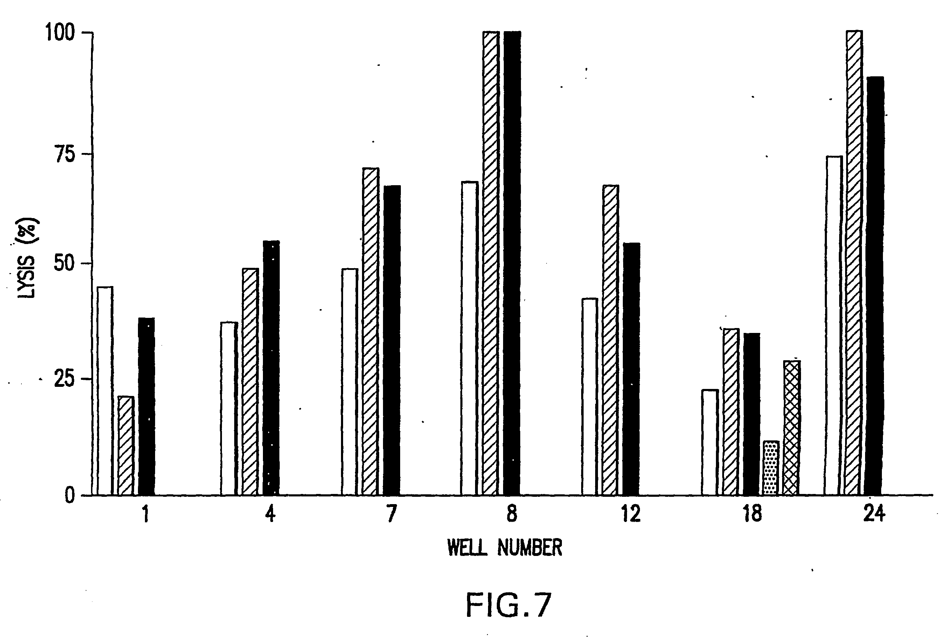

Figure 7 shows that p53.172B5K10-specific CTLs recognize analog and wildtype peptide-pulsed targets and transfected tumor target cells. Individual cultures were tested against EHM without peptide (open bar), EHM pulsed with p53.172B5K10 (hatched bar), and EHM pulsed with p53.172 (solid bar), SW403 (A3+/p53-, dotted bar) or SW403 transfected with p53 (A3+/p53+, crosshatched bar). A positive response was defined as one in which the specific lysis (sample - background) was 10% or higher. Well number 1 is negative and is included only for comparison.

### DETAILED DESCRIPTION OF THE INVENTION

This invention provides peptides that can be used to monitor an immune response to a tumor associated antigen or to create a cancer vaccine that stimulates the cellular arm of the immune system, especially when one or more peptides are combined. In particular embodiments, compositions mediate immune responses against tumors in individuals who bear at least one allele of HLA-A1, HLA-A1 supertype, and/or HLA-A2, HLA-A2 supertype, and/or HLA-A3, HLA-A3 supertype, and/or HLA-A24, HLA-A24 supertype, and/or, -B7, -B7 supertype, and/or -B44, -B44 supertype (see Table 5 for a listing of the members of these and other supertypes and types); such compositions will generally be referred to as A1, A2, A3, A24, B7, or B44, compositions (or combinations thereof).

An A2, A3, B7, A24, A1, and/or B44 composition may, for example, act as a vaccine to stimulate the immune system to recognize and kill tumor cells, leading to increased quality of life, and/or disease-free or overall survival rates for patients treated for cancer. In a preferred embodiment, a composition of the invention such as a vaccine will be administered to HLA-A₂ or HLA-A2 supertype, HLA-A3 or HLA-A3 supertype, -B7 or -B7 supertype, B-44 or -B44 supertype, -A24 or -A1 positive individuals who have a cancer that expresses at least one of the TAAs from which the epitopes or analogs were selected (e.g., CEA, p53, HER2/neu, MAGE2/3), examples of such cancers being breast, colon, lung, and gastric cancers and for MAGE 2/3, some melanomas. Alternative embodiments of a vaccine are directed at patients who bear additional HLA alleles, or who do not bear an A2, A3, B7, A24, B44, and/or A1 allele at all. Thereby, an A2, A3, B7, A24, B44, and/or A1 vaccine improves the standard of care for patients being treated for breast, colon, lung, or gastric cancers, or melanoma.

The peptides and corresponding nucleic acids and compositions of the present invention are useful for stimulating an immune response to TAAs by stimulating the production of CTL and optionally HTL responses, e.g. therapeutic prophylaxis, and are also useful for monitoring an immune response, e.g., diagnosis and prognosis. The peptides, which contain A2, A3, B7, A24, A1 and/or B44 epitopes derived directly or indirectly (i.e. by analoging) from native TAA protein amino acid sequences, are able to bind to HLA molecules and stimulate an immune response to TAAs. The complete sequence of the TAAs proteins to be analyzed can be obtained from GenBank. See Table 25.

The epitopes of the invention have been identified in a number of ways, as will be discussed below. Also discussed in greater detail is that analogs have been derived in which the binding activity for HLA molecules was modulated by modifying specific amino acid residues to create analogs which exhibit altered (*e.g.*, improved) immunogenicity. Further, the present invention provides peptides, polynucleotides, and compositions that are capable of interacting with HLA molecules encoded by various genetic alleles to provide broader population coverage than prior compositions, for prophylaxis, therapy, diagnosis, prognosis, etc.

### Definitions

The invention can be better understood with reference to the following definitions:

Throughout this disclosure, "binding data" results are often expressed in terms of "IC₅₀'s." IC₅₀ is the concentration of peptide in a binding assay at which 50% inhibition of binding of a reference peptide is observed. Given the conditions in which the assays are run (*i.e.*, limiting HLA proteins and labeled peptide concentrations), these values approximate K_{D} values. Assays for determining binding are described in detail, *e.g*., in PCT publications WO 94/20127 and WO 94/03205, and other publications such Sidney et al., Current Protocols in Immunology 18.3.1 (1998); Sidney, et al., J. Immunol. 1-54:247 (1995); and Sette, et al., Mol. Immunol. 31:813 (1994). It should be noted that IC₅₀ values can change, often dramatically, if the assay conditions are varied, and depending on the particular reagents used (*e.g.*, HLA preparation, *etc*.). For example, excessive concentrations of HLA molecules will increase the apparent measured IC₅₀ of a given ligand.

Alternatively, binding is expressed relative to a reference peptide. Although as a particular assay becomes more, or less, sensitive, the IC₅₀'s of the peptides tested may change somewhat; the binding relative to the reference peptide will not significantly change. For example, in an assay run under conditions such that the IC₅₀ of the reference peptide increases 10-fold, the IC₅₀ values of the test peptides will also shift approximately 10-fold. Therefore, to avoid ambiguities, the assessment of whether a peptide is a good (i.e. high), intermediate, weak, or negative binder is generally based on its IC₅₀, relative to the IC₅₀ of a standard peptide. The Tables included in this application present binding data in a preferred biologically relevant form of IC₅₀ nM.

Binding may also be determined using other assay systems including those using: live cells (*e.g.,* Ceppellini et al., Nature 339:392 (1989); Christnick et al., Nature 352:67 (1991); Busch et al., Int. Immunol. 2:443 (1990); Hill et al., J. Immunol. 147:189 (1991); del Guercio et al., J. Immunol. 154:685 (1995)), cell free systems using detergent lysates (*e.g.,* Cerundolo et al., J. Immunol. 21:2069 (1991)), immobilized purified MHC (*e.g.,* Hill et al., J. Immunol. 152, 2890 (1994); Marshall et al., J. Immunol. 152:4946 (1994)), ELISA systems (*e.g.*, Reay et al., EMBO J. 11:2829 (1992)), surface plasmon resonance (*e.g.,* Khilko et al., J. Biol. Chem. 268:15425 (1993)); high flux soluble phase assays (Hammer et al., J. Exp. Med. 180:2353 (1994)), and measurement of class I MHC stabilization or assembly (*e.g.*, Ljunggren et al., Nature 346:476 (1990); Schumacher et al., Cell 62:563 (1990); Townsend et al., Cell 62:285 (1990); Parker et al., J. Immunol. 149:1896 (1992)).

As used herein, "high affinity" with respect to HLA class I molecules is defined as binding with an IC₅₀ or K_{D} value, of 50 nM or less, "intermediate affinity" is binding with an IC₅₀ or K_{D} value of between 50 and about 500 nM, weak affinity is binding with an IC₅₀ or K_{D} value of between about 500 and about 5000 nM. "High affinity" with repect to binding to HLA class II molecules is defined as binding with an IC₅₀ or K_{D} value of 100 nM or less; "intermediate affinity" is binding with an IC₅₀ or K_{D} value of between about 100 and about 1000 nM.

A "computer" or "computer system" generally includes: a processor and related computer programs; at least one information storage/retrieval apparatus such as a hard drive, a disk drive or a tape drive; at least one input apparatus such as a keyboard, a mouse, a touch screen, or a microphone; and display structure, such as a screen or a printer. Additionally, the computer may include a communication channel in communication with a network. Such a computer may include more or less than what is listed above.

"Cross-reactive binding" indicates that a peptide is bound by more than one HLA molecule; a synonym is degenerate binding.

A "cryptic epitope" elicits a response by immunization with an isolated peptide, but the response is not cross-reactive *in vitro* when intact whole protein, which comprises the epitope, is used as an antigen.

The term "derived" when used to discuss an epitope is a synonym for "prepared." A derived epitope can be isolated from a natural source, or it can be synthesized in accordance with standard protocols in the art. Synthetic epitopes can comprise artificial amino acids "amino acid mimetics," such as D isomers of natural occurring L amino acids or non-natural amino acids such as cyclohexylalanine. A derived/prepared epitope can be an analog of a native epitope.

A "diluent" includes sterile liquids, such as water and oils, including those of petroleum, animal, vegetable or synthetic origin, such as peanut oil, soybean oil, mineral oil, sesame oil and the like. Water is a preferred diluent for pharmaceutical compositions. Saline solutions and aqueous dextrose and glycerol solutions can also be employed as diluents, particularly for injectable solutions.

A "dominant epitope" is an epitope that induces an immune response upon immunization with a whole native antigen (see, *e.g.,* Sercarz, et al., Annu. Rev. Immunol. 11:729-766, 1993). Such a response is cross-reactive *in vitro* with an isolated peptide epitope.

An "epitope" is the collective features of a molecule, such as primary, secondary and tertiary peptide structure, and charge, that together form a site recognized by an immunoglobulin, T cell receptor or HLA molecule. Alternatively, an epitope can be defined as a set of amino acid residues which is involved in recognition by a particular immunoglobulin, or in the context of T cells, those residues necessary for recognition by T cell receptor proteins and/or Major Histocompatibility Complex (MHC) receptors. Epitopes are present in nature, and can be isolated, purified or otherwise prepared/derived by humans. For example, epitopes can be prepared by isolation from a natural source, or they can be synthesized in accordance with standard protocols in the art. Synthetic epitopes can comprise artificial amino acids, "amino acid mimetics," such as D isomers of naturally-occurring L amino acids or non-naturally-occuring amino acids such as cyclohexylalanine. Throughout this disclosure, epitopes may be referred to in some cases as peptides. The epitopes and analogs of the invention are set forth in Tables 16A-23 and B44 Table.

It is to be appreciated that proteins or peptides that comprise an epitope or an analog of the invention as well as additional amino acid(s) are still within the bounds of the invention. In certain embodiments, the peptide comprises a fragment of an antigen. A "fragment of an antigen" or "antigenic fragment" or simply "fragment" is a portion of an antigen which has 100% identity with a wild type antigen or naturally-ocurring variant thereof. The fragment may or may not comprise an epitope of the invention. The fragment may be less than or equal to 600 amino acids, less than or equal to 500 amino acids, less than or equal to 400 amino acids, less than or equal to 250 amino acids, less than or equal to 100 amino acids, less than or equal to 85 amino acids, less than or equal to 75 amino acids, less than or equal to 65 amino acids, or less than or equal to 50 amino acids in length. In certain embodiments, a fragment is e.g., less than 101 or less than 51 amino acids in length, in any increment down to 5 amino acids in length. For example, the fragment may be 5, 6, 7, 8, 9,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length.

In certain embodiments, there is a limitation on the length of a peptide of the invention. The embodiment that is length-limited occurs when the protein/peptide comprising an epitope of the invention comprises a region (i.e., a contiguous series of amino acids) having 100% identity with a native sequence. In order to avoid the definition of epitope from reading, *e.g.*, on whole natural molecules, there is a limitation on the length of any region that has 100% identity with a native peptide sequence. Thus, for a peptide comprising an epitope of the invention and a region with 100% identity with a native peptide sequence, the region with 100% identity to a native sequence generally has a length of: less than or equal to 600 amino acids, often less than or equal to 500 amino acids, often less than or equal to 400 amino acids, often less than or equal to 250 amino acids, often less than or equal to 100 amino acids, often less than or equal to 85 amino acids, often less than or equal to 75 amino acids, often less than or equal to 65 amino acids, and often less than or equal to 50 amino acids. In certain embodiments, an "epitope" of the invention is comprised by a peptide having a region with less than 51 amino acids that has 100% identity to a native peptide sequence, in any increment down to 5 amino acids.

Accordingly, peptide or protein sequences longer than 600 amino acids are within the scope of the invention, so long as they do not comprise any contiguous sequence of more than 600 amino acids that have 100% identity with a native peptide sequence. For any peptide that has five contiguous residues or less that correspond to a native sequence, there is no limitation on the maximal length of that peptide in order to fall within the scope of the invention. It is presently preferred that a peptide of the invention (*e.g.*, a peptide comprising an epitope of the invention) be less than 600 residues long in any increment down to eight amino acid residues.

"Human Leukocyte Antigen" or "HLA" is a human class I or class II Major Histocompatibility Complex (MHC) protein (*see, e.g.,* Stites, et al., IMMUNOLOGY, 8TH ED., Lange Publishing, Los Altos, CA (1994).

An "HLA supertype or HLA family", as used herein, describes sets of HLA molecules grouped on the basis of shared peptide-binding specificities. HLA class I molecules that share somewhat similar binding affinity for peptides bearing certain amino acid motifs are grouped into such HLA supertypes. The terms HLA superfamily, HLA supertype family, HLA family, and HLA xx-like molecules (where "xx" denotes a particular HLA type), are synonyms. See Tables 14-23 plus B44 Table.

As used herein, "high affinity" with respect to HLA class I molecules is defined as binding with an IC₅₀, or K_{D} value, of 50 nM or less; "intermediate affinity" is binding with an IC₅₀ or K_{D} value of between about 50 and about 500 nM; "weak affinity" is binding with an IC₅₀ or K_{D} value between about 500 and about 5000 nM. "High affinity" with respect to binding to HLA class II molecules is defined as binding with an IC₅₀ or K_{D} value of 100 nM or less; "intermediate affinity" is binding with an IC₅₀ or K_{D} value of between about 100 and about 1000 nM. See "binding data."

An "IC₅₀" is the concentration of peptide in a binding assay at which 50% inhibition of binding of a reference peptide is observed. Given the conditions in which the assays are run (*i.e.,* limiting HLA proteins and labeled peptide concentrations), these values approximate K_{D} values. See "binding data."

The terms "identical" or percent "identity," in the context of two or more peptide sequences or antigen fragments, refer to two or more sequences or subsequences that are the same or have a specified percentage of amino acid residues that are the same, when compared and aligned for maximum correspondence over a comparison window, as measured using a sequence comparison algorithm or by manual alignment and visual inspection.

An "immunogenic" peptide or an "immunogenic" epitope or "peptide epitope" is a peptide that comprises an allele-specific motif or supermotif such that the peptide will bind an HLA molecule and induce a CTL and/or HTL response. Thus, immunogenic peptides of the invention are capable of binding to an appropriate HLA molecule and thereafter inducing a cytotoxic T lymphocyte (CTL) response, or a helper T lymphocyte (HTL) response, to the peptide.

The phrases "isolated" or "biologically pure" refer to material which is substantially or essentially free from components which normally accompany the material as it is found in its native state. Thus, isolated peptides in accordance with the invention preferably do not contain materials normally associated with the peptides in their *in situ* environment. An "isolated" epitope refers to an epitope that does not include the whole sequence of the antigen or polypeptide from which the epitope was derived. Typically the "isolated" epitope does not have attached thereto additional amino acids that result in a sequence that has 100% identity with a native sequence. The native sequence can be a sequence such as a tumor-associated antigen from which the epitope is derived. Thus, the term "isolated" means that the material is removed from its original environment (*e*.*g*., the natural environment if it is naturally occurring). For example, a naturally-occurring polynucleotide or peptide present in a living animal is not isolated, but the same polynucleotide or peptide, separated from some or all of the coexisting materials in the natural system, is isolated. Such a polynucleotide could be part of a vector, and/or such a polynucleotide or peptide could be part of a composition, and still be "isolated" in that such vector or composition is not part of its natural environment. Isolated RNA molecules include *in vivo* or *in vitro* RNA transcripts of the DNA molecules of the present invention, and further include such molecules produced synthetically.

"Major Histocompatibility Complex" or "MHC" is a cluster of genes that plays a role in control of the cellular interactions responsible for physiologic immune responses. In humans, the MHC complex is also known as the human leukocyte antigen (HLA) complex. For a detailed description of the MHC and HLA complexes, see, Paul, FUNDAMENTAL IMMUNOLOGY, 3RD ED., Raven Press, New York (1993).

The term "motif" refers to a pattern of residues in an amino acid sequence of defined length, preferably a peptide of less than about 15 amino acids in length, or less than about 13 amino acids in length, usually from about 8 to about 13 amino acids (*e.g.*, 8, 9, 10, 11, 12, or 13) for a class I HLA motif and from about 6 to about 25 amino acids (*e.g.*, 6, 7, 8, 9, 10, 11, 12, 13, 14, 15, 16, 17, 18, 19, 20, 21, 22, 23, 24, or 25) for a class II HLA motif, which is recognized by a particular HLA molecule. Motifs are typically different for each HLA protein encoded by a given human HLA allele. These motifs often differ in their pattern of the primary and secondary anchor residues. See Tables 2-4.

A "native" or a "wild type" sequence refers to a sequence found in nature.

A "negative binding residue" or "deleterious residue" is an amino acid which, if present at certain positions (typically not primary anchor positions) in a peptide epitope, results in decreased binding affinity of the peptide for the peptide's corresponding HLA molecule.

The term "peptide" is used interchangeably with "oligopeptide" in the present specification to designate a series of residues, typically L-amino acids, connected one to the other, typically by peptide bonds between the α-amino and carboxyl groups of adjacent amino acids.

A "PanDR binding" peptide or "PADRE^{®}" peptide (Epimmune, San Diego, CA) is a member of a family of molecules that binds more than one HLA class II DR molecule. The pattern that defines the PADRE^{®} family of molecules can be referred to as an HLA Class II supermotif. A PADRE^{®} molecule binds to HLA-DR molecules and stimulates *in vitro* and *in vivo* human helper T lymphocyte (HTL) responses. For a further definition of the PADRE^{®} family, see copending application US serial Nos. 09/709,774, filed November 11, 2000; and 09/707,738, filed November 6, 2000; PCT publication Nos WO 95/07707, and WO 97/26784; U.S. Patent Nos. 5,736,142 issued April 7, 1998; 5,679,640, issued October 21, 1997; and 6,413,935, issued July 2, 2002.

"Pharmaceutically acceptable" refers to a generally non-toxic, inert, and/or physiologically compatible composition or component of a composition.

A "pharmaceutical excipient" or "excipient" comprises a material such as an adjuvant, a carrier, pH-adjusting and buffering agents, tonicity adjusting agents, wetting agents, preservatives, and the like. A "pharmaceutical excipient" is an excipient which is pharmaceutically acceptable.

A "primary anchor residue" is an amino acid at a specific position along a peptide sequence which is understood to provide a contact point between the immunogenic peptide and the HLA molecule. One, two or three, primary anchor residues within a peptide of defined length generally defines a "motif" for an immunogenic peptide. These residues are understood to fit in close contact with peptide binding grooves of an HLA molecule, with their side chains buried in specific pockets of the binding grooves themselves. In one embodiment of an HLA class I motif, the primary anchor residues are located at position 2 (from the amino terminal position) and at the carboxyl terminal position of a peptide epitope in accordance with the invention. The primary anchor positions for each motif and supermotif of HLA Class I are set forth in Table 14. For example, analog peptides can be created by altering the presence or absence of particular residues in these anchor positions. Such analogs are used to modulate the binding affinity of an epitope comprising a particular motif or supermotif.

"Promiscuous recognition" by a TCR is where a distinct peptide is recognized by the various T cell clones in the context of various HLA molecules. Promiscuous binding by an HLA molecule is synonymous with cross-reactive binding.

A "protective immune response" or "therapeutic immune response" refers to a CTL and/or an HTL response to an antigen derived from an pathogenic antigen (*e.g.*, an antigen from an infectious agent or a tumor antigen), which in some way prevents or at least partially arrests disease symptoms, side effects or progression. The immune response may also include an antibody response which has been facilitated by the stimulation of helper T cells.

The term "residue" refers to an amino acid or amino acid mimetic incorporated into a peptide or protein by an amide bond or amide bond mimetic.

A "secondary anchor residue" is an amino acid at a position other than a primary anchor position in a peptide which may influence peptide binding. A secondary anchor residue occurs at a significantly higher frequency amongst HLA-bound peptides than would be expected by random distribution of amino acids at a given position. A secondary anchor residue can be identified as a residue which is present at a higher frequency among high or intermediate affinity binding peptides, or a residue otherwise associated with high or intermediate affinity binding. The secondary anchor residues are said to occur at "secondary anchor positions." For example, analog peptides can be created by altering the presence or absence of particular residues in these secondary anchor positions. Such analogs are used to finely modulate the binding affinity of an epitope comprising a particular motif or supermotif. The terminology "fixed peptide" is generally used to refer to an analog peptide that has changes in primary anchore position; not secondary.

A "subdominant epitope" is an epitope which evokes little or no response upon immunization with a whole antigen or a fragment of the whole antigen comprising a subdominant epitope and a dominant epitope, which comprise the epitope, but for which a response can be obtained by immunization with an isolated peptide, and this response (unlike the case of cryptic epitopes) is detected when whole antigen or a fragment of the whole antigen comprising a subdominant epitope and a dominant epitope is used to recall the response *in vitro* or *in vivo.*

A "supermotif' is a peptide binding specificity shared by HLA molecules encoded by two or more HLA alleles. Preferably, a supermotif-bearing peptide is recognized with high or intermediate affinity (as defined herein) by two or more HLA antigens.

"Synthetic peptide" refers to a peptide that is abtained from a non-natural source, *e.g.*, is man-made. Such peptides may be produced using such methods as chemical synthesis or recombinant DNA technology. "Synthetic peptides" include "fusion proteins."

As used herein, a "vaccine" is a.composition used for vaccination, e.g., for prophylaxis or therapy, that comprises one or more peptides of the invention. There are numerous embodiments of vaccines in accordance with the invention, such as by a cocktail of one or more peptides; one or more peptides of the invention comprised by a polyepitopic peptide; or nucleic acids that encode such peptides or polypeptides, *e.g.*, a minigene that encodes a polyepitopic peptide. The "one or more peptides" can include any whole unit integer from 1-150, e.g., at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80, 85, 90, 95, 100, 105, 110, 115, 120, 125, 130, 135, 140, 145, or 150 or more peptides of the invention. The peptides or polypeptides can optionally be modified, such as by lipidation, addition of targeting or other sequences. HLA class I-binding peptides of the invention can be linked to HLA class II-binding peptides, e.g., a PADRE^{®} universal HTL-bindind peptide, to facilitate activation of both cytotoxic T lymphocytes and helper T lymphocytes. Vaccines can comprise peptide pulsed antigen presenting cells, *e.g.*, dendritic cells.

The nomenclature used to describe peptides/proteins follows the conventional practice wherein the amino group is presented to the left (the N-terminus) and the carboxyl group to the right (the C-terminus) of each amino acid residue. When amino acid residue positions are referred to in a peptide w epitope they are numbered in an amino to carboxyl direction with position one being the position closest to the amino terminal end of the epitope, or the peptide or protein of which it may be a part. In the formulae representing selected specific embodiments of the present invention, the amino- and carboxyl-terminal groups, although not specifically shown, are in the form they would assume at physiologic pH values, unless otherwise specified. In the amino acid structure formulae, each residue is generally represented by standard three letter or single letter designations: The L-form of an amino acid residue is represented by a capital single letter or a capital first letter of a three-letter symbol, and the D-form for those amino acids having D-forms is represented by a lower case single letter or a lower case three letter symbol. However, when three letter symbols or full names are used without capitals, they may refer to L amino acids. Glycine has no asymmetric carbon atom and is simply referred to as "Gly" or "G". The amino acid sequences of peptides set forth herein are generally designated using the standard single letter symbol. (A, Alanine; C, Cysteine; D, Aspartic Acid; E, Glutamic Acid; F, Phenylalanine; G, Glycine; H, Histidine; I, Isoleucine; K, Lysine; L, Leucine; M, Methionine; N, Asparagine; P, Proline; Q, Glutamine; R, Arginine; S, Serine; T, Threonine; V, Valine; W, Tryptophan; and Y, Tyrosine.) In addition to these symbols, "B"in the single letter abbreviations used herein designates α-amino butyric acid. In some embodiments, α-amino butyric acid may be replaced with cysteine.
Acronyms used herein are as follows:
- APC:: Antigen presenting cell
- CD3:: Pan T cell marker
- CD4:: Helper T lymphocyte marker
- CD8:: Cytotoxic T lymphocyte marker
- CEA:: Carcinoembryonic antigen (see, e.g., SEQ ID NO: 363)
- CTL:: Cytotoxic T lymphocyte
- DC:: Dendritic cells. DC functioned as potent antigen presenting cells by stimulating cytokine release from CTL lines that were specific for a model peptide derived from hepatitis B virus. *In vivo* experiments using DC pulsed *ex vivo* with an HBV peptide epitope have stimulated CTL immune responses *in vivo* following delivery to naive mice.
- DLT:: Dose-limiting toxicity, an adverse event related to therapy.
- DMSO:: Dimethylsulfoxide
- ELISA:: Enzyme-linked immunosorbant assay
- E:T:: Effector:Target ratio
- G-CSF:: Granulocyte colony-stimulating factor
- GM-CSF:: Granulocyte-macrophage (monocyte)-colony stimulating factor
- HBV:: Hepatitis B virus
- HER2/neu:: A tumor associated antigen; c-erbB-2 is a synonym (see, e.g., SEQ ID NO: 364)
- HLA:: Human leukocyte antigen
- HLA-DR:: Human leukocyte antigen class II
- HPLC:: High Performance Liquid Chromatography
- HTC:: Helper T Cell
- HTL:: Helper T Lymphocyte. A synonym for HTC.
- ID:: Identity
- IFNγ:: Interferon gamma
- IL-4:: Interleukin-4
- IV:: Intravenous
- LU_{30%}:: Cytotoxic activity for 10⁶ effector cells required to achieve 30% lysis of a target cell population, at a 100:1 (E:T) ratio.
- MAb:: Monoclonal antibody
- MAGE:: Melanoma antigen (see, e.g., SEQ ID NO: 365 and 366 for MAGE2 and MAGE3)
- MLR:: Mixed lymphocyte reaction
- MNC:: Mononuclear cells
- PB:: Peripheral blood
- PBMC:: Peripheral blood mononuclear cell
- ProGP^{™}:: Progenipoietin^{™} product (Searle, St. Louis, MO), a chimeric flt3/G-CSF receptor agonist.
- SC:: Subcutaneous
- S.E.M.:: Standard error of the mean
- QD:: Once a day dosing
- TAA:: Tumor Associated Antigen
- TNF:: Tumor necrosis factor
- WBC:: White blood cells

The following describes the peptides, corresponding nucleic acid molecules, compositions, and methods of the invention in more detail.

### A2, A3, B7, A1, A24 AND B44 PEPTIDES AND POLYNUCLEOTIDES OF TUMOR ASSOCIATED ANTIGENS

**A2, A3, B7, A1, A24 and B44 Epitopes and Analogs.** In some embodiments, the invention is directed to an isolated peptide comprising or consisting of an epitope and/or analog. In some embodiments, the invention is directed to an isolated polynucleotide encoding such a peptide.

The isolated epitopes and analogs of the invention are all class I binding peptides, i.e., CTL peptides. In particular, the epitopes and analogs of the invention comprise an A2 motif or supermotif, an A3 motif or supermotif, a B7 motif or supermotif, a B44 motif or supermotif, an A1 motif, or an A24 motif. Epitopes and analogs of the invention are those set forth in Tables 6, 9 and 10 (SEQ ID Nos:1-25), 16a-23 (SEQ ID NOs:42-362) and 26-30 (SEQ ID Nos:368-745). Preferred epitopes and analogs are set forth in Tables 10 (SEQ ID Nos:1, 3, 4, 5, 10, 17, 19, 20, 21, and 25) and 20-23 (SEQ ID NOs: 42, 44, 46, 51, 52, 54, 55, 57, 60, 62, 67, 68, 69, 70, 73, 75, 77, 82, 90, 91, 96, 99, 102, 103 104, 107, 111, 114, 116, 119, and 124; 133, 136, 140, 146, 153, 155, and 362; 161, 167, 170, 172, 178, 180, 181, 182, 186, 188, 189, 191, 194, 198, 200, 201, 108, 211, 216, 219, 221, 228, 230, 234, 236, 238, 239, 240, 242, and 246; and 256, 263, 265, 269, 271, 278, 279, 281, 282, 285, 287, 290, 292, 293, 304, 305, 308, 310, 316, 321,324, 325, 331-336, 344, 345, 351, 356, 361, and 362; respectively). A1, A2, A3, A24, B7 and B44 epitopes and analogs of the invention may be referred to herein as "epitopes" and "analogs" or referred to by Table or referred to by SEQ ID NO. Other epitopes and analogs are referred to herein as CTL epitopes or CTL peptides and HTL epitopes or HTL peptides.

Peptides and Polynucleotides. In some embodiments, the invention is directed to an isolated peptide comprising or consisting of an epitope and/or analog, wherein the epitope or analog consists of a sequence selected from those in tables 6, 9, 10 (SEQ ID Nos:1-25), 16a-23 (SEQ ID NOs:42-362) and 26-30 (SEQ ID Nos:368-745).

Preferably, the peptide comprises or consists of an epitope or analog consisting of a sequence in Tables 10, 20-23, or 26-30.

Peptides of the invention may be fusion proteins of epitope(s) and/or analog(s) to CTL epitope(s), and/or HTL epitope(s), and/or linker(s), and/or spacer(s), and/or carrier(s), and/or additional amino acid(s), and/or may comprise or consist of homopolymers of an epitope or analog or heteropolymers of epitopes and/or analogs, as is described in detail below.

Peptides which comprise an epitope and/or analog of the invention may comprise or consist of a fragment of an antigen ("fragment" or "antigenic fragment"), wherein the fragment comprises an epitope and/or analog. The fragment may be a portion of CEA, HER2/neu MAGE2, MAGE3, and/or p53 (SEQ ID Nos:363-367, respectively). The epitope of the invention may be within the fragment or may be linked directly or indirectly, to the fragment.

The fragment may comprise or consist of a region of a native antigen that contains a high concentration of class I and/or class II epitopes, preferably it contains the greatest number of epitopes per amino acid length. Such epitopes can be present in a frame-shifted manner, *e.g.* a 10 amino acid long peptide could contain two 9 amino acid long epitopes and one 10 amino acid long epitope.

The fragment may be less than or equal to 600 amino acids, less than or equal to 500 amino acids, less than or equal to 400 amino acids, less than or equal to 250 amino acids, less than or equal to 100 amino acids, less than or equal to 85 amino-acids, less than or equal to 75 amino acids, less than or equal to 65 amino acids, or less than or equal to 50 amino acids in length. In certain embodiments, a fragment is less than 101 amino acids in length, in any increment down to 5 amino acids in length. For example, the fragment may be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length. (See Table 33). Fragments of full length antigens may be fragments from about residue 1-20, 21-40, 41-60, 61-80, 81-100, 101-120, 121-140, 141-160, 161-180, 181-200, 201-220, 221-240, 241-260, 261-280, 281-300, 301-320, 321-340, 341-360, 361-380, 381-400, 401-420, 421-440, 441-460, 461-480, 481-500, 501-520, 521-540, 541-560, 561-580, 581-600, 601-620, 621-680, 681-700, 701-720, 721-740, 741-780, 781-800, 801-820, 821-840, 841-860, 861-880, 881-900, 901-920, 921-940, 941-960, 961-980, 981 to the C-terminus of the antigen.

Peptides which comprise an epitope and/or analog of the invention may be a fusion protein comprising one or more amino acid residues in addition to the epitope, analog, or fragment. Fusion proteins include homopolymers and heteropolymers, as described below.

In some embodiments, the peptide comprises or consists of multiple epitopes and/or analogs, *e.g.*, 2, 3, 4, 5, 6, 7, 8, 9, 10, 11, 12, 13, 14, 15, 16, 17, 18, 19, 20, 21, 22, 23, 24, or 25 epitopes and/or analogs of the invention. In other embodiments, the peptide comprises or consists of 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 epitopes and/or analogs of the invention. In some embodiments, the peptide comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25 epitopes and/or analogs of the invention.

For example, the peptide may comprise or consist of at least 1, at least 2, at least 3, at least 4, or all 5 CEA epitopes and/or analogs from Table 6; at least 1, at least 2, at least 3, at least 4, at least 5, at least 6, at least 7, at least 8, at least 9, or all 10 HER2/neu epitopes and/or analogs of Table 6; at least 1, at least 2, at least 3, at least 4, or all 5 MAGE2/3 epitopes and/or analogs from Table 6; at least 1, at least 2, at least 3, at least 4, or all 5 p53 epitopes and/or analogs from Table 6. The peptide may comprise or consist of at least 1, at least 2, at least 3, at least 4, at least 5, at least 6, at least 7, at least 8, at least 9, at least 10, at least 11, at least 12, at least 13, or all 14 epitopes and/or analogs from Table 16a; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or all 20 epitopes and/or analogs from Table 16b; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, or all 28 epitopes and/or analogs from Table 16c; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, or all 25 epitopes and/or analogs from Table 16d. The peptide may comprise or consist of at least 1, at least 2, at least 3, at least 4, or all 5 epitopes and/or analogs from Table 17a; at least 1, at least 2, at least 3, at least 4, at least 5, or all 6 epitopes and/or analogs from Table 17b; at least 1, at least 2, at least 3, at least 4, at least 5, at least 6, at least 7, at least 8, at least 9, at least 10, at least 11, at least 12, at least 13, or all 14 epitopes and/or analogs from Table 17c; at least 1, at least 2, at least 3, or all 4 epitopes and/or analogs from Table 17d. The peptide may comprise or consist of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, or all 27 epitopes and/or analogs from Table 18a; at least 1, at least 2, at least 3, at least 4, at least 5, at least 6, at least 7, at least 8, at least 9, at least 10, at least 11, at least 12; at least 13, at least 14, at least 15, at least 16, at least 17, at least 18, at least 19, at least 20, at least 21, at least 22, at least 23, or all 24 epitopes and/or analogs from Table 18b; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, or all 28 epitopes and/or analogs from Table 18c; at least 1, at least 2, at least 3, at least 4, at least 5, at least 6, at least 7, at least 8, at least 9, or all 10 epitopes and/or analogs from Table 18d. The peptide may comprise or consist of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, or all 45 epitopes and/or analogs from Table 19a; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or all 21 epitopes and/or analogs from Table 19b; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, or all 40 epitopes and/or analogs from Table 19c; at least 1, at least 2, at least 3, at least 4, at least 5, at least 6, at least 7, at least 8, or all 9 epitopes and/or analogs from Table 19d. The peptide may comprise or consist of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, or at least 26 of the epitopes and/or analogs from Table 26, 27, 28, 29, or 30.

The peptide may preferably comprise or consist of at least 1 or all 2 CEA epitopes/analogs of Table 9; at least 1 or all 2 HER2/neu epitopes/analogs of Table 9; at least 1 or all 2 MAGE2/3 epitopes/analogs of Table 9; at least 1 or all 2 p53 epitopes/analogs of Table 9. The peptide may preferably comprise or consist of at least 1, at least 2, at least 3, at least 4, at least 5, at least 6, or all 7 CEA epitopes/analogs of Table 20; at least 1, at least 2, at least 3, at least 4, at least 5, at least 6, at least 7, at least 8, or all 9 HER2/neu epitopes/analogs of Table 20; at least 1, at least 2, at least 3, at least 4, at least 5, at least 6, at least 7, or all 8 MAGE2/3 epitopes/analogs of Table 20; at least 1, at least 2, at least 3, at least 4, at least 5, at least 6, or all 7 p53 epitopes/analogs of Table 20. The peptide may preferably comprise or consist of at least the CEA epitope/analog of Table 21; at least the HER2/neu epitope/analog of Table 21; at least 1, at least 2, at least 3, or all 4 MAGE2/3 epitopes/analogs of Table 21; at least the p53 epitope/analog of Table 21. The peptide may preferably comprise or consist of at least 1, at least 2, at least 3, at least 4, at least 5, at least 6, at least 7, or all 8 CEA epitopes/analogs of Table 22; at least 1, at least 2, at least 3, at least 4, at least 5, at least 6, at least 7, at least 8, or all 9 HER2/neu epitopes/analogs of Table 22; at least 1, at least 2, at least 3, at least 4, at least 5, at least 6, at least 7, or all 8 MAGE2/3 epitopes/analogs of Table 22; at least 1, at least 2, at least 3, at least 4, or all 5 p53 epitopes/analogs of Table 22. The peptide may preferably comprise or consist of at least 1, at least 2, at least 3, at least 4, at least 5, at least 6, at least 7, at least 9, qat least 10, at least 11, or all 12 CEA epitopes/analogs of Table 23; at least 1, at least 2, at least 3, at least 4, at least 5, or all 6 HER2/neu epitopes/analogs of Table 23; at least 1, at least 2, at least 3, at least 4, at least 5, at least 6, at least 7, at least 8, at least 9, at least 10, at least 11, at least 12, or all 13 MAGE2/3 epitopes/analogs of Table 23; at least 1, or all 2 p53 epitopes/analogs of Table 23.

The peptide may comprise or consist of the combinations above and below, and may also exclude any one or several epitopes and/or analogs selected from those in Tables 6, 9, 10 (SEQ ID Nos: 1-25), 16a-23 (SEQ ID NOS:42 362) and 26-30 (SEQ ID Nos:368-745). Epitopes/analogs which may preferably be excluded from peptides of the invention are SEQ ID Nos:42, 60, 62, 67, 82, 86, 101, 116, 153, 362, 230, 265, 290, 321, 334, and 345.

The peptide of the invention may comprise or consist of combinations of epitopes and/or analogs including:
A3 CEA combinations such as: (a) SEQ ID NOs:42, 44, 46, 51, 52, 54, and 55; (b) SEQ ID NOs: 44, 46, 51, 52, 54, and 55; (c) SEQ ID NOs:46, 51, 52, 54, and 55; (d) SEQ ID NOs: 51, 52, 54, and 55; (e) SEQ ID NO: 52, 54, and 55; (f) SEQ ID NO: 54 and 55;
   (g) SEQ ID NO: 44, 46, 51, 52, and 54; (h) SEQ ID NO: 44, 46, 51, and 52; (i) SEQ ID NO: 44, 46, and 51; and (j) SEQ ID NO: 44 and 46;
   (1) SEQ ID NO: 44, 51, 52, 54, and 55; (m) SEQ ID NO: 44, 46, 52, 54, and 55; (n) SEQ ID NO: 44,46, 51, 54, and 55; and (o) SEQ ID NO: 44, 46, 51, 52, and 55;
A3 HER2/neu combinations such as: (a) SEQ ID NO:57, 60, 62, 67, 68, 69, 70, 73, and 75; (b) SEQ ID NO: 60, 62, 67, 68, 69, 70, 73, and 75; (c) SEQ ID NO: 62, 67, 68, 69, 70; 73, and 75; (d) 67, 68, 69, 70, 73, and 75; (e) 68, 69, 70, 73, and 75; (f) SEQ ID NO: 69, 70, 73; and 75;
   (g) SEQ ID NO: 70, 73, and 75; (h) SEQ ID NO: 73 and 75;
   (i) SEQ ID NO: 57, 60, 62, 67, 68, 69, 70, and 73; (j) SEQ ID NO: 57, 60, 62, 67, 68, 69, and 70; (k) SEQ ID NO: 57, 60, 62, 67, 68, and 69; (l) SEQ ID NO: 57, 60, 62, 67, and 68;
   (m) SEQ ID NO: 57, 60, 62, and 67; (n) SEQ ID NO: 57, 60, and 62; (o) SEQ ID NO: 57 and 60; (p) SEQ ID NO: 57, 68, 69, 70, 73, and 75; (q) SEQ ID NO: 57, 60, 68, 69, 70, 73, and 75; (r) SEQ ID NO: 57, 60, 62, 69, 70, 73, and 75;
   and (s) SEQ ID NO: 57, 60, 62, 67, 68, 73, and 75;
A3 MAGE2/3 combinations such as: (a) SEQ ID NO:82, 90, 91, 96; 99, 102, and 103; (b) SEQ ID NO: 90, 91, 96, 99, 102, and 103; (c) SEQ ID NO: 91, 96, 99, 102, and 103; (d) SEQ ID NO: 96, 99, 102, and 103; (e) SEQ ID NO: 99, 102, and 103; (f) SEQ ID NO: 102 and 103;
   (g) SEQ ID NO: 77, 82, 90, 91, 96, 99, and 102; (h) SEQ ID NO: 77, 82, 90, 91, 96, and 99; (i) SEQ ID NO: 77, 82, 90, 91, and 96; (j) SEQ ID NO: 77, 82, 90, and 91; (k) SEQ ID NO: 77, 82, 90, and 91, 96, 99, 102, and 103; (1) SEQ ID ON: 77, 82, and 90; and (m) SEQ ID NO: 77 and 82;
A3 p53 combinations such as: (a) SEQ ID NO: 107, 111, 114, 116, 119, and 124; (b) SEQ ID NO: 111, 114, 116, 119, and 124; (c) SEQ ID NO: 114, 116, 119, and 124; (d) SEQ ID NO: 116, 119, and 124; (e) SEQ ID NO: 119 and 124;
   (f) SEQ ID NO:104, 107, 111, 114, 116, and 119; (g) SEQ ID NO:104, 107, 111, 114, and 116; (h) SEQ ID NO:104, 107, 111, and 114; (i) SEQ ID NO:104, 107, and 111; (j) SEQ ID NO: 104 and 107;
   (k) SEQ ID NO: 104, 111, 114, 116, 119, and 124; (1) SEQ ID NO: 104,107, 114, 116, 119, and 124; (m) SEQ ID NO:104, 107, 111, 116, 119, and 124; (n) SEQ ID NO:104, 107, 111, 114, 116, and 124;
   B7 MAGE2/3 combinations such as: (a) SEQ ID NO: 146, 153, and 364; (b) SEQ ID NO: 153 and 364; (d) SEQ ID NO: 140, 146, and 153; (e) SEQ ID NO: 140,146, and 364;
B7 combinations such as: (a) SEQ ID NO:133, 136, 140, 146, 153, and 155; (b) SEQ ID NO: 136, 140, 146, 153, and 155; (c) SEQ ID NO: 140, 146, 153, and 155; (d) SEQ ID NO: 153 and 155;
A1 CEA combinations such as: (a) SEQ ID NO: 167, 170, 172, 178, 180, 181, and 182; (b) SEQ ID NO: 170, 172, 178, 180, 181, and 182; (c) SEQ ID NO: 172, 178, 180, 181, and 182; (d) SEQ ID NO: 178, 180, 181, and 182; (e) SEQ ID NO: 180, 181, and 182; (f) SEQ ID NO: 181 and 182; (g) SEQ ID NO: 161, 167, 170, 172, 178, 180, and 181; (h) SEQ ID NO: 161, 167, 170, 172, 178, and 180; (i) SEQ ID NO: 161, 167, 170, 172, and 178; (j) SEQ ID NO: 161, 167, and 170; (k) SEQ ID NO: 181 and 182;
A1 HER2/neu combinations such as : (a) SEQ ID NO:188, 189, 191, 194, 198, 200, 201, and 208; (b) SEQ ID NO: 189, 191, 194, 198, 200, 201, and 208; (c) SEQ ID NO: 191, 194, 198, 200, 201, and 208; (d) SEQ ID NO: 194, 198; 200, 201, and 208; (e) SEQ ID NO: 198,200,201, and 208; (f) SEQ ID NO: 200,201, and 208; (g) SEQ ID NO:201 and 208;
   (h) SEQ ID NO:186, 188, 189, 191, 194, 198, 200, and 201; (i) SEQ ID NO:186, 188, 189, 191, 194, 198, and 200; (j) SEQ ID NO:186, 188, 189, 191, 194, and 198; (k) SEQ ID NO:186, 188, 189, 191, and 194; (l) SEQ ID NO:186, 188, 189, and 191; (m) SEQ ID NO:186, 188, and 189; (n) SEQ ID NO: 186 and 188;
A1 MAGE2/3 combinations such as: (a) SEQ ID NO: 216, 219, 221, 228, 230, 234, and 236; (b) SEQ ID NO: 219, 221, 228, 230, 234, and 236; (c) SEQ ID NO: 221, 228, 230, 234, and 236; (d) SEQ ID NO: 228, 230, 234, and 236; (e) SEQ ID NO: 230, 234, and 236; (f) SEQ ID NO: 234 and 236; (g) SEQ ID NO:211, 216, 219, 221, 228, 230, and 234; (h) SEQ ID NO:211, 216, 219, 221, 228, and 230; (i) SEQ ID NO:211, 216, 219, 221, and 228; (j) SEQ ID NO:211, 216, 219, and 221; (k) SEQ ID NO:211, 216, and 219; (l) SEQ ID NO:211 and 216; (m) SEQ ID NO:211, 216, 219, 221, 228, 234, and 236;
A1 p53 combinations such as: (a) SEQ ID NO: 239, 240, 242, and 246; (b) SEQ ID NO: 240, 242, and 246; (c) SEQ ID NO: 242 and 246; (d) SEQ ID NO:238, 239, 240, and 242; (e) SEQ ID NO:238, 239, and 240; (f) SEQ ID NO:238 and 239; (g) SEQ ID NO:238, 240, 242, and 246; (h) SEQ ID NO:238, 239, 242, and 246; (i) SEQ ID NO:238, 239, 240, and 246;
A24 CEA combinations such as: (a) SEQ ID NO: 263, 265, 269, 272, 278, 279, 281, 282, 285, 287, and 290; (b) SEQ ID NO: 265, 269, 272, 278, 279, 281, 282, 285, 287, and 290; (c) SEQ ID NO: 269, 272, 278, 279, 281, 282, 285, 287, and 290; (d) SEQ ID NO: 272, 278, 279, 281, 282, 285, 287, and 290; (e) SEQ ID NO: 278, 279, 281, 282, 285, 287, and 290; (f) SEQ ID NO: 279, 281, 282, 285, 287, and 290; (g) SEQ ID NO: 281, 282, 285, 287, and 290; (h) SEQ ID NO: 282, 285, 287, and 290;
   (i) SEQ ID NO: 285, 287, and 290; (j) SEQ ID NO: 287 and 290;
   (k) SEQ ID NO:256, 263, 265, 269, 272, 278, 279, 281, 282, 285, and 287; (l) SEQ ID NO:256, 263, 265, 269, 272, 278, 279, 281, 282, and 285; (m) SEQ ID NO:256, 263, 265, 269, 272, 278, 279, 281, and 282; (n) SEQ ID NO:256, 263, 265, 269, 272, 278, 279, and 281; (o) SEQ ID NO:256, 263, 265, 269, 272, 278, and 279; (p) SEQ ID NO:256, 263, 265, 269, 272, and 278; (q) SEQ ID NO:256, 263, 265, 269, and 272; (r) SEQ ID NO:256, 263, 265, and 269; (s) SEQ ID NO:256, 263, and 265; (t) SEQ ID NO:256 and 263; (u) SEQ ID NO:256, 263, 269, 272, 278, 279, 281, 282, 285, and 287;
A24 HER2/neu combinations such as: (a) SEQ ID NO: 293, 304, 305, 308, and 310; (b) SEQ ID NO: 304, 305, 308, and 310; (c) SEQ ID NO: 305, 308, and 310; (d) SEQ ID NO: 308 and 310; (e) SEQ ID NO:292, 293, 304, 305, and 308; (f) SEQ ID NO:292, 293, 304, and 305; (g) SEQ ID NO:292, 293, and 304; (h) SEQ ID NO:292 and 293; (i) SEQ ID NO:292, 304, 305, 308, and 310; (j) SEQ ID NO:292, 293, 305, 308, and 310; (k) SEQ ID NO:292, 293, 304, 308, and 310; (l) SEQ ID NO:292, 293, 304, 305, and 310;
A24 MAGE2/3 combinations such as: (a) SEQ ID NO: 321, 324, 325, 331, 332, 333, 334, 335, 336, 344, 345, and 351; (b) SEQ ID NO: 324, 325, 331, 332, 333, 334, 335, 336, 344, 345, and 351; (c) SEQ ID NO: 325, 331, 332, 333, 334, 335, 336, 344, 345, and 351; (d) SEQ ID NO: 331, 332, 333, 334, 335, 336, 344, 345, and 351; (e) SEQ ID NO: 332, 333, 334, 335, 336, 344, 345, and 351; (f) SEQ ID NO: 333, 334, 335, 336, 344, 345, and 351; (g) SEQ ID NO: 333, 334, 335, 336, 344, 345, and 351; (h) SEQ ID NO: 334, 335, 336, 344, 345, and 351; (i) SEQ ID NO: 335, 336, 344, 345, and 351; (j) SEQ ID NO: 336, 344, 345, and 351; (k) SEQ ID NO: 344, 345, and 351; (l) SEQ ID NO:345 and 351;
   (m) SEQ ID NO:316, 321, 324, 325, 331, 332, 333, 334, 335, 336, 344, and 345; (n) SEQ ID NO:316, 321, 324, 325, 331, 332, 333, 334, 335, 336, and 344; (o) SEQ ID NO:316, 321, 324, 325, 331, 332, 333, 334, 335, and 336; (p) SEQ ID NO:316, 321, 324, 325, 331, 332, 333, 334, and 335; (q) SEQ ID NO:316, 321, 324, 325, 331, 332, 333, and 334; (r) SEQ ID NO:316, 321, 324, 325, 331, 332, and 333; (s) SEQ ID NO:316, 321; 324, 325, 331, and 332; (t) SEQ ID NO:316, 321, 324, 325, and 331; (u) SEQ ID NO:316, 321, 324, and 325; (v) SEQ ID NO:316, 321, and 324; (w) SEQ ID NO:316 and 321; (x) SEQ ID NO:316, 324, 325, 331, 332, 333, 335, 336, 344, and 351;
A24 p53 combinations such as: SEQ ID NO:356 and 361;
B44 CEA combinations such as: (a) SEQ ID NO:368, 369, 390, 399, and 403; (b) SEQ ID NO:369, 370, 375, 376, 377, and 420; and (c) SEQ ID NO:370, 375, 379, 386, and 429;
B44 HER2/neu combinations such as: (a) SEQ ID NO:432, 435, 436, 443, 448, 460, 466, 467, and 488; (b) SEQ ID NO: 439, 473, 490, and 499; (c) SEQ ID NO:432, 433, 440, 441, 447, 456, 459, and 471; (d) SEQ ID NO: 477, 490, 499, 508, 527, and 535;
B44 MAGE2 combinations such as: (a) SEQ ID NO: 645, 646, 647, 653, 665, 670, 698, 718, and 716; (b) SEQ ID NO: 663, 688, 692, and 701; (c) SEQ ID NO:648, 655, 669, 677, 691, and 700; (d) SEQ ID NO: 651 and 673;
B44 MAGE3 combinations such as: (a) SEQ ID NO: 719, 720, 726, 732, and 740; (b) SEQ ID NO: 721, 725, 726, and 737; (c) SEQ ID NO: 726, 739, and 744; (d) SEQ ID NO: 722, 723, 728 and 735; (e) SEQ ID NO: 720, 728, 731, 736, and 741;
B44 p53 combinations such as: (a) SEQ ID NO: 598, 602, 603, and 617; (b) SEQ ID NO: 589, 599, 600, and 605; (c) SEQ ID NO:600, 603, 604, and 607; (d) SEQ ID NO: 601, 602, 604, and 609;
A2 combinations such as: (a) SEQ ID NO: 6, 8, 16, 18, 22, 23, and 24; (b) SEQ ID NO: 8, 16, 18, 22, 23, and 24; (c) SEQ ID NO: 16, 18, 22, 23, and 24; (d) SEQ ID NO: 18, 22, 23, and 24; (e) SEQ ID NO: 23 and 24; (f) SEQ ID NO: 1, 19, 3, and 4; (g) SEQ ID NO: 2, 6, 8, 16, 18, 22, and 23; (h) SEQ ID NO: 2, 6, 8, 16, 18, and 22; (i) SEQ ID NO: 2, 6, 8, 16, 18, and 22; (j) SEQ ID NO: 2, 6, 8, 16, and 18; (k) SEQ ID NO: 2, 6, 8, and 16; (l) SEQ ID NO: 2, 6, and 8; and (m) SEQ ID NO: 2 and 6; (n) SEQ ID NO:3, 4, 5, and 17; (o) SEQ ID NO: 20, 21, and 25; (p) SEQ ID NO: 1, 10, 17, and 25; (q) SEQ ID NO: 4,5, 10, 17, and 25;
TAA combinations such as: (a) SEQ ID NO: 1, 17, 22, 104, 114, 133, 136, 146, 170, 189, 221, 310, 336, 361, and 399; (b) SEQ ID NO:111, 124, 133, 140, 155, 180, 194, 228, 246, and 281; (c) SEQ ID NO: 16, 18, 25, 43, 68, 117, 309, and 499; (d) SEQ ID NO: 48, 55, 97, 369, 409, and 512; (e) SEQ ID NO: 55, 99, 135, 238, and 602; (f) SEQ ID NO: 1, 58, 77, 104, 128, 166, 207, 240, 360, and 403; (g) SEQ ID NO:17, 50, 72, 130, 161, 199, 300, and 627; (h) SEQ ID NO: 10, 55, 82, 104, 198, 400, 433, and 501; (i) SEQ ID NO: 3, 22, 122, 196, 211, 301, 360, and 667; (j) SEQ ID NO: 1, 21, 44, 100, 207, 405, and 661;

Peptides of the invention may also comprise or consist of combinations of the above combinations, including:
A24 combinations such as: A24 CEA (a) and A24 HER2/neu (a); A24 CEA (a) and A24 MAGE2/3 (a); A24 CEA (a) and A24 p53; A24 CEA (c) and A24 HER2/neu (e); A24 CEA (i) and A24 MAGE2/3 (a); A24 CEA (n) and A24 p53 (k);
A3 combinations such as: A3 CEA (a) and A3 HER2/neu (a); A3 CEA (a) and A3 MAGE2/3 (a); A3 CEA (a) and A3 p53 (a); A3 CEA (d) and A3 HER2/neu (b); A3 CEA (f) and A3 MAGE2/3 (i); A3 CEA (e) and A3 p53 (a);
CEA combinations such as: A24 CEA (a) and A1 CEA (a); A24 CEA (b) and A1 CEA (a); A24 CEA (c) and A1 CEA (a); A24 CEA (c) and A1 CEA (a); A3 CEA (a) and A1 CEA (a); A3 CEA (b) and A1 CEA (a); A3 CEA (c) and A24 CEA (a); B7 CEA (c) and A1 CEA (a);
   B7 CEA (a) and A3 CEA (a); B44 CEA (b) and A1 CEA (a); A3 CEA (e) and A1 CEA (g); A3 CEA (i) and A1 CEA (m);.
A1 CEA (a), (b) (c), (d), (e), (f) (g), (h), (i), (j), or (k) epitopes/analogs, and A3 CEA (a), (b) (c), (d), (e), (f) (g), (h), (i), (j), or (k) epitopes/analogs, and B7 CEA (a), (b) (c), (d), (e), (f) (g), (h), (i), (j), or (k) epitopes/analogs, and A3 p53 (a), (b) (c), (d), (e), (f) (g), (h), (i), (j), or (k) epitopes/analogs, and B44 MAGE2 (a), (b) (c), (d), (e), (f) (g), (h), (i), (j), or (k) epitopes/analogs, and A3 MAGE2 (a), (b) (c), (d), (e), (f) (g), (h), (i), (j), or (k) epitopes/analogs;
A24 CEA (a), (b) (c), (d), (e), (f) (g), (h), (i), (j), or (k) epitopes/analogs, and A2 CEA (a), (b) (c), (d), (e), (f) (g), (h), (i), (j), or (k) epitopes/analogs, and B7 MAGE3 (a), (b) (c), (d), (e), (f) (g), (h), (i), (j), or (k) epitopes/analogs, and B44 p53 (a), (b) (c), (d), (e), (f) (g), (h), (i), (j), or (k) epitopes/analogs;
A3 CEA (a), (b) (c), (d), (e), (f) (g), (h), (i), (j), or (k) epitopes/analogs, and B7 p53 (a), (b) (c), (d), (e), (f) (g), (h), (i), (j), or (k) epitopes/analogs, and B44 MAGE3 (a), (b) (c), (d), (e), (f) (g), (h), (i), (j), or (k) epitopes/analogs, and A24 HER2/neu (a), (b) (c), (d), (e), (f) (g), (h), (i), (j), or (k) epitopes/analogs.

The peptide may also comprise or consist of at least 1, at least 2, at least 3, at least 4, at least 5, at least 6, at least 7, or at least 8 peptides selected from the group consisting of the combinations set forth above.

The peptide may also be a homopolymer of one epitope or analog or the peptide may be a heteropolymer which contains at least two different epitopes and/or analogs. Polymers have the advantage of increased probability for immunological reaction and, where different epitopes/analogs are used to make up the polymer, the ability to induce antibodies and/or T cells that react with different antigenic determinants of the antigen(s) targeted for an immune response.

A homopolymer may comprise 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90, 95, 100, 105, 110, 115, 120, 125, 130, 135, 140, 145, or 150 copies of the same epitope or analog.

A heteropolymer may comprise one or more copies of an individual epitope or analog and one or more copies of one or more different epitopes and/or analogs of the invention. The epitopes and/or analogs that form a heteropolymer may all be from the same antigen, e.g., may be from CEA, p53, MAGE2/3, HER2/neu or other antigens herein or known in the art, or may be from different antigens, preferably TAAs. Combinations of epitopes and/or analogs that may form a heteropolymer include those combinations described above. Heteropolymers may contain multiple copies of one or more epitopes and/or analogs.

Thus, peptides of the invention such as heteropolymers may comprise a first epitope and/or analog and at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35,36, 37, 38, 39, 40, 41, 42, 43, 44, 45, 46, 47, 48, 49, or 50 other (different).

Peptides of the invention may also comprise additional amino acids.

In some embodiments, the peptides may comprise a number of CTL and/or HTL epitopes, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 CTL and/or HTL epitopes.

The CTL and/or HTL epitope and the epitope/analog of the invention may be from the same TAA or from different TAAs. Thus, for example, if the epitope and/or analog is from CEA, the CTL peptide and/or HTL peptide may also be from CEA. Alternatively, the CTL peptide and/or HTL peptide may be from another antigen, preferably a TAA antigen such as p53, MAGE2/3 or HER2/neu. As another example, if the epitope and/or analog is from p53, the CTL peptide and/or HTL peptide may be from p53 or, alternatively, may be from MAGE2/3, HER2/neu, or CEA.

The CTL peptide and/or HTL peptide may be from tumor-associated antigens such as but not limited to, melanoma antigens MAGE-1, MAGE-2, MAGE-3, MAGE-11, MAGE-A10, as well as BAGE, GAGE, RAGE, MAGE-C1, LAGE-1, CAG-3, DAM, MUC1, MUC2, MUC18, NY-ESO-1, MUM-1, CDK4, BRCA2, NY-LU-1, NY-LU-7, NY-LU-12, CASP8, RAS, KIAA-2-5, SCCs, p53, p73, CEA, HER2/neu, Melan-A, gp100, tyrosinase, TRP2, gp75/TRP1, kallikrein, prostate-specific membrane antigen (PSM), prostatic acid phosphatase (PAP), prostate-specific antigen (PSA), PT1-1, ∃-catenin, PRAME, Telomerase, FAK, cyclin D1 protein, NOEY2, EGF-R, SART-1, CAPB, HPVE7, p15, Folate receptor CDC27, PAGE-1, and PAGE-4.

Alternatively, the CTL peptide and/or HTL peptide may be from other antigens including hepatitis B core and surface antigens (HBVc, HBVs), hepatitis C antigens, Epstein-Barr virus antigens, human immunodeficiency virus (HIV) antigens and human papilloma virus (HPV) antigens (in particular anitgens from HPV-16, HPV-18, HPV-31, HPV-33, HPV-45, HPV-52, HPV-56 and HPV-58, *Mycobacterium tuberculosis* and *Chamydia.* Examples of suitable fungal antigens include those derived from *Candida albicans, Cryptococcus neoformans, Coccidoides spp., Histoplasma spp,* and *Aspergillus fumigatis.* Examples of suitable protozoan parasitic antigens include those derived from. *Plasmodium spp.,* including *P. falciparum, Trypanosoma spp., Schistosoma spp., Leishmania spp* and the like.

A CTL epitope may comprise a sequence selected from the group consisting of: SEQ ID Nos:1-25.

Examples of CTL peptides and HTL peptides are disclosed in WO 01/42270, published 14 June 2001; WO 01/41788, publidhes 14 June 2001; WO 01/42270, published 14 June 2001; WO 01/45728, published 28 June 2001; and WO 01/41787, published 14 June 2001.

The HTL peptide may comprise a "loosely HLA-restricted" or "promiscuous" sequence. Examples of amino acid sequences that are promiscuous include sequences from antigens such as tetanus toxoid at positions 830-843 (QYIKANSKFIGITE; SEQ ID NO: 627), *Plasmodium falciparum* CS protein at positions 378-398 (DIEKKIAKMEKASSVFNWNS; SEQ ID NO: 628), and Streptococcus 18kD protein at positions 116-131 (GAVDSILGGVATYGAA; SEQ ID NO: 629). Other examples include peptides bearing a DR 1-4-7 supermotif, or either of the DR3 motifs.

The HTL peptide may comprise a synthetic peptide such as a Pan-DR-binding epitope (*e.g.*, a PADRE^{®} peptide, Epimmune Inc., San Diego, CA, described, for example, in U.S. Patent Number 5,736,142), for example, having the formula aKXVAAZTLKAAa, where "X" is either cyclohexylalanine, phenylalanine, or tyrosine; "Z" is either tryptophan, tyrosine, histidine or asparagine; and "a" is either D-alanine or L-alanine (SEQ ID NO: 746). Certain pan-DR binding epitopes comprise all "L" natural amino acids; these molecules can be provided as peptides or in the form of nucleic acids that encode the peptide. See also, U.S. Patent Nos. 5,679,640 and 6,413,935.

The peptide may comprise additional amino acids. Such additional amino acids may be Ala, Arg, Asn, Asp, Cys, Gln, Gly, Glu, His, Ile, Leu, Lys, Met, Phe, Pro, Ser, Thr, Tyr, Trp, Val, amino acid mimetics, and other unnatural amino acids such as those described below. Additional amino acids may provide for ease of linking peptides one to another, for linking epitopes and/or analogs to one another, for linking epitopes and/or analogs to CTL and/or HTL epitopes, for coupling to a carrier support or larger peptide, for modifying the physical or chemical properties of the peptide or oligopeptide, or the like. Amino acids such as Ala, Arg, Asn, Asp, Cys, Gln, Gly, Glu, His, Ile, Leu, Lys, Met, Phe, Pro, Ser, Thr, Tyr, Trp, or Val, or the like, can be introduced at the C- and/or N-terminus of the peptide and/or can be introduced internally.

The peptide may comprise an amino acid spacer, which may be joined to the epitopes, analogs, CTL epitopes, HTL epitopes, carriers, etc. within a peptide or may be joined to the peptide at the N-and/or C-terminus. Thus, spacers may be at the N-terminus or C-terminus of peptide, or may be internal such that they link or join epitopes, analogs, CTL epitopes, HTL epitopes, carriers, additional amino acids, and/or antigenic fragments one to the other.

The spacer is typically comprised of one or more relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer may be composed of the same residues or may be composed of one or more different residues and thus may be a homo- or hetero-oligomer of spacer residues. Thus, the spacer may contain more than one Ala residue (poly-alanine) or more than one Gly residue (poly-glycine), or may contain both Ala and Gly residues, e.g., Gly, Gly-Gly-, Ser,Ser-Ser-, Gly-Ser-, Ser-Gly-, etc. When present, the spacer will usually be at least one or two residues, more usually three to six residues and sometimes 10 or more residues, e.g., 3, 4, 5, 6, 7, 8, 9, or 10, or even more residues. (Livingston, B.D. et al. Vaccine 19:4652-4660 (2000)).

Peptides may comprise carriers such as those well known in the art, *e*.*g*., thyroglobulin, albumins such as human serum albumin, tetanus toxoid, polyamino acids such as poly L-lysine, poly L-glutamic acid, influenza virus proteins, hepatitis B virus core protein, and the like. (See Table 31).

In addition, the peptide may be modified by terminal-NH₂ acylation, *e.g.*, by alkanoyl (C₁-C₂₀) or thioglycolyl acetylation, terminal-carboxyl amidation, *e.g.*, ammonia, methylamine, *etc*. In some instances these modifications may provide sites for linking to a support or other molecule.

The peptides in accordance with the invention can contain modifications such as but not limited to glycosylation, side chain oxidation, biotinylation, phosphorylation, addition of a surface active material, *e.g.* a lipid, or can be chemically modified, *e.g.*, acetylation, *etc*. Moreover, bonds in the peptide can be other than peptide bonds, *e*.*g*., covalent bonds, ester or ether bonds, disulfide bonds, hydrogen bonds, ionic bonds, *etc.*

Peptides of the present invention may contain substitutions to modify a physical property (*e.g.*, stability or solubility) of the resulting peptide. For example, peptides may be modified by the substitution of a cysteine (C) with α-amino butyric acid ("B"). Due to its chemical nature, cysteine has the propensity to form disulfide bridges and sufficiently alter the peptide structurally so as to reduce binding capacity. Substituting α-amino butyric acid for C not only alleviates this problem, but actually improves binding and crossbinding capability in certain instances. Substitution of cysteine with α-amino butyric acid may occur at any residue of a peptide, *e.g.*, at either anchor or non-anchor positions of an epitope or analog within a peptide, or at other positions of a peptide.

The peptides can comprise amino acid mimetics or unnatural amino acids, *e.g.* D- or L-naphylalanine; D- or L-phenylglycine; D- or L-2-thieneylalanine; D- or L-1, -2, 3-, or 4-pyreneylalanine; D- or L-3 thieneylalanine; D- or L-(2-pyridinyl)-alanine; D- or L-(3-pyridinyl)-alanine; D-or L-(2-pyrazinyl)-alanine; D- or L-(4-isopropyl)-phenylglycine; D-(trifluoromethyl)-phenylglycine; D-(trifluoromethyl)-phenylalanine; D-ρ-fluorophenylalanine; D- or L-ρ-biphenylphenylalanine; D- or L-ρ-methoxybiphenylphenylalanine; D- or L-2-indole(alkyl)alanines; and, D- or L-alkylalanines, where the alkyl group can be a substituted or unsubstituted methyl, ethyl, propyl, hexyl, butyl, pentyl, isopropyl, iso-butyl, sec-isotyl, iso-pentyl, or a non-acidic amino acids. Aromatic rings of a non-natural amino acid include, *e.g.*, thiazolyl, thiophenyl, pyrazolyl, benzimidazolyl, naphthyl, furanyl, pyrrolyl, and pyridyl aromatic rings. Modified peptides that have various amino acid mimetics or unnatural amino acids are particularly useful, as they tend to manifest increased stability *in vivo.* Such peptides may also possess improved shelf-life or manufacturing properties.

Peptide stability can be assayed in a number of ways. For instance, peptidases and various biological media, such as human plasma and serum, have been used to test stability. *See, e.g.,* Verhoef, et al., Eur. J. Drug Metab. Pharmacokinetics 11:291 (1986). Half-life of the peptides of the present invention is conveniently determined using a 25% human serum (v/v) assay. The protocol is generally as follows: Pooled human serum (Type AB, non-heat inactivated) is delipidated by centrifugation before use. The serum is then diluted to 25% with RPMI-1640 or another suitable tissue culture medium. At predetermined time intervals, a small amount of reaction solution is removed and added to either 6% aqueous trichloroacetic acid (TCA) or ethanol. The cloudy reaction sample is cooled (4°C) for 15 minutes and then spun to pellet the precipitated serum proteins. The presence of the peptides is then determined by reversed-phase HPLC using stability-specific chromatography conditions.

The peptides in accordance with the invention can be a variety of lengths, and either in their neutral (uncharged) forms or in forms which are salts. The peptides in accordance with the invention can contain modifications such as glycosylation, side chain oxidation, or phosphorylation, generally subject to the condition that modifications do not destroy the biological activity of the peptides.

The peptides of the invention may be lyophylized, or may be in crystal form.

It is generally preferable that the epitope be as small as possible while still maintaining substantially all of the immunologic activity of the native protein. When possible, it may be desirable to optimize HLA class I binding epitopes of the invention to a length of about 8 to about 13 amino acid residues, for example, 8, 9, 10, 11, 12 or 13, preferably 9 to 10. It is to be appreciated that one or more epitopes in this size range can be comprised by a longer peptide (see the Definition Section for the term "epitope" for further discussion of peptide length). HLA class II binding epitopes are preferably optimized to a length of about 6 to about 30 amino acids in length, *e.g.*, 6, 7, 8, 9, 10, 11, 12, 13, 14, 15, 16, 17, 18, 19, 20, 21, 22, 23, 24, 25, 26, 27, 28, 29 or 30, preferably to between about 13 and about 20 residues, *e.g.*, 13, 14, 15, 16, 17, 18, 19 or 20. Preferably, the epitopes are commensurate in size with endogenously processed pathogen-derived peptides or tumor cell peptides that are bound to the relevant HLA molecules. The identification and preparation of peptides of various lengths can be carried out using the techniques described herein.

Peptides in accordance with the invention can be prepared synthetically, by recombinant DNA technology or chemical synthesis, or can be isolated from natural sources such as native tumors or pathogenic organisms. Epitopes may be synthesized individually or joined directly or indirectly in a peptide. Although the peptide will preferably be substantially free of other naturally occurring host cell proteins and fragments thereof, in some embodiments the peptides may be synthetically conjugated to be joined to native fragments or particles.

The peptides of the invention can be prepared in a wide variety of ways. For relatively short sizes, the peptides can be synthesized in solution or on a solid support in accordance with conventional techniques. Various automatic synthesizers are commercially available and can be used in accordance with known protocols. (*See*, for example, Stewart & Young, SOLID PHASE PEPTIDE SYNTHESIS, 2D. ED., Pierce Chemical Co., 1984). Further, individual peptides can be joined using chemical ligation to produce larger peptides that are still within the bounds of the invention.

Alternatively, recombinant DNA technology can be employed wherein a nucleotide sequence which encodes a peptide inserted into an expression vector, transformed or transfected into an appropriate host cell and cultivated under conditions suitable for expression. These procedures are generally known in the art, as described generally in Sambrook et al., MOLECULAR CLONING, A LABORATORY MANUAL, Cold Spring Harbor Press, Cold Spring Harbor, New York (1989). Thus, recombinant peptides, which comprise or consist of one or more epitopes of the invention, can be used to present the appropriate T cell epitope.

Polynucleotides encoding each of the peptides above are also part of the invention. As appreciated by one of ordinary skill in the art, various nucleic acids will encode the same peptide due to the redundancy of the genetic code. Each of these nucleic acids falls within the scope of the present invention. This embodiment of the invention comprises DNA and RNA, and in certain embodiments a combination of DNA and RNA. It is to be appreciated that any polynucleotide that encodes a peptide in accordance with the invention falls within the scope of this invention.

The polynucleotides encoding peptides contemplated herein can be synthesized by chemical techniques, for example, the phosphotriester method of Matteucci, et al., J. Am. Chem. Soc. 103:3185 (1981). Polynucleotides encoding peptides comprising or consisting of an analog can be made simply by substituting the appropriate and desired nucleic acid base(s) for those that encode the native epitope.

The polynucleotide, e.g. minigene (see below), may be produced by assembling oligonucleotides that encode the plus and minus strands of the polynucleotide, e.g. minigene. Overlapping oligonucleotides (15-100 bases long) may be synthesized, phosphorylated, purified and annealed under appropriate conditions using well known techniques. The ends of the oligonucleotides can be joined, for example, using T4 DNA ligase. A polynucleotide, e.g. minigene, encoding the peptide of the invention, can be cloned into a desired vector such as an expression vector. The coding sequence can then be provided with appropriate linkers and ligated into expression vectors commonly available in the art, and the vectors used to transform suitable hosts to produce the desired peptide such as a fusion protein.

A large number of such vectors and suitable host systems are known to those of skill in the art, and are commercially available. The following vectors are provided by way of example. Bacterial: pQE70, pQE60, pQE-9 (Qiagen), pBS, pD10, phagescript, psiX174, pBluescript SK, pbsks, pNH8A, pNH16a, - pNH18A, pNH46A (Stratagene); ptrc99a, pKK223-3, pKK233-3, pDR540, pRIT5 (Pharmacia); pCR (Invitrogen). Eukaryotic: pWLNEO, pSV2CAT, pOG44, pXT1, pSG (Stratagene) pSVK3, pBPV, pMSG, pSVL (Pharmacia); p75.6 (valentis); pCEP (Invitrogen); pCEI (Epimmune). However, any other plasmid or vector can be used as long as it is replicable and viable in the host.

As representative examples of appropriate hosts, there can be mentioned: bacterial cells, such as *E. coli, Bacillus subtilis, Salmonella typhimurium* and various species within the genera Pseudomonas, Streptomyces, and Staphylococcus; fungal cells, such as yeast; insect cells such as Drosophila and Sf9; animal cells such as COS-7 lines of monkey kidney fibroblasts, described by Gluzman, Cell 23:175 (1981), and other cell lines capable of expressing a compatible vector, for example, the C127, 3T3, CHO, HeLa and BHK cell lines or Bowes melanoma; plant cells, etc. The selection of an appropriate host is deemed to be within the scope of those skilled in the art from the teachings herein.

Thus, the present invention is also directed to vectors, preferably expression vectors useful for the production of the peptides of the present invention, and to host cells comprising such vectors.

Host cells are genetically engineered (transduced or transformed or transfected) with the vectors of this invention which can be, for example, a cloning vector or an expression vector. The vector can be, for example, in the form of a plasmid, a viral particle, a phage, etc. The engineered host cells can be cultured in conventional nutrient media modified as appropriate for activating promoters, selecting transformants or amplifying the polynucletides. The culture conditions, such as temperature, pH and the like, are those previously used with the host cell selected for expression, and will be apparent to the ordinarily skilled artisan.

For expression of the peptides, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts.

Generally, recombinant expression vectors will include origins of replication and selectable markers permitting transformation of the host cell, *e.g.,* the ampicillin resistance gene of *E. coli* and *S. cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), ∀-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, *e.g.*, stabilization or simplified purification of expressed recombinant product.

Yeast, insect or mammalian cell hosts may also be used, employing suitable vectors and control sequences. Examples of mammalian expression systems include the COS-7 lines of monkey kidney fibroblasts, described by Gluzman, Cell 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. Such promoters may also be derived from viral sources, such as, e.g., human cytomegalovirus (CMV-IE promoter) or herpes simplex virus type-1 (HSV TK promoter). Nucleic acid sequences derived from the SV40 splice, and polyadenylation sites can be used to provide the required nontranscribed genetic elements.

Polynucleotides encoding peptides of the invention may also comprise a ubiquitination signal sequence, and/or a targeting sequence such as an endoplasmic reticulum (ER) signal sequence to facilitate movement of the resulting peptide into the endoplasmic reticulum.

Polynucleotides of the invention, e.g., minigenes, may be expressed in human cells. A human codon usage table can be used to guide the codon choice for each amino acid. Such polynucleotides preferably comprise spacer amino acid residues between epitopes and/or analogs, such as those described above, or may comprise naturally-occurring flanking sequences adjacent to the epitopes and/or analogs (and/or CTL and HTL epitopes).

The peptides of the invention can also be expressed by viral or bacterial vectors. Examples of expression vectors include attenuated viral hosts, such as vaccinia or fowlpox. As an example of this approach, vaccinia virus is used as a vector to express nucleotide sequences that encode the peptides of the invention. Vaccinia vectors and methods useful in immunization protocols are described in, *e.g.,* U.S. Patent No. 4,722,848. Another vector is BCG (Bacille Calmette Guerin). BCG vectors are described in Stover et al., Nature 351:456-460 (1991). A wide variety of other vectors useful for therapeutic administration or immunization of the polypeptides of the invention, *e.g.* adeno and adeno-associated virus vectors, retroviral vectors, *Salmonella typhi* vectors, detoxified anthrax toxin vectors, and the like, will be apparent to those skilled in the art from the description herein. A preferred vector is Modified Vaccinia Ankara (MVA) (*e.g.*, Bavarian Noridic (MVA-BN)).

Standard regulatory sequences well known to those of skill in the art are preferably included in the vector to ensure expression in the human target cells. Several vector elements are desirable: a promoter with a downstream cloning site for polynucleotide, *e.g.*, minigene insertion; a polyadenylation signal for efficient transcription termination; an *E*. *coli* origin of replication; and an *E. coli* selectable marker (*e.g.* ampicillin or kanamycin resistance). Numerous promoters can be used for this purpose, *e.g.*, the human cytomegalovirus (hCMV) promoter. See, *e.g.*, U.S. Patent Nos. 5,580,859 and 5,589,466 for other suitable promoter sequences. A preferred promoter is the CMV-IE promoter.

Polynucleotides, e.g. minigenes, may comprise one or more synthetic or naturally-occurring introns in the transcribed region. The inclusion of mRNA stabilization sequences and sequences for replication in mammalian cells may also be considered for increasing polynucleotide, e.g. minigene, expression.

In addition, the polynucleotide, e.g. minigene, may comprise immunostimulatory sequences (ISSs or CpGs). These sequences may be included in the vector, outside the polynucleotide (e.g. minigene) coding sequence to enhance immunogenicity.

In some embodiments, a bi-cistronic expression vector which allows production of both the polynucleotide- (e.g. minigene-) encoded peptides of the invention and a second protein (*e.g.*, one that modulates immunogenicity) can be used. Examples of proteins or polypeptides that, if co-expressed with peptides of the invention, can enhance an immune response include cytokines (*e.g.,* IL-2, IL-12, GM-CSF), cytokine-inducing molecules (*e.g.,* LeIF), costimulatory molecules, or pan-DR binding proteins (PADRE^{®} molecules, Epimmune, San Diego, CA). Helper T cell (HTL) epitopes such as PADRE^{®} molecules can be joined to intracellular targeting signals and expressed separately,from expressed peptides of the invention. Specifically decreasing the immune response by co-expression of immunosuppressive molecules (*e.g.* TGF-β) may be beneficial in certain diseases.

Once an expression vector is selected, the polynucleotide, e.g. minigene, is cloned into the polylinker region downstream of the promoter. This plasmid is transformed into an appropriate bacterial strain, and DNA is prepared using standard techniques. The orientation and DNA sequence of the polynucleotide, e.g. minigene, as well as all other elements included in the vector, are confirmed using restriction mapping, DNA sequence analysis, and/or PCR analysis. Bacterial cells harboring the correct plasmid can be stored as cell banks.

Therapeutic/prophylactic quantities of DNA can be produced for example, by fermentation in *E*. *coli,* followed by purification. Aliquots from the working cell bank are used to inoculate growth medium, and are grown to saturation in shaker flasks or a bioreactor according to well known techniques. Plasmid DNA is purified using standard bioseparation technologies such as solid phase anion-exchange resins available, *e.g.,* from QIAGEN, Inc. (Valencia, California). If required, supercoiled DNA can be isolated from the open circular and linear forms using gel electrophoresis or other methods.

Purified polynucleotides, e.g. minigenes, can be prepared for injection using a variety of formulations. The simplest of these is reconstitution of lyophilized polynucleotide, e.g. DNA, in sterile phosphate-buffer saline (PBS). This approach, known as "naked DNA," is currently being used for intramuscular (IM) administration in clinical trials. To maximize the immunotherapeutic effects of polynucleotide vaccines, alternative methods of formulating purified plasmid DNA may be used. A variety of such methods have been described, and new techniques may become available. Cationic lipids, glycolipids, and fusogenic liposomes can also be used in the formulation (see, *e.g.*, WO 93/24640; Mannino & Gould-Fogerite, BioTechniques 6(7): 682 (1988); U.S. Patent No. 5,279,833; WO 91/06309; and Felgner, et al., Proc. Nat'l Acad. Sci. USA 84:7413 (1987). In addition, peptides and compounds referred to collectively as protective, interactive, non-condensing compounds (PINC) can also be complexed to purified plasmid DNA to influence variables such as stability, intramuscular dispersion, or trafficking to specific organs or cell types.

Known methods in the art can be used to enhance delivery and uptake of a polynucleotide *in vivo.* For example, the polynucleotide can be complexed to polyvinylpyrrolidone (PVP), to prolong the localized bioavailability of the polynucleotide, thereby enhancing uptake of the polynucleotide by the organisum (*see e.g.,* U.S. Patent No. 6,040,295; EP 0 465 529; WO 98/17814). PVP is a polyamide that is known to form complexes with a wide variety of substances, and is chemically and physiologically inert.

Target cell sensitization can be used as a functional assay of the expression and HLA class I presentation of polynucleotide- (e.g. minigene-) encoded peptides. For example, the polynucleotide, e.g. plasmid DNA, is introduced into a mammalian cell line that is a suitable target for standard CTL chromium release assays. The transfection method used will be dependent on the final formulation. For example, electroporation can be used for "naked" DNA, whereas cationic lipids or PVP-formulated DNA allow direct *in vitro* transfection. A plasmid expressing green fluorescent protein (GFP) can be co-transfected to allow enrichment of transfected cells using fluorescence activated cell sorting (FACS). The transfected cells are then chromium-51 (⁵¹Cr) labeled and used as targets for epitope-specific CTLs. Cytolysis of the target cells, detected by ⁵¹Cr release, indicates both production and HLA presentation of, polynucleotide-, e.g. minigene-, encoded epitopes and/or analogs of the invention, or peptides comprising them. Expression of HTL epitopes may be evaluated in an analogous manner using assays to assess HTL activity.

*In vivo* immunogenicity is a second approach for functional testing of polynucleotides, e.g. minigenes. Transgenic mice expressing appropriate human HLA proteins are immunized with the polynucleotide, e.g. DNA, product. The dose and route of administration are formulation dependent (*e.g.*, IM for polynucleotide (*e.g.*, naked DNA or PVP-formulated DNA) in PBS, intraperitoneal (IP) for lipid-complexed polynucleotide (*e.g.*, DNA)). Eleven to twenty-one days after immunization, splenocytes are harvested and restimulated for one week in the presence of polynucleotides encoding each peptide being tested. Thereafter, for peptides comprising or consisting of epitopes and/or analogs, standard assays are conducted to determine if there is cytolysis of peptide-loaded, ⁵¹Cr-labeled target cells. Once again, lysis of target cells that were exposed to epitopes and/or analogs corresponding to those encoded by the polynucleotide, e.g. minigene, demonstrates polynucleotide, e.g., DNA, vaccine function and induction of CTLs. Immunogenicity of HTL epitopes is evaluated in transgenic mice in an analogous manner.

Alternatively, the nucleic acids can be administered using ballistic delivery as described, for instance, in U.S. Patent No. 5,204,253. Using this technique, particles comprised solely of a polynucleotide such as DNA are administered. In a further alternative embodiment for ballistic delivery, polynucleotides such as DNA can be adhered to particles, such as gold particles.

The use of polynucleotides such as multi-epitope minigenes is described herein and in, *e.g.* co-pending application U.S.S.N. 09/311,784; Ishioka et al., J. Immunol. 162:3915-3925, 1999; An, L. and Whitton, J. L., J. Virol. 71:2292, 1997; Thomson, S. A. et al., J. Immunol. 157:822, 1996; Whitton, J. L. et al., J. Virol. 67:348, 1993; Hanke, R. et al., Vaccine 16:426, 1998. For example, a polynucleotide such as a multi-epitope DNA plasmid can be engineered which encodes an epitope derived from multiple regions of a TAA (*e.g.,* p53, HER2/nev, MAGE-2/3, or CEA), a pan-DR binding peptide such as the PADRE^{®} universal helper T cell epitope, and an endoplasmic reticulum-translocating signal sequence. As descibed in the sections above, a peptide/polynucleotide may also comprise/encode epitopes that are derived from other TAAs.

Thus, the invention includes peptides as described herein, polynucleotides encoding each of said peptides, as well as compositions comprising the peptides and polynucleotides, and includes methods for producing and methods of using the peptides, polynucleotides, and compositions, as further described below.

Compositions. In other embodiments, the invention is directed to a composition comprising one or more peptides and/or a polynucleotide of the invention and optionally another component(s).

In some embodiments, the composition comprises or consists of multiple peptides, *e.g.*, 2, 3, 4, 5, 6, 7, 8, 9, 10, 11, 12, 13, 14, 15, 16, 17, 18, 19, 20, 21, 22, 23, 24, or 25 peptides of the invention. In other embodiments, the composition comprises or consists of 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145 or 150 or more peptides of the invention. In some embodiments, the composition comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 44, at least 45, at least 46, at least 47, at least 48, at least 49, at least 50, peptides of the invention.

For example, compositions may comprise or consist of combinations of epitopes and/or analogs, *e*.*g*., 2, 3, 4, 5, 6, 7, 8, 9, 10, 11, 12, 13, 14, 15, 16, 17, 18, 19, 20, 21, 22, 23, 24, or 25 epitopes and/or analogs of the invention. In other embodiments, the composition comprises or consists of 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 epitopes and/or analogs of the invention. In some embodiments, the composition comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25 epitopes and/or analogs of the invention.

For example, the composition may comprise or consist of at least 1, at least 2, at least 3, at least 4, or all 5 CEA epitopes and/or analogs from Table 6; at least 1, at least 2, at least 3, at least 4, at least 5, at least 6, at least 7, at least 8, at least 9, or all 10 HER2/neu epitopes and/or analogs of Table 6; at least 1, at least 2, at least 3, at least 4, or all 5 MAGE2/3 epitopes and/or analogs from Table 6; at least 1, at least 2, at least 3, at least 4, or all 5 p53 epitopes and/or analogs from Table 6. The composition may comprise or consist of at least 1, at least 2, at least 3, at least 4, at least 5, at least 6, at least 7, at least 8, at least 9, at least 10, at least 11, at least 12, at least 13, or all 14 epitopes and/or analogs from Table 16a; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or all 20 epitopes and/or analogs from Table 16b; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, or all 28 epitopes and/or analogs from Table 16c; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, or all 25 epitopes and/or analogs from Table 16d. The composition may comprise or consist of at least 1, at least 2, at least 3, at least 4, or all 5 epitopes and/or analogs from Table 17a; at least 1, at least 2, at least 3, at least 4, at least 5, or all 6 epitopes and/or analogs from Table 17b; at least 1, at least 2, at least 3, at least 4, at least 5, at least 6, at least 7, at least 8, at least 9, at least 10, at least 11, at least 12, at least 13, or all 14 epitopes and/or analogs from Table 17c; at least 1, at least 2, at least 3, or all 4 epitopes and/or analogs from Table 17d. The composition may comprise or consist of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, or all 27 epitopes and/or analogs from Table 18a; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, or all 24 epitopes and/or analogs from Table 18b; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, or all 28 epitopes and/or analogs from Table 18c; at least 1, at least 2, at least 3, at least 4, at least 5, at least 6, at least 7, at least 8, at least 9, or all 10 epitopes and/or analogs from Table 18d. The composition may comprise or consist of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, or all 45 epitopes and/or analogs from Table 19a; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or all 21 epitopes and/or analogs from Table 19b; at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32,_at least 33, at least 34, at least 35, at least 36, at least 37, at least 38, at least 39, or all 40 epitopes and/or analogs from Table 19c; at least 1, at least 2, at least 3, at least 4, at least 5, at least 6, at least 7, at least 8, or all 9 epitopes and/or analogs from Table 19d. The composition may comprise or consist of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, or at least 26 of the epitopes and/or analogs from Table 26, 27, 28, 29, or 30.

The composition may preferably comprise or consist of at least 1 or all 2 CEA epitopes/analogs of Table 9; at least 1 or all 2 HER2/neu epitopes/analogs of Table 9; at least 1 or all 2 MAGE2/3 epitopes/analogs of Table 9; at least 1 or all 2 p53 epitopes/analogs of Table 9. The composition may preferably comprise or consist of at least 1, at least 2, at least 3, at least 4, at least 5, at least 6, or all 7 CEA epitopes/analogs of Table 20; at least 1, at least 2, at least 3, at least 4, at least 5, at least 6, at least 7, at least 8, or all 9 HER2/neu epitopes/analogs of Table 20; at least 1, at least 2, at least 3, at least 4, at least 5, at least 6, at least 7, or all 8 MAGE2/3 epitopes/analogs of Table 20; at least 1, at least 2, at least 3, at least 4, at least 5, at least 6, or all 7 p53 epitopes/analogs of Table 20. The composition may preferably comprise or consist of at least the CEA epitope/analog of Table 21; at least the HER2/neu epitope/analog of Table 21; at least 1, at least 2, at least 3, or all 4 MAGE2/3 epitopes/analogs of Table 21; at least the p53 epitope/analog of Table 21. The composition may preferably comprise or consist of at least 1, at least 2, at least 3, at least 4, at least 5, at least 6, at least 7, or all 8 CEA epitopes/analogs of Table 22; at least 1, at least 2, at least 3, at least 4, at least 5, at least 6, at least 7, at least 8, or all 9 HER2/neu epitopes/analogs of Table 22; at least 1, at least 2, at least 3, at least 4, at least 5, at least 6, at least 7, or all 8 MAGE2/3 epitopes/analogs of Table 22; at least 1, at least 2, at least 3, at least 4, or all 5 p53 epitopes/analogs of Table 22. The composition may preferably comprise or consist of at least 1, at least 2, at least 3, at least 4, at least 5, at least 6, at least 7, at least 9, qat least 10, at least 11, or all 12 CEA epitopes/analogs of Table 23; at least 1, at least 2, at least 3, at least 4, at least 5, or all 6 HER2/neu epitopes/analogs of Table 23; at least 1, at least 2, at least 3, at least 4, at least 5, at least 6, at least 7, at least 8, at least 9, at least 10, at least 11, at least 12, or all 13 MAGE2/3 epitopes/analogs of Table 23; at least 1, or all 2 p53 epitopes/analogs of Table 23.

The composition may comprise or consist of the combinations above and below, and may also exclude any one or several epitopes and/or analogs selected from those in Tables 6, 9, 10 (SEQ ID Nos:1-25), 16a-23 (SEQ ID NOS:42-362) and 26-30 (SEQ ID Nos:368-745). Epitopes/analogs which may preferably be excluded from composition of the invention are SEQ ID Nos:42, 60, 62, 67, 82, 86, 101, 116, 153, 362, 230, 265, 290, 321, 334, and 345.

The composition of the invention may comprise or consist of combinations of epitopes and/or analogs including:
A3 CEA combinations such as: (a) SEQ ID NOs:42, 44, 46, 51, 52, 54, and 55; (b) SEQ ID NOs: 44, 46, 51, 52, 54, and 55; (c) SEQ ID NOs:46, 51, 52, 54, and 55; (d) SEQ ID NOs: 51, 52, 54, and 55; (e) SEQ ID NO: 52, 54, and 55; (f) SEQ ID NO: 54 and 55;
   (g) SEQ ID NO: 44, 46, 51, 52, and 54; (h) SEQ ID NO: 44, 46, 51, and 52; (i) SEQ ID NO: 44, 46, and 51; and (j) SEQ ID NO: 44 and 46;
   (1) SEQ ID NO: 44, 51, 52, 54, and 55; (m) SEQ ID NO: 44, 46, 52, 54, and 55; (n) SEQ ID NO: 44,46, 51, 54, and 55; and (o) SEQ ID NO: 44, 46, 51, 52, and 55;
A3 HER2/neu combinations such as: (a) SEQ ID NO:57, 60, 62, 67, 68, 69, 70, 73, and 75; (b) SEQ ID NO: 60, 62, 67, 68, 69, 70, 73, and 75; (c) SEQ ID NO: 62, 67, 68, 69, 70, 73, and 75; (d) 67, 68, 69, 70, 73, and 75; (e) 68, 69, 70, 73, and 75; (f) SEQ ID NO: 69, 70, 73, and 75;
   (g) SEQ ID NO: 70, 73, and 75; (h) SEQ ID NO: 73 and 75;
   (i) SEQ ID NO: 57, 60, 62, 67, 68, 69, 70, and 73; (j) SEQ ID NO: 57, 60, 62, 67, 68, 69, and 70; (k) SEQ ID NO: 57, 60, 62, 67, 68, and 69; (1) SEQ ID NO: 57, 60, 62, 67, and 68;
   (m) SEQ ID NO: 57, 60, 62, and 67; (n) SEQ ID NO: 57, 60, and 62; (o) SEQ ID NO: 57 and 60; (p) SEQ ID NO: 57, 68, 69, 70, 73, and 75; (q) SEQ ID NO: 57, 60, 68, 69, 70, 73, and 75; (r) SEQ ID NO: 57, 60, 62, 69, 70, 73, and 75;
   and (s) SEQ ID NO: 57, 60, 62, 67, 68, 73, and 75;
A3 MAGE2/3 combinations such as: (a) SEQ ID NO:82, 90, 91, 96, 99, 102, and 103; (b) SEQ ID NO: 90, 91, 96, 99, 102, and 103; (c) SEQ ID NO: 91, 96, 99, 102, and 103; (d) SEQ ID NO: 96, 99, 102, and 103; (e) SEQ ID NO: 99, 102, and 103; (f) SEQ ID NO: 102 and 103;
   (g) SEQ ID NO: 77, 82, 90, 91, 96, 99, and 102; (h) SEQ ID NO: 77, 82, 90, 91, 96, and 99; (i) SEQ ID NO: 77, 82, 90, 91, and 96; (j) SEQ ID NO: 77, 82, 90, and 91; (k) SEQ ID NO: 77, 82, 90, and 91, 96, 99, 102, and 103; (1) SEQ ID ON: 77, 82, and 90; and (m) SEQ ID NO: 77 and 82;
A3 p53 combinations such as: (a) SEQ ID NO: 107,111,114, 116, 119, and 124; (b) SEQ ID NO: 111, 114, 116, 119, and 124; (c) SEQ ID NO: 114, 116, 119, and 124; (d) SEQ ID NO: 116, 119, and 124; (e) SEQ ID NO: 119 and 124;
   (f) SEQ ID NO:104, 107, 111, 114, 116, and 119; (g) SEQ ID NO:104, 107, 111, 114, and 116; (h) SEQ ID NO:104, 107, 111, and 114; (i) SEQ ID NO: 104, 107, and 111; (j) SEQ ID NO:104 and 107;
   (k) SEQ ID NO: 104,111,114,116,119, and 124; (1) SEQ ID NO:104,107,114,116, 119, and 124; (m) SEQ ID NO:104, 107, 111, 116, 119, and 124; (n) SEQ ID NO:104, 107, 111, 114, 116, and 124;
   B7 MAGE2/3 combinations such as: (a) SEQ ID NO: 146, 153, and 364; (b) SEQ ID NO: 153 and 364; (d) SEQ ID NO: 140, 146, and 153; (e) SEQ ID NO: 140, 146, and 364;
B7 combinations such as: (a) SEQ ID NO:133, 136, 140, 146, 153, and 155; (b) SEQ ID NO: 136, 140, 146, 153, and 155; (c) SEQ ID NO: 140, 146, 153, and 155; (d) SEQ ID NO: 153 and 155;
A1 CEA combinations such as: (a) SEQ ID NO: 167, 170, 172, 178, 180, 181, and 182; (b) SEQ ID NO: 170, 172, 178, 180, 181, and 182; (c) SEQ ID NO: 172, 178, 180, 181, and 182; (d) SEQ ID NO: 178, 180, 181, and 182; (e) SEQ ID NO:180, 181, and 182; (f) SEQ ID NO: 181 and 182; (g) SEQ ID NO: 161, 167, 170, 172, 178, 180, and 181; (h) SEQ ID NO: 161, 167, 170, 172, 178, and 180; (i) SEQ ID NO: 161, 167, 170, 172, and 178; (j) SEQ ID NO: 161, 167, and 170; (k) SEQ ID NO: 181 and 182;
A1 HER2/neu combinations such as : (a) SEQ ID NO:188, 189, 191, 194, 198, 200, 201, and 208; (b) SEQ ID NO: 189, 191, 194, 198, 200, 201, and 208; (c) SEQ ID NO: 191, 194, 198, 200, 201, and 208; (d) SEQ ID NO: 194, 198, 200, 201, and 208; (e) SEQ ID NO: 198, 200, 201, and 208; (f) SEQ ID NO: 200, 201, and 208; (g) SEQ ID NO:201 and 208;
   (h) SEQ ID NO:186, 188, 189, 191, 194, 198, 200, and 201; (i) SEQ ID NO:186, 188, 189, 191, 194, 198, and 200; (j) SEQ ID NO:186, 188, 189, 191, 194, and 198; (k) SEQ ID NO:186, 188, 189, 191, and 194; (l) SEQ ID NO:186, 188, 189, and 191; (m) SEQ ID NO:186, 188, and 189; (n) SEQ ID NO:186 and 188;
A1 MAGE2/3 combinations such as: (a) SEQ ID NO: 216, 219, 221, 228, 230, 234, and 236; (b) SEQ ID NO: 219, 221, 228, 230, 234, and 236; (c) SEQ ID NO: 221, 228, 230, 234, and 236; (d) SEQ ID NO: 228, 230, 234, and 236; (e) SEQ ID NO: 230, 234, and 236; (f) SEQ ID NO: 234 and 236; (g) SEQ ID NO:211, 216, 219, 221, 228, 230, and 234; (h) SEQ ID NO:211, 216, 219, 221, 228, and 230; (i) SEQ ID NO:211, 216, 219, 221, and 228; (j) SEQ ID NO:211, 216, 219, and 221; (k) SEQ ID NO:211, 216, and 219; (1) SEQ ID NO:211 and 216; (m) SEQ ID NO:211, 216, 219, 221, 228, 234, and 236;
A1 p53 combinations such as: (a) SEQ ID NO: 239, 240, 242, and 246; (b) SEQ ID NO: 240, 242, and 246; (c) SEQ ID NO: 242 and 246; (d) SEQ ID NO:238, 239, 240, and 242; (e) SEQ ID NO:238, 239, and 240; (f) SEQ ID NO:238 and 239; (g) SEQ ID NO:238, 240, 242, and 246; (h) SEQ ID NO:238, 239, 242, and 246; (i) SEQ ID NO:238, 239, 240, and 246;
A24 CEA combinations such as: (a) SEQ ID NO: 263, 265, 269, 272, 278, 279, 281, 282, 285, 287, and 290; (b) SEQ ID NO: 265, 269, 272, 278, 279, 281, 282, 285, 287, and 290; (c) SEQ ID NO: 269, 272, 278, 279, 281, 282, 285, 287, and 290; (d) SEQ ID NO: 272, 278, 279, 281, 282, 285, 287, and 290; (e) SEQ ID NO: 278, 279, 281, 282, 285, 287, and 290; (f) SEQ ID NO: 279, 281, 282, 285, 287, and 290; (g) SEQ ID NO: 281, 282, 285, 287, and 290; (h) SEQ ID NO: 282, 285, 287, and 290; (i) SEQ ID NO: 285, 287, and 290; (j) SEQ ID NO: 287 and 290;
   (k) SEQ ID NO:256, 263, 265, 269, 272, 278, 279, 281, 282, 285, and 287; (1) SEQ ID NO:256, 263, 265, 269, 272, 278, 279, 281, 282, and 285; (m) SEQ m NO:256, 263, 265, 269, 272, 278, 279, 281, and 282; (n) SEQ ID N0:256, 263, 265, 269, 272, 278, 279, and 281; (o) SEQ ID NO:256, 263, 265, 269, 272, 278, and 279; (p) SEQ ID NO:256, 263, 265, 269, 272, and 278; (q) SEQ ID NO:256, 263, 265, 269, and 272; (r) SEQ ID NO:256, 263, 265, and 269; (s) SEQ ID NO:256, 263, and 265; (t) SEQ ID NO:256 and 263; (u) SEQ ID NO:256, 263, 269, 272, 278, 279, 281, 282, 285, and 287;
A24 HER2/neu combinations such as: (a) SEQ ID NO: 293, 304, 305, 308, and 310; (b) SEQ ID NO: 304, 305, 308, and 310; (c) SEQ ID NO: 305, 308, and 310; (d) SEQ ID NO: 308 and 310; (e) SEQ ID NO:292, 293, 304, 305, and 308; (f) SEQ ID NO:292, 293, 304, and 305; (g) SEQ ID NO:292, 293, and 304; (h) SEQ ID NO:292 and 293; (i) SEQ ID NO:292, 304, 305, 308, and 310; (j) SEQ ID NO:292, 293, 305, 308, and 310; (k) SEQ ID NO:292, 293, 304, 308, and 310; (1) SEQ ID NO:292, 293, 304, 305, and 310;
A24 MAGE2/3 combinations such as: (a) SEQ ID NO: 321, 324, 325, 331, 332, 333, 334, 335, 336, 344, 345, and 35 1; (b) SEQ ID NO: 324, 325, 331, 332, 333, 334, 335, 336, 344, 345, and 351; (c) SEQ ID NO: 325, 331, 332, 333, 334, 335, 336, 344, 345, and 351; (d) SEQ ID NO: 331, 332, 333, 334, 335, 336, 344, 345, and 351; (e) SEQ ID NO: 332, 333, 334, 335, 336, 344, 345, and 351; (f) SEQ ID NO: 333, 334, 335, 336, 344, 345, and 351; (g) SEQ ID NO: 333, 334, 335, 336, 344, 345, and 351; (h) SEQ ID NO: 334, 335, 336, 344, 345, and 351; (i) SEQ ID NO: 335, 336, 344, 345, and 351; (j) SEQ ID NO: 336, 344, 345, and 351; (k) SEQ ID NO: 344, 345, and 351; (l) SEQ ID NO:345 and 351;
   (m) SEQ ID NO:316, 321, 324, 325, 331, 332, 333, 334, 335, 336, 344, and 345; (n) SEQ ID NO:316, 321, 324, 325, 331, 332, 333, 334, 335, 336, and 344; (o) SEQ ID NO:316, 321, 324, 325, 331, 332, 333, 334, 335, and 336; (p) SEQ ID NO:316, 321, 324, 325, 331, 332, 333, 334, and 335; (q) SEQ ID N0:316, 321, 324, 325, 331, 332, 333, and 334; (r) SEQ ID NO:316, 321, 324, 325, 331, 332, and 333; (s) SEQ ID NO:316, 321, 324, 325, 331, and 332; (t) SEQ ID NO:316, 321, 324, 325, and 331;
   (u) SEQ ID NO:316, 321, 324, and 325; (v) SEQ ID NO:316, 321, and 324; (w) SEQ ID NO:316 and 321; (x) SEQ ID NO:316, 324, 325, 331, 332, 333, 335, 336, 344, and 351;
A24 p53 combinations such as: SEQ ID NO:356 and 361;
B44 CEA combinations such as: (a) SEQ ID NO:368, 369, 390, 399, and 403; (b) SEQ ID NO:369, 370, 375, 376, 377, and 420; and (c) SEQ ID NO:370, 375, 379, 386, and 429;
B44 HER2/neu combinations such as: (a) SEQ ID NO:432, 435, 436, 443, 448, 460, 466, 467, and 488; (b) SEQ ID NO: 439, 473, 490, and 499; (c) SEQ ID NO:432, 433, 440, 441, 447, 456, 459, and 471; (d) SEQ ID NO: 477, 490, 499, 508, 527, and 535;
B44 MAGE2 combinations such as: (a) SEQ ID NO: 645, 646, 647, 653, 665, 670, 698, 718, and 716; (b) SEQ ID NO: 663, 688, 692, and 701; (c) SEQ ID NO:648, 655, 669, 677, 691, and 700; (d) SEQ ID NO: 651 and 673;
B44 MAGE3 combinations such as: (a) SEQ ID NO: 719, 720, 726, 732, and 740; (b) SEQ ID NO: 721, 725, 726, and 737; (c) SEQ ID NO: 726, 739, and 744; (d) SEQ ID NO: 722, 723, 728 and 735; (e) SEQ ID NO: 720, 728, 731, 736, and 741;
B44 p53 combinations such as: (a) SEQ ID NO: 598, 602, 603, and 617; (b) SEQ ID NO: 589, 599, 600, and 605; (c) SEQ ID NO:600, 603, 604, and 607; (d) SEQ ID NO: 601, 602, 604, and 609;
A2 combinations such as: (a) SEQ ID NO: 6, 8, 16, 18, 22, 23, and 24; (b) SEQ ID NO: 8, 16, 18, 22, 23, and 24; (c) SEQ ID NO: 16, 18, 22, 23, and 24; (d) SEQ ID NO: 18, 22, 23, and 24; (e) SEQ ID NO: 23 and 24; (f) SEQ ID NO: 1, 19, 3, and 4; (g) SEQ ID NO: 2, 6, 8, 16, 18, 22, and 23; (h) SEQ ID NO: 2, 6, 8, 16, 18, and 22; (i) SEQ ID NO: 2, 6, 8, 16, 18, and 22; (j) SEQ ID NO: 2, 6, 8, 16, and 18; (k) SEQ ID NO: 2, 6, 8, and 16; (l) SEQ ID NO: 2, 6, and 8; and (m) SEQ ID NO: 2 and 6; (n) SEQ ID NO:3, 4, 5, and 17; (o) SEQ ID NO: 20, 21, and 25; (p) SEQ ID NO: 1, 10, 17, and 25; (q) SEQ ID NO: 4, 5, 10, 17, and 25;
TAA combinations such as: (a) SEQ ID NO: 1, 17, 22, 104, 114, 133, 136, 146, 170, 189, 221, 310, 336, 361, and 399; (b) SEQ ID NO:111, 124, 133, 140, 155, 180, 194, 228, 246, and 281; (c) SEQ m NO: 16, 18, 25, 43, 68, 117, 309, and 499; (d) SEQ ID NO: 48, 55, 97, 369, 409, and 512; (e) SEQ ID NO: 55, 99, 135, 238, and 602; (f) SEQ ID NO: 1, 58, 77, 104, 128, 166, 207, 240, 360, and 403; (g) SEQ ID NO:17,50, 72, 130, 161, 199, 300, and 627; (h) SEQ ID NO: 10, 55, 82, 104, 198, 400, 433, and 501; (i) SEQ ID NO: 3, 22, 122, 196, 211, 301, 360, and 667; (j) SEQ ID NO: 1, 21, 44, 100, 207, 405, and 661.

Compositions of the invention may also comprise or consist of combinations of the above combinations, including:
A24 combinations such as: A24 CEA (a) and A24 HER2/neu (a); A24 CEA (a) and
A24 MAGE2/3 (a); A24 CEA (a) and A24 p53; A24 CEA (c) and A24 HER2/neu (e);
A24 CEA (i) and A24 MAGE2/3 (a); A24 CEA (n) and A24 p53 (k);
A3 combinations such as: A3 CEA (a) and A3 HER2/neu (a); A3 CEA (a) and A3 MAGE2/3 (a); A3 CEA (a) and A3 p53 (a); A3 CEA (d) and A3 HER2/neu (b); A3 CEA (f) and A3 MAGE2/3 (i); A3 CEA (e) and A3 p53 (a);
CEA combinations such as: A24 CEA (a) and A1 CEA (a); A24 CEA (b) and A1 CEA (a); A24 CEA (c) and A1 CEA (a); A24 CEA (c) and A1 CEA (a); A3 CEA (a) and A1 CEA (a); A3 CEA (b) and A1 CEA (a); A3 CEA (c) and A24 CEA (a); B7 CEA (c) and A1 CEA (a);
B7 CEA (a) and A3 CEA (a); B44 CEA (b) and A1 CEA (a); A3 CEA (e) and A1 CEA (g); A3 CEA (i) and A1 CEA (m);.
A1 CEA (a), (b) (c), (d), (e), (f) (g), (h), (i), (j), or (k) epitopes/analogs, and A3 CEA (a), (b) (c), (d), (e), (f) (g), (h), (i), (j), or (k) epitopes/analogs, and B7 CEA (a), (b) (c), (d), (e), (f) (g), (h), (i), (j), or (k) epitopes/analogs, and A3 p53 (a), (b) (c), (d), (e), (f) (g), (h), (i), (j), or (k) epitopes/analogs, and B44 MAGE2 (a), (b) (c), (d), (e), (f) (g), (h), (i), (j), or (k) epitopes/analogs, and A3 MAGE2 (a), (b) (c), (d), (e), (f) (g), (h), (i), (j), or (k) epitopes/analogs;
A24 CEA (a), (b) (c), (d), (e), (f) (g), (h), (i), (j), or (k) epitopes/analogs, and A2 CEA (a), (b) (c), (d), (e), (f) (g), (h), (i), (j), or (k) epitopes/analogs, and B7 MAGE3 (a), (b) (c), (d), (e), (f) (g), (h), (i), (j), or (k) epitopes/analogs, and B44 p53 (a), (b) (c), (d), (e), (f) (g), (h), (i), (j), or (k) epitopes/analogs;
A3 CEA (a), (b) (c), (d), (e), (f) (g), (h), (i), (j), or (k) epitopes/analogs, and B7 p53 (a), (b) (c), (d), (e), (f) (g), (h), (i), (j), or (k) epitopes/analogs, and B44 MAGE3 (a), (b) (c), (d), (e), (f) (g), (h), (i), (j), or (k) epitopes/analogs, and A24 HER2/neu (a), (b) (c), (d), (e), (f) (g), (h), (i), (j), or (k) epitopes/analogs.

Compositions of the invention may comprise polynucleotides encoding the above peptides, and/or combinations of polynucleotides encoding the above combinations of peptides.

The composition can comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 44, at least 45, at least 46, at least 47, at least 48, at least 49, at least 50, peptides or polynucleotides selected from those described above or below. At least one of the one or more peptides can be a heteropolymer or a homopolymer. Additionally, the composition can comprise a CTL and/or HTL epitope, which can be derived from a tumor-associated antigen. The additional epitope can also be a PanDR binding molecule, (*e.g.*, a PADRE^{®} universal helper T cell epitope)..

Optional components include excipients, diluents, proteins such as peptides comprising a CTL epitope, and/or an HTL epitope such as a pan-DR binding peptide (*e.g.*, a PADRE^{®} universal helper T cell epitope), and/or a carrier, polynucleotides encoding such proteins, lipids, or liposomes, as well as other components described herein. There are numerous embodiments of compositions in accordance with the invention, such as a cocktail of one or more peptides and/or polynucleotides; one or more peptides and/or analogs and one or more CTL and/or HTL epitopes; and/or nucleic acids that encode such peptides, e.g., minigenes.

Compositions may comprise one or more peptides (or polynucleotides such as minigenes) of the invention, along with one or more other components as described above and herein. "One or more" refers to any whole unit integer from 1-150, *e.g.*, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 , 45, 50, 55, 60, 65, 70, 75, 80, 85, 90, 95, 100, 105, 110, 115, 120, 125, 130, 135, 140, 145, or 150 peptides, polynucleotides, or other components.

Compositions of the invention may be, for example, polynucleotides or polypeptides of the invention combined with or complexed to cationic lipid formulations; lipopeptides (*e.g.*, Vitiello, A. et al., J. Clin. Invest. 95:341, 1995), encapsulated *e.g.*, in poly(DL-lactide-co-glycolide) ("PLG") microspheres (*see, e.g.,* Eldridge, et al., Molec. Immunol. 28:287-294, 1991: Alonso et al., Vaccine 12:299-306, 1994; Jones et al., Vaccine 13:675-681; 1995); peptide compositions contained in immune stimulating complexes (ISCOMS) (*see, e.g.,* Takahashi et al., Nature 344:873-875, 1990; Hu et al., Clin Exp Immunol. 113:235-243, 1998); multiple antigen peptide systems (MAPs) (*see e.g.,* Tam, J. P., Proc. Natl. Acad. Sci. U.S.A. 85:5409-5413, 1988; Tam, J.P., J. Immunol. Methods 196:17-32, 1996); viral, bacterial, or, fungal delivery vectors (Perkus, M. E. et al., In: Concepts in vaccine development, Kaufmann, S. H. E., ed., p. 379, 1996; Chakrabarti, S. et al., Nature 320:535, 1986; Hu, S. L. et al., Nature 320:537, 1986; Kieny, M.-P. et al., AIDS Bio/Technology 4:790, 1986; Top, F. H. et al., J. Infect. Dis. 124:148, 1971; Chanda, P. K. et al., Virology 175:535, 1990); particles of viral or synthetic origin (*e.g.,* Kofler, N. et al., J. Immunol. Methods. 192:25, 1996; Eldridge, J. H. et al., Sem. Hematol. 30:16, 1993; Falo, L. D., Jr. et al., Nature Med. 7:649, 1995); adjuvants (*e.g.,* incomplete Freund's adjuvant) (Warren, H. S., Vogel, F. R., and Chedid, L. A. Annu. Rev. Immunol. 4:369, 1986; Gupta, R. K. et al., Vaccine 11:293, 1993); liposomes (Reddy, R. et al., J. Immunol. 148:1585, 1992; Rock, K. L., Immunol. Today 17:131, 1996); or, particle-absorbed cDNA or other polynucleotides of the invention (Ulmer, J. B. et al., Science 259:1745, 1993; Robinson, H. L., Hunt, L. A., and Webster, R. G., Vaccine 11:957, 1993; Shiver, J. W. et al., In: Concepts in vaccine development, Kaufmann, S. H. E., ed., p. 423, 1996; Cease, K. B., and Berzofsky, J. A., Annu. Rev. Immunol. 12:923, 1994 and Eldridge, J. H. et al., Sem. Hematol. 30:16, 1993), etc. Toxin-targeted delivery technologies, also known as receptor mediated targeting; such as those of Avant Immunotherapeutics, Inc. (Needham, Massachusetts) or attached to a stress protein, e.g., HSP 96 (Stressgen Biotechnologies Corp., Victoria, BC, Canada) can also be used.

Compositions of the invention comprise polynucleotide-mediated modalities. DNA or RNA encoding one or more of the peptides of the invention can be administered to a patient. This approach is described, for instance, in Wolff et. al., Science 247:1465 (1990) as well as U.S. Patent Nos. 5,580,859; 5,589,466; 5,804,566; 5,739,118; 5,736,524; 5,679,647; and, WO 98/04720. Examples of DNA-based delivery technologies include "naked DNA", facilitated (bupivicaine, polymers (*e.g.*, PVP, PINC, etc.), peptide mediated) delivery, cationic lipid complexes, and particle-mediated ("gene gun") or pressure-mediated delivery (*see, e.g.,* U.S. Patent No. 5,922,687). Accordingly, peptides of the invention can be expressed by viral or bacterial vectors. Examples of expression vectors include attenuated viral hosts, such as Modified Vaccinia Ankara (MVA) (*e.g.*, Bavarian Noridic), vaccinia or fowlpox. For example, vaccinia virus is used as a vector to express nucleotide sequences that encode the peptides of the invention. Upon introduction into an acutely or chronically infected host or into a non-infected host, the recombinant vaccinia virus expresses the immunogenic peptide, and thereby elicits an immune response. Vaccinia vectors and methods useful in immunization protocols are described in, *e.g.*, U.S. Patent No. 4,722,848. Another vector is BCG (Bacille Calmette Guerin). BCG vectors are described in Stover et al., Nature 351:456-460 (1991). A wide variety of other vectors useful for therapeutic administration or immunization of the peptides of the invention, *e.g.* adeno and adeno-associated virus vectors, alpha virus vectors, retroviral vectors, *Salmonella typhi* vectors, detoxified anthrax toxin vectors, and the like, are apparent to those skilled in the art from the description herein.

In certain embodiments, components that induce T cell responses are combined with components that induce antibody responses to the target antigen of interest. A preferred embodiment of such a composition comprises class I and class II epitopes in accordance with the invention. Alternatively, a composition comprises a class I and/or class II epitope in accordance with the invention, along with a PADRE^{®} molecule (Epimmune, San Diego, CA).

Compositions of the invention can comprise antigen presenting cells, such as dendritic cells. Antigen presenting cells, *e.g.*, dendritic cells, may be transfected, *e.g.*, with a polynucleotide such as a minigene construct in accordance with the invention, in order to elicit immune responses. The peptide can be bound to an HLA molecule on the antigen-resenting cell, whereby when an HLA-restricted cytotoxic T lymphocyte (CTL) is present, a receptor of the CTL binds to a complex of the HLA molecule and the peptide.

The compositions of the invention may also comprise antiviral drugs such as interferon-α, or immune adjuvants such as IL-12, GM-CSF, etc.

Compositions may comprise an HLA heavy chain, β₂-microglobulin, streptavidin, and/or biotin. The streptavidin may be fluorescently labeled. Compositions may comprise tetramers (*see e.g.,* U.S. Pat. No. 5,635,363; Science 274:94-96 (1996)). A tetramer composition comprising an HLA heavy chain, β₂-microglobulin, streptavidin, and biotin. The streptavidin may be fluorescently labeled. Compositions may also comprise dimers. A dimer composition comprises as MHC molecule and an Ig molecule (*see e.g.,* PNAS 95:7568-73 (1998)).

In some embodiments it may be desirable to include in the compositions of the invention at least one component which primes cytotoxic T lymphocytes. Lipids have been identified as agents capable of priming CTL *in vivo* against viral antigens. For example, palmitic acid residues can be attached to the ε-and α- amino groups of a lysine residue and then linked, *e.g.*, via one or more linking residues such as Gly, Gly-Gly-, Ser, Ser-Ser, or the like, to an immunogenic peptide. The lipidated peptide can then be administered either directly in a micelle or particle, incorporated into a liposome, or emulsified in an adjuvant, *e.g.*, incomplete Freund's adjuvant. A preferred composition comprises palmitic acid attached to ε- and α- amino groups of Lys, which is attached via linkage, *e.g.*, Ser-Ser, to the amino terminus of the peptide.

As another example of lipid priming of CTL responses, *E. coli* lipoproteins, such as tripalmitoyl-S-glycerylcysteinlyseryl-serine (P₃CSS) can be used to prime virus specific CTL when covalently attached to an appropriate peptide (*see, e.g.,* Deres, et al., Nature 342:561, 1989). Peptides of the invention can be coupled to P₃CSS, for example, and the lipopeptide administered to an individual to specifically prime a CTL response to the target antigen. Moreover, because the induction of neutralizing antibodies can also be primed with P₃CSS-conjugated epitopes, two such compositions can be combined to more effectively elicit both humoral and cell-mediated responses.

Another preferred embodiment is a composition comprising one or more peptides of the invention emulsified in IFA.

Compositions of the invention may also comprise CTL and/or HTL peptides. Such CTL and HTL peptides can be modified by the addition of amino acids to the termini of a peptide to provide for ease of linking peptides one to another, for coupling to a carrier support or larger peptide, for modifying the physical or chemical properties of the peptide or oligopeptide, or the like. Amino acids such as tyrosine, cysteine, lysine, glutamic or aspartic acid, or naturally or unnaturally occuring amino acid residues, can be introduced at the carboxyl- or amino-terminus of the peptide or oligopeptide, particularly class I peptides. However, it is to be noted that modification at the carboxyl terminus of a CTL epitope may, in some cases, alter binding characteristics of the peptide. In addition, the peptide or oligopeptide sequences can differ from the natural sequence by being modified by terminal-NH₂ acylation, *e.g.,* by alkanoyl (C₁-C₂₀) or thioglycolyl acetylation, terminal-carboxyl amidation, *e.g.*, ammonia, methylamine, *etc*. In some instances these modifications may provide sites for linking to a support or other molecule. CTL and HTL epitopes may comprise additional amino acids, such as those described above including spacers.

A further embodiment of a composition in accordance with the invention is an antigen presenting cell that comprises one or more peptides in accordance with the invention. The antigen presenting cell can be a "professional" antigen presenting cell, such as a dendritic cell. The antigen presenting cell can comprise the peptide of the invention by any means known or to be determined in the art. Such means include pulsing of dendritic cells with one or more individual peptides, by nucleic acid administration such as ballistic nucleic acid delivery or by other techniques in the art for administration of nucleic acids, including vector-based, *e.g.* viral vector, delivery of nucleic acids.

Compositions may comprise carriers. Carriers that can be used with compositions of the invention are well known in the art, and include, *e.g.*, thyroglobulin, albumins such as human serum albumin, tetanus toxoid, polyamino acids such as poly L-lysine, poly L-glutamic acid, influenza virus proteins, hepatitis B virus core protein, and the like.

The compositions (e.g. pharmaceutical compositions) can contain a physiologically tolerable diluent such as water, or a saline solution, preferably phosphate buffered saline. Additionally, as disclosed herein, CTL responses can be primed by conjugating peptides of the invention to lipids, such as tripalmitoyl-S-glyceryl-eysteinyl-seryl-serine (P₃CSS).

Compositions of the invention may be pharmaceutically acceptable compositions. Pharmaceutical compositions preferably contain an immunologically effective amount of one or more peptides and/or polynucleotides of the invention, and optionally one or more other components which are pharmaceutically acceptable. A preferred composition comprises one or more peptides of the invention and IFA. A more preferred composition of the invention comprises one or more peptides of the invention, one or more peptides, and IFA.

Upon immunization with a peptide and/or polynucleotide and/or composition in accordance with the invention, via injection (*e.g.*, SC, ID, IM), aerosol, oral, transdermal, transmucosal, intrapleural, intrathecal, or other suitable routes, the immune system of the host responds to the vaccine by an immune response comprising the production of antibodies, CTLs and/or HTLs specific for the desired antigen(s). Consequently, the host becomes at least partially immune to subsequent exposure to the TAA(s), or at least partially resistant to further development of TAA-bearing cells and thereby derives a prophylactic or therapeutic benefit.

Furthermore, the peptides, primers, and epitopes of the invention can be used in any desired immunization or administration regimen; e.g., as part of periodic vaccinations such as annual vaccinations as in the veterinary arts or as in periodic vaccinations as in the human medical arts, or as in a prime-boost regime wherein an inventive vector or recombinant is administered either before or after the administration of the same or of a different epitope of interest or recombinant or vector expressing such as a same or different epitope of interest (including an inventive recombinant or vector expressing such as a same or different epitope of interest), see, e.g., U.S. Pat. Nos. 5,997,878; 6,130,066; 6,180,398; 6,267,965; and 6,348,450. An useful viral vector of the present invention is Modified Vaccinia Ankara (MVA) (*e.g.,* Bavarian Noridic (MVA-BN)).

Recent studies have indicated that a prime-boost protocol, whereby immunization with a poxvirus recombinant expressing a foreign gene product is followed by a boost using a purifired subunit preparation form of that gene product, elicits an enhanced immune response relative to the response elicited with either product alone. Human volunteers immunized with a vaccinia recombinant expressing the HIV-1 envelope glycoprotein and boosted with purified HIV-1 envelope glycoprotein subunit preparation exhibit higher HIV-1 neutralizing antibody titers than individuals immunized with just the vaccinia recombinant or purified envelope glycoprotein alone (Graham et al., J. Infect. Dis., 167:533-537 (1993); Cooney et al., Proc. Natl. Acad. Sci. USA, 90:1882-1886 (1993)). Humans immunized with two injections of an ALVAC-HIV-1 env recombinant (vCP125) failed to develop HIV specific antibodies. Boosting with purified rgp160 from a vaccinia virus recombinant resulting in detectable HIV-1 neutralizing antibodies. Furthermore, specific lymphocyte T cell proliferation to rgp160 was clearly increased by the boost with rgp160. Envelope specific cytotoxic lymphocyte activity was also detected with this vaccination regimen (Pialoux et al., AIDS Res. and Hum. Retroviruses, 11:272-381 (1995)). Marcaques immunized with a vaccinia recombinant expressing the simian immunodeficiency virus (SIV) envelope glycoprotein and boosted with SIV envelope glycoprotein from a baculovirus recombinant are protected against SIV challenge (Hu et al., AID Res. and Hum. Retroviruses, 3:615-620 (1991); Hu et al., Science 255:456-459 (1992)). In the same fashion, purified HCMVgB protein can be used in prime-boost protocols with NYVAC or ALVAC-gB recombinants.

In certain embodiments, the polynucleotides are complexed in a liposome preparation. Liposomal preparations for use in the instant invention include cationic (positively charged), anionic (negatively charged) and neutral preparations. However, cationic liposomes are particularly preferred because a tight charge complex can be formed between the cationic liposome and the polyanionic nucleic acid. Cationic liposomes have been shown to mediate intracellular delivery of plasmid DNA (Felgner et al., Proc. Natl. Acad. Sci. USA 84:74137416 (1987), which is herein incorporated by reference); mRNA (Malone et al., Proc. Natl. Acad. Sci. USA 86:60776081 (1989), which is herein incorporated by reference); and purified transcription factors (Debs et al., J. Biol. Chem. 265:1018910192 (1990), which is herein incorporated by reference), in functional form.

Cationic liposomes are readily available. For example, N-[12,3-dioleyloxy)-propyl]-N,N,N-triethylammonium (DOTMA) liposomes are particularly useful and are available under the trademark Lipofectin, from GIBCO BRL, Grand Island, N.Y. (See, also, Felgner et al., Proc. Natl Acad. Sci. USA 84:74137416 (1987)). Other commercially available liposomes include transfectace (DDAB/DOPE) and DOTAP/DOPE (Boehringer).

Other cationic liposomes can be prepared from readily available materials using techniques well known in the art. See, e.g. PCT Publication No. WO 90/11092 for a description of the synthesis of DOTAP (1,2-bis(oleoyloxy)-3-(trimethylmmonio)propane) liposomes. Preparation of DOTMA liposomes is explained in the literature, see, e.g., P. Felgner et al., Proc. Natl. Acad. Sci. USA 84:74137417. Similar methods can be used to prepare liposomes from other cationic lipid materials.

Similarly, anionic and neutral liposomes are readily available, such as from Avanti Polar Lipids (Birmingham, Ala.), or can be easily prepared using readily available materials. Such materials include phosphatidyl, choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

For example, commercially available dioleoylphosphatidyl choline (DOPC), dioleoylphosphatidyl glycerol (DOPG), and dioleoylphosphatidyl ethanolamine (DOPE) can be used in various combinations to make conventional liposomes, with or without the addition of cholesterol. Thus, for example, DOPG/DOPC vesicles can be prepared by drying 50 mg each of DOPG and DOPC under a stream of nitrogen gas into a sonication vial. The sample is placed under a vacuum pump overnight and is hydrated the following day with deionized water. The sample is then sonicated for 2 hours in a capped vial, using a Heat Systems model 350 sonicator equipped with an inverted cup (bath type) probe at the maximum setting while the bath is circulated at 15EC. Alternatively, negatively charged vesicles can be prepared without sonication to produce multilamellar vesicles or by extrusion through nucleopore membranes to produce unilamellar vesicles of discrete size. Other methods are known and available to those of skill in the art.

The liposomes can comprise multilamellar vesicles (MLVs), small unilamellar vesicles (SWs), or large unilamellar vesicles (LUVs), with SUVs being preferred. The various liposome nucleic acid complexes are prepared using methods well known in the art. See, e.g., Straubinger et al., Methods of Immunology 101:512527 (1983). For example, MLVs containing nucleic acid can be prepared by depositing a thin film of phospholipid on the walls of a glass tube and subsequently hydrating with a solution of the material to be encapsulated. SUVs are prepared by extended sonication of MLVs to produce a homogeneous population of unilamellar liposomes. The material to be entrapped is added to a suspension of preformed MLVs and then sonicated. When using liposomes containing cationic lipids, the dried lipid film is resuspended in an appropriate solution such as sterile water or an isotonic buffer solution such as 10 mM Tris/NaCl, sonicated, and then the preformed liposomes are mixed directly with the DNA. The liposome and DNA form a very stable complex due to binding of the positively charged liposomes to the cationic DNA. SUVs find use with small nucleic acid fragments. LUVs are prepared by a number of methods, well known in the art. Commonly used methods include Ca²⁺-EDTA chelation (Papahadjopoulos et al., Biochim. Biophys. Acta 394:483 (1975); Wilson et al., Cell 17:77 (1979)); ether injection (Deamer, D. and Bangham, A., Biochim. Biophys. Acta 443:629 (1976); Ostro et al., Biochem. Biophys. Res. Commun. 76:836 (1977); Fraley et al., Proc. Natl. Acad. Sci. USA 76:3348 (1979)); detergent dialysis (Enoch, H. and Strittmatter, P., Proc. Natl. Acad. Sci. USA 76:145 (1979)); and reversephase evaporation (REV) (Fraley et al., J. Biol. Chem. 255:10431 (1980); Szoka, F. and Papahadjopoulos, D., Proc. Natl. Acad. Sci. USA 75:145 (1978); SchaeferRidderera et al., Science 215:166 (1982)).

Generally, the ratio of DNA to liposomes will be from about 10:1 to about 1:10. Preferably, the ration will be from about 5:1 to about 1:5. More preferably, the ration will be about 3:1 to about 1:3. Still more preferably, the ratio will be about 1:1.

U.S. Patent No. 5,676,954 reports on the injection of genetic material, complexed with cationic liposomes carriers, into mice. U.S. Patent Nos. 4,897,355, 4,946,787, 5,049,386, 5,459,127, 5,589,466, 5,693,622, 5,580,859, 5,703,055, and international publication no. WO 94/9469 provide cationic lipids for use in transfecting DNA into cells and mammals. U.S. Patent Nos. 5,589,466, 5,693,622, 5,580,859, 5,703,055, and international publication no. WO 94/9469 provide methods for delivering DNA-cationic lipid complexes to mammals.

### Binding Affinity of Epitopes and Analogs for HLA Molecules

As indicated herein, the large degree of HLA polymorphism is an important factor to be taken into account with the epitope-based approach to developing therapeutics and diagnostics. To address this factor, epitope selection encompassing identification of peptides capable of binding at high or intermediate affinity to multiple HLA molecules is preferably utilized, most preferably these epitopes bind at high or intermediate affinity to two or more allele-specific HLA molecules. However, in some embodiments, it is preferred that all epitopes in a given composition bind to the alleles of a single HLA supertype or a single HLA molecule.

Epitopes and analogs of the invention preferably include those that have an IC₅₀ or binding affinity value for a class I HLA molecule(s) of 500 nM or better (*i.e.*, the value is ≤ 500 nM). In certain embodiments of the invention, peptides of interest have an IC₅₀ or binding affinity value for a class I HLA molecule(s) of 200 nM or better. In certain embodiments of the invention, peptides of interest, such as A1 and A24 peptides, have an IC₅₀ or binding affinity value for a class I HLA molecule(s) of 100 nM or better. If HTL epitopes are included, they preferably are HTL epitopes that have an IC₅₀ or binding affinity value for class II HLA molecules of 1000 nM or better, (*i.e.,* the value is ≤ 1,000 nM). For example, peptide binding is assessed by testing the capacity of a candidate peptide to bind to a purified HLA molecule *in vitro.* Peptides exhibiting high or intermediate affinity are then considered for further analysis. Selected peptides are generally tested on other members of the supertype family. In preferred embodiments, peptides that exhibit cross-reactive binding are then used in cellular screening analyses or vaccines.

The relationship between binding affinity for HLA class I molecules and immunogenicity of discrete peptide epitopes on bound antigens was determined for the first time by inventors at Epimmune. As disclosed in greater detail herein, higher HLA binding affinity is correlated with greater immunogenicity.

Greater immunogenicity can be manifested in several different ways. Immunogenicity corresponds to whether an immune response is elicited at all, and to the vigor of any particular response, as well as to the extent of a population in which a response is elicited. For example, a peptide might elicit an immune response in a diverse array of the population, yet in no instance produce a vigorous response. In accordance with these principles, close to 90% of high binding peptides have been found to elicit a response and thus be "immunogenic," as contrasted with about 50% of the peptides that bind with intermediate affinity. (See, e.g., Schaeffer et al. PNAS (1988)) High affinity-binding class I peptides generally have an affinity of less than or equal to 100 nM. Moreover, not only did peptides with higher binding affinity have an enhanced probability of generating an immune response, the generated response tended to be more vigorous than the response seen with weaker binding peptides. As a result, less peptide is required to elicit a similar biological effect if a high affinity binding peptide is used rather than a lower affinity one. Thus, in some preferred embodiments of the invention, high affinity binding epitopes are used.

The correlation between binding affinity and immunogenicity was analyzed by the present inventors by two different experimental approaches (*see, e.g.,* Sette, et al., J. Immunol. 153:5586-5592 (1994)). In the first approach, the immunogenicity of potential epitopes ranging in HLA binding affinity over a 10,000-fold range was analyzed in HLA-A*0201 transgenic mice. In the second approach, the antigenicity of approximately 100 different hepatitis B virus (HBV)-derived potential epitopes, all carrying A*0201 binding motifs, was assessed by using PBL from acute hepatitis patients. Pursuant to these approaches, it was determined that an affinity threshold value of approximately 500 nM (preferably 50 nM or less) determines the capacity of a peptide epitope to elicit a CTL response. These data are true for class I binding affinity measurements for naturally processed peptides and for synthesized T cell epitopes. These data also indicate the important role of determinant selection in the shaping of T cell responses (*see, e.g.,* Schaeffer et al. Proc. Natl. Acad. Sci. USA 86:4649-4653 (1989)).

An affinity threshold associated with immunogenicity in the context of HLA class II (*i.e.,* HLA DR) molecules has also been delineated (*see, e.g.,* Southwood et al. J. Immunology 160:3363-3373 (1998), and U.S. Patent No. 6,413,527, issued July 2, 2002). In order to define a biologically significant threshold of HLA class II binding affinity, a database of the binding affinities of 32 DR-restricted epitopes for their restricting element (*i.e.*, the HLA molecule that binds the epitope) was compiled. In approximately half of the cases (15 of 32 epitopes), DR restriction was associated with high binding affinities, *i.e.* binding affinity values of 100 nM or less. In the other half of the cases (16 of 32), DR restriction was associated with intermediate affinity (binding affinity values in the 100-1000 nM range). In only one of 32 cases was DR restriction associated with an IC₅₀ of 1000 nM or greater. Thus, 1000 nM is defined as an affinity threshold associated with immunogenicity in the context of DR molecules.

The binding affinity of peptides for HLA molecules can be determined as described in Example 3, below.

### Epitope Binding Motifs and Supermotifs

Through the study of single amino acid substituted antigen analogs and the sequencing of endogenously bound, naturally processed peptides, critical residues required for allele-specific binding to HLA molecules have been identified.- The presence of these residues in a peptide correlates with both the probability of binding and with binding affinity for HLA molecules.

The identification of motifs and/or supermotifs that correlate with high and intermediate affinity binding is important when identifying immunogenic peptide epitopes for the inclusion in a vaccine. Kast et al. (J. Immunol. 152:3904-3912 (1994)) have shown that motif-bearing peptides account for 90% of the epitopes that bind to allele-specific HLA class I molecules. In the Kast study, all possible 9 amino acid long peptides, each overlapping by eight amino acids, which cover the entire sequence of the E6 and E7 proteins of human papillomavirus type 16 were generated, which produced 240 peptides. All 240 peptides were evaluated for binding to five allele-specific HLA molecules that are expressed at high frequency among different ethnic groups. This unbiased set of peptides allowed an evaluation of the predictive values of HLA class I motifs. From the set of 240 peptides, 22 peptides were identified that bound to an allele-specific HLA molecule with high or intermediate affinity. Of these 22 peptides, 20 (*i.e.* 91%) were motif-bearing. Thus, this study demonstrated the value of motifs for identification of peptide epitopes to be included in a vaccine.

Accordingly, the use of motif-based identification techniques identifies approximately 90% of all potential epitopes in a target protein sequence. Without the disclosed motif analysis, the ability to practically identify immunogenic peptide(s) for use in diagnostics or therapeutics is seriously impaired.

Peptides, pharmaceutical compositions and vaccines of the present invention may also comprise epitopes that bind to MHC class II DR molecules. A greater degree of heterogeneity in both size and binding frame position of the motif, relative to the N- and C- termini of the peptide, exists for class II peptide ligands. This increased heterogeneity of HLA class II peptide ligands is due to the structure of the binding groove of the HLA class II molecule which, unlike its class I counterpart, is less physically constricted at both ends. Crystallographic analysis of HLA class II DRB*0101-peptide complexes to identify the residues associated with major binding energy identified those residues complexed with complementary pockets- on the DRBI*0101 molecules. An important anchor residue engages the deepest hydrophobic pocket (*see, e.g.,* Madden, D.R. Ann. Rev. Immunol. 13:587 (1995)) and is referred to as position 1 (P1). P1 may represent the N-terminal residue of a class II binding peptide epitope, but more typically is flanked towards the N-terminus by one or more residues. Other studies have also pointed to an important role for the peptide residue in the sixth position towards the C-terminus, relative to P1, for binding to various DR molecules. See, *e.g.*, U.S. Patent 5,736,142, and co-pending applications entitled Alteration Of Immune Responses Using Pan DR Binding Peptides, U.S.S.N. 09/709,774, filed November 8, 2000 and 09/707,738, filed November 6, 2000.

Thus; a large fraction of HLA class I and class II molecules can be classified into a relatively few supertypes, each respective supertype characterized by largely overlapping peptide binding repertoires, and consensus structures of the main peptide binding pockets. Thus, peptides of the present invention are preferably identified by any one of several HLA-specific amino acid motifs (*see, e.g.,* Tables 2-4), or if the presence of the motif corresponds to the ability to bind several allele-specific HLA antigens, a supermotif (*see, e.g.,* Tables 14-16d).

The primary anchor residues of the HLA class I peptide epitope supermotifs and motifs are summarized in Tables 2 and 14. The HLA class I motifs set out in Tables 2, 2a and 14 are particularly relevant to the invention claimed here. Primary and secondary anchor positions for HLA Class I are summarized in Table 3. Allele-specific HLA molecules that are comprised by the various HLA class I supertypes are listed in Table 5. In some cases, patterns of amino acid residues are present in both a motif and a supermotif. The relationship of a particular motif and any related supermotif is indicated in the description of the individual motifs.

Thus, the peptide motifs and supermotifs described below, and summarized in Tables 2-4 and 14, provide guidance for the identification and use of peptide epitopes in accordance with the invention.

### HLA-A1 supermotif

The HLA-A1 supermotif is characterized by the presence in peptide ligands of a small (T or S) or hydrophobic (L, I, V, or M) primary anchor residue in position 2, and an aromatic (Y, F, or W) primary anchor residue at the C-terminal position of the epitope. The corresponding family of HLA molecules that bind to the A1 supermotif (*i.e.,* the HLA-A1 supertype) is comprised of at least A*0101, A*2601, A*2602, A*2501, A*2902 and A*3201 (Sette, et al. Immunogenetics 50:201 (1999)). Other allele-specific HLA molecules predicted to be members of the A1 superfamily are shown in Table 5.

### HLA-A1 motif

The HLA-A1 motif is characterized by the presence in peptide ligands of T, S, or M as a primary anchor residue at position 2 and the presence of Y as a primary anchor residue at the C-terminal position of the epitope. An alternative allele-specific A1 motif is characterized by a primary anchor residue at position 3 rather than position 2. This motif is characterized by the presence of D, E, A, or S as a primary anchor residue in position 3, and a Y as a primary anchor residue at the C-terminal position of the epitope (see, e.g., DiBrino et al., J. Imnnunol., 152:620, 1994; Kondo et al., Immunogenetics 45:249, 1997; and Kubo et al., J. Immunol. 152:3913, 1994 for reviews of relevant data).

### HLA-A3 supermotif

The HLA-A3 supermotif is characterized by the presence in peptide ligands of A, L, I, V, M, S, or, T as a primary anchor at position 2, and a positively charged residue, R or K, at the C-terminal position of the epitope, *e.g.*, in position 9 of 9-mers (*see, e.g.,* Sidney et al., Hum. Immunol. 45:79, 1996). Exemplary members of the corresponding family of HLA molecules (the HLA-A3 supertype) that bind the A3 supermotif include at least A*0301, A*1101, A*3101, A*3301, and A*6801. Other allele-specific HLA molecules predicted to be members of the A3 supertype are shown in Table 5.

### HLA-A3 motif

The HLA-A3 motif is characterized by the presence in peptide ligands of L, M, V, I, S, A, T, F, C, G, or D as a primary anchor residue at position 2, and the presence of K, Y, R, H, F, or A as a primary anchor residue at the C-terminal position of the epitope (*see, e.g.,* DiBrino et al., Proc. Natl. Acad. Sci USA 90:1508, 1993; and Kubo et al., J. Immunol. 152:3913-3924, 1994).

### HLA-A24 supermotif

The HLA-A24 supermotif is characterized by the presence in peptide ligands of an aromatic (F, W, or Y) or hydrophobic aliphatic (L, I, V, M, or T) residue as a primary anchor in position 2, and Y, F, W, L, I, or M as primary anchor at the C-terminal position of the epitope (*see, e.g.,* Sette and Sidney, *Immunogenetics, 50*:201-212,1999). The corresponding family of HLA. molecules that bind to the A24 supermotif (*i.e.*, the A24 supertype) includes at least A*2402, A*3001, A*2301 and A*3002. Other allele-specific HLA molecules predicted to be members of the A24 supertype are shown in Table 5.

### HLA-B7 supermotif

The HLA-B7 supermotif is characterized by peptides bearing proline in position 2 as a primary anchor, and a hydrophobic or aliphatic amino acid (L, I, V, M, A, F, W, or Y) as the primary anchor at the C-terminal position of the epitope. The corresponding family of HLA molecules that bind the B7 supermotif (*i.e.*, the HLA-B7 supertype) is comprised of at least twenty six HLA-B proteins including: B*0702, B*0703, B*0704, B*0705, B*1508, B*3501, B*3502, B*3503, B*3504, B*3505, B*3506, B*3507, B*3508, B*5101, B*5102, B*5103, B*5104, B*5105, B*5301, B*5401, B*5501, B*5502, B*5601, B*5602, B*6701, and B*7801 (*see*, *e.g.*, Sidney, et al., J. Immunol. 154:247, 1995; Barber, et al., Curr. Biol. 5:179, 1995; Hill, et al., Nature 360:434, 1992; Rammensee, et al., Immunogenetics 41:178, 1995 for reviews of relevant data). Other allele-specific HLA molecules predicted to be members of the B7 supertype are shown in Table 5.

### HLA-B44 supermotif

The HLA-B44 supermotif is characterized by the presence in peptide ligands of negatively charged (D or E) residues as a primary anchor in position 2, and a hydrophobic residues (F, W, Y, I, M, T, L, A, or V) as the primary anchor of the C-terminal position of the epitope (see, e.g., Sidney et al. Immunol. Today 1 7:261, 1996). Exemplary members of the corresponding family of HLA molecules that bind to the B44 supermotif (i.e., the HLA-B44 supertype) includes at least: B*1801, B*4001(B60), B*4002 (B61), B*4402, B*4403, and B*4501. Other allele-specific HLA molecules predicted to be members of the B44 supertype are shown in Table 5.

### HLA-A2 supermotif

Primary anchor specificities for allele-specific HLA-A2.1 molecules (*see, e.g.,* Falk et al., Nature 351:290-296 (1991); Hunt et al., Science 255:1261-1263 (1992); Parker et al., J. Immunol. 149:3580-3587 (1992); Ruppert et al., Cell 74:929-937 (1993)) and cross-reactive binding among HLA-A2 and -A28 molecules have been described. (*See, e.g.,* Fruci et al., Human Immunol. 38:187-192 (1993); Tanigaki et al., Human Immunol. 39:155-162 (1994); del Guercio et al., J. Immunol. 154:685-693 (1995); Kast et al., J. Immunol. 152:3904-3912 (1994) for reviews of relevant data.) These primary anchor residues define the HLA-A2 supermotif; which when present in peptide ligands corresponds to the ability to bind several different HLA-A2 and -A28 molecules. The HLA-A2 supermotif comprises peptide ligands with L, I, V, M, A, T, or Q as a primary anchor residue at position 2 and L, I, V, M, A, or T as a primary anchor residue at the C-terminal position of the epitope.

The corresponding family of HLA molecules (*i.e.,* the HLA-A2 supertype that binds these peptides) is comprised of at least: A*0201, A*0202, A*0203, A*0204, A*0205, A*0206, A*0207, A*0209, A*0214, A*6802, and A*6901. Other allele-specific HLA molecules predicted to be members of the A2 superfamily are shown in Table 5. As explained in detail below, binding to each of the individual allele-specific HLA molecules can be modulated by substitutions at the primary anchor and/or secondary anchor positions, preferably choosing respective residues specified for the supermotif.

### HLA-A*0201 motif

An HLA-A2*0201 motif was determined to be characterized by the presence in peptide ligands of L or M as a primary anchor residue in position 2, and L or V as a primary anchor residue at the C-terminal position of a 9-residue peptide (*see, e.g.,* Falk et al., Nature 351:290-296 (1991)) and was further found to comprise an I at position 2 and I or A at the C-terminal position of a nine amino acid peptide (*see, e.g.,* Hunt et al., Science 255:1261-1263, March 6, 1992; Parker et al., J. Immunol. 149:3580-3587 (1992)) and position 10 of a decamer peptide. The A*0201 allele-specific motif has also been defined by the present inventors to additionally comprise V, A, T, or Q as a primary anchor residue at position 2, and M or T as a primary anchor residue at the C-terminal position of the epitope (*see, e.g.,* Kast et al., J. Immunol. 152:3904-3912, 1994).

Thus, the HLA-A*0201 motif comprises peptide ligands with L, I, V, M, A, T, or Q as primary anchor residues at position 2 and L, I, V, M, A, or T as a primary anchor residue at the C-terminal position of the epitope. For this motif-supermotif relationship the preferred and less preferred/tolerated residues that characterize the primary anchor positions of the HLA-A*0201 motif are identical to the residues describing the A2 supermotif. (For reviews of relevant data, *see, e.g.,* del Guercio et al., J. Immunol. 154:685-693, 1995; Ruppert et al., Cell 74:929-937, 1993; Sidney et al., Immunol. Today 17:261-266, 1996; Sette and Sidney, Curr. Opin. in Immunol. 10:478-482, 1998). Secondary anchor residues that characterize the A*0201 motif have additionally been defined (*see, e.g.,* Ruppert et al., Cell 74:929-937, 1993). These secondary anchors are shown in Table 3. Peptide binding to HLA-A*0201 molecules can be modulated by substitutions at primary and/or secondary anchor positions, preferably choosing respective residues specified for the motif.

### HLA-A24 motif

The HLA-A24 motif is characterized by the presence in peptide ligands of Y, F, W, or M as a primary anchor residue in position 2, and F, L, I, or W as a primary anchor residue at the C-terminal position of the epitope (*see, e.g.,* Kondo et al., J. Immunol. 155:4307-4312, 1995; and Kubo et al., J. Immunol. 152:3913-3924, 1994).

### Motifs Indicative of Class II HTL Inducing Peptide Epitopes

The primary and secondary anchor residues of the HLA class II peptide epitope supermotifs and motifs are summarized in Table 4. Also see, U.S. Patent 5,736,142, 5,679,640 and 6,413,935; co-pending applications entitled Alteration Of Immune Responses Using Pan DR Binding Peptides, U.S.S.N. 09/709,774, filed November 8, 2000 and 09/707,738, filed November 6, 2000; and PCT publication Nos. WO 95/07707 and WO 97/26784.

### Enhancing Population Coverage of the Vaccine

As set forth in Tables 2 through 4, there are neumerous additional superinotifs and motifs in addition to the A2 supermotif and the A2.1-allel specific motif that presently are a focus of the present application. By inclusion of one or more epitopes from other motifs or supermotifs, enhanced population coverage for major global ethnicities can be obtained.

### Immune Response-Stimulating Peptide Analogs

In general, CTL and HTL responses are not directed against all possible epitopes. Rather, they are restricted to a few "immunodominant" determinants (Zinkernagel, et al., Adv. Immunol. 27:5159, 1979; Bennink, et al., J. Exp. Med. 168:19351939, 1988; Rawle, et al., J. Immunol. 146:3977-3984, 1991). It has been recognized that immunodominance (Benacerraf, et al., Science 175:273-279, 1972) could be explained by either the ability of a given epitope to selectively bind a particular HLA protein (determinant selection theory) (Vitiello, et al., J. Immunol. 131:1635, 1983); Rosenthal, et al., Nature 267:156-158, 1977), or to be selectively recognized by the existing TCR (T cell receptor) specificities (repertoire theory) (Klein, J., IMMUNOLOGY, THE SCIENCE OF SELFNONSELF DISCRIMINATION, John Wiley & Sons, New York, pp. 270-310, 1982). It has been demonstrated that additional factors, mostly linked to processing events, can also play a key role in dictating, beyond strict immunogenicity, which of the many potential determinants will be presented as immunodominant (Sercarz, et al., Annu. Rev. Immunol. 11:729-766, 1993).

The concept of dominance and subdominance is relevant to immunotherapy of both infectious diseases and malignancies. For example, in the course of chronic viral disease, recruitment of subdominant epitopes can be important for successful clearance of the infection, especially if dominant CTL or HTL specificities have been inactivated by functional tolerance, suppression, mutation of viruses and other mechanisms (Franco, et al., Curr. Opin. Immunol. 7:524-531, 1995). In the case of cancer and tumor antigens, CTLs recognizing at least some of the highest binding affinity peptides might be functionally inactivated. Lower binding affinity peptides are preferentially recognized at these times, and may therefore be preferred in therapeutic or prophylactic anti-cancer vaccines.

In particular, it has been noted that a significant number of epitopes derived from known non-viral tumor associated antigens (TAA) bind HLA class I with intermediate affinity (IC₅₀ in the 50-500 nM range) rather than at high affinity (IC₅₀ of less than 50 nM).

For example, it has been found that 8 of 15 known TAA peptides recognized by tumor infiltrating lymphocytes (TIL) or CTL bound in the 50-500 nM range. (These data are in contrast with estimates that 90% of known viral antigens were bound by HLA class I molecules with IC₅₀ of 50 nM or less, while only approximately 10% bound in the 50-500 nM range (Sette, et al., J. Immunol., 153:558-5592, 1994). In the cancer setting this phenomenon is probably due to elimination or functional inhibition of the CTL recognizing several of the highest binding peptides, presumably because of T cell tolerization events.

Without intending to be bound by theory, it is believed that because T cells to dominant epitopes may have been clonally deleted, and selecting subdominant epitopes may allow existing T cells to be recruited, which will then lead to a therapeutic or prophylactic response. However, the binding of HLA molecules to subdominant epitopes is often less vigorous than to dominant ones.

Accordingly, there is a need to be able to modulate the binding affinity of particular immunogenic epitopes for one or more HLA molecules, to thereby modulate the immune response elicited by the peptide, for example to prepare analog peptides which elicit a more vigorous response. This ability to modulate both binding affinity and the resulting immune response in accordance with the present invention greatly enhances the usefulness of peptide epitope-based vaccines and therapeutic agents.

Although peptides with suitable cross-reactivity among all alleles of a superfamily are identified by the screening procedures described above, cross-reactivity is not always as complete as possible, and in certain cases procedures to increase cross-reactivity of peptides can be useful; moreover, such procedures can also be used to modify other properties of the peptides such as binding affinity or peptide stability. Having established the general rules that govern cross-reactivity of peptides for HLA alleles within a given motif or supermotif, modification-(i.e., analoging) of the structure of peptides of particular interest in order to achieve broader (or otherwise modified) HLA binding capacity can be performed. More specifically, peptides that exhibit the broadest cross-reactivity patterns, can be produced in accordance with the teachings herein. The present concepts related to analog generation are set forth in greater detail in co-pending U.S.S.N. 09/226,775 filed 6 January 1999.

In brief, the analoging strategy utilizes the motifs or supermotifs that correlate with binding to certain HLA molecules. Analog peptides can be created by substituting amino acid residues at primary anchor, secondary anchor, or at primary and secondary anchor positions. Generally, analogs are made for peptides that already bear a motif or supermotif. As noted herein, preferred primary and secondary anchor residues of supermotifs and motifs for HLA class I and HLA class II binding peptides are shown in Tables 3 and 4, respectively. For a number of the motifs or supermotifs in accordance with the invention, residues are defined which are deleterious to binding to allele-specific HLA molecules or members of HLA supertypes that bind the respective motif or supermotif (Tables 3 and 4). Accordingly, removal of such residues that are detrimental to binding can be performed in accordance with the present invention. For example, in the case of the A3 supertype, when all peptides that have such deleterious residues are removed from the population of peptides used in the analysis, the incidence of cross-reactivity increased from 22% to 37% (*see, e.g.,* Sidney, J. et al., Hu. Immunol. 45:79, 1996).

Thus, one strategy to improve the cross-reactivity of peptides within a given supermotif is simply to delete one or more of the deleterious residues present within a peptide and substitute a small "neutral" residue such as Ala (that may not influence T cell recognition of the peptide). An enhanced likelihood of cross-reactivity is expected if, together with elimination of detrimental residues within a peptide, "preferred" residues associated with high affinity binding to an allele-specific HLA molecule or to multiple HLA molecules within a superfamily are inserted.

To ensure that an analog peptide, when used as a vaccine, actually elicits a CTL response to the native epitope *in vivo* (or, in the case of class II epitopes, elicits helper T cells that cross-react with the wild type peptides), the analog peptide may be used to induce T cells *in vitro* from individuals of the appropriate HLA allele. Thereafter, the immunized cells' capacity to lyse wild type peptide sensitized target cells is evaluated. Alternatively, evaluation of the cells' activity can be evaluated by monitoring IFN release. Each of these cell monitoring strategies evaluate the recognition of the APC by the CTL. It will be desirable to use as antigen presenting cells, cells that have been either infected, or transfected with the appropriate genes, or, (generally only for class II epitopes, due to the different peptide processing pathway for HLA class II), cells that have been pulsed with whole protein antigens, to establish whether endogenously produced antigen is also recognized by the T cells induced by the analog peptide. It is to be noted that peptide/protein-pulsed dendritic cells can be used to present whole protein antigens for both HLA class I and class II.

Another embodiment of the invention is to create analogs of weak binding peptides, to thereby ensure adequate numbers of cellular binders. Class I binding peptides exhibiting binding affinities of 500-5000 nM, and carrying an acceptable but suboptimal primary anchor residue at one or both positions can be "fixed" by substituting preferred anchor residues in accordance with the respective supertype. The analog peptides can then be tested for binding and/or cross-binding capacity.

Another embodiment of the invention is to create analogs of peptides that are already cross-reactive binders and are vaccine candidates, but which bind weakly to one or more alleles of a supertype. If the cross-reactive binder carries a suboptimal residue (less preferred or deleterious) at a primary or secondary anchor position, the peptide can be analoged by substituting out a deleterious residue and replacing it with a preferred or less preferred one, or by substituting out a less preferred reside and replacing it with a preferred one. The analog peptide can then be tested for cross-binding capacity.

Another embodiment for generating effective peptide analogs involves the substitution of residues that have an adverse impact on peptide stability or solubility in, *e.g.,* a liquid environment. This substitution may occur at any position of the peptide epitope. For example, a cysteine (C) can be substituted in favor of α-amino butyric acid. Due to its chemical nature, cysteine has the propensity to form disulfide bridges and sufficiently alter the peptide structurally so as to reduce binding capacity. Substituting α-amino butyric acid for C not only alleviates this problem, but actually improves binding and crossbinding capability in certain instances (*see, e.g.,* the review by Sette et al., In: Persistent Viral Infections, Eds. R. Ahmed and I. Chen, John Wiley & Sons, England, 1999). Substitution of cysteine with α-amino butyric acid may occur at any residue of a peptide epitope, *i.e.* at either anchor or non-anchor positions.

Moreover, it has been shown that in sets of A*0201 motif-bearing peptides containing at least one preferred secondary anchor residue while avoiding the presence of any deleterious secondary anchor residues, 69% of the peptides will bind A*0201 with an IC₅₀ less than 500 nM (Ruppert, J. et al. Cell 74:929, 1993). The determination of what was a preferred or deleterious residue in Ruppert can be used to generate algorithms. Such algorithms are flexible in that cut-off scores may be adjusted to select sets of peptides with greater or lower predicted binding properties, as desired.

In accordance with the procedures described herein, tumor associated antigen peptide epitopes and analogs thereof that were found to bind HLA-A1, -A2, -A3, -A11, -A24, -B7 and -B44 allele-specific molecules and to members of the HLA-A2, -A11, -B7 and -B44 supertypes have been identified.

Furthermore, additional amino acids can be added to the termini of a peptide to provide for ease of linking peptides one to another, for coupling to a carrier support or larger peptide, for modifying the physical or chemical properties of the peptide or oligopeptide, or the like. Amino acids such as tyrosine, cysteine, lysine, glutamic or aspartic acid, or any naturally occuring or any non-naturally occuring amino acid residues, can be introduced at the C-and/or N-terminus of the peptide or oligopeptide, particularly class I peptides. It is to be noted that modification at the carboxyl terminus of a CTL epitope may, in some cases, alter binding characteristics of the peptide. In addition, the peptide or oligopeptide sequences can differ from the natural sequence by being modified by terminal-NH₂ acylation, *e.g.*, by alkanoyl (C₁-C₂₀) or thioglycolyl acetylation, terminal-carboxyl amidatibn, *e.g.*, ammonia, methylamine, *etc.* In some instances these modifications may provide sites for linking to a support or other molecule.

### Assays to Detect T-Cell Responses

Once HLA binding peptides are identified, they can be tested for the ability to elicit a T-cell response. The preparation and evaluation of motif-bearing peptides are described, *e.g.*, in PCT publications WO 94/20127 and WO 94/03205. Briefly, peptides comprising epitopes from a particular antigen are synthesized and tested for their ability to bind to relevant HLA proteins. These assays may involve evaluation of peptide binding to purified HLA class I molecules in relation to the binding of a radioiodinated reference peptide. Alternatively, cells expressing empty class I molecules (*i.e.* cell surface HLA molecules that lack any bound peptide) may be evaluated for peptide binding by immunofluorescent staining and flow microfluorimetry. Other assays that may be used to evaluate peptide binding include peptide-dependent class I assembly assays and/or the inhibition of CTL recognition by peptide competition. Those peptides that bind to an HLA class I molecule, typically with an affinity of 500 nM or less, are further evaluated for their ability to serve as targets for CTLs derived from infected or immunized individuals, as well as for their capacity to induce primary *in vitro* or *in vivo* CTL responses that can give rise to CTL populations capable of reacting with selected target cells associated with pathology.

Analogous assays are used for evaluation of HLA class II binding peptides. HLA class II motif-bearing peptides that are shown to bind, typically at an affinity of 1000 nM or less, are further evaluated for the ability to stimulate HTL responses.

Conventional assays utilized to detect T cell responses include proliferation assays, lymphokine secretion assays, direct cytotoxicity assays, and limiting dilution assays. For example, antigen-presenting cells that have been incubated with a peptide can be assayed for the ability to induce CTL responses in responder cell populations. Antigen-presenting cells can be normal cells such as peripheral blood mononuclear cells or dendritic cells. Alternatively, mutant, non-human mammalian cell lines that have been transfected with a human class I MHC gene, and that are deficient in their ability to load class I molecules with internally processed peptides, are used to evaluate the capacity of the peptide to induce *in vitro* primary CTL responses. Peripheral blood mononuclear cells (PBMCs) can be used as the source of CTL precursors. Antigen presenting cells are incubated with peptide, after which the peptide-loaded antigen-presenting cells are then incubated with the responder cell population under optimized culture conditions. Positive CTL activation can be determined by assaying the culture for the presence of CTLs that lyse radio-labeled target cells, either specific peptide-pulsed targets or target cells that express endogenously processed antigen from which the specific peptide was derived. Alternatively, the presence of epitope-specific CTLs can be determined by IFNγ *in situ* ELISA.

In an embodiment of the invention, directed to diagnostics, a method has been devised which allows direct quantification of antigen-specific T cells by staining with fluorescein-labelled HLA tetrameric complexes (Altman, J. D. et al., Proc. Natl. Acad. Sci. USA 90:10330, 1993; Altman, J. D. et al., Science 274:94, 1996). Other options include staining for intracellular lymphokines, and interferon release assays or ELISPOT assays. Tetramer staining, intracellular lymphokine staining and ELISPOT assays all appear to be at least 10-fold more sensitive than more conventional assays (Lalvani, A. et al., J. Exp. Med. 186:859, 1997; Dunbar, P. R. et al., Curr. Biol. 8:413, 1998; Murali-Krishna, K. et al., Immunity 8:177, 1998). Additionally, DimerX technology can be used as a means of quantitation (*see, e.g.,* Science 274:94-99 (1996) and Proc. Natl. Acad. Sci. 95:7568-73 (1998)).

HTL activation may also be assessed using techniques known to those in the art, such as T cell proliferation or lymphokine secretion (*see, e.g.* Alexander et al., Immunity 1:751-761, 1994).

Alternatively, immunization of HLA transgenic mice can be used to determine immunogenicity of peptide epitopes. Several transgenic mouse strains, *e.g.*, mice with human A2.1, A11 (which can additionally be used to analyze HLA-A3 epitopes), and B7 alleles have been characterized. Other transgenic mice strains (*e.g.*, transgenic mice for HLA-A1 and A24) are being developed. Moreover, HLA-DR1 and-HLA-DR3 mouse models have been developed. In accordance with principles in the art, additional transgenic mouse models with other HLA alleles are generated as necessary.

Such mice can be immunized with peptides emulsified in Incomplete Freund's Adjuvant; thereafter any resulting T cells can be tested for their capacity to recognize target cells that have been peptide-pulsed or transfected with genes encoding the peptide of interest. CTL responses can be analyzed using cytotoxicity assays described above. Similarly, HTL responses can be analyzed using, *e.g.*, T cell proliferation or lymphokine secretion assays.

### Enhancing Population Coverage of the Vaccine

As set forth in Tables 2 through 4, there are numerous additional supermotifs and motifs in addition to the A3, B7 and B44 supermotifs and motifs and A1, A2, A24 and B44 motifs that presently are a focus of the present application. By inclusion of one or more epitopes from other motifs or supermotifs, enhanced population coverage for major global ethnicities can be obtained. (See Tables 11, 13a, 13b, and 32).

### Minigenes

A number of different approaches are available which allow simultaneous delivery of multiple epitopes. Nucleic acids encoding multiple epitopes are a useful embodiment of the invention; discrete peptide epitopes or polyepitopic peptides can be encoded. The epitopes to be included in a minigene are preferably selected according to the guidelines set forth in the previous section. Examples of amino acid sequences that can be included in a minigene include: HLA class I epitopes, HLA class II epitopes, a ubiquitination signal sequence, and/or a targeting sequence such as an endoplasmic reticulum (ER) signal sequence to facilitate movement of the resulting peptide into the endoplasmic reticulum.

The use of multi-epitope minigenes is also described in, *e.g.*, co-pending applications U.S.S.N. 09/311,784, 09/894,018, 60/419,973, 60/415,463; Ishioka et al., J. Immunol. 162:3915-3925, 1999; An, L. and Whitton, J. L., J. Virol. 71:2292, 1997; Thomson, S. A. et al., J. Immunol. 157:822, 1996; Whitton, J. L. et al., J. Virol. 67:348, 1993; Hanke, R. et al., Vaccine 16:426, 1998. For example, a multi-epitope DNA plasmid encoding nine dominant HLA-A*0201- and A11-restricted CTL epitopes derived from the polymerase, envelope, and core proteins of HBV and human immunodeficiency virus (HIV), a PADRE^{®} universal helper T cell (HTL) epitope, and an endoplasmic reticulum-translocating signal sequence has been engineered. Immunization of HLA transgenic mice with this plasmid construct resulted in strong CTL induction responses against the nine CTL epitopes tested. This CTL response was similar to that observed with a lipopeptide of known immunogenicity in humans, and significantly greater than immunization using peptides in oil-based adjuvants. Moreover, the immunogenicity of DNA-encoded epitopes *in vitro* was also correlated with the *in vitro* responses of specific CTL lines against target cells transfected with the DNA plasmid. These data show that the minigene served: 1.) to generate a CTL response and 2.) to generate CTLs that recognized cells expressing the encoded epitopes. A similar approach can be used to develop minigenes encoding TAA epitopes.

For example, to create a DNA sequence encoding the selected epitopes (minigene) for expression in human cells, the amino acid sequences of the epitopes may be reverse translated. A human codon usage table can be used to guide the codon choice for each amino acid. These epitope-encoding DNA sequences may be directly adjoined, so that when translated, a continuous peptide sequence is created. However, to optimize expression and/or immunogenicity, additional elements can be incorporated into the minigene design such as spacer amino acid residues between epitopes. HLA presentation of CTL and HTL epitopes may be improved by including synthetic (*e.g.* poly-alanine) or naturally-occurring flanking sequences adjacent to the CTL or HTL epitopes; these larger peptides comprising the epitope(s) are within the scope of the invention. In one embodiment, spacer amino acid residues between one or more CTL and/or HTL epitopes are designed so as to minimize junctional epitopes that may result from the juxtaposition of 2 CTL and/or HTL epitopes.

The minigene sequence may be converted to DNA by assembling oligonucleotides that encode the plus and minus strands of the minigene. Overlapping oligonucleotides (30-100 bases long) may be synthesized, phosphorylated, purified and annealed under appropriate conditions using well known techniques. The ends of the oligonucleotides can be joined, for example, using T4 DNA ligase. This synthetic minigene, encoding the epitope peptide, can then be cloned into a desired expression vector.

Standard regulatory sequences well known to those of skill in the art are preferably included in the vector to ensure expression in the target cells. Several vector elements are desirable: a promoter with a downstream cloning site for minigene insertion; a polyadenylation signal for efficient transcription termination; an *E. coli* origin of replication; and an *E. coli* selectable marker (*e.g.* ampicillin or kanamycin resistance). Numerous promoters can be used for this purpose, *e.g.*, the human cytomegalovirus (hCMV) CMV-IE promoter. See, *e.g.*, U.S. Patent Nos. 5,580,859 and 5,589,466 for other suitable promoter sequences.

Optimized peptide expression and immunogenicity can be achieved by certain modifications to a minigene construct. For example, in some cases introns facilitate efficient gene expression, thus one or more synthetic or naturally-occurring introns can be incorporated into the transcribed region of the minigene. The inclusion of mRNA stabilization sequences and sequences for replication in mammalian cells may also be considered for increasing minigene expression.

Once an expression vector is selected, the minigene is cloned into the polylinker region downstream of the promoter. This plasmid is transformed into an appropriate bacterial strain, and DNA is prepared using standard techniques. The orientation and DNA sequence of the minigene, as well as all other elements included in the vector, are confirmed using restriction mapping, PCR and/or DNA sequence analysis. Bacterial cells harboring the correct plasmid can be stored as cell banks.

In addition, immunostimulatory sequences (ISSs or CpGs) appear to play a role in the immunogenicity of DNA vaccines. These sequences may be included in the vector, outside the minigene coding sequence to enhance immunogenicity.

In some embodiments, a bi-cistronic expression vector which allows production of both the minigene-encoded epitopes and a second protein (*e.g.*, one that modulates immunogenicity) can be used. Examples of proteins or polypeptides that, if co-expressed with epitopes, can enhance an immune response include cytokines (*e.g.*, IL-2, IL-12, GM-CSF), cytokine-inducing molecules (*e.g.*, LeIF), costimulatory molecules, or pan-DR binding proteins (PADRE^{®}, Epimmune, San Diego, CA). Helper T cell (HTL) epitopes such as PADRE^{®} molecules can be joined to intracellular targeting signals and expressed separately from expressed CTL epitopes. This can be done in order to direct HTL epitopes to a cell compartment different than that of the CTL epitopes, one that provides for more efficient entry of HTL epitopes into the HLA class II pathway, thereby improving HTL induction. In contrast to HTL or CTL induction, specifically decreasing the immune response by co-expression of immunosuppressive molecules (*e.g.* TGF-β) may be beneficial in certain diseases.

Therapeutic quantities of plasmid DNA can be produced for example, by fermentation in *E*. *coli,* followed by purification. Aliquots from the working cell bank are used to inoculate growth medium, and are grown to saturation in shaker flasks or a bioreactor according to well known techniques. Plasmid DNA is purified using standard bioseparation technologies such as solid phase anion-exchange resins available, *e.g.*, from QIAGEN, Inc. (Valencia, California). If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). This approach, known as "naked DNA," is currently being used for intramuscular (IM) administration in clinical trials. To maximize the immunotherapeutic effects of minigene vaccines, alternative methods of formulating purified plasmid DNA may be used. A variety of such methods have been described, and new techniques may become available. Cationic lipids, glycolipids, and fusogenic liposomes can also be used in the formulation (see, *e.g.,* WO 93/24640; Mannino & Gould-Fogerite, BioTechniques 6(7): 682 (1988); U.S. Patent No. 5,279,833; WO 91/06309; and Felgner, et al., Proc. Nat'l Acad. Sci. USA 84:7413 (1987). In addition, peptides and compounds referred to collectively as protective, interactive, non-condensing compounds (PINC) can also be complexed to purified plasmid DNA to influence variables such as stability, intramuscular dispersion, or trafficking to specific organs or cell types.

Known methods in the art can be used to enhance delivery and uptake of a polynucleotide *in vivo.* For example, the polynucleotide can be complexed to polyvinylpyrrolidone (PVP), to prolong the localized bioavailability of the polynucleotide, thereby enhancing uptake of the polynucleotide by the organisum (*see e.g.,* U.S. Patent No. 6,040,295; EP 0 465 529; WO 98/17814). PVP is a polyamide that is known to form complexes with a wide variety of substances, and is chemically and physiologically inert.

Target cell sensitization can be used as a functional assay of the expression and HLA class I presentation of minigene-encoded epitopes. For example, the plasmid DNA is introduced into a mammalian cell line that is a suitable target for standard CTL chromium release assays. The transfection method used will be dependent on the final formulation, electroporation can be used for "naked" DNA, whereas cationic lipids or DNA:PVP compositions allow direct *in vitro* transfection. A plasmid expressing green fluorescent protein (GFP) can be co-transfected to allow enrichment of transfected cells using fluorescence activated cell sorting (FACS). The transfected cells are then chromium-51 (⁵¹Cr) labeled and used as targets for epitope-specific CTLs. Cytolysis of the target cells, detected by ⁵¹Cr release, indicates both the production and HLA presentation of, minigene-encoded CTL epitopes. Expression of HTL epitopes may be evaluated in an analogous manner using assays to assess HTL activity.

*In vivo* immunogenicity is a second approach for functional testing of minigene DNA formulations. Transgenic mice expressing appropriate human HLA proteins are immunized with the DNA product. The dose and route of administration are formulation dependent (*e.g.*, IM for DNA in PBS, intraperitoneal (IP) for lipid-complexed DNA). Eleven to twenty-one days after immunization, splenocytes are harvested and restimulated for one week in the presence of peptides encoding each epitope being tested. Thereafter, for CTLs, standard assays are conducted to determine if there is cytolysis of peptide-loaded, ⁵¹Cr-labeled target cells. Once again, lysis of target cells that were exposed to epitopes corresponding to those in the minigene, demonstrates DNA vaccine function and induction of CTLs. Immunogenicity of HTL epitopes is evaluated in transgenic mice in an analogous manner.

Alternatively, the nucleic acids can be administered using ballistic delivery as described, for instance, in U.S. Patent No. 5,204,253. Using this technique, particles comprised solely of DNA are administered. In a further alternative embodiment for ballistic delivery, DNA can be adhered to particles, such as gold particles.

### Vaccine Compositions

Vaccines that contain an immunologically effective amount of one or more peptides or polynucleotides of the invention are a further embodiment of the invention. The peptides can be delivered by various means or formulations, all collectively referred to as "vaccine" compositions. Such vaccine compositions, and/or modes of administration, can include, for example, naked DNA, DNA formulated with PVP, DNA in cationic lipid formulations; lipopeptides (*e.g.*,Vitiello, A. et al., J. Clin. Invest. 95:341, 1995), DNA or peptides, encapsulated *e.g.,* in poly(DL-lactide-co-glycolide); ("PLG") microspheres (*see, e.g.,* Eldridge, et al., Molec. Immunol. 28:287-294, 1991: Aloriso et al., Vaccine 12:299-306, 1994; Jones et al., Vaccine 13:675-681, 1995); peptide compositions contained in immune stimulating complexes (ISCOMS) (*see, e.g.,* Takahashi et al., Nature 344:873-875, 1990; Hu et al., Clin Exp Immunol. 113:235-243, 1998); multiple antigen peptide systems (MAPs) (*see e.g.,* Tam, J. P., Proc. Natl. Acad. Sci. U.S.A. 85:5409-5413, 1988; Tam, J.P., J. Immunol. Methods 196:17-32, 1996); viral, bacterial, or, fungal delivery vectors (Perkus, M. E. et al., In: Concepts in vaccine development, Kaufmann, S. H. E., ed., p. 379, 1996; Chakrabarti, S. et al., Nature 320:535, 1986; Hu, S. L. et al., Nature 320:537, 1986; Kieny, M.-P. et al., AIDS Bio/Technology 4:790, 1986; Top, F. H. et al., J. Infect. Dis. 124:148, 1971; Chanda, P. K. et al., Virology 175:535, 1990); particles of viral or synthetic origin (*e.g.,* Kofler, N. et al., J. Immunol. Methods. 192:25, 1996; Eldridge, J. H. et al., Sem. Hematol. 30:16, 1993; Falo, L. D., Jr. et al., Nature Med. 7:649, 1995); adjuvants (*e.g.,* incomplete freund's advjuvant) (Warren, H. S., Vogel, F. R., and Chedid, L. A. Annu. Rev. Immunol. 4:369, 1986; Gupta, R. K. et al., Vaccine 11:293, 1993); liposomes (Reddy, R. et al., J. Immunol. 148:1585, 1992; Rock, K. L., Immunol. Today 17:131, 1996); or, particle-absorbed DNA (Ulmer, J. B. et al., Science 259:1745, 1993; Robinson, H. L., Hunt, L. A., and Webster, R. G., Vaccine 11:957, 1993; Shiver, J. W. et al., In: Concepts in vaccine development, Kaufmann, S. H. E., ed., p. 423, 1996; Cease, K. B., and Berzofsky, J. A., Annu. Rev. Immunol. 12:923, 1994 and Eldridge, J. H. et al., Sem. Hematol. 30:16, 1993), etc. Toxin-targeted delivery technologies, also known as receptor mediated targeting, such as those of Avant Immunotherapeutics, Inc. (Needham, Massachusetts) or attached to a stress protein, e.g., HSP 96 (Stressgen Biotechnologies Corp., Victoria, BC, Canada) can also be used.

Vaccines of the invention comprise nucleic acid mediated modalities. DNA or RNA encoding one or more of the peptides of the invention can be administered to a patient. This approach is described, for instance, in Wolff et. al., Science 247:1465 (1990) as well as U.S. Patent Nos. 5,580,859; 5,589,466; 5,804,566; 5,739,118; 5,736,524; 5,679,647; and, WO 98/04720. Examples of DNA-based delivery technologies include "naked DNA", facilitated (bupivicaine, polymers (*e.g.*, PVP), peptide-mediated) delivery, cationic lipid complexes, and particle-mediated ("gene gun") or pressure-mediated delivery (*see, e.g.,* U.S. Patent No. 5,922,687). Accordingly, peptide vaccines of the invention can be expressed by viral or bacterial vectors. Examples of expression vectors include attenuated viral hosts, such as vaccinia or fowlpox. For example, vaccinia virus is used as a vector to express nucleotide sequences that encode the peptides of the invention (*e.g.*, MVA). Upon introduction into an acutely or chronically infected host or into a non-infected host, the recombinant vaccinia virus expresses the immunogenic peptide, and thereby elicits an immune response. Vaccinia vectors and methods useful in immunization protocols are described in, *e.g.*, U.S. Patent No. 4,722,848. Another vector is BCG (Bacille Calmette Guerin). BCG vectors are described in Stover et al., Nature 351:456-460 (1991). A wide variety of other vectors useful for therapeutic administration or immunization of the peptides of the invention, *e.g.* adeno and adeno-associated virus vectors, alpha virus vectors, retroviral vectors, *Salmonella typhi* vectors, detoxified anthrax toxin vectors, and the like, are apparent to those skilled in the art from the description herein.

Furthermore, vaccines in accordance with the invention can comprise one or more peptides of the invention. Accordingly, a peptide can be present in a vaccine individually; alternatively, the peptide can exist as a homopolymer comprising multiple copies of the same peptide, or as a heteropolymer of various peptides. Polymers have the advantage of increased probability for immunological reaction and, where different peptide epitopes are used to make up the polymer, the ability to induce antibodies and/or T cells that react with different antigenic determinants of the antigen targeted for an immune response. The composition may be a naturally occurring region of an antigen or can be prepared, e.g., recombinantly or by chemical synthesis.

Carriers that can be used with vaccines of the invention are well known in the art, and include, *e*.*g*., thyroglobulin, albumins such as human serum albumin, tetanus toxoid, polyamino acids such as poly L-lysine, poly L-glutamic acid, influenza virus proteins, hepatitis B virus core protein, and the like. The vaccines can contain a physiologically tolerable diluent such as water, or a saline solution, preferably phosphate buffered saline. Generally, the vaccines also include an adjuvant. Adjuvants such as incomplete Freund's adjuvant, aluminum phosphate, aluminum hydroxide, or alum are examples of materials well known in the art. Additionally, as disclosed herein, CTL responses can be primed by conjugating peptides of the invention to lipids, such as tripalmitoyl-S-glyceryl-cysteinyl-seryl-serine (P₃CSS).

Upon immunization with a peptide composition in accordance with the invention, via injection (*e.g.,* SC, ID, IM), aerosol, oral, transdermal, transmucosal, intrapleural, intrathecal, or other suitable routes, the immune system of the host responds to the vaccine by producing antibodies, CTLs and/or HTLs specific for the desired antigen. Consequently, the host becomes at least partially immune to subsequent exposure to the TAA, or at least partially resistant to further development of TAA-bearing cells and thereby derives a prophylactic or therapeutic benefit.

In certain embodiments, components that induce T cell responses are combined with components that induce antibody responses to the target antigen of interest. A preferred embodiment of such a composition comprises class I and class II epitopes in accordance with the invention. Alternatively, a composition comprises a class I and/or class II epitope in accordance with the invention, along with a PADRE^{®} molecule (Epimmune, San Diego, CA).

Vaccines of the invention can comprise antigen presenting cells, such as dendritic cells, as a vehicle to present peptides of the invention. For example, dendritic cells are transfected, *e.g.,* with a minigene construct in accordance with the invention, in order to elicit immune responses. Minigenes are discussed in greater detail in a following section. Vaccine compositions can be created *in vitro,* following dendritic cell mobilization and harvesting, whereby loading of dendritic cells occurs *in vitro.*

The vaccine compositions of the invention may also be used in combination with antiviral drugs such as interferon-α, or immune adjuvants such as IL-12, GM-CSF, etc.

Preferably, the following' principles are utilized when selecting epitope(s) and/or analogs for inclusion in a vaccine, either peptide-based or nucleic acid-based formulations. Exemplary epitopes and analogs that may be utilized in a vaccine to treat or prevent TAA-associated disease are set out in Table 6. Each of the following principles can be balanced in order to make the selection. When multiple epitopes are to be used in a vaccine, the epitopes may be, but need not be, contiguous in sequence in the native antigen from which the epitopes are derived. Such multiple epitotes can refer to the order of epitopes within a peptide, or to the selection of epitopes that come from the same reagion, for use in either individual peptides or in a multi-epitopic peptide.
1.) Epitopes and/or analogs are selected which, upon administration, mimic immune responses that have been observed to be correlated with prevention or clearance of TAA-expressing tumors. For HLA Class I, this generally includes 3-4 epitopes and/or analogs from at least one TAA.
2.) Epitopes and/or analogs are selected that have the requisite binding affinity established to be correlated with immunogenicity: for HLA Class I an IC₅₀ of 500 nM or less, or for Class II an IC₅₀ of 1000 nM or less. For HLA Class I it is presently preferred to select a peptide having an IC₅₀ of 200 nM or less, as this is believed to better correlate not only to induction of an immune response, but to *in vitro* tumor cell killing as well. For HLA A1 and A24, it is especially preferred to select a peptide having an IC₅₀ of 100 nM or less.
3.) Supermotif bearing-epitopes and/or analogs, or a sufficient array of allele-specific motif-bearing epitopes and/or analogs, are selected to give broad population coverage. In general, it is preferable to have at least 80% population coverage. A Monte Carlo analysis, a statistical evaluation known in the art, can be employed to assess the breadth of population coverage.
4.) For cancer-related antigens, it can be preferable to select analogs instead of or in addition to epitopes, because the patient may have developed tolerance to the native epitope.
5.) Of particular relevance are "nested epitopes." Nested epitopes occur where at least two epitopes overlap in a given peptide sequence. For example, a nested epitope can be a fragment of an antigen from a region that contains multiple epitopes that are overleapping, or one epitope that is completely encompassed by another, e.g., A2 peptides MAGE3.159 and MAGE3.160 are nested.A peptide comprising "transcendent nested epitopes" is a peptide that has both HLA class. I and HLA class II epitopes in it. When providing nested epitopes, it is preferable to provide a sequence that has the greatest number of epitopes per provided sequence. Preferably, one avoids providing a peptide that is any longer than the amino terminus of the amino terminal epitope and the carboxyl terminus of the carboxyl terminal epitope in the peptide. When providing a sequence comprising nested epitopes, it is important to evaluate the sequence in order to insure that it does not have pathological or other deleterious biological properties; this is particularly relevant for vaccines directed to infectious organisms.
6.) If a protein with multiple epitopes or a polynucleotide (e.g., minigene) is created, an objective is to generate the smallest peptide that encompasses the epitopes of interest. This principle is similar, if not the same as that employed when selecting a peptide comprising nested epitopes. However, with an artificial peptide comprising multipe epitopes, the size minimization objective is balanced against the need to integrate any spacer sequences between epitopes in the polyepitopic protein. Spacer amino acid residues can be introduced to avoid junctionaLepitopes (an epitope recognized by the immune system, not present in the target antigen, and only created by the man-made juxtaposition of epitopes), or to facilitate cleavage between epitopes and thereby enhance epitope presentation. Junctional epitopes are generally to be avoided because the recipient may generate an immune response to that non-native epitope. Of particular concern is a junctional epitope that is a "dominant epitope." A dominant epitope may lead to such a zealous response that immune responses to other epitopes are diminished or suppressed.

The principles are the same, except junctional epitopes applies to the sequences surrounding the epitope. One must also take care with other sequences in construct to avoid immune response.

### T CELL PRIMING MATERIALS

In some embodiments it may be desirable to include in the pharmaceutical compositions of the invention at least one component which primes cytotoxic T lymphocytes. Lipids have been identified as agents capable of facilitating the priming *in vitro* CTL response against viral antigens. For example, palmitic acid residues can be attached to the ε-and α- amino groups of a lysine residue and then linked to an immunogenic peptide. One or more linking moieties can be used such as Gly, Gly-Gly-, Ser, Ser-Ser, or the like. The lipidated peptide can then be administered directly in a micelle or particle, incorporated into a liposome, or emulsified in an adjuvant, *e.g.*, incomplete Freund's adjuvant. A preferred immunogenic composition comprises palmitic acid attached to ε- and α- amino groups of Lys via a linking moiety, *e.g.*, Ser-Ser, added to the amino terminus of an immunogenic peptide.

In another embodiment of lipid-facilitated priming of CTL responses, *E. coli* lipoproteins, such as tripalinitoyl-S-glyceryl-cysteinyl-seryl-serine (P₃CSS) can be used to prime CTL when covalently attached to an appropriate peptide. (*See, e.g.,* Deres, et al., Nature 342:561, 1989). Thus, peptides of the invention can be coupled to P₃CSS, and the lipopeptide administered to an individual to specifically prime a CTL response to the target antigen. Moreover, because the induction of neutralizing antibodies can also be primed with P₃CSS-conjugated epitopes, two such compositions can be combined to elicit both humoral and cell-mediated responses.

### DENDRITIC CELLS PULSED WITH CTL AND/OR HTL PEPTIDES

An embodiment of a vaccine composition in accordance with the invention comprises *ex vivo* administration of a cocktail of epitope-bearing peptides to PBMC, or isolated DC therefrom, from the patient's blood. A pharmaceutical to facilitate harvesting of DC can be used, such as Progenipoietin™ (Monsanto, St. Louis, MO) or GM-CSF/IL-4. After pulsing the DC with peptides and prior to reinfusion into patients, the DC are washed to remove unbound peptides. In this embodiment, a vaccine comprises peptide-pulsed DCs which present the pulsed peptide epitopes in HLA molecules on their surfaces.

The DC can be pulsed *ex vivo* with a cocktail of peptides, some of which stimulate CTL responses to one or more antigens of interest, *e.g.*, tumor associated antigens (TAA) such as HER2/neu, p53, MAGE 2, MAGE3, and/or carcinoembryonic antigen (CEA). Collectively, these TAA are associated with breast, colon and lung cancers. Optionally, a helper T cell (HTL) peptide such as PADRE^{®}, can be included to facilitate the CTL response. Thus, a vaccine in accordance with the invention comprising epitopes from HER2/neu, p53, MAGE 2, MAGE3, and carcinoembryonic antigen (CEA) is used to treat minimal or residual disease in patients with malignancies such as breast, colon, lung or ovarian cancer; any malignancies that bear any of these TAAs can also be treated with the vaccine: A TAA vaccine can be used following debulking procedures such as surgery, radiation therapy or chemotherapy, whereupon the vaccine provides the benefit of increasing disease free survival and overall survival in the recipients.

Thus, in preferred embodiments, a vaccine of the invention is a product that treats a majority of patients across a number of different tumor types. A vaccine comprising a plurality of epitopes, preferably supermotif-bearing epitopes, offers such an advantage.

### DIAGNOSTIC AND PROGNOSTIC USES

In one embodiment of the invention, HLA class I and class II binding peptides can be used as reagents to evaluate an immune response. Preferably, the following principles are utilized when selecting an epitope(s) and/or analog(s) for diagnostic, prognostic and similar uses. Potential principles include having the binding affinities described earlier, and/or matching the HLA-motif/supermotif of a peptide with the HLA-type of a patient.

The evaluated immune response can be induced by any immunogen. For example, the immunogen may result in the production of antigen-specific CTLs or HTLs that recognize the peptide epitope(s) employed as the reagent. Thus, a peptide of the invention may or may not be used as the immunogen. Assay systems that can be used for such analyses include tetramer-based protocols (*e.g.*, DimerX technology (*see, e.g.,* Science 274:94-99 (1996) and Proc. Natl. Acad. Sci. 95:7568-73 (1998)), staining for intracellular lymphokines, interferon release assays, or ELISPOT assays.

For example, following exposure to a putative immunogen, a peptide of the invention can be used in a tetramer staining assay to assess peripheral blood mononuclear cells for the presence of any antigen-specific CTLs. The HLA-tetrameric complex is used to directly visualize antigen-specific CTLs and thereby determine the frequency of such antigen-specific CTLs in a sample of peripheral blood mononuclear cells (*see, e.g.,* Ogg et al., Science 279:2103-2106, 1998; and Altman et al., Science 174:94-96, 1996).

A tetramer reagent comprising a peptide of the invention is generated as follows: A peptide that binds to an HLA molecule is refolded in the presence of the corresponding HLA heavy chain and β₂-microglobulin to generate a trimolecular complex. The complex is biotinylated at the carboxyl terminal end of the HLA heavy chain, at a site that was previously engineered into the protein. Tetramer formation is then induced by adding streptavidin. When fluorescently labeled streptavidin is used, the tetrameric complex is used to stain antigen-specific cells. The labeled cells are then readily identified, *e.g.*, by flow cytometry. Such procedures are used for diagnostic or prognostic purposes; the cells identified by the procedure can be used for therapeutic purposes.

Peptides of the invention are also used as reagents to evaluate immune recall responses. (*see, e.g.,* Bertoni et al., J. Clin. Invest. 100:503-513, 1997 and Penna et al., J. Exp. Med. 174:1565-1570, 1991.) For example, a PBMC sample from an individual expressing a disease-associated antigen (e.g. a tumor-associated antigen such as CEA, p53, MAGE2/3,HER2neu, or an organism associated with neoplasia such as HPV or HSV) can be analyzed for the presence of antigen-specific CTLs or HTLs using specific peptides. A blood sample containing mononuclear cells may be evaluated by cultivating the PBMCs and stimulating the cells with a peptide of the invention. After an appropriate cultivation period, the expanded cell population may be analyzed, for example, for CTL or for HTL activity.

Thus, the peptides can be used to evaluate the efficacy of a vaccine. PBMCs obtained from a patient vaccinated with an immunogen may be analyzed by methods such as those described herein. The patient is HLA typed, and peptide epitopes that are bound by the HLA molecule(s) present in that patient are selected for analysis. The immunogenicity of the vaccine is indicated by the presence of CTLs and/or HTLs directed to epitopes present in the vaccine.

The peptides of the invention may also be used to make antibodies, using techniques well known in the art (see, *e.g.* CURRENT PROTOCOLS IN IMMUNOLOGY, Wiley/Greene, NY; and Antibodies A Laboratory Manual Harlow, Harlow and Lane, Cold Spring Harbor Laboratory Press, 1989). Such antibodies are useful as reagents to determine the presence of disease-associated antigens. Antibodies in this category include those that recognize a peptide when bound by an HLA molecule, *i.e.,* antibodies that bind to a peptide-MHC complex.

### ADMIMSTRATION FOR THERAPEUTIC OR PROPHYLACTIC PURPOSES

The peptides and polynucleotides of the present invention, including compositions thereof, are useful for administration to mammals, particularly humans, to treat and/or prevent disease. In one embodiment, peptides, polynucleotides, or vaccine compositions (peptide or nucleic acid) of the invention are administered to a patient who has a malignancy associated with expression of one or more TAAs, or to an individual susceptible to, or otherwise at risk for developing TAA-related disease. Upon administration an immune response is elicited against the TAAs, thereby enhancing the patient's own immune response capabilities. In therapeutic applications, peptide and/or nucleic acid compositions are administered to a patient in an amount sufficient to elicit an effective immune response to the TAA-expressing cells and to thereby cure, arrest or slow symptoms and/or complications. An amount adequate to accomplish this is defined as "therapeutically effective dose." Amounts effective for this use will depend on, e.g., the particular composition administered, the manner of administration, the stage and severity of the disease being treated, the weight and general state of health of the patient, and the judgment of the prescribing physician.

The vaccine compositions of the invention can be used purely as prophylactic agents. Generally the dosage for an initial prophylactic immunization generally occurs in a unit dosage range where the lower value is about 1, 5, 50, 500, or 1000 µg of peptide and the higher value is about 10,000; 20,000; 30,000; or 50,000 µg of peptide. Dosage values for a human typically range from about 500 µg to about 50,000 µg of peptide per 70 kilogram patient. This is followed by boosting dosages of between about 1.0 µg to about 50,000 µg of peptide, administered at defined intervals from about four weeks to six months after the initial administration of vaccine. The immunogenicity of the vaccine may be assessed by measuring the specific activity of CTL and HTL obtained from a sample of the patient's blood.

As noted above, peptides comprising CTL and/or HTL epitopes of the invention induce immune responses when presented by HLA molecules and contacted with a CTL or HTL specific for an epitope comprised by the peptide. The manner in which the peptide is contacted with the CTL or HTL is not critical to the invention. For instance, the peptide can be contacted with the CTL or HTL either *in vitro* or *in vivo.* If the contacting occurs *in vivo,* peptide can be administered directly, or in other forms/vehicles, *e.g.*, DNA vectors encoding one or more peptides, viral vectors encoding the peptide(s), liposomes, antigen presenting cells such as dendritic cells, and the like.

Accordingly, for pharmaceutical compositions of the invention in the form of peptides or polypeptides, the peptides or polypeptides can be administered directly. Alternatively, the peptide/polypeptides can be administered indirectly presented on APCs, or as DNA encoding them. Furthermore, the peptides or DNA encoding them can be administered individually or as fusions of one or more peptide sequences.

For therapeutic use, administration should generally begin at the first diagnosis of TAA-related disease. This is followed by boosting doses at least until symptoms are substantially abated and for a period thereafter. In chronic disease states, loading doses followed by boosting doses may be required.

The dosage for an initial therapeutic immunization generally occurs in a unit dosage range where the lower value is about 1, 5, 50, 500, or 1,000 µg of peptide and the higher value is about 10,000; 20,000; 30,000; or 50,000 µg of peptide. Dosage values for a human typically range from about 500 µg to about 50,000 µg of peptide per 70 kilogram patient. Boosting dosages of between about 1.0 µg to about 50,000 µg of peptide, administered pursuant to a boosting regimen over weeks to months, can be administered depending upon the patient's response and condition. Patient response can be determined by measuring the specific activity of CTL and HTL obtained from the patient's blood.

In certain embodiments, peptides and compositions of the present invention are used in serious disease states. In such cases, as a result of the minimal amounts of extraneous substances and the relative nontoxic nature of the peptides, it is possible and may be desirable to administer substantial excesses of these peptide compositions relative to these stated dosage amounts.

For treatment of chronic disease, a representative dose is in the range disclosed above, namely where the lower value is about 1, 5, 50, 500, or 1,000 µg of peptide and the higher value is about 10,000; 20,000; 30,000; or 50,000 µg of peptide, preferably from about 500 µg to about 50,000 µg of peptide per 70 kilogram patient. Initial doses followed by boosting doses at established intervals, *e.g.*, from four weeks to six months, may be required, possibly for a prolonged period of time to effectively immunize an individual. In the case of chronic disease, administration should continue until at least clinical symptoms or laboratory tests indicate that the disease has been eliminated or substantially abated, and for a follow-up period thereafter. The dosages, routes of administration, and dose schedules are adjusted in accordance with methodologies known in the art.

The pharmaceutical compositions for therapeutic treatment are intended for parenteral, topical, oral, intrathecal, or local administration. Preferably, the pharmaceutical compositions are administered parentally, *e.g.*, intravenously, subcutaneously, intradermally, or intramuscularly.

Thus, in a preferred embodiment the invention provides compositions for parenteral administration which comprise a solution of the immunogenic peptides dissolved or suspended in an acceptable carrier, preferably an aqueous carrier. A variety of aqueous carriers may be used, *e.g.*, water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances or pharmaceutical excipients as may be required to approximate physiological conditions, such as pH-adjusting and buffering agents, tonicity adjusting agents, wetting agents, preservatives, and the like, for example, sodium acetate, sodium lactate, sodium chloride, potassium chloride, calcium chloride, sorbitan monolaurate, triethanolamine oleate, *etc.*

The concentration of peptides of the invention in the pharmaceutical formulations can vary widely, *i.e.,* from less than about 0.1%, usually at or at least about 2% to as much as 20% to 50% or more by weight, and will be selected primarily by fluid volumes, viscosities, *etc.,* in accordance with the particular mode of administration selected.

A human unit dose form of the peptide composition is typically included in a pharmaceutical composition that also comprises a human unit dose of an acceptable carrier, preferably an aqueous carrier, and is administered in a volume of fluid that is known by those of skill in the art to be used for administration of such compositions to humans (*see, e.g.,* Remington's Pharmaceutical Sciences, 17th Edition, A. Gennaro, Editor, Mack Publishing Co., Easton, Pennsylvania, 1985).

The peptides of the invention can also be administered via liposomes, which serve to target the peptides to a particular tissue, such as lymphoid tissue, or to target selectively to infected cells, as well as to increase the half-life of the peptide composition. Liposomes include emulsions, foams, micelles, insoluble monolayers, liquid crystals, phospholipid dispersions, lamellar layers and the like. In these preparations, the peptide to be delivered is incorporated as part of a liposome, alone or in conjunction with a molecule which binds to a receptor prevalent among lymphoid cells (such as monoclonal antibodies which bind to the CD45 antigen) or with other therapeutic or immunogenic compositions. Thus, liposomes either filled or decorated with a desired peptide of the invention can be directed to the site of lymphoid cells, where the liposomes then deliver the peptide compositions. Liposomes for use in accordance with the invention are formed from standard vesicle-forming lipids, which generally include neutral and negatively charged phospholipids and a sterol, such as cholesterol. The selection of lipids is generally guided by consideration of, *e.g.*, liposome size, acid lability and stability of the liposomes in the blood stream. A variety of methods are available for preparing liposomes, as described in, *e.g.*, Szoka, et al., Ann. Rev. Biophys. Bioeng. 9:467 (1980), and U.S. Patent Nos. 4,235,871, 4,501,728, 4,837,028, and 5,019,369.

For targeting compositions of the invention to cells of the immune system, a ligand can be incorporated into the liposome, e.g., antibodies or fragments thereof specific for cell surface determinants of the desired immune system cells. A liposome suspension containing a peptide may be administered intravenously, locally, topically, *etc.* in a dose which varies according to, *inter alia,* the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, often at a concentration of 25%-75%.

For aerosol administration, the immunogenic peptides are preferably supplied in finely divided form, along with a surfactant and propellant. Typical percentages of peptides are 0.01%-20% by weight, often 1%-10%. The surfactant must, of course, be pharmaceutically acceptable, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant, although an atomizer may be used in which no propellant is necessary and other percentages are adjusted accordingly. A carrier can also be included, e.g., lecithin for intranasal delivery.

Antigenic peptides of the invention have been used to elicit a CTL and/or HTL response *ex vivo,* as well. The resulting CTLs or HTLs can be used to treat chronic infections, or tumors, in patients that do not respond to other conventional forms of therapy, or who do not respond to a therapeutic peptide or nucleic acid vaccine in accordance with the invention. *Ex vivo* CTL or HTL responses to a particular antigen (infectious or tumor-associated) are induced by incubating in tissue culture the patient's, or genetically compatible, CTL or HTL precursor cells together with a source of antigen-presenting cells (APC), such as dendritic cells, and the appropriate immunogenic peptide. After an appropriate incubation time (typically about 7-28 days), in which the precursor cells are activated and expanded into effector cells, the cells are infused back into the patient, where they will destroy (CTL) or facilitate destruction (HTL) of their specific target cell (an infected cell or a tumor cell).

### KITS

The peptide and nucleic acid compositions of this invention can be provided in kit form together with instructions for vaccine administration. Typically the kit would include desired composition(s) of the invention in a container, preferably in unit dosage form and instructions for administration. For example, a kit would include an APC, such as a dendritic cell, previously exposed to and now presenting peptides of the invention in a container, preferably in unit dosage form together with instructions for administration. An alternative kit would include a minigene construct with desired nucleic acids of the invention in a container, preferably in unit dosage form together with instructions for administration. Lymphokines such as IL-2 or IL-12 may also be included in the kit. Other kit components that may also be desirable include, for example, a sterile syringe, booster dosages, and other desired excipients.

The invention will be described in greater detail by way of specific examples. The following examples are offered for illustrative purposes, and are not intended to limit the invention in any manner. Those of skill in the art will readily recognize a variety of non-critical parameters that can be changed or modified to yield alternative embodiments in accordance with the invention.

### EXAMPLES

### EXAMPLE 1

### SELECTION OF TUMOR ASSOCIATED ANTIGENS

Vaccines which bind to HLA supertypes, A2, A3, and B7, will afford broad, non-ethnically biased population coverage (83-88%). Vaccines which bind to HLA supertypes, A1, A11, and B44, will afford broad, non-ethnically biased population coverage (99-100%). Since the A2 supertype is broadly expressed in the population (39-49%), peptides which bind to this family of molecules provide a reasonable starting point for the use of peptide-based vaccines. While the A2 vaccine targets patients that express HLA-A2 molecules, the approach can be readily extended to include peptide(s) that bind to additional alleles or supertype groups thereof.

Whole proteins often induce an immune response limited to specific epitopes that may be ineffective in mediating effective anti-tumor immune responses (Disis et al., J. Immunology 156:3151-3158 (1996); Manca et al., J. Immunology 146:1964-1971 (1991)). An epitope-based vaccine circumvents this limitation through the identification of peptide epitopes embedded in TAAs. Exemplary TAAs are set forth in Table 12.

Peptides were evaluated based upon MHC binding motifs, on the capacity to bind MHC molecules, and the ability to activate tumor-reactive CTL *in vitro* using lymphocyte cultures from normal individuals. This approach has several advantages. First, it does not require the isolation of patient-derived cells such as CTL or tumor cells. Secondly, the identification of epitopes that stimulate CTL in normal individuals permits the identification of a broad range of epitopes, including subdominant as well as dominant epitopes.

Four tumor-associated antigens, CEA, p53, MAGE 2/3 and HER2/neu, are expressed in various tumor types (Kawashima et al., Human Immunology 59:1-14 (1998); Tomlinson, et al., Advanced Drug Delivery Reviews, Vol. 32(3) (6 July 1998)). In a preferred embodiment, a vaccine comprises epitopes (as one or more peptides or as nucleic acids encoding them) from among these four, or any other, TAAs. Accordingly, this vaccine induces CTL responses against several major cancer types.

Carcinoembryonic antigen is a 180 kDmw cell surface and secreted glycoprotein overexpressed on most human adenocarcinomas. These include colon, rectal, pancreatic and gastric (Muraro, 1985) as well as 50% of breast (Steward, 1974) and 70% of non-small cell lung carcinomas (Vincent, 1978). This antigen is also expressed on normal epithelium and in some fetal tissue (Thompson, 1991).

The HER2/neu antigen (185 kDa) is a transmembrane glycoprotein with tyrosine kinase activity whose structure is similar to the epidermal growth factor receptor (Coussens, 1985; Bargmann, 1986; Yamamoto, 1986). Amplification of the HER2/neu gene and/or overexpression of the associated protein have been reported in many human adenocarcinomas of the breast (Slamon, 1987 and 1989; Borg, 1990), ovary (Slamon, 1989), uterus (Berchuck, 1991; Lukes, 1994), prostate (Kuhn, 1993; Sadasivan, 1993), stomach (Yonemura, 1991; Kameda, 1990; Houldsworth, 1990), esophagus (Houldsworth, 1990), pancreas (Yamanaka, 1993), kidney (Weidner, 1990) and lung (Kern, 1990; Rachwal, 1995).

The MAGE, melanoma antigen genes, are a family of related proteins that were first described in 1991. Van der Bruggen and co-workers were able to identify the MAGE gene after isolating CTLs from a patient who demonstrated spontaneous tumor regression. These CTLs recognized melanoma cell lines as well as tumor lines from other patients all expressing the same HLA-A1 restricted gene (van der Bruggen, 1991; De Plaen, 1994). The MAGE genes are expressed in metastatic melanomas (Brasseur, 1995), non-small lung (Weynants, 1994), gastric (Inoue, 1995), hepatocellular (Chen, 1999), renal (Yamanaka, 1998) colorectal (Mori, 1996), and esophageal (Quillien, 1997) carcinomas as wells as tumors of the head and neck (Lee, 1996), ovaries (Gillespie, 1998; Yamada, 1995), bladder (Chaux, 1998) and bone (Sudo, 1997). They are also expressed on normal tissue, specifically placenta and male germ cells (De Plaen, 1994). However, these normal cells do not express MHC Class I molecules and therefore do not present MAGE peptides on their surface.

In this study and previous work to identify A2 superfamily epitopes (Kawashima, 1998), MAGE-2 and MAGE-3 were considered a single TAA, based on the expression patterns and predicted primary amino acid sequences of the two genes. These two members of the MAGE family appear to be coordinately regulated (Zakut, 1993), resulting in a distribution in cancers that appears to be very similar, if not identical. Therefore, immune responses directed at either antigen should provide coverage for treatment of the cancers expected to express these TAA. The MAGE-2 and MAGE-3 proteins are 84% identical at the primary amino acid level. As a result, some epitopes are identical in the two antigens, while others are unique to one or the other. It should be noted that two subtypes of MAGE-2, designated "a" and b", have been reported (Zakut, 1993). The gene referred to herein as MAGE-2 corresponds to the MAGE-2a subtype (C. Dahlberg personal communication, NB 1056, p.16; Van der Bruggen, 1991; Zakut, 1993).

The fourth TAA selected for use in the vaccine is p53. In normal cells the p53 gene induces a cell cycle arrest which allows DNA to be checked for irregularities and maintains DNA integrity (Kuerbitz, 1992). Mutations in the gene abolish its suppressor function and allow escape of transformed cells from the restriction of controlled growth. At the same time, these mutations lead to overexpression of both wildtype and mutated p53 (Levine, 1991) making it more likely that epitopes within the protein may be recognized by the immune system. The most common mutations are at positions 175, 248, 273 and 282 and have been observed in colon (Rodrigues, 1990), lung (Fujino, 1995), prostate (Eastham, 1995), bladder (Vet, 1995) and bone cancers (Abudu, 1999; Hung, 1997).

Other TAAs that can be included in a vaccine composition are associated with prostate cancer *(see, e.g.,* copending U.S. Patent Application USSN 09/633,364, filed 8 July 2000).

Table 7 below delineates the tumor antigen expression in breast, colon and lung. By targeting four TAA, the likelihood of the mutation of tumor cells (tumor escape) into cells which do not express any of the tumor antigens is decreased. Preferably, the inclusion of two or more epitopes from each TAA serves to increase the likelihood that individuals of different ethnicity will respond to the vaccine and provides broadened population coverage.

This rational approach to vaccine compositions can be focused on a particular HLA allele, or extended to various HLA molecules or supertypes to further extend population coverage.

Table 8 shows the incidence, 5-year survival rates, and the estimated number of deaths per year for these tumors in the U.S for each type of cancer in Table 7. In terms of estimated new cases, estimated deaths and 5 year survival rates each of these tumor types has a large unmet need. Globally, the incidence of these tumors is significantly greater.

### EXAMPLE 2:

### IDENTIFICATION OF A2 SUPERMOTIF/MOTIF-BEARING PEPTIDES

Protein sequences from the four targeted tumor antigens (CEA, p53, MAGE 2/3 and HER2/neu) were analyzed, to identify 8-, 9-, 10-, and 11-mer sequences containing the HLA-A2 supertype binding motif. This motif [leucine (L), isoleucine (I), valine (V), methionine (M), alanine (A), threonine (T), or glutamine (Q) at position 2, and leucine (L), isoleucine (I), valine (V), methionine (M), alanine (A), or threonine (T) at the C-terminus; see Table 2] is the predominant factor in determining peptide binding to the HLA molecules within the A2 supertype (see, *e.g.*, del Guercio et al., J. Immunol., 154:685-693 (1995); Sette, A. and Sidney, J., Cur. Opin. Immunol., 10: 478-482 (1998); Sidney et al., Immunology Today, 17:261-266 (1996)). Nonamer and decamer sequences were further characterized using an A2-specific algorithm to evaluate secondary anchor residues (Ruppert et al., Cell 74:929-937 (1993); Gulukota et al., J. Mol. Biol. 267:1258-1267 (1997)).

### EXAMPLE 3

### MOLECULAR BINDING ASSAYS

Native sequences containing HLA-A2 peptide motifs were tested directly for binding to human class I HLA molecules, since a subset of motif-bearing peptides bind with a biologically significant affinity, data depicted in Table 6. An affinity threshold ≤ 500 nM to the HLA-A2 molecule was previously shown to define the capacity of a peptide epitope to elicit a CTL response (Sette et al., J. Immunol. 153:5586-5592 (1994)). A competitive inhibition assay using purified HLA molecules was used to quantify peptide binding. Motif-bearing peptides were initially tested for binding to HLA-A*0201, the prototype member of the HLA-A2 supertype. Peptides binding to A*0201 with an IC₅₀ ≤ 500 nM were subsequently tested for their capacity to bind other predominant molecules of the A2 supertype: A*0202, A*0203, A*0206 and A*6802 (del Guercio et al., J. Immunol., 154:685-693 (1995); Sette, A. and Sidney, J., Cur. Opin. Immunol., 10: 478-482 (1998); Sidney et al., Immunology Today, 17:261-266 (1996)). A*0201-binding peptides found to bind at least one additional A2 supertype member were selected for further testing. Analogs of the native sequences for the CEA and p53 were evaluated to identify additional CTL peptide epitopes, as described below.

### EXAMPLE 4

### A2 EPITOPE IDENTIFICATION

Since HLA-A2 is a species restricted molecule, the binding and functional activities of the A2 vaccine epitopes were measured *in vitro* using human molecules and cells. CTL epitopes were identified that demonstrated high or intermediate HLA-A2 binding affinity (IC₅₀ of ≤ 500 nM). These epitopes also bound to at least one additional member of the HLA-A2 supertype family with an IC₅₀ ≤ 500 nM. Each epitope stimulated the *in vitro* induction of a specific human CTL that recognized and lysed peptide-pulsed target cells and tumor cell lines expressing the relevant TAA. A PADRE® molecule is optionally included in the vaccine to promote the induction of long lasting CTL responses (Alexander et al., Immunol. Res. 18(2):79-92 (1998)).

Immunological responses were demonstrated by *in vitro* induction of human CTL that were capable of recognizing both peptide-pulsed cells and TAA-expressing tumor cell lines. In certain cases, analog peptides were selected based on either improved binding affinity or supertype coverage relative to the native peptide and in one case, substitution of a cysteine with another amino acid.

Analogous assays can be used for other HLA types.

### EXAMPLE 5

### PEPTIDE ANALOGS INCREASE SUPERTYPE CROSS-REACTIVITY OR IMPROVE CHEMICAL CHARACTERISTICS

Class I HLA peptides can be modified, or "analoged" by substitution of amino acids at a given position to increase their HLA binding affinity and/or supertype cross-reactivity (see, *e.g.*, Table 2, and Zitvogel et al., J Exp Med 183:87-97 (1996); Sette, et al., J. Immunol. 153:5586-5592 (1994)). The amino acids at position 2 and the C terminus of a peptide are the primary contact or "anchor" residues that interact with the HLA-A2 binding pocket. In order to identify analogs for inclusion in a composition of the invention, anchor residues were modified by substitution with a presently preferred or less preferred anchor residue, at position 2 and/or at the C-terminus.

Another type of modification utilized involved the substitution of α-amino butyric acid (B) for endogenous cysteine (C) residues to avoid the potential complication of disulfide bridge formation during experimentation and development.

For example, two criteria that were used to select native peptides to be analoged: 1) presence of a suboptimal anchor residue; and 2) at least weak binding (IC₅₀ = 500-5000 nM) of the parent peptide to at least two or three alleles of a supertype.

Peptides can also be analoged by modification of a secondary anchor residue. For example, in preferred approaches, a peptide can be analoged by removal of a deleterious residue in favor of an acceptable or preferred one; an acceptable residue can be exchanged for a different acceptable residue or a preferred residue, or a preferred residue can be exchanged for another preferred one.

Accordingly, peptide sequences were modified using one or more of the strategies described above. The peptides were tested for HLA-A2 supertype binding using the molecular binding assay. Supertype-binding data for analog peptides are shown in Table 6.

### EXAMPLE 6

### CELLULAR IMMUNOGENICITY SCREENING

The peptides of the invention were also evaluated for their potential to stimulate CTL precursor responses to the TAA-derived peptide (*in vitro* primary CTL induction) and CTL recognition of tumor cells expressing the target TAA peptide epitope (recognition of endogenous targets). These criteria provided evidence that the peptides are functional epitopes.

### In Vitro Primary CTL Induction

Peripheral blood monocytic cell-derived (or bone-marrow-derived) human DC, generated *in vitro* using GM-CSF and IL-4 and pulsed with a peptide of interest, were used as antigen presenting cells (APCs) in primary CTL induction cultures. The peptide pulsed DC were incubated with CD8 T cells (positively selected from normal donor lymphocytes using magnetic beads) which served as the source of CTL precursors. One week after stimulation with peptide, primary cultures were tested for epitope-specific CTL activity using either a standard chromium-release assay which measures cytotoxicity or a sandwich ELISA-based interferon gamma (IFNγ) production assay. Each of the CTL epitopes of Table 6 stimulated CTL induction from CD8 T cells of normal donors.

### Recognition of Endogenous Targets

As described herein, T cell cultures testing positive for recognition of peptide-pulsed targets were expanded and evaluated for their ability to recognize human tumor cells that endogenously express the TAA. The chromium-release and IFNγ production assays were used for these evaluations, with tumor cell lines serving as the targets. Tumor cell lines lacking expression of either the TAA or the HLA-A2.1 molecule served as the negative control for non-specific activity. CTL cultures were generated which recognized tumor cells in a peptide-specific and HLA-A2-restricted manner (Table 6).

The HLA receptor binding and immunogenicity characteristics of CTL peptides are summarized in Table 6.

### EXAMPLE 7

### A PADRE^{®} MOLECULE AS A HELPER EPITOPE FOR ENHANCEMENT OF CTL INDUCTION

There is increasing evidence that HTL activity is critical for the induction of long lasting CTL responses (Livingston et al. J. Immunol 162:3088-3095 (1999); Walter et al., New Engl. J. Med. 333:1038-1044 (1995); Hu et al., J. Exp. Med. 177:1681-1690 (1993)). Therefore, one or more peptides that bind to HLA class II molecules and stimulate HTLs can be used in accordance with the invention. Accordingly, a preferred embodiment of a vaccine includes a molecule from the PADRE^{®} family of universal T helper cell epitopes (HTL) that target most DR molecules in a manner designed to stimulate helper T cells. For instance, a pan-DR-binding epitope peptide having the formula: aKXVAAZTLKAAa, where "X" is either cyclohexylalanine, phenylalanine, or tyrosine; "Z" is either tryptophan, tyrosine, histidine or asparagine; and "a" is either D-alanine or L-alanine (SEQ ID NO:29), has been found to bind to most HLA-DR alleles, and to stimulate the response of T helper lymphocytes from most individuals, regardless of their HLA type.

A particularly preferred PADRE^{®} molecule is a synthetic peptide, aKXVAAWTLKAAa (a = D-alanine, X = cyclohexylalanine), containing non-natural amino acids, specifically engineered to maximize both HLA-DR binding capacity and induction of T cell immune responses.

Alternative preferred PADRE^{®} molecules are the peptides, aKFVAAWTLKAAa, aKYVAAWTLKAAa, aKFVAAYTLKAAa, aKXVAAYTLKAAa, aKYVAAYTLKAAa, aKFVAAHTLKAAa, aKXVAAHTLKAAa, aKYVAAHTLKAAa, aKFVAANTLKAAa, aKXVAANTLKAAa, aKYVAANTLKAAa, AKXVAAWTLKAAA (SEQ ID NO:30), AKFVAAWTLKAA.A (SEQ ID NO:31), AKYVAAWTLKAAA (SEQ ID NO:32), AKFVAAYTLKAAA (SEQ ID NO:33), AKXVAAYTLKAAA (SEQ ID NO:34), AKYVAAYTLKAAA (SEQ ID NO:35), AKFVAAHTLKAAA (SEQ ID NO:36), AKXVAAHTLKAAA (SEQ ID NO:37), AKYVAAHTLKAAA (SEQ ID NO:38), AKFVAANTLKAAA (SEQ ID NO:39), AKXVAANTLKAAA (SEQ ID NO:40), AKYVAANTLKAAA (SEQ ID NO:41) (a = D-alanine, X = cyclohexylalanine).

In a presently preferred embodiment, the PADRE^{®} peptide is amidated. For example, a particularly preferred amidated embodiment of a PADRE^{®} molecule is conventionally written aKXVAAWTLKAAa-NH₂.

Competitive inhibition assays with purified HLA-DR molecules demonstrated that the PADRE^{®} molecule aKXVAAWTLKAAa-NH₂ binds with high or intermediate affinity (IC₅₀ ≤1,000 nM) to 15 out of 16 of the most prevalent HLA-DR molecules ((Kawashima et al., Human Immunology 59:1-14 (1998); Alexander et al., Immunity 1:751-761 (1994)). A comparison of the DR binding capacity of PADRE^{®} and tetanus toxoid (TT) peptide 830-843, a "universal" epitope has been published (Panina-Bordignon et al., Eur. J. Immunology 19:2237-2242 (1989)). The TT 830-843 peptide bound to only seven of 16 DR molecules tested, while PADRE^{®} bound 15 of 16. At least 1 of the 15 DR molecules that bind PADRE^{®} is predicted to be present in >95% of all humans. Therefore, this PADRE^{®} molecule is anticipated to induce an HTL response in virtually all patients, despite the extensive polymorphism of HLA-DR molecules in the human population.

PADRE® has been specifically engineered for optimal immunogenicity for human T cells. Representative data from *in vitro* primary immunizations of normal human T cells with TT 830-843 antigen and the PADRE^{®} molecule aKXVAAWTLKAAa-NH₂ are shown in Figure 1. Peripheral blood mononuclear cells (PBMC) from three normal donors were stimulated with the peptides *in vitro.* Following the third round of stimulation, it was observed that PADRE^{®} generated significant primary T cell responses for all three donors as measured in a standard T cell proliferation assay. With the PADRE^{®} peptide, the 10,000 cpm proliferation level was generally reached with 10 to 100 ng/ml of antigen. In contrast, TT 830-843 antigen generated responses for only 2 out of 3 of the individuals tested. Responses approaching the 10,000 cpm range were reached with about 10,000 ng/ml of antigen. In this respect, it was noted that PADRE^{®} was, on a molar basis, about 100-fold more potent than TT 830-843 antigen for activation of T cell responses.

Early data from a phase I/II investigator-sponsored trial, conducted at the University of Leiden (C.J.M. Melief), support the principle that the PADRE^{®} molecule aKXVAAWTLKAAa, possibly the amidated aKXVAAWTLKAAa -NH₂, is highly immunogenic in humans (Ressing et al., Detection of immune responses to helper peptide, but not to viral CTL epitopes, following peptide vaccination of immunocompromised patients with recurrent cervical carcinoma. (J. Immunother. 23(2):255-66 (2000)). In this trial, a PADRE^{®} molecule was co-emulsified with various human papilloma virus (HPV)-derived CTL epitopes and was injected into patients with recurrent or residual cervical carcinoma. However, because of the late stage of carcinoma with the study patients, it was expected that these patients were immunocompromised. The patients' immunocompromised status was demonstrated by their low frequency of influenza virus-specific CTL, reduced levels of CD3 expression, and low incidence of proliferative recall responses after *in vitro* stimulation with conventional antigens. Thus, no efficacy was anticipated in the University of Leiden trial, rather the goal of that trial was essentially to evaluate safety. Safety was, in fact, demonstrated. In addition to a favorable safety profile, PADRE^{®} T cell reactivity was detected in four of 12 patients (Figure 2) in spite of the reduced immune competence of these patients.

Thus, the PADRE^{®} peptide component(s) of the vaccine bind with broad specificity to multiple allelic forms of HLA-DR molecules. Moreover, PADRE^{®} peptide component(s) bind with high affinity (IC₅₀ ≤1000 nM), i.e., at a level of affinity correlated with being immunogenic for HLA Class II restricted T cells. The *in vivo* administration of PADRE^{®} peptide(s) stimulates the proliferation of HTL in normal humans as well as patient populations.

### EXAMPLE 8

### FUNCTIONAL COMPETENCE OF PROGP-DERIVED DC

One embodiment of a vaccine in accordance with the invention comprises epitope-bearing peptides of the invention delivered via dendritic cells (DC). Accordingly, DC were evaluated in both *in vitro* and *in vivo* immune function assays. These assays include the stimulation of CTL hybridomas and CTL cell lines, and the *in vivo* activation of CTL.

### DC Purification

ProGP-mobilized DC were purified from peripheral blood (PB) and spleens of ProGP-treated C57B1/6 mice to evaluate their ability to present antigen and to elicit cellular immune responses. Briefly, DC were purified from total WBC and spleen using a positive selection strategy employing magnetic beads coated with a CD11c specific antibody (Miltenyi Biotec, Auburn CA). For comparison, *ex vivo* expanded DC were generated by culturing bone marrow cells from untreated C57B1/6 mice with the standard cocktail of GM-CSF and IL-4 (R&D Systems, Minneapolis, MN) for a period of 7-8 days (Mayordomo et al., Nature Med. 1:1297-1302 (1995)). Recent studies have revealed that this *ex vivo* expanded DC population contains effective antigen presenting cells, with the capacity to stimulate anti-tumor immune responses (Celluzzi et al., J. Exp. Med. 83:283-287 (1996)).

The purities of ProGP-derived DC (100 µg/day, 10 days, SC) and GM-CSF/IL-4 *ex vivo* expanded DC were determined by flow cytometry. DC populations were defined as cells expressing both CD11c and MHC Class II molecules. Following purification of DC from magnetic CD11c microbeads, the percentage of double positive PB-derived DC, isolated from ProGP-troated mice, was enriched from approximately 4% to a range from 48-57% (average yield = 4.5 x 10⁶ DC/animal). The percentage of purified splenic DC isolated from ProGP treated mice was enriched from a range of 12-17% to a range of 67-77%. The purity of GM-CSF/IL-4 *ex vivo* expanded DC ranged from 31-41% (Wong et al., J. Immunother., 21:32040 (1998)).

### In Vitro Stimulation of CTL Hybridomas and CTL Cell Lines: Presentation of Specific CTL Epitopes

The ability of ProGP generated DC to stimulate a CTL cell line was demonstrated *in vitro* using a viral-derived epitope and a corresponding epitope responsive CTL cell line. Transgenic mice expressing human HLA-A2.1 were treated with ProGP. Splenic DC isolated from these mice were pulsed with a peptide epitope derived from hepatitis B virus (HBV Pol 455) and then incubated with a CTL cell line that responds to the HBV Pol 455 epitope/HLA-A2.1 complex by producing IFNγ. The capacity of ProGP-derived splenic DC to present the HBV Pol 455 epitope was greater than that of two positive control populations: GM-CSF and IL-4 expanded DC cultures, or purified splenic B cells (Figure 3). The left shift in the response curve for ProGP-derived spleen cells versus the other antigen presenting cells reveal that these ProGP-derived cells require less epitope to stimulate maximal IFNγ release by the responder cell line.

### EXAMPLE 9

### PEPTIDE-PULSED PROGP-DERIVED DC PROMOTE IN VIVO CTL RESPONSES

The ability of *ex vivo* peptide-pulsed DC to stimulate CTL responses *in vivo* was also evaluated using the HLA-A2.1 transgenic mouse model. DC derived from ProGP-treated animals or control DC derived from bone marrow cells after expansion with GM-CSF and IL-4 were pulsed *ex vivo* with the HBV Pol 455 CTL epitope, washed and injected (IV) into such mice. At seven days post immunization, spleens were removed and splenocytes containing DC and CTL were restimulated twice *in vitro* in the presence of the HBV Pol 455 peptide. The CTL activity of three independent cultures of restimulated spleen cell cultures was assessed by measuring the ability of the CTL to lyse ⁵¹Cr-labeled target cells pulsed with or without peptide. Vigorous CTL responses were generated in animals immunized with the epitope-pulsed ProGP derived DC as well as epitope-pulsed GM-CSF/IL-4 DC (Figure 4). In contrast, animals that were immunized with mock-pulsed ProGP-generated DC (no peptide) exhibited no evidence of CTL induction. These data confirm that DC derived from ProGP treated mice can be pulsed *ex vivo* with epitope and used to induce specific CTL responses *in vivo.* Thus, these data support the principle that ProGP-derived DC promote CTL responses in a model that manifests human MHC Class I molecules.

*In vivo* pharmacology studies in mice have demonstrated no apparent toxicity of reinfusion of pulsed autologous DC into animals.

### EXAMPLE 10

### MANUFACTURING OF SYNTHETIC PEPTIDES

### Physical/Chemical Properties of the Bulk A2 Vaccine Peptides

In one embodiment, each peptide of the invention is prepared by chemical synthesis and is isolated as a solid by lyophilization. Peptides are manufactured in compliance with Good Manufacturing Practices.

Bulk peptides of the invention, following identity and release testing, are formulated as an aqueous or non-aqueous solution, sterile filtered, and aseptically filled into sterile, depyrogenated vials. Sterile rubber stoppers are inserted and overseals applied to the vials. The vialed formulations undergo 100% visual inspection and specified release testing. The released vials are labeled and packaged before delivery for administration.

Table 6 summarizes the identifying source number, the amino acid sequence, binding data, and properties of CTLs induced by each peptide.

### EXAMPLE 11

### DENDRITIC CELL ISOLATION, PULSING, TESTING AND ADMINISTRATION

A presently preferred procedure for vaccination is set forth herein. In brief, patients are treated with ProGP to expand and mobilize DC into the circulation. On the day of peak DC mobilization, determined in accordance with procedures known in the art, patients undergo leukapheresis (approximately 15L process, possibly repeated once if required to collect sufficient mononuclear cells). The mononuclear cell product is admixed with peptides of the invention by injection through micropore filters (this admixing protocol is not needed if sterile peptides are used). After incubation and washing to remove residual unbound peptides, the cell product vaccine embodiment is resuspended in cryopreservative solution (final 10% DMSO) and, for those protocols involving multiple vaccination boosts, divided into aliquots. The pulsed mononuclear cell product(s) are frozen and stored according to accepted procedures for hematopoietic stem cells.

Vaccination is performed by injection or intravenous infusion of thawed cell product after the hematologic effects of ProGP in the patient have dissipated (*i.e.*, the hemogram has returned to baseline). Figure 5 provides a flow chart of *ex vivo* pulsing of DC with peptides, washing of DC, DC testing, and cryopreservation. A more detailed description of the process is provided in the following Examples.

### EXAMPLE 12

### ADMINISTRATION OF PROGP AND COLLECTION OF MONONUCLEAR CELLS BY LEUIKAPHERESIS

Patients are treated with ProGP daily by subcutaneous injection (dose and schedule determined in accordance with standard medical procedures). On the evening before leukapheresis, patients are assessed by an apheresis physician or nurse/technologist for adequacy of intravenous access for large-bore apheresis catheters. If peripheral venous access is deemed inadequate to maintain rapid blood flow for apheresis, then central venous catheters (inguinal, subclavian or internal jugular sites) can be inserted by appropriate medical/surgical personnel. On the day of predicted peak DC mobilization, leukapheresis (approximately 3 blood volumes or 15L) is performed, for example, on a Cobe Spectra or Fenwal CS3000 (flow rate ≥35mL/min) to obtain mononuclear cells. The number of DC in the leukapheresis product is estimated by flow cytometric counting of mononuclear cells possessing the immunophenotypes lin-/HLA-DR+/CD11c+ and lin-/HLA-DR+/CD 123+ in a 1mL sample aseptically withdrawn from the apheresis product. The numbers of granulocytes and lymphocytes in the leukapheresis product are counted by automated cytometry (CBC/differential). CBC/differential is performed immediately after the leukapheresis procedure and every other day for ten days to monitor resolution of the hematologic effects of the hematopoietin treatment and apheresis.

### EXAMPLE 13

### A PROCEDURE FOR DENDRITIC CELL PULSING

Plasma is removed from the leukapheresis product by centrifugation and expression of supernatant. The cells from the centrifugation pellet are resuspended in OptiMEM medium with 1% Human Serum Albumin (HSA) at a cell density of 10⁷ DC/ml in up to 100 ml.

The peptide(s) of the invention, preferably as individual sterile A2 peptide formulations, are administered directly into the DC culture bag through an injection port, using aseptic technique. After mixing, e.g., by repeated squeezing and inversion, the cell suspension is incubated for four hours at ambient temperature. Cryopreservative solution is prepared by dissolving 50 mL pharmaceutical grade dimethylsulfoxide (DMSO) in 200 mL Plasmalyte^{®}. After the pulsing period, the cell suspension is washed by centrifugation and resuspension in an equal volume of phosphate buffered saline solution. The washing procedure is repeated a defined number of times, *e.g.*, until studies validate that peptides have been removed. Samples of one milliliter each are removed for viability testing and microbiological testing. The cells are then prepared for freezing by centrifugation and resuspension in an equal volume of cryopreservative solution (final 10% DMSO). The cell suspension in cryopreservative is then divided into six equal aliquots, transferred to 50 ml freezing bags (Fenwal) and frozen at controlled rate of 1°C/min for storage in liquid nitrogen until needed for vaccination procedure.

### Assay to Evaluate the Pulsing Procedure

Antigen presenting cells, long-term stimulated T cells corresponding to peptides of the invention, or T cell hybridomas, are used to determine the optimal procedure for incubating the peptide reagents of a vaccine with human cells. Pulsing studies are done using one or more of the following cell sources: purified DC from ProGP treated HLA- A2.1 transgenic mice; human tumor cell lines that express HLA-A2; peripheral blood mononuclear cells from normal human volunteers; peripheral blood mononuclear cells from ProGP treated patients; and/or DC obtained from normal human HLA-A2 volunteers following the *ex vivo* culture of their peripheral blood mononuclear cells with GM-CSF and IL-4.

Evaluated conditions include, *e.g.*:
Cellular isolation procedure and cell number
Concentration of vaccine peptides
Washing conditions to remove ancillary reagents
Post-pulsing manipulations (resuspension, freezing)

Accordingly, these studies demonstrate the ability of the procedure to produce functional HLA-A2/peptide complexes on the surface of the human cells. The validation of the pulsing procedure is established using HLA-A2.1-specific T cell lines after which the Phase I clinical trial occurs.

This Example may also be performed using A1, A3, A24, B7 or B44-restricted peptides by substituting appropriate HLA-related reagents. It will be clear to one of skill in the art how to make such substitutions.

### EXAMPLE 14

### VALIDATION OF PEPTIDE REMOVAL FROM THE DC PRODUCT

Following pulsing with the peptide reagents, DC from the patient are washed several times to remove excess peptides prior to infusing the cells back into the patient. In this embodiment of a vaccine of the invention, the washing procedure removes unbound peptides. Accordingly, there is no, or negligible, systemic exposure of the patient to the peptides. Alternative vaccines of the invention involve direct administration of peptides of the invention to a patient, administration of a multiepitopic polypeptide comprising one or more peptides of the invention, administration of the peptides in a form of nucleic acids which encode them, *e.g.*, by use of minigene constructs, or by viral vectors.

### Assay for Vaccine Peptides in the Dendritic Cell Wash Buffer

After the DC are incubated with the peptides, the cells are washed with multiple volumes of wash buffer. An aliquot of the last wash is placed onto a nonpolar solid-phase extraction cartridge and washed to reduce the salt content of the sample. Any peptides contained in the buffer will be eluted from the extraction cartridge and evaporated to dryness. The sample is then reconstituted in High Performance Liquid Chromatography (HPLC) mobile phase, injected onto a polymer based reverse-phase HPLC column, and eluted using reverse-phase gradient elution chromatography. Residual peptides are detected using a mass spectrometer set-up to monitor the protonated molecular ions of each peptide as they elute from the HPLC column.. The peptides are quantified by comparing the area response ratio of analyte and internal standard to that obtained for standards in a calibration curve.

### EXAMPLE 15

### VALIDATION OF TRIFLUOROACETIC ACID REMOVAL FROM THE DC PRODUCT

In a particular embodiment, peptide reagents may be formulated using 0.1% trifluoroacetic acid (TFA). The washing procedure developed to remove residual peptide also removes residual TFA.

### EXAMPLE 16

### DENDRITIC CELL RELEASE TESTING

### Identity

The number of DC in the leukapheresis product is estimated by flow cytometric counting of mononuclear cells possessing the immunophenotypes lin⁻/HLA-DR⁺/CD11c⁺ and lin⁻/HLA-DR⁺/CD123⁺ in a 1 ml sample aseptically withdrawn from the apheresis product. Lin⁻ cells excludes monocytes, T-lymphocytes, B-lymphocytes, and granulocytes, by using a cocktail of antibodies to lineage markers CD3, CD14, DC16, CD19, CD20, CD56.

### Cell Viability

Viability of mononuclear cells is assessed after pulsing and washing, prior to suspension in cryopreservative, by trypan blue dye exclusion. In general, if the cell product contains more than 50% trypan blue-positive cells, the product is not administered to a patient.

### Microbiological Testing

The cell suspension in cryopreservative is examined for microbial contamination by gram stain and routine clinical bacterial and fungal culture/sensitivity. If tests are positive for bacterial or fungal contamination, implicit evidence of significant contamination, the product is not infused. If, e.g., a gram stain is negative, the product may be infused for the first vaccination while awaiting results of culture/sensitivity. Antibiotic therapy based on culture results is instituted at the discretion of the treating physician if the patient shows appropriate signs of infection that could be clinically attributable to the infused contaminant.

### EXAMPLE 17

### PATIENT VACCINATION

In a preferred embodiment, an aliquot of frozen pulsed dendritic cell product is removed from a liquid nitrogen freezer and kept frozen in an insulated vessel containing liquid nitrogen during transport to the infusion site. The product is thawed by immersion with gentle agitation in a water bath at 37°C. Immediately on thawing, the cell suspension is infused through intravenous line by gravity or by syringe pump. Alternatively, the vaccine is administered by injection, *e.g.*, subcutaneously, intradermally, or intramuscularly. The patient's vital signs are monitored before infusion/injection and at 5 minute intervals during an infusion, then at 15 minute intervals for 1 hour after infusion/injection.

Infusion protocols in accordance with knowledge in the art are carried out for alternative vaccine embodiments of the invention, such as direct peptide infusion or nucleic acid administration.

### EXAMPLE 18

### AN A2 VACCINE

A vaccine in accordance with the invention comprises eight peptide epitopes bearing the HLA-A2 supermotif. Collectively, these eight epitopes are derived from the tumor associated antigens (TAAs) HER2/neu, p53, MAGE 2, MAGE3, and carcinoembryonic antigen (CEA), and stimulate CTL responses to these TAAs. (see Table 9) These eight peptides, which are also presented in Table 6, bear an HLA-A2 supermotif. Optionally, a ninth peptide, an HTL epitope that enhances CTL responses such as a pan-DR-binding peptide (PADRE^{®}, Epimmune, San Diego, CA), is included.

The eight HLA-A2 peptide components of the A2 vaccine bind to multiple HLA-A2 superfamily molecules with high or intermediate affinity (IC₅₀ ≤ 500 nM). HLA-A2-specific analog and native peptide components of the A2 vaccine stimulate CTL from the peripheral blood of normal human volunteers. These CTL recognize native peptides that have been pulsed onto HLA-A2 expressing APCs, as well as endogenous peptides presented by HLA-matched tumor cell lines. Thus, the A2 vaccine is effective in stimulating the cellular arm of the immune system to mediate immune responses against tumors.

It is to be appreciated that vaccines comprising peptides bearing other motifs, or nucleic acids encoding such peptides, are also used in accordance with the principles set forth herein, and are within the scope of the present invention.

In a preferred embodiment, an A2 vaccine comprises DC pulsed *ex vivo* with the nine peptides. This embodiment of a vaccine can be used with progenipoietin (ProGP)-mobilized DC.

### EXAMPLE 19

### AN A2 VACCINE

An A2 vaccine comprises a cocktail of 12 peptides, 10 of which stimulate CTL responses to the tumor associated antigens (TAA) HER2/neu, p53, MAGE 2/3, and carcinoembryonic antigen (CEA). The remaining two peptides are both members of the PADRE^{®} family of peptides that are HTL epitopes that enhance CTL responses (see Table 10). This embodiment of an A2 Vaccine is used in combination with an emulsion-based adjuvant such as Montanide® ISA51 or ISA720 (Seppic, Paris, France) or an Incomplete Freund's Adjuvant, preferably administered by injection. As appreciated by those of skill in the art, alternative modes of administration can also be used. Many adjuvants are known in the art, and are used in accordance with the present invention, see, *e.g.,* Tomlinson, et al., Advanced Drug Delivery Reviews, Vol. 32(3) (6 July 1998).

The eight HLA-A2 CTL peptide components of this vaccine embodiment bind to multiple HLA-A2 superfamily molecules with high or intermediate affinity (IC₅₀ ≤ 500 nM). The HLA-A2-specific analog and native peptide components of the present vaccine stimulate CTL from patient's blood. These CTL recognize native peptides that were pulsed onto HLA-A2 expressing APCs, as well as endogenous peptides presented by HLA-matched tumor cell lines.

Two peptides that stimulate HLA class II are also used in accordance with the invention. For instance, a pan-DR-binding epitope peptide having the formula: aKXVAAZTLKAAa, where "X" is either cyclohexylalanine, phenylalanine, or tyrosine; "Z" is either tryptophan, tyrosine, histidine or asparagine; and "a" is either D-alanine or L-alanine (SEQ ID NO:29), has been found to bind to most HLA-DR alleles, and to stimulate the response of T helper lymphocytes from most individuals, regardless of their HLA type. Two particularly preferred PADRE^{®} molecules are the peptides, aKFVAAYTLKAAa-NH₂ and aKXVAAHTLKAAa-NH₂ (a = D-alanine, X = cyclohexylalanine), the latter containing a non-natural amino acid, specifically engineered to maximize both HLA-DR binding capacity and induction of T cell immune responses.

The PADRE^{®} peptide components of the A2 vaccine bind with high affinity and broad specificity to multiple allelic forms of HLA-DR molecules (IC₅₀ ≤1000 nM). The *in vivo* administration of PADRE^{®} peptide stimulates the proliferation of HTL in normal humans as well as patient populations. Thus, this vaccine embodiment is effective in stimulating the cellular arm of the immune system to mediate immune responses against tumors.

### EXAMPLE 20:

### IDENTIFICATION OF HLA-A1, -A3, -A24 AND -B7 MOTIF BEARING PEPTIDES

Cytotoxic T cells (CTLs) play a major role in anti-tumor immune responses by directly lysing tumor cells and also by secreting cytokines such as interleukin-2, TNFα (tumor necrosis factor), GM-CSF (granulocyte-macrophage colony stimulating factor) and interferon gamma (IFNγ) which can contribute to the anti-tumor effect. These CTLs recognize small peptides, 8-11 amino acids long, that are derived from antigens expressed specifically by tumor cells and bound to MHC Class I molecules (Zinkernagel, 1997; York, 1996; Rammensee, 1993). The role of CTLs in tumor regression has been documented in both mouse models and patients. The CTLs constitute a major component of immune lymphocytes infiltrating tumor sites (TIL cells). These cells have been associated with spontaneous tumor regression in humans (Zorn, 1999). *In vivo* CTL induction in transgenic mice gives rise to CTLs that recognize tumor cells resulting in tumor regression. Toes et al (1996) and Vierboom et al (1997) also performed adoptive transfer experiments in mice and observed protection from tumor development. Adoptive transfer experiments in humans have also demonstrated the efficacy of anti-tumor CTL (Greenberg, 1991; Kawakami, 1995). Human trials have demonstrated that epitope-specific CTLs can be induced in cancer patients and in several instances correlated their induction with partial or complete tumor responses (Murphy, 1996; Nestlé, 1998; Rosenberg, 1998).

An important first step in the development of a cancer vaccine is the identification of these peptide epitopes from tumor-associated antigens (TAA). There are numerous tumor-associated antigens expressed by tumor cells and a tumor cell may express multiple epitopes from several TAA (Tsang, 1995; Rongcun, 1999; Soussi, 1996). Several investigators (Shu, 1997; Wang, 1997; Gilboa, 1999 and Berlyn, 1999) have attempted to categorize the tumor antigens identified to date. Briefly, these are differentiation antigens that correspond to normal tissue-specific gene products such as tyrosinase, gp100 and MART-1; mutations of tumor suppressor genes such as p53, ras and bcr/abl; overexpressed normal or embryonic gene products represented by MAGE (a family of melanoma associated antigens), Her2/neu and CEA; and antigens derived from viruses such as human papilloma virus. Tumor cells may be expressed by more than one TAA, and a tumor cell may express multiple epitopes from a particular TAA (Van den Eynde, 1989; Tsang, 1995; Rongcun, 1999; Soussi, 1996).

For the purpose of developing a broadly effective cancer vaccine, four TAA (CEA, HER2/neu, MAGE2/3, and p53) expressed by many tumors such as colon, breast, lung and gastric cancers, and in the case of MAGE, some melanomas, were selected. The use of multiple TAA should address the potential problem in developing a cancer immunotherapeutic; namely, that tumor escape can occur through the selection of antigen-negative variants (Boon, 1989a,b; Melief, 1989).

Using peptide epitopes in a vaccine composition has distinct advantages over using whole antigen. The whole antigen may include immunosuppressive epitopes or might have undesired intrinsic biological activity. An additional advantage to an epitope-based vaccine is the ability to combine both CTL and helper epitopes, or epitopes from multiple TAA or HLA types into a single formulation.

One obstacle in the development of CTL epitope based vaccines is the large degree of MHC polymorphism (Sette, 1998). The Class I MHC displaying these epitopes in humans are termed human leukocyte antigens, or HLA. While there are over 125 HLA Class I molecules, it has been discovered that most can be grouped into one of several families or "supertypes" based on their ability to bind similar repertoires of peptides. Nine major class I supertypes have been described (Sette and Sidney, 1999). Of these, the five most prevalent are HLA-A2.1, -A3, -B7, -A1 and -A24. Together these 5 supertypes cover, on average, 98.8 % of the Caucasian, African American, Japanese, Chinese and Hispanic populations (Table 1a). The A2 superfamily and the corresponding peptide motifs have been characterized elsewhere (Sette, 1998). Therefore, this work will focus on HLA-A3, -B7, -A1 and - A24. The A3 superfamily comprises A*03, A*11, A*3101, A*3301 and A*6801, of which A*03 and A*11 are the most predominant. The A3 supertype provides an average coverage of 44.2% amongst the 5 major ethnicities: Caucasian, Black, Japanese, Chinese and Hispanic populations (Table 11, Sidney, 1996a). A B7 superfamily has also been identified and ccomprises HLA-B*0702, B*3501-3, B*51, B*5301 and B*5401, and has an average population coverage of 44.7% (Table 11, Sidney, 1996b). Work done at Epimmune and by others has demonstrated that many peptides exhibit degenerate (crossreactive) binding (Tanigaki, 1994; del Guercio, 1995; Sidney, 1995; Sidney, 1996b) which would allow us to identify supertype cross-reactive epitopes that would extend the breadth of coverage.

Until binding assays for all of the HLA-A1 and -A24 superfamily alleles are developed, identification of HLA-A1 and -A24 restricted candidate peptides relies on motif analysis and binding affinity assays for the primary alleles, A*0101 and A*2402. These 2 alleles would provide average population coverage of 11.9% and 28.7%, respectively (Table 11). HLA-A*01 increases coverage of black, Chinese, and Hispanic populations and A*24 provides significant coverage of the Asian and Hispanic populations.

Analysis of HCV-derived peptides revealed that peptides binding the predominant allele of the supertype (i.e. A*0201 for the A2 supertype and A*0301 for the A3 supertype) with an IC₅₀ ≤100nM showed cross-reactive binding and were recognized by infected patients. Hepatitis B virus-derived peptides that fit the same criteria were also demonstrated to be immunogenic 89% of the time either in transgenic mice, HBV-infected patients, or human primary PBL cultures (data not shown). A significant body of work was done with infectious disease antigens that demonstrated that immunogenicity could be predicted on the basis of binding affinity of ≤500nM (Sette, 1994a; Wentworth, 1996, Alexander, 1997) and supertype cross-reactivity (Threlkeld, 1997; Bertoni, 1997; Doolan, 1997; Scognamiglio, 1999). A correlation between binding affinity and immunogenicity was also demonstrated for HLA-A2-restricted TAA (Keogh, et al. J. Immunol. 167(2):787-91 (2001)). In the case of TAA-derived wildtype peptides, binding affinity ≤200nM and supertype cross-reactivity were highly predictive of endogenous recognition (75%). The same success rate was achieved with analogs when primary immunogenicity was considered in addition to binding affinity and supertype binding.

Based on this knowledge, the principal strategy for epitope identification has been to first identify candidate peptides in the wildtype antigen sequence by their MHC binding motif, then to determine their binding affinity and supertype crossreactivity (DiBrino, 1993; Sette, 1994b). High affinity, crossreactive peptides are then tested for *in vitro* immunogenicity with PBMCs from normal donors and their ability to induce tumor-reactive CTLs (Celis, 1994a; Celis, 1994b; Kawashima, 1998, Feltkamp, 1994).

An extension of this strategy adopted to identify peptides for potential inclusion in vaccines is the development of primary anchor-substituted analogs (Ruppert, 1993). This strategy involves the identification of peptides carrying suboptimal residues at their primary anchor positions, and the replacement of one or more of these suboptimal residues with optimal anchor residues to enhance binding affinity to the predominant allele of the superfamily and/or crossreactivity to the other alleles in the superfamily. This strategy has been used to generate analogs of A2 restricted peptides (Keogh, et al. J. Immunol. 167(2):787-96 (2001)). The preferred and tolerated amino acids at each anchor position for A3, A1, A24 and B7 binding peptides have been identified in order to formulate analoging strategies for these alleles. The results of those efforts are briefly summarized herein and in Sidney, et al. Hum. Immunol. 62(11):1200-16 (2001).

The use of analogs is also relevant to address the problem of expanding the number of potential epitopes of a given tumor antigen, particularly in the case of small proteins such as p53. In addition, broadly crossreactive supertype binding analogs increase population coverage of a given epitope. Analogs can also be used to enhance the immunogenicity of known epitopes. Another advantage is to increase peptide manufacturability and stability by substituting, for instance, α-aminobutyric acid (B) for cysteine (Sette, Persistent Viral Infections, review).

Sarobe (1998), Vierboom (1998) and Irvine (1999) demonstrated that A2-restricted, anchor- analoged epitopes derived from TAA and infectious disease antigens showed improved immunogenicity in mice. Other investigators have demonstrated (Rosenberg, 1998; Zaremba, 1997) that when analogs with binding better than the corresponding wildtype peptide were used to stimulate cells from cancer patients *in vitro,* a peptide-specific CTL response was detected after far fewer restimulations than were required with the wildtype peptide (Rosenberg, 1998; Zaremba, 1997). Most importantly, tumor killing was also observed. Based on these results, a much stronger CTL response would be anticipated *in vivo.* Additionally, in a clinical trial, Rosenberg et al (1998) have observed tumor regression with a melanoma analog in conjunction with IL2 therapy, demonstrating the value of analog peptides as immunotherapeutics. Similar results were obtained with fixed anchor analogs and PBMCs from normal donors (data not shown). Those results clearly demonstrated that these peptides are strong immunogens capable of generating wildtype peptide and tumor cell reactive CTLs.

There has been little information in the literature describing TAA-derived epitopes for the non-A2 alleles (A3, B7, A1 and A24). Our strategy is to identify novel peptide epitopes that demonstrate high HLA-A*03, -B*07, -A*01 or -A*24 binding affinity and supertype binding where applicable in order to elicit a strong CTL response. Kawashima (1998); Castelli (1998), Kittlesen (1998), Tahara (1999) and others have demonstrated CTL responses to tumor epitopes in normal donors or cancer patients, which would indicate that tolerance is incomplete. It appears that immune tolerance at the CTL level does not completely eliminate or inactivate CTL precursors capable of recognizing high affinity class I peptides.

Here we report the identification of HLA-A3, -A1, -A24 and -B7- restricted vaccine candidate peptides from the CEA, MAGE2/3, HER2/neu and p53 tumor antigens. Candidates selected for *in vitro* immunogenicity assays on the basis of their HLA affinity and supertype cross-reactivity were also demonstrated to be immunogenic and capable of inducing CTL that recognize tumor target cells.

### MATERIALS AND METHODS

### MHC Sources

The following EBV-transformed cell lines were used as sources of class I major histocompatibility complex molecules: Steinlin (A*0101), AMAI (B*5301), GM3107 (A*0301, B*0702), BVR (A*1101), SPACH (A*3101), LWAGS (A*3301), KAS116 (B*51), and KT3 (A*2402, B*5401). A C1R transfectant was used for the isolation of A*6801, as well as for B*3501. These C1R transfectants were characterized by Dr. Walter Storkus and Dr. Masafumi Takaguchi, respectively.

Cells were maintained *in vitro* by culture in RPMI 1640 medium supplemented with 2mM L-glutamine and 10% heat-inactivated FCS. Cells were also supplemented with 100µg/ml of streptomycin [Irvine Scientific, Santa Ana, CA] and 100U/ml of penicillin [Life Technologies, Carlsbad, CA]. Large quantities of cells were grown in spinner cultures.
Affinity purification of HLA-A and -B molecules.

Cells were lysed at a concentration of 10⁸ cells/ml in PBS containing 1% NP-40 and 1mM PMSF. The lysates were cleared of debris and nuclei by centifugation at 10,000x *g* for 20min.

MHC molecules were then purified by affinity chromatography as previously described (Sette, 1998; Ruppert, 1993). Columns of inactivated Sepharose CL4B and Protein A Sepharose were used as pre-columns. Lysates were filtered through 0.8 and 0.4µM filters and then depleted of HLA-B and HLA-C molecules by repeated passage over Protein A Sepharose beads conjugated with the anti-HLA (B,C) antibody B1.23.2. Typically 2 to 4 passages were required for effective depletion. Subsequently, the anti-HLA (A,B,C) antibody W6/32 was used to capture HLA-A molecules.

Independently, both antibody columns were washed with 15-column volumes of 10mM TRIS in 1.0% NP-40, PBS and 2-column volumes of PBS containing 0.4% n-octylglucoside. Finally, the class I molecules were eluted with 50mM diethylamine in 0.15M NaCl containing 0.4% n-octylglucoside, pH 11.5. A 1/25 volume of 2.0M Tris; pH 6.8, was added to the eluate to reduce the pH to ~8.0, and then concentrated by centrifugation in Centriprep 30 concentrators (Amicon, Beverly, MA) at 2000rpm. Protein purity, concentration, and effectiveness of depletion steps were monitored by SDS-PAGE and BCA protein analysis (Sigma).
Class I peptide-binding assays.

Purified human class I molecules [5 to 500nM] were incubated with 1-10nM ¹²⁵I-radiolabeled probe peptide, iodinated by the Chloramine T method (Buus; 1987), for 48h at room temperature in the presence of 1 µM human β2M (Scripps Laboratories, San Diego, CA) and a cocktail of protease inhibitors. The final concentrations of protease inhibitors were: 1mM PMSF, 1.3nM 1.10 phenanthroline, 73µM pepstatin A, 8mM EDTA, and 200µM N alpha-tosyl-lysine chloromethyl ketone (TLCK).

Class I peptide complexes were separated from free peptide by gel filtration on TSK200 columns, and the fraction of bound peptide calculated as previously described (Sette, 1998). In preliminary experiments, the HLA class I preparation was titered in the presence of fixed amounts of radiolabeled peptides to determine the concentration of class I molecules necessary to bind 10-20% of the total radioactivity. All subsequent inhibition and direct binding assays were then performed using these class I concentrations. In the inhibition assays, peptide inhibitors were typically tested at concentrations ranging from 120µg/ml to 1.2ng/ml. The data were then plotted and the dose yielding 50% inhibition was measured. Peptides were tested in two to four completely independent experiments. Since under these conditions [label]<[MHC] and IC₅₀ ≤[MHC], the measured IC₅₀s are reasonable approximations of the true k_{D} values.

Radiolabeled probe and standard control peptides used are as follows:

An A3 non-natural consensus peptide (A3con; sequence KVFPYALINK) (SEQ ID NO: 747) (Sette 1994b; Kubo, 1994) was used as the radiolabeled probe for the A3, A11, A31, and A*6801 assays. An HBV 141.Y7 analog of HBVc 141-151 (sequence STLPETYVVRR) (SEQ ID NO: 748) was used as the radiolabeled probe for the A*3301 assay. The average IC₅₀'s of A3con for the A3, A11, A31, and A*6801 assays were 11nM, 6.0nM, 18nM, and 8.0nM, respectively. The average IC₅₀ of the HBVc 141-151 peptide in the A*3301 assay was 29nM.

B35con2 (sequence FPFKYAAAF) (SEQ ID NO: 749) was used as the radiolabeled probe and standard control peptide for the B*3501, B*5101, B*5301, and B*5401 assays. The IC₅₀'s of B35con2 for each of these assays were 7.2nM, 5.5nM, 9.3nM, and 10nM, respectively. The A*0201 Signal Sequence 5-13a.Y7 (APRTLVYLL) (SEQ ID NO: 750) (Huczko, 1993; Chen, 1994; Sidney, 1996a,b) was used as the radiolabeled probe and standard peptide for B*0702 assay. It had an average IC₅₀ of 5.5nM.

The human J chain peptide (sequence YTAVVPLVY) (SEQ ID NO: 751) was used as the radiolabeled probe for the A1 assay, utilizing an A1con peptide (sequence YLEPAIAKY) (SEQ ID NO: 752) as the standard control. It had an average IC₅₀ of 25nM. The A24con peptide (sequence AYIDNYNKF) (SEQ ID NO: 753) was used as the radiolabeled probe and standard control peptide for the A24 assay, having an average IC₅₀ of 12nM.
Peptide Synthesis.

Peptides were either synthesized at Epimmune, Inc. (San Diego, CA), as previously described (Sette, 1998), or, for large epitope libraries, purchased as crude material from Chiron Technologies Corp (Clayton, Victoria, Australia). Peptides synthesized at Epimmune were purified to >95% homogeneity by reverse-phase HPLC. The purity of these synthetic peptides was determined on an analytical reverse-phase column and their composition ascertained by amino acid analysis and/or mass spectrometry analysis.

### Identification of motif-positive peptides.

### HLA-A3 supertype

Protein sequences from the targeted four tumor antigens (p53, CEA, HER2/neu, and MAGE2/3) were scanned, utilizing a customized program, to identify 8-, 9-, 10-, and 11-mer sequences containing the HLA-A3 supertype main anchor motif. That motif is leucine (L), isoleucine (I), valine (V), methionine (M), alanine (A), serine (S) or threonine (T) at position 2, and either lysine (K) or arginine (R) at the C-terminus.

Naturally-occurring, or wild-type (WT) peptides identified as described above were tested for their capacity to bind purified HLA-A*0301 and A*1101 molecules *in vitro.* Peptides exhibiting high (IC₅₀ ≤50nM) or intermediate binding affinity (IC₅₀ 51-500nM) for either one or both of these primary A3 supertype alleles were then tested on other predominant molecules of the A3 supertype family (A*3101, A*3301, and A*6801) (Sidney, 1996a). Peptides binding at least three of the five alleles tested were classified as "crossbinders", and candidates for cellular screening analysis. The rationale for having co-primary alleles for the A3 supertype is based on similar peptide motifs as well as the dichotomy of these alleles' population coverage. A*0301 has the highest phenotypic frequencies for Caucasians and North American blacks, and A*1101 has the best coverage for Asians.

### HLA-B7 supertype

The targeted TAA sequences were scanned to identify 8-, 9-, 10-, and 11-mer sequences containing proline (P) at position 2, and L, I, V, M, A, phenylalanine (F), tryptophan (W), or tyrosine (Y) at the C-terminus (HLA-B7 supertype motif).

These peptides were tested for their capacity to bind purified HLA-B*0702 molecules *in vitro.* Peptides exhibiting high or intermediate binding affinity were then tested on other predominant molecules of the B7 supertype family (B*3501, B*5101, B*5301, and B*5401) (Sidney, 1995). Peptides that are B7 supertype crossbinders were candidates for cellular screening analysis.

### HLA-A1 and HLA-A24 motifs

For A24 sequences, peptides carrying Y, F, W, or M at position 2, and F, I, L, or W at the C terminus were identified as motif positive. The A1 motif is somewhat unique in that it possesses a dual primary anchor motif; one at position 2 and the C terminus, another at position 3 and the C terminus (Kubo, 1994; Kondo, 1997). The residues associated with the A1 motif are T, S, or M at position 2, aspartic acid (D), glutamic acid (E), A, or S at position 3, and Y at the C terminus.

Peptides were tested for their capacity to bind the appropriate purified HLA-A1 and A24 motif carrying molecule *in vitro.*

### Definition of distinct binding regions

When a protein sequence is scanned for the presence of motif-positive peptides, it is not uncommon for several peptides to be identified within a few residues of each other. Often times, a 9-mer peptide is nested within a 10- or 11-mer sequence. It is possible that some overlap exists amongst responses elicited by such nested or overlapping epitopes, and we thereby consider such epitopes as closely related. For vaccine development, we generally recommend inclusion of only one epitope from each such family of overlapping peptides. On the other hand, we define distinct regions as peptides having a first position at least 4 residues apart. It is safe to assume that upon binding to the MHC, these peptides would induce responses from different T cell receptors (TCR), and should therefore be considered as distinct for the purpose of epitope selection.
Primary anchor position residue substitution strategy.

It has been shown that class I peptide ligands can be modified to increase their binding affinity and/or degeneracy (Sidney, 1996b; Rosenberg, 1998). More importantly, modified peptides have also been shown to possess increased immunogenicity and crossreactive recognition by T cells specific for the WT epitope (Parkhurst, 1996; Pogue, 1995). This modification, sometimes referred to as "fixing", entails analoging peptides by replacing sub-optimal amino acids at primary anchor positions for optimal residues. What residues are optimal is dependent upon the allele under examination. This strategy was successfully employed in the A2 system as part of an effort to develop an A2 therapautic cancer vaccine (data not shown).

For the various supertypes and alleles addressed in this study, motifs are listed in Table 2. HLA binding data from peptides selected using these motifs was analyzed to develop a strategy for analoging TAA-derived peptides with suboptimal anchor residues at anchor positions.

For the A3 supertype, residues L, I, M, A, or S are suboptimal at position 2 and can be substituted with T or V. At the C terminus, both R and K are canonical residues, each displaying a propensity towards specific alleles within the A3 supertype with lysine preferred by A*0301 and A*1101, arginine is preferred by A*3101, A*3301, and A*6801.

For the B7 supertype, proline is absolutely required at position 2 and therefore only the C terminus is a candidate for analoging in terms of primary anchors. Residues L, M, A, V, F, W, or Y are suboptimal, and can be substituted with I. Additionally, we have observed that the presence of a bulky aromatic, specifically phenylalanine (F), at position 1 of a B7 supermotif peptide can significantly increase B7 binding and crossbinding capacity (Table 15).

For the A24 motif, residues F, W, or M at position 2 are suboptimal and should be substituted with Y. At the C terminus, I, L, or W are suboptimal and should be substituted with F.

The A1 allele is associated with a dual primary anchor motif (or 2 submotifs). For one submotif, T is preferred over S and M at position 2. The second submotif prefers D over E, A, and S at position 3. Additionally, Y is the optimal residue at the C terminus for both submotifs, but substituting for A, F, or W if both T₂ and D₃ are present is a viable alternative.

In addition, WT peptide candidates for analoging must exhibit at least weak binding (IC₅₀ of ≤5000nM) to the parent allele (A1 or A24), or weakly bind at least 3 of 5 alleles (A3 and B7 supertypes). The rationale for this requirement is that the WT peptide must be present endogenously in sufficient quantity to be biologically relevant.

Another analog utilized in these studies, unrelated to the primary anchor position, involves the substitution of α-amino butyric acid (B) for cysteine (C). Due to its chemical nature, cysteine has the propensity to form disulfide bridges and sufficiently alter the peptide structurally so as to reduce binding capacity. Substituting B for C not only alleviates this problem, but has been shown to actually improve binding and crossbinding capability in certain instances (Review: Sette, Persistent Viral Infections, Ed. R. Ahmed and I. Chen, John Wiley & Sons, England).

### Target Cell Lines for HLA-A3 and -B7 Cellular Screening

The Epstein-Barr transformed homozygous cell lines EHM (A3+, ASHI cell repository, currently inactive) or GM3107 (A3+, B7+; Human Genetic Mutant Repository) were used as the peptide-loaded target to measure activity of HLA-A3-restricted CTL. The JY cell line (B7+, a gift from L. Sherman at The Scripps Research Institute) was used as the peptide-loaded target cells to measure activity of HLA-B7-restricted CTL. The negative and positive tumor target cell lines used for each antigen were: SW480 (A3-, CEA+) (ATCC No. CCL-228) and SW403 (A3+, CEA+) (ATCC No. CCL-230) for CEA; SW480 (A3-, HER2/neu+) and SW403 (A3+, HER2/neu+) for HER2/neu; 938mel (A3-, MAGE2/3+) and 624mel (A3+, MAGE2/3+) for MAGE2/3; and SW403 (A3+, p53-) and SW403 transfected with p53 (A3+, p53+) for p53. The HLA-typed melanoma cell lines (624mel and 938mel) were a generous gift from Y. Kawakami and S. Rosenberg, National Cancer Institute, Bethesda, MD: The tumor cell lines, SW403 and SW480 were obtained from the American Type Culture Collection (Manassas, VA). The EBV transformed and melanoma cell lines were grown in RPMI-1640 medium supplemented with antibiotics, sodium pyruvate, nonessential amino acids and 10% (v/v) heat inactivated FCS. SW403 and SW480 were grown in DMEM with the same additives. The tumor target cells were treated with 100U/ml IFNγ (Genzyme) for 48 hours at 37°C prior to use as targets in the ⁵¹Cr release and *in situ* IFNγ assays.

### Primary CTL Induction Cultures

Generation of dendritic cells (DC): Monocytes were purified from previously frozen PBMCs by plating 10 X 10⁶ cells in 3 ml of complete medium (RPMI with 5% heat-inactivated human AB serum, penicillin, streptomycin, sodium pyruvate and non-essential amino acids) in each well of a 6-well plate. After 2 hrs at 37°C, the non-adherent cells were removed and three ml of complete medium containing 50 ng/ml of human rGM-CSF and 1,000U/ml of human rIL-4 were then added to each well. On day 6, the non-adherent cells were harvested, washed and cultured at 0.3-0.5x10⁶ cells/ml in complete medium with 75ng/ml TNFα (R&D Systems).

On day 8, the DC were collected, washed, and pulsed with 40µg/ml of peptide at a cell concentration of 1-2x10⁶/ml in the presence of 3µg/ml β₂microglobulin for 4 hours at 20°C. The DC were then irradiated (4,200 rads), washed 1 time with medium and counted again.

Induction of CTL with DC and Peptide: CD8+ T-cells were isolated by positive selection with Dynal immunomagnetic beads and detachabead reagent according to the manufacturer's instructions. Typically 200~250x10⁶ PBMC were processed to obtain 24x10⁶ CD8+ T cells (enough for a 48-well plate). 0.25ml of CD8+ T- cells (@ 2x10⁶ cell/ml) were co-cultured with

0.25 ml cytokine-generated DC (@1x10⁵ cells/ml) in each well of a 48-well plate in the presence of 10 ng/ml human rIL-7 (Endogen). Human rIL10 (Endogen) was added the next day at a final concentration of 10 ng/ml and human rIL2 was added on day 2 at 10IU/ml.

Restimulation of the induction cultures with peptide-pulsed adherent cells: Seven and fourteen days after the primary induction, the cells were restimulated with irradiated, peptide-pulsed adherent cells. Briefly, adherent cells were pulsed with 10µg/ml of peptide in the presence of 3µg/ml β₂microglobulin in RPMI/5% human AB serum for 2 hours at 37°C. The wells were washed once with RPMI. Most of the medium was aspirated from the induction cultures (CD8+ cells) and brought to 0.5 ml with fresh medium and the cells were transferred to the wells containing the peptide-pulsed adherent cells. Human rIL10 was added at a final concentration of 10 ng/ml 24 hours later and human rIL2 was added after 48 hours and again 2-3 days later at 50IU/ml (Tsai, 1998). Seven days after the second restimulation, the cultures were assayed for CTL activity in a ⁵¹Cr release assay or *in situ* IFNγ ELISA.

### Measurement of CTL lytic activity by ⁵¹Cr release

Cytotoxicity was determined in a standard ⁵¹Cr release assay by assaying individual wells at a single E:T ratio. Peptide-pulsed targets were prepared by incubating the cells with 10µg/ml peptide overnight at 37°C. Adherent target cells were removed from culture flasks with trypsin-EDTA. Target cells were labeled with 200µCi of ⁵¹Cr sodium chromate (Dupont, Wilmington, DE) for 1 hour at 37°C, washed twice, resuspended at 10⁶ per ml and diluted 1:10 with K562 cells (an NK- sensitive erythroblastoma cell line used to reduce non-specific lysis) at a concentration of 3.3x10⁶/ml. Target cells (100 µl) and 100µl of effectors were plated in 96 well round-bottom plates and incubated for 5 hours at 37°C. 100 µl of supernatant were collected from each well and percent lysis was determine according to the formula: [(cpm of the test sample - cpm of the spontaneous ⁵¹Cr release sample)/(cpm of the maximal ⁵¹Cr release sample - cpm of the .spontaneous ⁵¹Cr release sample)] x 100. Maximum and spontaneous release was determined by incubating the labeled targets with 1% Triton X-100 and medium alone, respectively. A positive culture was defined as one in which the specific lysis (sample - background) was 10% or higher in the case of individual wells and was 15% or more at the 2 highest E:T ratios when expanded cultures were assayed.

### In situ Measurement of Human IFNγ Production

In brief, Costar EIA plates were coated with mouse anti-human IFNγ monoclonal antibody (Pharmingen) overnight at 4°C. The plates were washed and blocked for 2 hours, after which the CTLs (100µl/well) and targets (100µl/well) were added to each well, leaving empty wells for the standards and blanks (which received medium only). For expanded cultures, 1x10⁵ CTL/well were mixed with 1x10⁵ targets (neg. control) or peptide-pulsed or endogenous targets. All wells were brought to 200µl with medium and incubated for 48 hours at 37°C with 5% CO₂.

Human rIFNγ (R&D Systems) was added to the standard wells starting at 400 pg/100ul/well and the plate incubated for 2 hours at 37°C. The plates were washed and 100µl biotinylated mouse anti-human IFNγ monoclonal antibody (Pharmingen) were added to each well and the plates incubated for 2 hours at room temp. After washing again, 100µl/well HRP-streptavidin (Zymed) were added and incubated for 1 hour at room temp. The plates were then washed 6x with wash buffer, 100µl/well TMB developing solution (KPL, mixed 1:1) was added and the plates allowed to develop for 5-15 min. The reaction was stopped with 50 µl/well 1M H₃PO₄ and read at OD450. A culture was considered positive if it measured at least 50 pg of IFNγ/well above background and was at least twice the background level of expression.

### CTL Expansion

Those cultures that demonstrated activity against peptide-pulsed targets and/or tumor targets were expanded over a two week period with anti-CD3 antibodies (Wang, 1998; Greenberg, 1991). Briefly, 5x10⁴ CD8+ cells were added to a T25 flask containing the following: 1x10⁶ irradiated (4,200 rad) PBMC (autologous or allogeneic) per ml, 2x10⁵ irradiated (8,000 rad) EBV-transformed cells per ml, and OKT3 at 30 ng per ml in RPMI-1640 containing 10% (v/v) human AB serum, non-essential AA, sodium pyruvate, 25µM 2-ME, L-glutamine and penicillin/streptomycin. Human rIL2 was added 24 hours later at a final concentration of 200IU/ml and every 3-4 days thereafter with fresh medium at 50 IU/ml. The cells were split if the concentration exceeded 1x10⁶/ml and the cultures assayed between days 13 and 15.

Alternatively, cultures were expanded by stimulation with peptide-pulsed autologous PBMCs in the absence of OKT3. All other conditions and cell numbers remained the same as those described above.

### RESULTS:

### Scanning for motif-positive peptides

As described in the Materials and Methods section, protein sequences from the four tumor antigen targets (p53, CEA, Her2/neu, and MAGE2/3) were scanned, utilizing a customized program, for 8-, 9-, 10-, and 11-mer sequences containing one of four motif types. Specifically, peptides carrying motifs of the HLA-A3 supertype, the HLA-B7 supertype, the HLA-A24 motif, and the HLA-A1 motif were identified. The specific residues associated with each of these motifs or supermotifs are listed in the Materials and Methods section. For the HER2/neu antigen, only the intracellular domain was examined.

A number of naturally occurring, or wildtype (WT) peptides identified utilizing these motifs are listed in Tables 6, 9, 10, 16a-d, 17a-d, 18a-d and 19a-d. Also presented is the number of analogs that could be synthesized. In general, WT peptides carrying suboptimal residues at primary anchor positions can be substituted with optimal residues, employing the analog strategies described in the Materials and Methods section. To be considered a candidate for analoging, a TAA-derived WT peptide must carry one preferred residue at either position 2 or the C terminus. In previous work in the A2 system, analogs tested for immunogenicity which had residues substituted at both anchor positions were able to induce peptide-specific CTLs *in vitro.* However, those CTLs were able to recognize tumor cell targets only 40% of the time as compared to 75% for analogs with a single anchor position substitution, and therefore were not pursued in the A3, B7, A1 and A24 systems. For the B7 supermotif-positive peptides, another analog strategy was discovered from previous unrelated studies that substituting the position 1 residue with phenylalanine (F) greatly improved B7 crossbinding capability. These analogs are also included in Table 15 and Table 17a-d. However, these F1 analogs will not be considered as candidates until this strategy has been validated. That is, induction of tumor-reactive CTLs must be demonstrated with these analogs.

Additionally, Table 15 presents, for each TAA, the number of WT peptides and analogs actually synthesized and tested for supertype or primary allele binding affinity. As seen, the vast majority of WT peptides have been tested for binding affinity. The synthesis and testing of analogs, however, are in various stages of completion dependent upon the TAA. Recall that for an analog to be a candidate for immunogenicity screening, its WT parent peptide must bind at least weakly, ie. IC₅₀ ≤5000 nM, to 3 of 5 alleles in the supertype, or to the parent allele in the cases of A1 and A24. The numbers presented in the analog, motif-positive columns are based solely on analoging potential, or the presence of suboptimal residues at anchor positions, and not on measured binding. For this reason, many of the analogs listed would not ultimately be synthesized.

To date, 695 CEA-derived motif positive peptides have been identified and 358 of these tested for supertype or primary binding. These include 64 A3 binding wildtype and analog peptides, 171 B7 peptides, 65 A1 peptides and 28 A24 peptides. Analysis of the intracellular domain of the HER2/neu protein identified 822 motif positive peptides. Of these, 364 (64 A3, 213 B7, 81 A1 and 47 A24) have been tested to determine their binding affinities. The MAGE2 and MAGE3 proteins were considered together and a total of 611 motif positive peptides were identified. A subset of these, including 80 A3 wildtype and analog peptides, 146 B7 peptides, 40 A1 peptides and 54 A24 peptides were tested for primary and/or supertype binding. Lastly, 566 motif positive, p53-derived peptides have been identified. The wildtype and analog peptides tested to determine their binding affinity included 102 A3-restricted peptides, 165 B7-restricted peptides, 19 A1-restricted peptides and 14 A24-restricted peptides. The results of the binding assays on these motif positive peptides and the subsequent identification of epitopes is the subject of this report.

### Analysis of HLA-A3 vaccine candidates

**CEA:** A total of 64 CEA-derived peptides were tested for binding to A3 supertype molecules to date. Fourteen CEA-derived peptides from 8 distinct regions of the protein sequence have been identified. These fourteen peptides have a binding affinity of ≤500nM to A*0301 and/or A*1101, the two most predominant alleles, and also crossbind to a total of at least 3 alleles of the A3 superfamily (Table 16a).

All fourteen bind HLA-A*1101 with high or intermediate affinity. Noteworthy is the case of the CEA.61 epitope that binds with high affinity (≤50nM) to all five alleles, is immunogenic and is generated by natural processing. High A*1101 binding affinity has also been observed in 5 WT peptides of the remaining six regions: CEA.376, CEA.418, CEA.419, CEA.554 and CEA.636. Three of these, CEA.419, CEA.554 and CEA.636, bind to an additional three alleles and are also good candidate peptides.

Five of the seven regions exhibited suboptimal A*0301 binding for all of the WT peptides encompassed. Peptides from three of these regions were analoged in an attempt to improve A3 binding and overall crossbinding capacity. The CEA.241K10 analog displayed a 26-fold improvement in A3 binding. This analog was demonstrated to be immunogenic in *in vitro* immunogenicity assays, and the resulting CTLs were shown to be cross-reactive with target cells pulsed with the wildtype peptide (Figure 1). A second analog within the same region, CEA.241V2, was associated with improved binding to A*0301 and A*1101, and bound 4 of 5 superfamily alleles. Therefore, this peptide is also a strong candidate. Significant increases were observed with CEA.420V2, which exhibited the most suitable binding profile of any peptide derived from that region, which included 3 different wild-type sequences. Lastly, CEA.376V2 and CEA.554V2 showed no improvement in binding capacity over their wildtype counterparts.

In conclusion, within the eight distinct regions of CEA identified to harbor motif positive peptide candidates, multiple peptides have been identified which bind each of the five supertype alleles. Two peptides bind all five alleles and another six bind four of the five. A vaccine could be designed with one peptide from each region, which would include at least 4 peptides specific for each allele of the A3 superfamily.

**HER2/neu**: When motif analysis was performed on the intracellular and transmembrane domains of the 1255 amino acid HER2/neu protein sequence and binding affinity of the motif positive peptides determined, candidate peptides from 11 different regions were identified (Table 16b). A total of sixteen wild-type peptides and analogs demonstrated binding affinities of ≤500nM to at least 3 alleles in the A3 superfamily. Eleven of these bound HLA-A*0301 and fifteen bound A*1101, and 10 peptides had a binding affinity of ≤500nM for both alleles. The HER2/neu.681 epitope was also considered here because primary CTL data indicates that this peptide was immunogenic and has been shown to induce tumor-reactive CTLs. Three additional peptides, HER2/neu.754 (Kawashima, 1999), HER2/neu.669 (9-mer), and HER2/neu.852 (Kawashima, 1999), bind with an affinity ≤500nM to ≥4 alleles and are immunogenic. Five additional WT peptides (HER2/neu.806, HER2/neu.846, HER2/neu.889, HER2/neu.972 and HER2/neu.997) also demonstrated high and intermediate binding to ≥3 alleles and therefore are candidate peptides.

Of the six analog peptides tested, 2 showed improvement as compared to their wildtype peptide counterparts. More specifically, HER2/neu.860V2 improved' binding affinity to A*6801, while HER2/neu.889V2 exhibited improved binding to HLA-A*0301 and A*6801.

For each of, the five supertype alleles, multiple peptides have been identified which bound with affinities of 500nM or less. Two peptides bind all five alleles and six additional candidates bind 4 alleles. In total, six of the ten regions have peptides that bind ≥ 4 of 5 alleles representing a substantial pool of A3 supertype peptides that could be considered for use in a vaccine.

**MAGE2/3:** Motif analysis of the MAGE 2/3 protein sequences (each 314 amino acids long) identified 22 peptides from 9 distinct regions which are high or intermediate cross-reactive binders of the A3 superfamily (Table 16c). More specifically, sixteen of these bind both A*0301 and A*1101. MAGE2.73 binds 5 alleles and induced CTL capable of recognizing both peptide-pulsed and tumor targets (Epimmune U.S. Patent No. 6,037,135). MAGE2.237, MAGE2.277 and MAGE3.189 bind to 3 of the 5 alleles. Interestingly, Reynolds and co-workers (Reynolds et al., J. Immunol. Methods 244:59-67 (2000) have demonstrated in an ELISPOT assay that MAGE3.189 is antigenic (eg. when melanoma patients were given a vaccine comprised of supernatant from a melanoma cell line, CTLs could be isolated that recognized MAGE3.189-pulsed target cells).

It can also be noted that the MAGE2.226 and MAGE3.226 peptides are homologous except for a M to V difference at position 2. Since the main MHC anchor residues are least likely to influence T cell receptor recognition (Zhang, 1992), these peptides are likely to induce overlapping T cell specificity and therefore could be considered variations of the same epitopic sequence. Of the two, MAGE3.226 is preferred at this point because it binds 2 of the 5 alleles with higher affinity. In particular, this epitope binds HLA-A*3301 more than 10-fold better than the K9 analog, which binds all 5 alleles but with significantly lower A*3301 affinity.

Additional analogs were generated with improved binding characteristics. Specifically, MAGE2.69K9 showed improved cross-reactivity in regard to A*0301, A*1101 and A*6801 binding. MAGE2.299V2 demonstrated improved A*0301 binding and introducing V at position 2 in MAGE3.138 significantly improved the A*0301, A*1101 and A*6801 binding affinities. Substituting K or R at position 9 of the MAGE3.116 peptide improved crossreactivity to 3 and 5 alleles, respectively.

For each of the five supertype alleles, multiple peptides have been identified which bound with affinities of 500nM or less. Three peptides bound all five alleles, one candidate bound 4 alleles and an additional six peptides bound 3 alleles. In total, all nine regions have peptides that bound ≥3 of 5 alleles.

**p53:** A total of 17 high and intermediate affinity cross-reactive motif positive peptides, 7 wildtype and 10 analogs, were identified as a result of scanning the 393 amino acid p53 protein sequence (Table 16d). These peptides are derived from 8 different regions of the protein. All seventeen bind A*0301 and 16/17 bind A*1101. Several of the wildtype peptides are potential candidate peptides. The p53.124 peptide binds 3 alleles and provides coverage of A*6801. The 8-mer of p53.273 binds 4 out of 5 alleles, including A*3101 and A*3301. p53.132 and p53.376 bind 3 alleles each but bind only A*0301 and A*1101 with high affinity.

The p53.101, p53.172 and two p53.240 peptides could also represent candidates for inclusion in an epitope based vaccine, depending on supertype binding results.

Six of the eight regions described have had WT peptides analoged. Two of these analogs, p53.172B5K10 and p53.240B3K9, demonstrated improved binding to one of the two primary alleles (A*0301 or A*1101). Additionally, the p53.172B5K10 epitope bound 3 alleles of the A3 superfamily, is immunogenic and able to induce CTLs that recognize both the wildtype sequence and p53 transfected tumor targets (Figure 2). The p53.240V2B3 is also a good candidate for inclusion in a multi-epitope vaccine based on the binding affinities observed, pending WT supertype binding results.

With regard to results generated for the other analogs, p53.156R9 showed improved binding to A*3301, an allele for which few p53-derived wildtype peptides bound. Finally, the p53.101K10 and p53.101V2 peptides are similar with respect to binding affinity and allelic coverage but immunogenicity has been demonstrated for the K10 analog (data not shown) making it a stronger candidate than the V2 analog.

Supertype crossreactive candidates from all eight protein regions have been identified and all the candidates described above bind 3 to 4 of the supertype alleles. Coverage of all five alleles can be achieved by including multiple epitopes in a vaccine.

### Analysis of HLA-B7 vaccine candidates

HLA-B7 supertype peptides are identified by their binding affinity for B*0702, the primary allele of the B7 superfamily, and two or more additional alleles. The overall number of B7 cross-reactive candidates is limited primarily because proline is the only tolerated amino acid at primary anchor position 2 while other supertypes (eg. A2 and A3) tolerate several different amino acids at their anchor positions (Sidney, 1995; 1996b). This restriction also limits the number of analogs that can be generated for a particular peptide.

Three regions of the CEA protein sequence are represented by 3 candidate peptides, one wildtype and 2 analogs (with isoleucine substituted at the C terminus), which bind B*0702 and 2-3 additional alleles with an affinity ≤500nM (Table 17a).

Three wildtype peptides derived from 3 regions encoded within the intracellular domain of HER2/neu have also been identified (Table 17b). Substituting I for A at position 8 of the HER2/neu.921 peptide increases the binding affinities for B*0702 and B*5101 and therefore the analog would be considered a preferred candidate of that region. The 4 candidate peptides listed in Table 17b bound 3 to 4 alleles of the B7 supertype with an affinity ≤500nM.

Motif analysis of the MAGE2 and MAGE3 protein sequences yielded a number of candidates from four discrete regions of these antigens which bind to ≥3 alleles of the B7 superfamily with an affinity ≤500nM (Table 17c). MAGE2.170 binds to 4 alleles of the superfamily and is capable of inducing peptide reactive CTLs *in vitro.* Three analogs (MAGE3.71I9, MAGE3.77I8 and MAGE3.196I10) exhibit increased crossreactivity and Class I binding affinity.

Another factor to be considered in the selection of vaccine candidates is the potential relevance of using nested peptides. The MAGE3.196 peptides can be used to illustrate this point. The 10-mer, while not cross-reactive, has high binding affinity for B*5401. It also has the advantage of containing the 8- and 9-mer peptides within its sequence. In theory, the 10-mer would undergo trimming in the endoplasmic reticulum to the 8-mer and/or the 9-mer peptide (Paz, 1999), each of which binds other common alleles in the B7 superfamily. In addition, Reynolds et al. (submitted 1999), demonstrated antigenicity for the 9-mer wildtype peptide.

Lastly, two p53-derived peptides have been identified, p53.76 and p53.84 (Table 17d). The p53.76 11-mer bound 3 alleles with higher affinity than the 9-mer. While the p53.84 wildtype peptide bound with only intermediate affinity, this candidate would be an improved candidate than the corresponding analog because of higher binding affinities for B*5301 and B*5401.

In summary, three CEA-derived peptides from 3 distinct regions, 4 Her2/neu peptides from 3 regions, and 4 p53 peptides from two regions, all with binding affinities ≤500nM and crossreactive for at least 3 supertype alleles, have been identified. An additional 10 MAGE2/3-derived high and intermediate affinity peptides from 4 regions were also identified.

### Analysis of HLA-A1 vaccine candidates

**CEA:** Six high and intermediate binding CEA wildtype peptides from 6 regions of the 702 amino acid protein sequence have been identified (Table 18a). Results with other supertype alleles (eg. A2 and A3) indicate that peptides with an IC₅₀ ≤100nM for the predominant allele of the superfamily also bound with high or intermediate affinity to the other alleles of the superfamily. Therefore, until binding assays are developed for the other alleles of the A1 superfamily, it is reasonable to employ the stricter 100nM cut-off for the identification of HLA-A1-restricted, potentially degenerate binding peptides. Accordingly, four WT peptides are potential candidates based on a binding affinity of ≤100nM to A*0101. These are CEA.225, CEA.403, CEA.581, and CEA.616.

CEA.616 and peptides from 4 additional regions were analoged at primary anchor positions, for 1 of the 2 submotifs, to improve coverage of this allele. The binding cut-off of the wildtype peptides to be analoged was 5000nM to ensure expression of the tumor antigen-derived peptide on the tumor cell and the cut-off for the analog candidates was again ≤100nM. Employing these criteria, an additional 5 analog peptides have been identified as candidates. The CEA.289D3, CEA.418D3, CEA.419D3, CEA.467D3, and CEA.616D3 peptides all demonstrate an HLA-A*0101 binding affinity ≤100nM.

A total of 4 wildtype and 5 analog peptides derived from 7 distinct regions and with binding affinities ≤100nM have been identified as candidates.

**HER2/neu:** The Her2/neu protein sequence was scanned to identify HLA-A1 motif positive peptides and the binding affinity of those peptides for HLA-A*0101 was determined. Wildtype peptides from 6 distinct regions were identified and 4 of these bind HLA-A*0101 with an affinity ≤100nM (Table 18b). These are HER2/neu.826, HER2/neu.869, HER2/neu.899, and HER2/neu.1213.

All of the wildtype peptides were analoged at primary anchor positions to improve coverage of this antigen. Eleven analogs, representing a total of 9 regions, were identified as potential candidates based on a binding affinity ≤100nM to HLA-A*0101. Of these, eight demonstrate significantly improved binding affinity. These are HER2/neu.773D3, HER2/neu.826T2, HER2/neu.996D3, HER2/neu.997T2, HER2/neu.1014T2, HER2/neu.1131D3, HER2/neu.1213T2, HER2/neu.1239D3.

In summary, sixteen wildtype and analog peptides representing 9 different protein regions meet the criteria for a vaccine candidate. That is, they bound to the primary allele of the A1 superfamily, HLA-A*0101 with an affinity of 100nM or less.

**MAGE2/3:** Five HLA-A1 motif positive peptides with binding affinities ≤100nM have been identified and are listed in Table 18c. These five wildtype peptides (MAGE3.246, MAGE2.247, MAGE3.68, MAGE3.166, and MAGE3.168) are from four non-overlapping regions of the two proteins. Additionally, Tuting et al (1998) demonstrated induction of MAGE3.168-specific CTLs as well as endogenous recognition of tumor targets expressingthe epitope. Additionally, in clinical trials, tumor regression was observed in melanoma patients vaccinated with peptide-pulsed DC (Nestle et al, 1998) or the peptide alone (Marchand, 1999).

Eight wildtype peptides with a binding affinity between 501 and 5000nM were analoged at primary anchor positions (Table 18c). Ten analogs, representative of 8 distinct regions, were identified as potential candidates. These are MAGE2.68D3, MAGE2.179D3, the T2 analogs of MAGE2.246/247, MAGE3.68D3, MAGE3.69T2, MAGE3.137T2, MAGE3.168T2, MAGE3.246D3 and MAGE3.293T2. Taken together, there are 15 peptides derived from 8 distinct regions of the MAGE2 and MAGE3 proteins.

**p53:** Four HLA-A1 motif positive wildtype peptides from 2 non-overlapping regions of the proteins were identified (Table 18d). These are p53.117, p53.225 (10-mer), p53.226 (9-mer) and p53.226 (11-mer), all of which bound HLA-A*0101 ≤100nM. These are derived from 2 of the 4 regions shown in Table 18d.

Four wildtype peptides with an HLA-A*0101 binding affinity ≤5000nM were analoged at primary anchor positions to improve coverage of this antigen (Table 18d). Four analogs, representing four distinct regions, were identified as potential candidates with an HLA-A*0101 binding affinity ≤100nM. Two of these, the T2 substitution in p53.98 and the D3 substitution in p53.196, improved binding affinity more than 24-fold.

Binding analysis of the p53 wildtype and analog motif positive peptides has led to the identification of 8 candidates from four protein regions.

### Analysis of HLA-A24 vaccine candidates

**CEA:** Twenty-one high and intermediate affinity HLA-A24 motif positive peptides, corresponding to 16. distinct regions of the CEA protein were identified (Table 19a). Only those peptides that bind with an affinity ≤100nM were considered to be potential degenerate binders and therefore vaccine candidates. Using this criterion, nine CEA wildtype peptides from 9 non-redundant regions have been identified as potential candidate peptides. In addition, Nukaya et al (1999) demonstrated that CEA.268 induced CTLs in normal donors that recognized peptide-pulsed targets and HLA matched tumor targets. Kim et al (1998) obtained the same results with CEA.652. Both of these peptides are high affinity (≤100nM) binders, further confirming the selection of vaccine candidates on the basis of their binding affinity.

Wildtype peptides from 10 regions were analoged at primary anchor residues to improve coverage of this allele (Table 19a). The binding cut-off for the wildtype peptides to be analoged was 5000nM to ensure expression of the tumor antigen-derived peptide on the tumor cell and the cut-off for the analog candidates was again ≤100nM. Employing these criteria, eight additional peptides have been identified as candidates. Six of these analogs demonstrated binding affinities similar to their wildtype counterparts, while 2 analogs significantly improved binding to the HLA-A*2402 allele. These are CEA.446F10 and CEA.604F10.

Nine wildtype and eight analog peptides from 11 regions have been identified, providing an opportunity for wide coverage of this antigen.

**HER2/neu:** Seven wildtype peptides derived from 3 regions of the intracellular domain of HER2/neu were identified as HLA-A*2402 binders of high or intermediate affinity (Table 19b). Four of the seven peptides bound HLA-A*2402 ≤100nM. These are HER2/neu.780, HER2/neu.907 and the 9- and 11-mers of HER2/neu.951.

Five wildtype peptides, with A*2402 binding affinities ≤5000nM, were analoged at primary anchor positions to improve coverage of both this allele and tumor antigen. Four analogs were identified as potential vaccine candidates: HER2/neu.780F9, HER2/neu.907F9, HER2/neu.951F9 and HER2/neu.968Y2. The HER2/neu.968Y2 analog demonstrated the most significant increase (>18-fold) in binding affinity.

Eight peptides, 4 WT and 4 analogs, derived from 4 distinct regions of the intracellular domain of the HER2/neu protein have been identified as potential vaccine candidates.

**MAGE2/3:** Fifteen high and intermediate affinity HLA-A24 motif positive peptides, from 11 non-overlapping regions of these proteins, were identified (Table 19c). Eight of the 15 bound HLA-A*2402 ≤100nM and represent 7 different regions of these proteins. Additionally, induction of CTLs and tumor target recognition were demonstrated for MAGE2.156 (Tahara, 1999) and MAGE3.195(Tanaka, 1997).

Seven of the wildtype peptides were analoged at primary anchor residues to improve coverage of these antigens (Table 19c). Seven analogs, each representing a distinct region, were identified as potential candidate peptides, and three of these (MAGE2.97Y2, MAGE2.175F10 and MAGE3.175F10) demonstrated significantly higher binding affinity than the corresponding WT peptides. Overall, there are 15 wildtype and analog peptides that are derived from a total of 10 regions of the MAGE2 and MAGE3 proteins. Additionally, 2 of these peptides have been confirmed to induce peptide and tumor reactive CTLs by outside investigators.

**p53:** Five HLA-A24 motif positive peptides from 3 distinct regions have been identified (Table 19d). Two of these 5 bind HLA-A*0101 ≤100nM. These are p53.102 (10-mer) and p53.125.

When the 10-mer of p53.102 was analoged at the C-terminus, a 3-fold improvement in binding affinity was observed. Analoging of p53.106 did increase the binding affinity 5-fold, but not to the imposed threshold of ≤100nM. Overall, there are 3 p53-derived peptides (2 wildtype and 1 analog), representing two regions that are potential candidate peptides for a vaccine.

### Binding to the primary HLA-A3 and B7 superfamily alleles is predictive of degenerate binding by TAA-derived peptides

Extensive analysis of infectious disease peptides demonstrated that a binding affinity ≤100nM to the primary allele of a supertype was highly predictive of the degeneracy of a peptide. In other words, a majority of peptides meeting this criterion bound at least 2 other alleles of that superfamily with an affinity ≤500nM (Bertoni, 1997; Doolan, 1997; Threlkeld, 1997). Based on this analysis, it is recommended that the 100nM binding cut-off be used to select HLA-A*0101 and HLA-A*2402 peptide candidates in the absence of binding assays for the other supertype alleles. However, a significant number of HLA-A3 restricted TAA peptides were identified for the studies described here and supertype binding performed. This information was analyzed to determine if primary binding of ≤100nM was predictive of supertype crossreactivity in the case of TAA-derived peptides. A total of 23 wildtype peptides (from Tables 16a-d) bound HLA-A*0301 or HLA-A*1101 with an affinity ≤100nM. Twenty-two of these (96%) bound ≥3 alleles with an affinity of ≤500nM. Eighteen of the 23 (78%) were cross-reactive when the more stringent cut-off of 200nM was applied.

Additionally, six peptides were tested for immunogenicity in 3-4 donors. When this data is taken into account for those peptides that bind at ≤100nM to the primary allele and at ≤200nM to at least 2 additional supertype alleles, 6/6 (CEA.241.KIO, CEA.61, CEA.61, HER2/nev.681, HER/nev.754, and p53.172.B5KIO) (100%) are immunogenic and 4/6 (CEA.61, HER2/nev.681, HER/nev.754, and p53.172.B5KIO) (67%) induce tumor-reactive CTLs. These results are similar to those observed with TAA-derived HLA-A2-specific peptides. Keogh, et al. J Immunol. 167(2):787-96 (2001).

The analog peptides were analyzed in the same way. Thirty-nine analogs with either HLA-A*0301 or A*1101 binding affinity ≤100nM were identified from Tables 4a-d. All 39 (100%) were demonstrated to be cross-reactive with at least 2 additional supertype alleles with an affinity ≤500nM. Applying a binding cut-off of 200nM for supertype cross-reactivity, 27/39 (69%) of the analog peptides were degenerate binders.

The same analysis was performed with the HLA-B7-restricted peptides (derived from Tables 17a-d) although the sample size was much smaller than that for A3. Five wildtype peptides and 5 analogs were identified to have binding affinities ≤100nM. All 10 (100%) were also cross-reactive with at least 2 other alleles of the B7 superfamily. When the binding affinity cut-off for cross-reactivity was set at 200nM or less, 2 out of 5 (40%) peptides were cross-reactive in the case of both wildtype and analog peptides. While this is lower than the 78% observed for A3, this is likely to be due to the small number of peptides available for analysis.

### Selection of HLA-A3, -B7, -A1 and -A24 candidate peptides for immunotherapy

### Criteria for selection of vaccine candidates

Desirable criteria for the inclusion of an epitope in an immunotherapeutic cancer vaccine are that the epitope bind to three or more alleles of a given superfamily with an affinity ≤500nM and that such an epitope induces a specific CTL response recognizing target tumor cells. An analysis of Epimmune's accumulated database and the results from primary immunogenicity screening of A2 supertype cancer antigen-derived epitopes in particular provided several important findings. First, a binding affinity of ≤500nM is highly predictive of immunogenicity. However, peptides that bind with affinities of 201-500nM did not induce tumor-reactive CTLs. Additionally, it was demonstrated that when a more strict binding affinity cut-off of ≤200nM was applied to wildtype peptides, 76% of the TAA peptides induced CTLs that recognize the endogenously processed and presented epitope. In the course of the same set of experiments, analogs which bound with an IC₅₀ of 200nM or less, and for which primary immunogenicity (recognition of wildtype peptide-pulsed targets) could be demonstrated, were studied. These analogs induced CTLs capable of recognizing tumor cell targets and/or wildtype peptides in at least 75% of the cases. It was concluded from this analysis that binding affinity was highly predictive of immunogenicity and that a binding affinity of 200nM was indicative of a peptides ability to induce tumor reactive CTLs. Therefore, utilizing a binding cut-off of ≤200nM to select the HLA-A3 and -B7 peptide candidates described herein increases the likelihood of identifying epitopes and reduces the number of peptides to be screened *in vitro.*

Currently, supertype binding assays are unavailable for A*0101 and A*2402. However, work done with HCV and HBV peptides by researchers at Epimmune as well as studies performed by Doolan (1997) and Threlked (1997) with malaria and HIV-derived peptides, respectively, demonstrated that peptides with a binding affinity ≤100nM were often degenerate binders. Therefore, A1 and A24 candidate peptides were selected on the basis of a stringent (≤100nM) binding affinity to the primary allele of the A1 and A24 superfamilies. These selection criteria should ensure that these peptides are not only likely to be cross-reactive, but also immunogenic.

Another factor we wished to consider in the selection of HLA-A3 and B7-restricted candidates was to allow for a combination of both wild-type peptides and analogs. Using a wildtype peptide increases the likelihood that the peptide is endogenously processed and presented. However, analogs may play an important role, particularly in the case of tumor antigens, because they could more easily overcome T cell tolerance and allow generation of a more multi-specific response. Furthermore, it would be predicted that a more vigorous CTL response can be induced by analoging at primary anchor positions of a peptide to increase binding affinity. In the case of HLA-A1 and HLA-A24-restricted peptides, wildtype peptides may be relatively preferred over analogs until assays are available to determine cross-reactive binding of the parent wildtype peptide. However, analogs should still be considered to provide the greatest coverage of each allele and tumor antigen.

To summarize, candidate peptides are selected on the basis of a binding affinity ≤200nM and crossreactive binding ≥3 alleles of the HLA-A3 or -B7 superfamilies, or a binding affinity ≤100nM for HLA-A1 and -A24 and primary immunogenicity and/or endogenous recognition for analogs.

### HLA-A3 cross-reactive vaccine candidates

Using a binding affinity threshold of 200nM or less for each allele to define supertype cross-reactivity and considering *in vitro* immunogenicity data where available, six to eight potential vaccine candidates have been identified for each antigen (Table 20).

**CEA:** When multiple peptides from a single region met the minimum criteria, the peptide that bound the greatest number of alleles was the preferred candidate. CEA.636, CEA.656, CEA.376, CEA.554, and CEA.420V2 were the most highly cross-reactive of the peptides from each of those regions. Lastly, CEA.61 and CEA.241K10 were selected on the basis of their demonstrated ability to induce CTLs *in vitro* that recognize endogenous targets (CEA.61) or the wildtype peptide (CEA.241K10). The V2 analog of the CEA.241 peptide is a back-up candidate because it meets the minimum criteria but immunogenicity has not yet been demonstrated. When considered as a group, four to seven of the different CEA-derived peptides bind each of the alleles of the A3 superfamily, providing broad population coverage of this antigen.

**HER2/neu:** For HER2/neu, 8 peptide candidates were identified from the transmembrane or intracellular domain of the protein. There are six candidates that met the criterion of cross-reactive binding to 3 or more alleles described in the previous section. Five of these are wildtype peptides and one is an analog. HER2/neu.669, HER2/neu.852, HER2/neu.860V2, HER2/neu.889, HER2/neu.972 and HER2/neu.997 bound to ≥3 alleles with an affinity ≤200nM and are therefore considered to be vaccine candidates representative of 6 distinct protein regions. HER2/neu.669 and HER2/neu.852 were also capable of inducing a CTL response in normal donors. It should be noted that HER2/neu.889 does not meet the binding criteria for either A*0301 or A*1101 but is included to increase coverage of A*3101, A*3301 and A*6801. Two additional wildtype peptides, HER2/neu.681 and HER2/neu.754, that are cross-reactive with 2 alleles and are also included because of available data demonstrating not only immunogenicity but also tumor target recognition by the CTLs induced (Kawashima, 1999). Three to eight of the candidate peptides within this group bound to each of the 5 alleles.

**MAGE2/3:** Six candidate peptides were identified for MAGE2/3 (four MAGE2 and two MAGE3), consisting of 3 wildtype and 3 analog peptides. Each allele within the supertype is covered by at least 2 peptides within this group. One of them, the MAGE2.73 peptide has been shown to be immunogenic and induce tumor reactive CTLs.

**p53:** Motif analysis of the p53 protein, determination of binding affinity, and analoging led to the identification of 6 non-redundant candidates. A somewhat greater fraction of candidates is represented by analogs in this case, probably reflective of the relatively small size of the p53 protein. All six peptides (2 wildtype and 4 analogs) bound the A*0301 allele, five bound A*1101 and A*3101, two bound A*3301 and three bound A*6801. Immunogenicity has already been demonstrated for two of the analogs, p53.101K10 and p53.172B5K10. Additionally it was demonstrated that p53.172B5K10-specific CTLs were able to lyse wildtype-peptide coated targets and p53 transfected tumor targets.

Summarizing, there are seven non-redundant CEA-derived peptides (5 wildtype and 2 analogs), 8 HER2/neu-derived peptides (including one analog), 6 peptides each from MAGE2/3 (3 wild-type and 3 analogs) and p53 which is represented by 2 wild-type peptides and 4 analogs.. HLA-B7 vaccine candidates

Using a binding affinity threshold of 200nM or less for each allele to define supertype cross-reactivity, one to three potential candidate peptides have been identified for each antigen (Table 21). One CEA-derived wildtype peptide binds 4 alleles, one HER2/neu-derived wildtype peptide binds four alleles, and one wildtype p53 peptide binds 3 alleles. One wildtype and 2 analog peptides were identified from MAGE2/3 that bind 3-4 alleles. MAGE3.196I10 is also included as a candidate (despite the fact that it did not meet the binding criteria for B*0702) because it provides coverage of B*5101, B*5301 and B*5401. Although, almost all the HLA-B7-restricted peptides have been assayed for binding, this allele remains underrepresented in the number of vaccine candidates identified.

### HLA-A1 vaccine candidates

The selection of HLA-A*0101 wildtype vaccine candidates was based on a binding affinity ≤100nM. In the case of analog candidates the same binding criteria was applied and, in addition, the parent (wildtype) peptide had to bind ≤5000nM.

**CEA:** When these criteria were applied to the peptides listed in Tables 6a-d, the choice of HLA-A*0101 restricted vaccine candidates was straightforward. They include seven non-overlapping CEA peptides (Table 22). Of these, 4 are wildtype peptides and 3 are analog peptides. CEA.418D3 was selected as the candidate from the group of candidates around that protein position because the affinity of the wildtype peptide was ≤500nM. In the case of CEA.616, the wildtype peptide is the preferred candidate because the analog provides only a marginal (less than 2-fold) increase in binding affinity.

**HER2/neu:** Four wildtype candidates and 5 analogs derived from HER2/neu met the criteria for the selection of candidate peptides (Table 22). Wherever possible the wildtype peptide was the preferred candidate (eg. HER2/neu.826.T2, HER2/neu.869, HER2/neu.899 and HER2/neu.1213). In the case of the analog peptides, the 5 chosen were clearly associated with improved binding affinities. HER2/neu.997T2 was preferred over HLA-B7 vaccine candidates

Using a binding affinity threshold of 200nM or less for each allele to define supertype cross-reactivity, one to three potential candidate peptides have been identified for each antigen (Table 21). One CEA-derived wildtype peptide binds 4 alleles, one HER2/neu-derived wildtype peptide binds four alleles, and one wildtype p53 peptide binds 3 alleles. One wildtype and 2 analog peptides were identified from MAGE2/3 that bind 3-4 alleles. MAGE3.196I10 is also included as a candidate (despite the fact that it did not meet the binding criteria for B*0702) because it provides coverage of B*5101, B*5301 and B*5401. Although, almost all the HLA-B7-restricted peptides have been assayed for binding, this allele remains underrepresented in the number of vaccine candidates identified.

### HLA-A1 vaccine candidates

The selection of HLA-A*0101 wildtype vaccine candidates was based on a binding affinity ≤100nM. In the case of analog candidates the same binding criteria was applied and, in addition, the parent (wildtype) peptide had to bind ≤5000nM.

**CEA:** When these criteria were applied to the peptides listed in Tables 6a-d, the choice of HLA-A*0101 restricted vaccine candidates was straightforward. They include seven non-overlapping CEA peptides (Table 22). Of these, 4 are wildtype peptides and 3 are analog peptides. CEA.418D3 was selected as the candidate from the group of candidates around that protein position because the affinity of the wildtype peptide was ≤500nM. In the case of CEA.616, the wildtype peptide is the preferred candidate because the analog provides only a marginal (less than 2-fold) increase in binding affinity.

**HER2/neu:** Four wildtype candidates and 5 analogs derived from HER2/neu met the criteria for the selection of candidate peptides (Table 22). Wherever possible the wildtype peptide was the preferred candidate (eg. HER2/neu.826.T2, HER2/neu.869, HER2/neu.899 and HER2/neu.1213). In the case of the analog peptides, the 5 chosen were clearly associated with improved binding affinities. HER2/neu.997T2 was preferred over HER2/neu.996D3 because the higher affinity of the 9-mer wildtype peptide makes it more likely to be presented by the MHC class I molecules and recognized by HER2/neu.996-specific CTLs.

**MAGE2/3:** Table 22 also lists the eight A1-restricted selected epitopes (four wildtype candidates and 4 analogs) derived from MAGE2 and MAGE3 that met the criteria for the selection of candidates. Once again, wherever possible the wildtype peptide was the preferred candidate (eg. MAGE2.247, MAGE3.246, MAGE3.68 and MAGE3.168). MAGE3.168 has also been previously demonstrated to be an epitope (Tuting,1998). In the case of the analog peptides, the four chosen are clearly associated with improved binding affinity (≤100nM).

**p53:** Finally, a total of four p53-derived peptides have been selected, each representing a discrete region of the protein (Table 22). p53.117 and p53.226 both have a binding affinity ≤100nM and were therefore chosen as the wildtype candidate peptides. In the case of p53.98T2 and p53.196D3 an approximately 25 to 50-fold increase in binding affinity over their wildtype counterparts was demonstrated. Correspondingly, these analogs were also selected as candidates.

### HLA-A24 vaccine candidates

As was described above for HLA-A1 epitopes, the selection of HLA-A*2402 wildtype vaccine candidates was based on binding ≤100nM and immunogenicity data generated at Epimmune or described in the literature. Analog candidates had to both bind ≤100nM and be associated with a wildtype peptide that binds ≤5000nM. When a wildtype peptide and corresponding analog both bound ≤100nM, the wildtype was the preferred choice.

When these criteria were applied to the peptides listed in Tables 7a-d, the HLA-A*2401 restricted vaccine candidate peptides listed in Table 11 were obtained. These included eleven non-overlapping CEA peptides. Of these, 9 are wildtype peptides and 2 are analog peptides.

Similarly, three wildtype candidates and 1 analog derived from Her2/neu met the criteria for the selection of candidate peptides (Table 23). As stated above, the wildtype peptide was the preferred candidate (eg. HER2/neu.780, HER2/neu.907 and HER2/neu.951 (11-mer)). In the case of the analog selected, HER2/neu.968Y2, binding affinity was significantly improved as compared to the wildtype parent counterpart.

Seven wildtype candidate peptides and 3 analog peptides derived from MAGE2 and MAGE3 were identified (Table 23). It can be noted that, the 3 analog peptides chosen (MAGE2.97Y2, MAGE2.175F10 and MAGE3.175F10) were all associated with binding affinity increased more than approximately 15-fold.

Lastly, two p53-derived peptides have also been selected, each representing a discrete region of the protein (Table 23). p53.102 and p53.125 both have a binding affinity ≤100nM.

By applying the criterion of a 100nM cut-off to identify HLA-A24-restricted peptides, a number of candidates have been identified for each antigen. The CEA candidate list includes 9 wildtype and 2 analog peptides. One analog and 3 wildtype peptides derived from the transmembrane and/or intracellular domain of HER2/neu have been identified. A total of 10 MAGE2/3-derived peptides (7 wildtype and 3 analogs) and 2 wildtype p53-derived peptides were identified.

### DISCUSSION:

These studies describe the selection of candidates for a therapeutic vaccine utilizing peptides carrying motifs representative of the A3, B7, A1 and A24 superfamilies. In the A3 and B7 supertypes, candidate selection is based on cross-reactive binding affinity. For HLA-A1 and -A24, selection is based solely on binding to A*0101 and A*2402, respectively.

Analoging strategies for HLA-A3, -B7, -A1 and -A24-restricted peptides were also described. Sidney, *et al.* (2001) (*supra*). Briefly, the Epimmune analog strategy was first documented for HLA-A2-restricted peptides. Ruppert, et al. PNAS 90(7):2671-75 (1993), also, Grey, et al. Clin. Exp. Rheumatol. Suppl. 8:S47-50 (1993), and researchers at Epimmune (EPI-45-99) demonstrated that substituting preferred amino acids for sub-optimal residues at primary anchor positions increased binding to the 5 predominant alleles of the A2 superfamily. Enhanced immunogenicity of analogs was demonstrated both in mice (Sarobe, 1998) and patients (Rosenberg, 1998). Tumor cell recognition was also observed, indicating that these peptide modifications do not affect the ability of the analog-specific CTL to recognize the endogenously expressed peptide. For A3 supertype moledules, T or V were determined to be optimal at position 2 and K or R optimal at the C-terminus to utilize as substitution residues with the greatest potential for improved crossbinding capacity. For B7 supertype alleles, proline is required at position 2. Although each molecule of the B7 superfamily prefers a different residue at the C-terminus (L, Y, I, W or A), isoleucine is the optimal C-terminal substitution residue to improve B7 superfamily crossbinding. Optimal residues for HLA-A1-restricted peptides are T at position 2, D at position 3, and Y at the C-terminus. Lastly, Y at position 2 and F at the C-terminus are recommended for the A24 allele. These strategies were implemented to identify additional binders to supplement population coverage for the CEA, HER2/neu, MAGE2/3 and p53 antigens.

Using cross-reactive binding affinities ≤200nM for A3 and B7-restricted peptides, binding affinities ≤100nM for A1 and A24-restricted peptides, and primary immunogenicity and tumor recognition when that data was available, a total of 88 wildtype and analog peptides were selected as candidates for a therapeutic vaccine (Table 24). There are 6-8 A3-restricted candidates for each of the four tumor antigens. In the B7 supertype arena, only 1-3 candidates were identified for each TAA. This is due in part to the requirement that proline be present at primary anchor position 2, which is severely limiting by the number of tolerated residues alone. Couple this with the fact that B7 peptides bind on average three-fold less well that other supertypes, roughly 10% of motif-positive peptides, the low number of identified TAA-derived B7 candidates is expected. However, including A1 and A24-resticted peptides in a non-A2 vaccine would increase coverage of black, Hispanic, and Asian populations and compensate for the paucity of B7 candidate peptides. Four to nine A1-restricted peptides and two to eleven A24-restricted peptides binding ≤100nM were identified for each antigen. Overall, there are 26 CEA, 22 Her2/neu, 27 MAGE2/3 and 13 p53 potential candidates.

Analysis of A2 peptides that were able to induce both peptide and tumor reactive CTLs led to the observation that when a binding cut-off of ≤200nM and supertype crossreactivity ≥3 alleles was employed, 76% of the wildtype peptides and 50% of the analogs induced tumor reactive CTLs. When primary immunogenicity data (wildtype recognition) was included, 81% of wildtype peptides and 75% of analogs were identified as tumor epitopes. When a 200nM cut-off was used to define cross-reactivity to the A3 superfamily alleles, 78% of wildtype peptides and 69% of analogs that bound A*0301 or A*1101 with an IC₅₀ ≤100nM were degenerate binders. A subset of A3, B7, A1 and A24 peptides described herein were tested for immunogenicity and tumor cell reactivity. When binding and cross-reactivity, as previously described for A2 peptides, were considered, 12/13 (92%) induce CTLs that recognize the wildtype peptide and 8/11 (72%) induce CTLs that recognize the endogenously processed peptide confirming the correlation between binding affinity and immunogenicity.

Assays have been established and validated to measure the capacity of a peptide to bind the 5 most predominant alleles of the A3 and B7 superfamilies, and subsequently select the cross-reactive (degenerate) candidate peptides. Supertypes for A1 and A24 have been proposed, but binding assays are currently available only for the primary alleles of those superfamilies, A*0101 and A*2402. Analysis of infectious disease-derived peptides indicates that binding affinity ≤100nM is predictive of degenerate binding (≤500nM) to at least 2 other alleles of the superfamily. The same analysis of the A3 and B7 TAA-derived peptides described in this example validates this finding. Of 28 wildtype peptides with a binding affinity ≤100nM for A*0301/A*1101 or B*0702, 27 (96%) bound 2 or more additional alleles with an affinity ≤500nM. This finding indicates that TAA-derived peptides behave in a similar fashion to infectious disease-derived peptides and that a binding affinity of 100nM or less for the primary allele of the superfamily is highly predictive of cross-reactivity.

In conclusion, this example describes the identification of peptide candidates derived from 4 TAA and covering multiple alleles. These candidates include both wildtype peptides and fixed anchor analogs which are either immunogenic or have a binding affinity predicted to be conducive to immunogenicity.

It is understood that the examples and embodiments described herein are for illustrative purposes only and that various modifications or changes in light thereof will be suggested to persons skilled in the art and are to be included within the spirit and purview of this application and scope of the appended claims. All publications, patents, patent applications and sequence listings cited herein are hereby incorporated by reference in their entirety for all purposes.

**Table 1. Overview of current cancer vaccine approaches.**

| APPROACH | DESCRIPTION | ISSUES | STRENGTHS |
|---|---|---|---|
| Whole Cell Vaccines | Involve the administration of whole cancer cells with adjuvants which serve to potentiate the immune response | • Often difficult to obtain tumor cells | • Likely to have novel TAA |
| | | • Patient variability | |
| | | • Single patient product | |
| | | • Has relatively low concentration of relevant TAA epitopes | |
| Cell Lysate Vaccines | Consist of lysed allogeneic cancer cell membrane particles that are ingested by macrophages and presented as tumor antigens to effector cells | • Often difficult to obtain tumor cells | • Likely to have novel TAA |
| | | • Patient variability | • |
| | | • Single patient product | |
| | | • Has relatively low concentration of relevant TAA epitopes | |
| Idiotypic Vaccines | Contain proteins derived from individual patient tumors or from specific tumor types | • Often difficult to obtain tumor cells | • Specific TAA |
| | | | • |
| | | • Patient variability | |
| | | • Single patient product | |
| | | • Has relatively low concentration of relevant TAA epitopes | |
| Whole Antigen Vaccines | | • Limited disease coverage | • Complex "natural" immune responses may be elicited |
| | | • Difficult to break tolerance | • Relatively easy single compound manufacture |
| Viral oncolysate vaccines | Consist of vaccinia virus infected cancer cell, lysed to form membrane segments expressing both vaccinia and cancer cell antigens | • Often difficult to obtain tumor cells | |
| | | • Not always possible to infect cancer cells | |
| | | • Patient specific treatment | |
| | | • Has relatively low concentration of relevant TAA epitopes | |
| Shed antigen vaccines | Similar to whole cell and lysate vaccines but are partially purified | • Difficult to purify antigens | • Likely to have novel TAA |
| | | • Patient specific treatment | • |
| | | • Has relatively low concentration of relevant TAA epitopes | |
| Genetically modified tumor cell vaccines | A number of avenues are being explored including the transduction of cells with GM-CSF | • Very difficult to obtain tumor tissues and grow to allow stable transduction | • Cells contain novel TAA and adjuvants |
| | | • Patient specific treatment | |
| Peptide Vaccines | Synthetic peptides are produced that correspond to tumor associated antigens. Designed to stimulate a cytotoxic T-Cell response (CTL) | • Need to choose correct peptides to elicit an effective immune response | • Single preparation used for multiple patients and possibly multiple diseases |
| | | • Restriction to HLA subtype or HLA supertypes | |
| | | | • Possible to combine various antigens/ targets |
| | | | • Reproducible antigen production |
| | | | • Able to break tolerance |
| | | | • Able. to elicit responses to subdominant epitopes |
| | | | • Can be directed to supertypes for broad population coverage |
| Carbohydrate vaccines | Synthetically produced tumor associated carbohydrates, designed to stimulate an antibody response against the carbohydrate antigens | • May need CTL response as well as humoral response | • Single preparation used for multiple patients and possibly multiple diseases |
| | | • Carbohydrate antigens are HTL dependent | |

**TABLE 2**

| SUPERMOTIFS | POSITION | POSITION | POSITION |
|---|---|---|---|
| | 2 (Primary Anchor) | 3 (Primary Anchor) | C Terminus (Primary Anchor) |
| A1 | **T, I,** *L, V, M, S* | | **F, W, Y** |
| A2 | **L, I, V, M,** *A*, *T, Q* | | **I, V,** *M, A, T, L* |
| A3 | **V, S, M, A,** *T, L, I* | | **R,K** |
| A24 | **Y, F,** *W, I, V, L, M, T* | | **F*,* I*****,** Y, W, L, M* |
| B7 | **P** | | **V, I, L, F,** *M*, *W, Y, A* |
| B27 | **R, H, K** | | **F, Y, L,** *W, M, I, V, A* |
| B44 | **E**, *D* | | **F, W, L, I, M, V, A** |
| B58 | **A, T, S** | | **F, W, Y,** *L, I, V, M, A* |
| B62 | **Q, L,** *I, V, M, P* | | **F, W, Y,** *M, I, V, L, A* |
| | | | |
| MOTIFS | | | |
| A1 | **T, S, M** | | **Y** |
| A1 | | **D, E,** *A*, *S* | **Y** |
| A2.1 | **L, M,** *V, Q, I, A, T* | | **V,** *L, I, M, A, T* |
| A3 | **L, M, V, I, S, A, T, F,** *C, G, D* | | **K, Y, R,** *H*, *F, A* |
| A11 | **V, T, M, L, I, S, A,** ***G, N,** C, D, F* | | **K,** *R, Y, H* |
| A24 | **Y, F, W,** *M* | | **F, L, I, W** |
| A*3101 | **M**, **V**, **T***, A ,L, I, S* | | **R***, K* |
| A*3301 | **M, V, A, L, F**, *I*, *S*, *T* | | **R, K** |
| A*6801 | **A, V, T,** *M, S, L, I* | | **R, K** |
| B*0702 | **P** | | **L**, **M**, **F**, *W, Y, A, I, V* |
| B*3501 | **P** | | **L**, **M**, **F**, **W**, **Y**, *I, V, A* |
| B51 | **P** | | **L**, **I**, **V**, **F**, *W, Y, A, M* |
| B*5301 | **P** | | **I, M, F, W, Y,** *A, L, V* |
| B*5401 | **P** | | **A, T, I, V,** *L, M, F, W, Y* |

Bolded residues are preferred, italicized residues are less preferred: A peptide is considered motif-bearing if it has primary anchors at each primary anchor position for a motif or supermotif as specified in the above table.

**TABLE 2a**

| SUPERMOTIFS | POSITION | POSITION | POSITION |
|---|---|---|---|
| | 2 (Primary Anchor) | 3 (Primary Anchor) | C Terminus (Primary Anchor) |
| A1 | **T, I,** *L, V, M, S* | | **F, W, Y** |
| A2 | **V,** *Q, A, T* | | **I, V,** *L, M, A, T* |
| A3 | **V,** *S,* **M, A,** *T, L, I* | | **R, K** |
| A24 | **Y, F,** *W, I, V, L, M, T* | | **F, I,** *Y, W, L, M* |
| B7 | **P** | | **V, I, L, F,** *M, W, Y, A* |
| B27 | **R, H, K** | | **F, Y, L,** *W, M, I, V, A* |
| B58 | **A, T, S** | | **F, W, Y,** *L, I, V, M, A* |
| B62 | **Q, L,** *I*, *V, M, P* | | **F, W, Y,** *M*, *I*, *V, L, A* |
| | | | |
| MOTIFS | | | |
| A1 | **T, S, M** | | **Y** |
| A1 | | **D, E,** *A, S* | **Y** |
| A2.1 | *V, Q, A, T** | | **V,** *L, I, M, A, T* |
| A3.2 | **L, M, V, I, S, A, T, F,** *C, G, D* | | **K, Y, R,** *H, F, A* |
| A11 | **V, T, M, L, I, S, A, G, N,** *C, D, F* | | **K,** *R, H, Y* |
| A24 | **Y ,F, W** | | **F, L, I, W** |

| | | | |
|---|---|---|---|
| *If 2 is V, or Q, the C-term is not L | | | |

Bolded residues are preferred, italicized residues are less preferred: A peptide is considered motif-bearing if it has primary anchors at each primary anchor position for a motif or supermotif as specified in the above table.

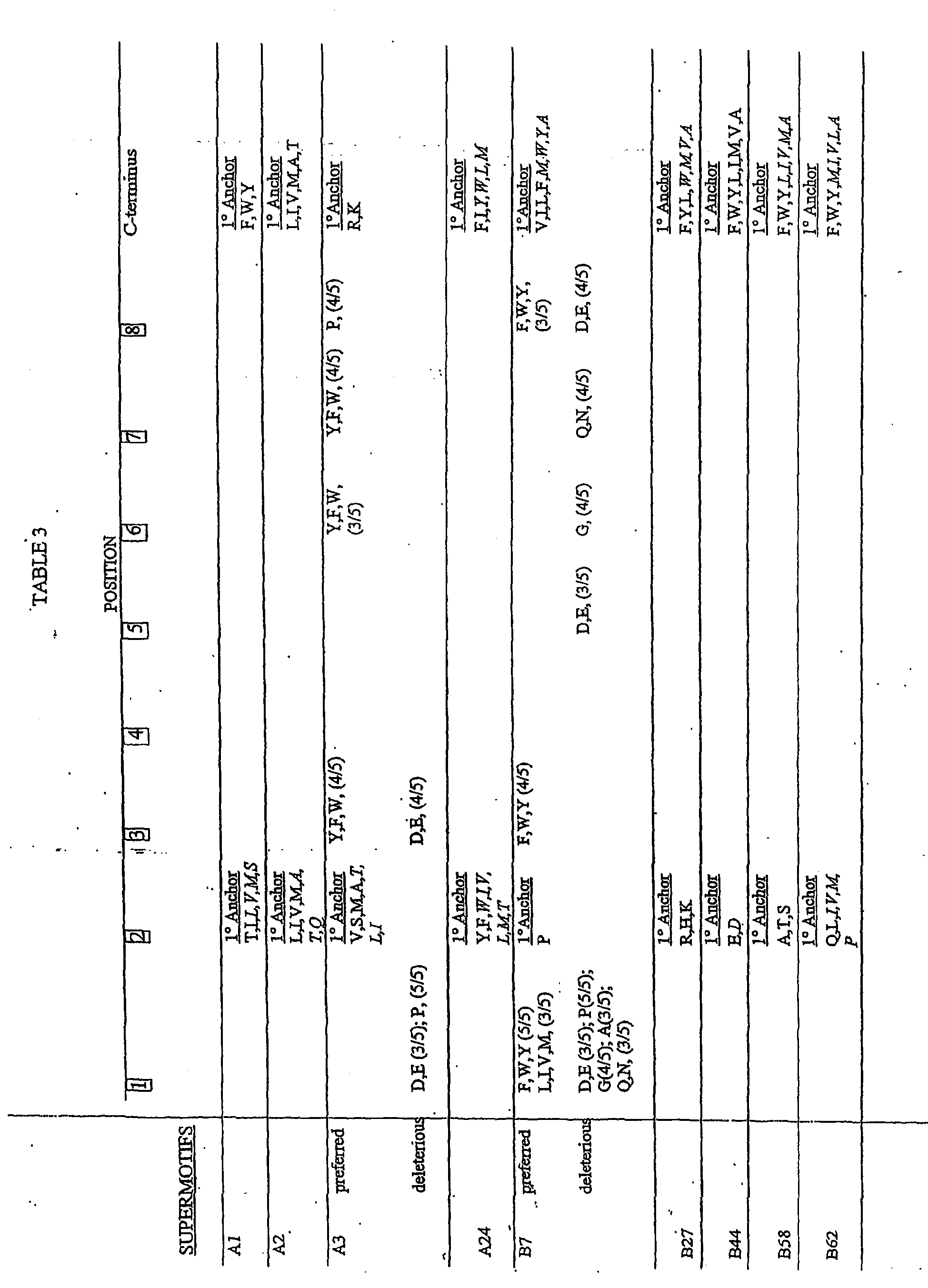

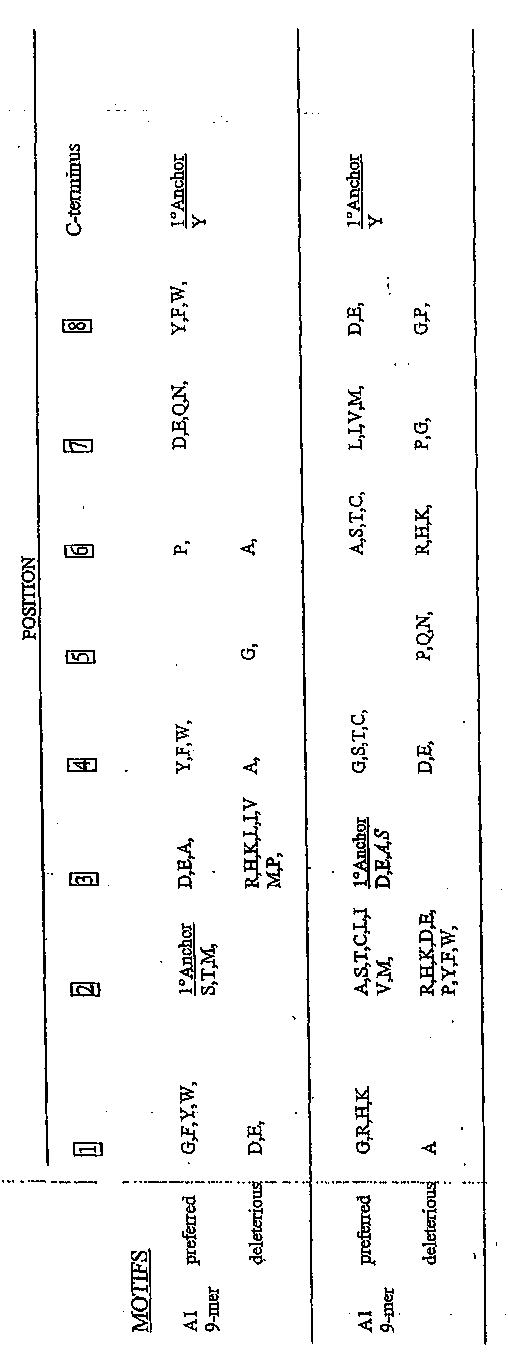

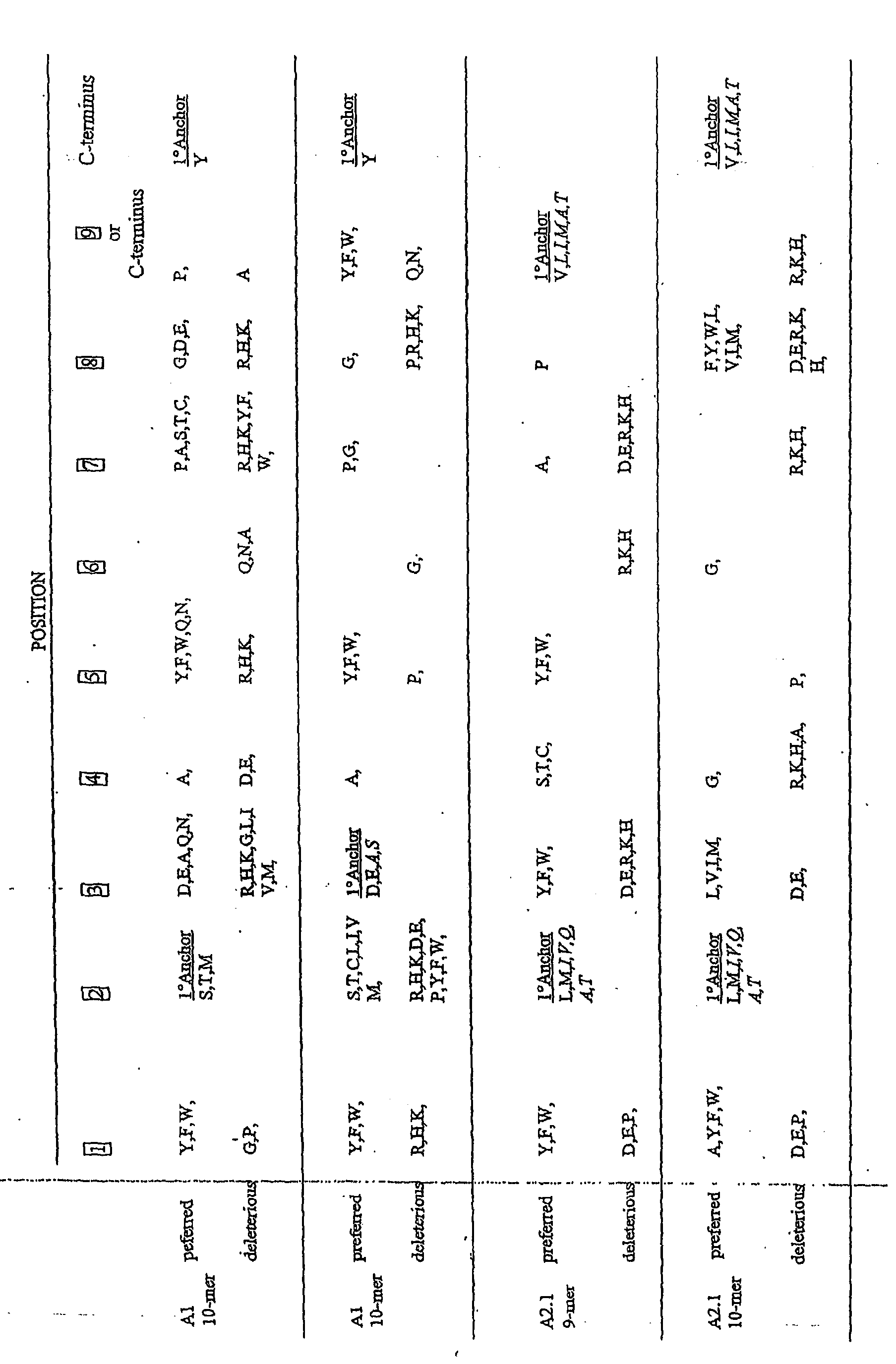

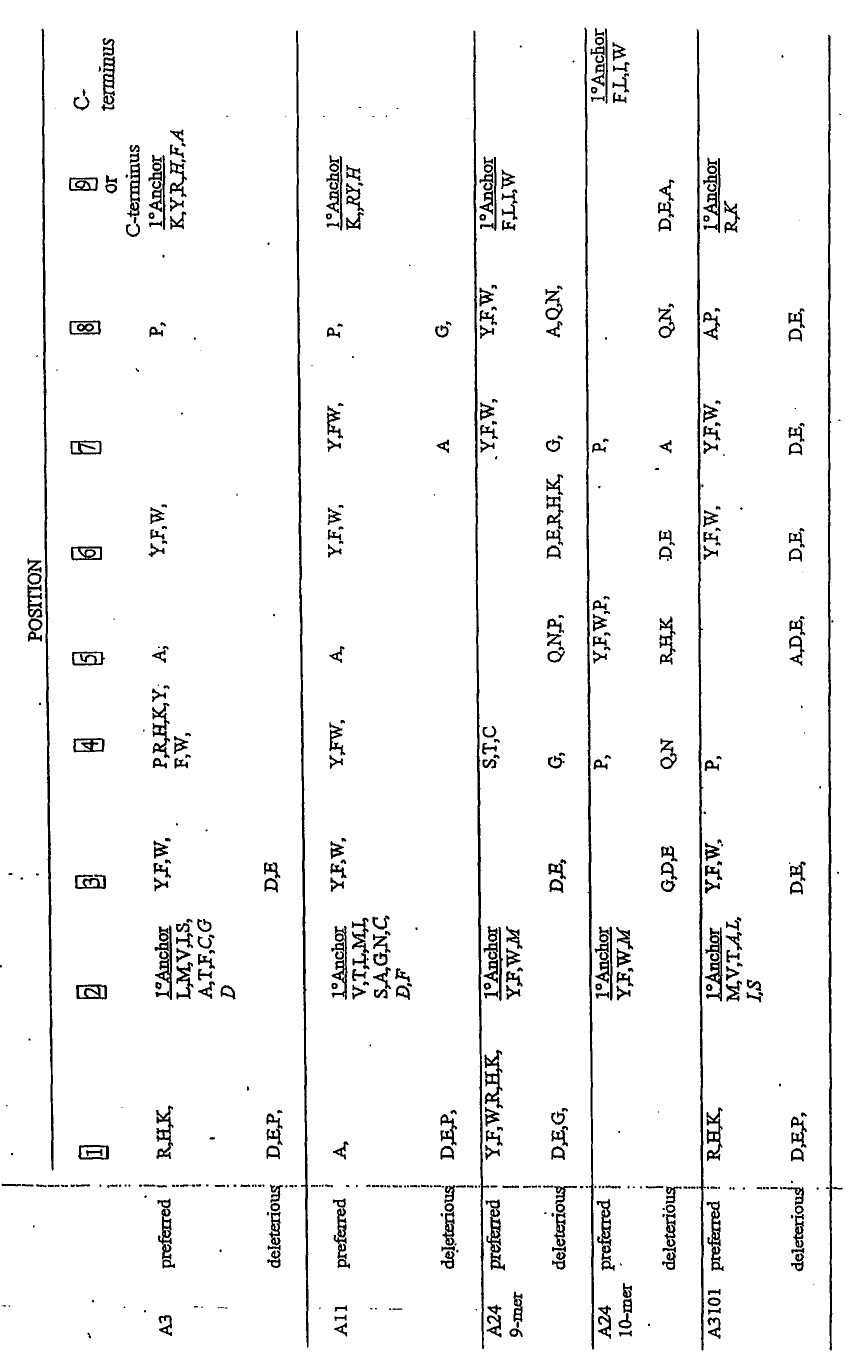

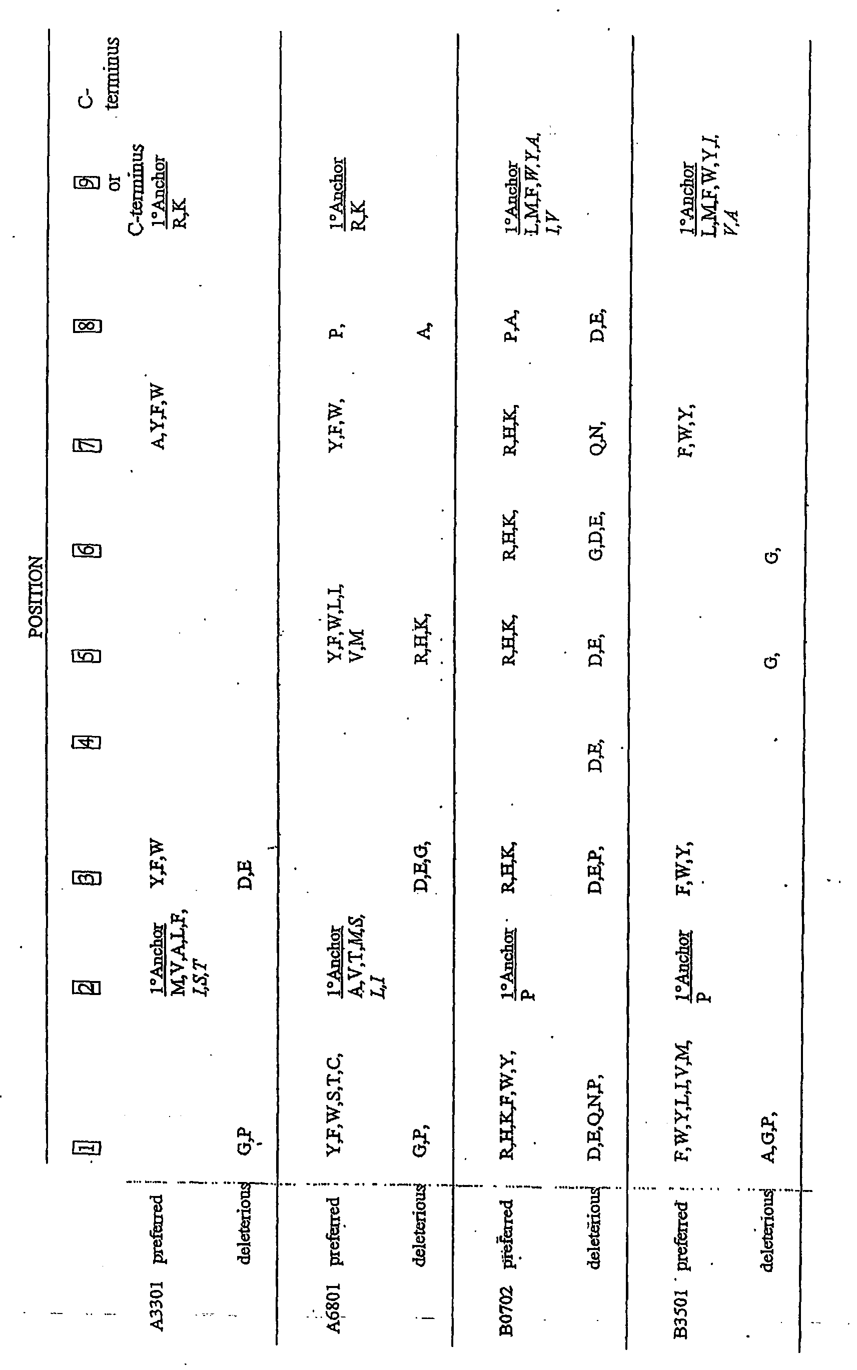

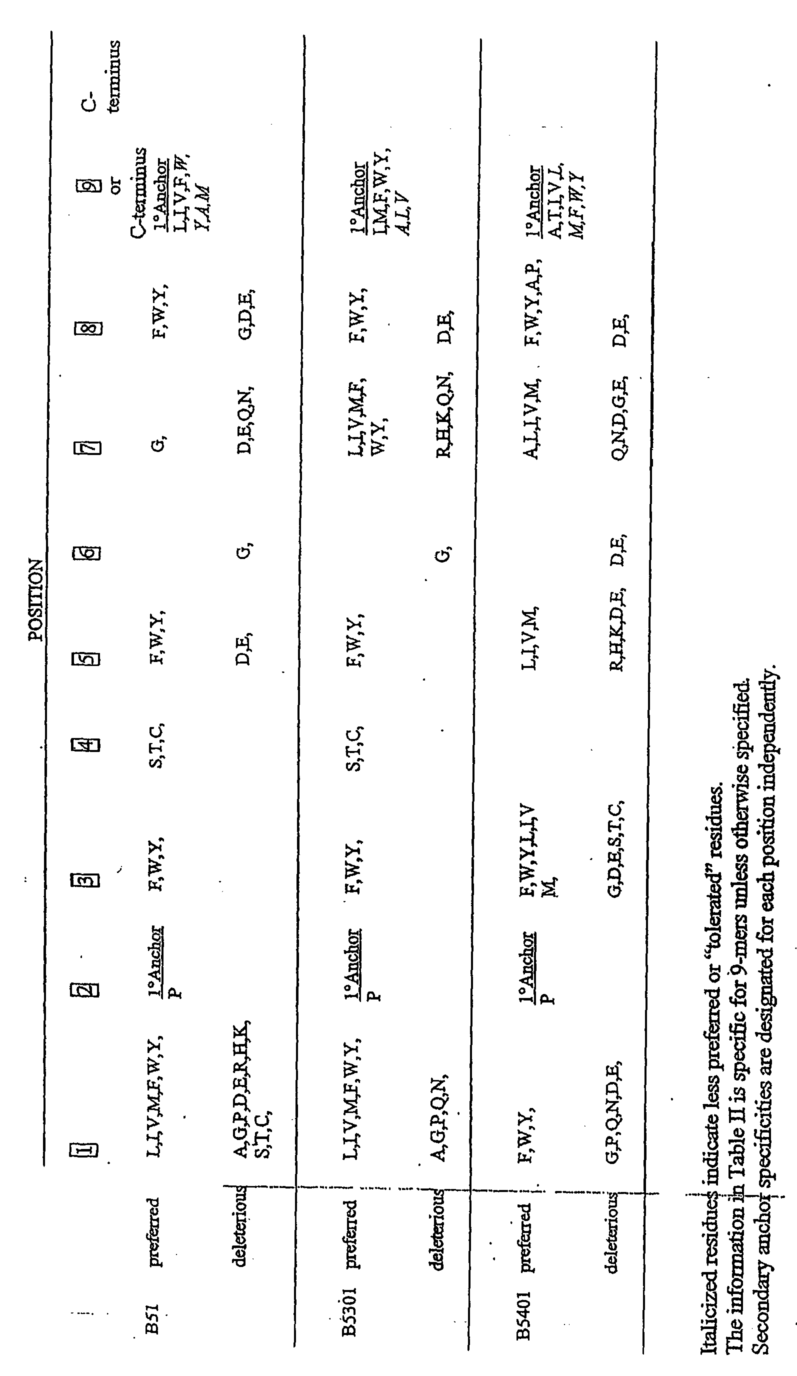

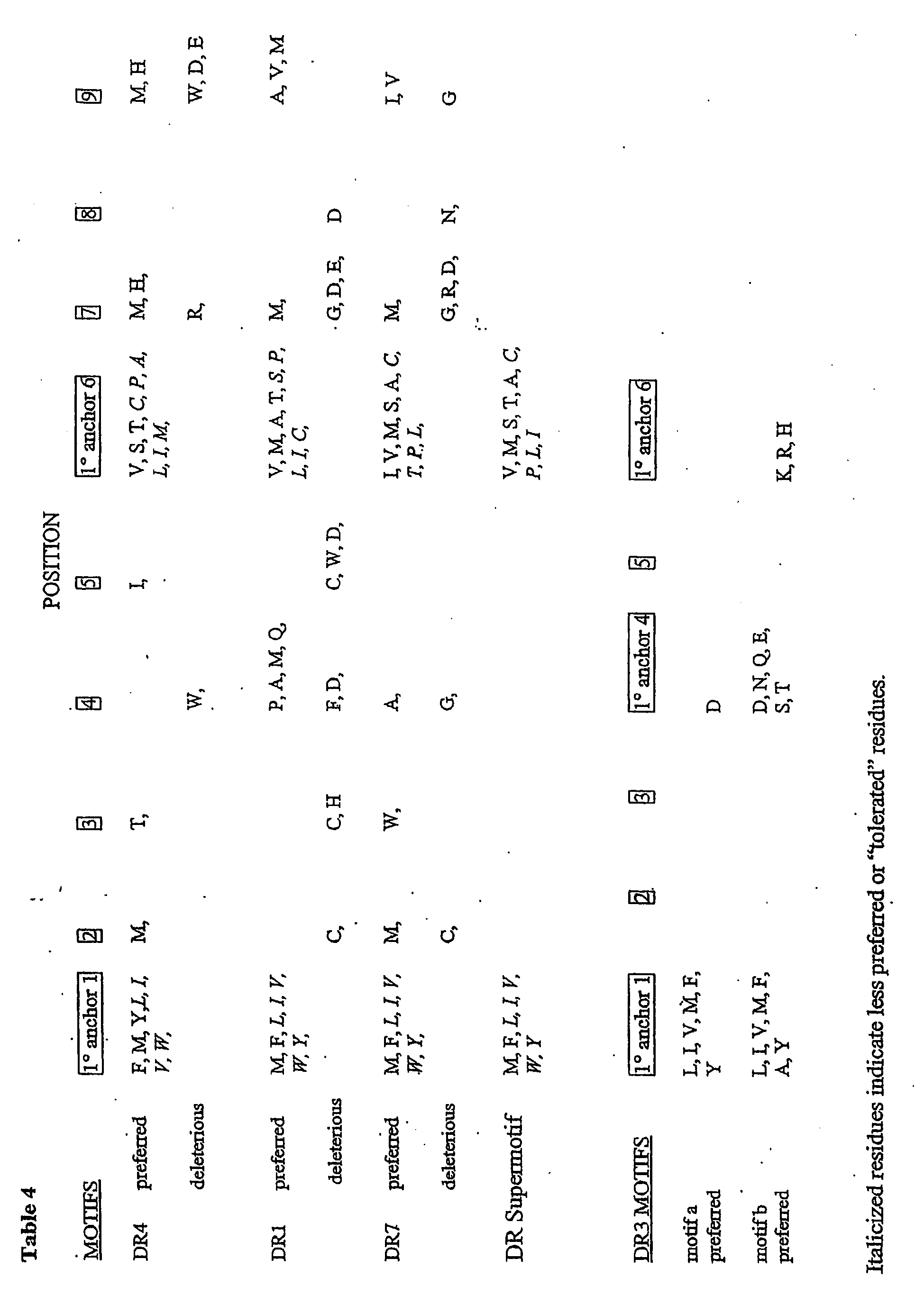

**Table 5**

| | | |
|---|---|---|
| | Allelle-specific HLA-supertype members | |
| HLA-supertype | Verified^{a} | Predicted^{b} |
| A1 | A*0101, A*2501, A*2601, A*2602, | A*0102, A*2604, A*3601, A*4301, |
| | A*3201, A*2902 | A*8001 |
| A2 | A*0201, A*0202, A*0203, A*0204, | A*0208, A*0210, A*0211, A*0212, |
| | A*0205, A*0206, A*0207, A*0209, | A*0213 |
| | A*0214, A*6802, A*6901 | |
| A3 | A*0301, A*1101, A*3101, A*3301, A*6801 | A*0302, A*1102, A*2603, A*3302, |
| | | A*3303, A*3401, A*3402, A*6601, |
| | | A*6602, A*7401 |
| A24 | A*2301, A*2402, A*3001 | A*2403, A*2404, A*3002, A*3003 |
| B7 | B*0702, B*0703, B*0704, B*0705, B*1508, | B*1511, B*4201, B*5901 |
| | B*3501, B*3502, B*3503, B*3503, B*3504, | |
| | B*3505, B*3506, B*3507, B*3508, B*5101, | |
| | B*5102, B*5103, B*5104, B*5105, B*5301, | |
| | B*5401, B*5501, B*5502, B*5601, B*5602, | |
| | B*6701, B*7801 | |
| B27 | B*1401, B*1402, B*1509, B*2702, B*2703, | B*2701, B*2707, B*2708, B*3802, |
| | B*2704, B*2705, B*2706, B*3801, B*3901, | B*3903, B*3904, B*3905, B*4801, |
| | B*3902, B*7301 | B*4802, B*1510, B*1518, B*1503 |
| B44 | B*1801, B*1802, B*3701, B*4402, B*4403, | B*4101, B*4501, B*4701, B*4901, |
| | B*4404, B*4001, B*4002, B*4006 | B*5001 |
| B58 | B*5701, B*5702, B*5801, B*5802, B*1516, | |
| | B*1517 | |
| B62 | B*1501, B*1502, B*1513, B*5201 | B*1301, B*1302, B*1504, B*1505, |
| | | B*1506, B*1507, B*1515, B*1520, |
| | | B*1521, B*1512, B*1514, B*1510 |

| | | |
|---|---|---|
| a. Verified alleles include alleles whose specificity has been determined by pool sequencing analysis, peptide binding assays, or by analysis of the sequences of CTL epitopes. b. Predicted alleles are alleles whose specificity is predicted on the basis of B and F pocket structure to overlap with the supertype specificity. | | |

**Table 6: Identified CTL Epitopes for an A2 Vaccine**

| Source | Sequence (SEQ ID NO:) | HLA-A2 Binding Affinity (IC50 nM) | | | | | | CTL¹ | | |
|---|---|---|---|---|---|---|---|---|---|---|
| | | A*0201 | A*0202 | A*0203 | A*0206 | A*6802 | No. A2 Alleles Bound | Sequence | Wild-type Sequence⁴ | Tumor |
| CEA.24V9 | LLTFWNPPV (19) | 16 | 307 | 26 | 56 | 952 | 4 | 1/1 | TBD² | 1/1 |
| CEA.233V10 | VLYGPDAPTV (1) | 26 | 430 | 16 | 206 | 952 | 4 | 3/4 | 2/2 | 1/4 |
| CEA.605V9 | YLSGANLNV (2) | 73 | 13 | 13 | 80 | 1600 | 4 | 4/4 | 3/4 | 1/4 |
| CEA.687 | ATVGIMIGV (3) | 36 | 8.8 | 20 | 11 | 0.80 | 5 | 1/1 | 1/1 | 1/1 |
| CEA.691 | IMIGVLVGV (16) | 69 | 62 | 13 | 106 | 89 | 5 | 8/8 | 8/8 | 4/7 |
| p53.25V11 | LLPENNVLSPV (4) | 38 | 4 | 4 | 9 | 30 | 5 | 2/3 | 1/3 | 1/3 |
| p53.139L2 | KLCPVQLWV (5) | 122 | 239 | 29 | 23 | -³ | 4 | 2/5 | 2/3 | 1/3 |
| p53.139L2B3 | KLBPVQLWV (6) | 34 | 8.7 | 20 | 11 | - | 4 | 3/4 | 2/3 | 1/2 |
| p53.149L2 | SLPPPGTRV (7) | 122 | 226 | 13 | 9250 | 140 | 4 | 2/3 | 1/3 | 0/3 |
| p53.149M2 | SMPPPGTRV (8) | 172 | 215 | 13 | 425 | 667 | 4 | 2/4 | 2/4 | 2/4 |
| Her2/neu.5 | ALCRWGLLL (12) | 100 | -² | 278 | - | - | 2 | 2/2 | 2/2 | 2/2 |
| Her2/neu.48 | HLYQGCQVV (14) | 139 | 307 | 13 | 514 | 1143 | 3 | 3/4 | 3/4 | 1/3 |
| Her2/neu.369 | KIFGSLAFL (17) | 36 | 9 | 19 | 23 | 3333 | 4 | 10/11 | 10/11 | 7/11 |
| Her2/neu. | KLFGSLAFV (9) | 5.8 | 7.5 | 19 | 17 | 1270 | 4 | 4/4 | 3/4 | 2/4 |
| 369L2V9 | | | | | | | | | | |
| Her2/neu. | KVFGSLAFV (10) | 20 | 19 | 769 | 15 | 29 | 4 | 4/4 | 3/4 | 2/4 |
| 369V2V9 | | | | | | | | | | |
| Her2/neu.435 | ILHNGAYSL (15) | 75 | 358 | 100 | 569 | - | 3 | 5/5 | 5/5 | 3/5 |
| Her2/neu.665 | VVLGVVFGI (23) | 14 | - | 2500 | 430 | 2000 | 2 | 4/8 | 4/8 | 1/1 |
| Her2/neu.689 | RLLQETELV (22) | 21 | - | 625 | 34 | - | 2 | 4/8 | 4/8 | 1/1 |
| Her2/neu.773 | VMAGVGSPYV (11) | 200 | 391 | 13 | 3700 | - | 3 | 2/4 | 2/4 | 1/4 |
| Her2/neu.952, | YMIMVKCWMI (25) | 20 | 307 | 83 | 116 | 267 | 5 | 2/3 | 2/3 | 2/3 |
| MAGE2157 | YLQLVFGIEV (24) | 50 | 165 | 345 | 370 | 9302 | 4 | 3/3 | 3/3 | 1/3 |
| MAGE3.159 | QLVFGIELMEV (21) | 7.9 | 74 | 217 | 185 | 267 | 5 | 3/3 | 3/3 | 1/3 |
| MAGE3.112 | KVAELVHFL (18) | 69 | 29 | 14 | 168 | 17 | 5 | 3/4 | 3/4 | 3/4 |
| MAGE3.160 | LVFGIELMEV (20) | 29 | 20 | 7.7 | 28 | 14 | 5 | 4/4 | 4/4 | 1/4 |
| MAGE3.271 | FLWGPRALV (13) | 31 | 43 | 14 | 336 | 40 | 5 | 4/4 | 4/4 | 2/4 |

| | | | | | | | | | | |
|---|---|---|---|---|---|---|---|---|---|---|
| 1) Number of donors yielding a positive response/ total tested. 2) To be determined 3) - indicates binding affinity ≤ 10,000 nM. 4) For peptides that are not analogs, "Sequence" and "Wild-type Sequence" provide the same information | | | | | | | | | | |

**Table 7. Expression of Tumor Associated Antigen (TAA)**

| **TAA** | **% of Tumors Expressing the TAA** | | |
|---|---|---|---|
| | **Colon Cancer** | **Breast Cancer** | **Lung Cancer** |
| CEA | 95 | 50 | 70 |
| P53 | 50 | 50 | 40-60 |
| MAGE 2/3 | 20-30 | 20-30 | 35 |
| HER2/neu | 28-50 | 30-50 | 20-30 |
| Total | 99 | 86-91 | 91-95 |

**Table 8. Incidence and survival rate of patients with breast, colon, or lung cancer in the United States**

| | **Estimated New Cases 1998** | **Estimate Deaths 1998** | **5-Year relative survival rates** | | |
|---|---|---|---|---|---|
| | | | **1974-76** | **1980-82** | **1986-1993** |
| Breast | 180,300 | 43,900 | 75% | 77% | 80% |
| Colon | 95,600 | 47,700 | 50% | 56% | 63% |
| Lung | 171,500 | 160,100 | 12% | 14% | 14% |

| | | | | | |
|---|---|---|---|---|---|
| Source: Cancer Statistics 1998. January/February 1998, Vol. 48, No. 1 | | | | | |

**Table 9: Summary of CTL Epitopes for an A2 Vaccine**

| Epitope ¹ | Sequence | A*0201 | A*0202 | A*0203 | A*0206 | A*6802 | No. A2 Members Cross-bound | CTL Recognition | |
|---|---|---|---|---|---|---|---|---|---|
| | (SEQ ID NO.) | IC₅₀ (nM)² | IC₅₀ (nM)² | IC₅₀ (nM)² | IC₅₀ (nM)² | IC₅₀ (nM)² | | Native Pulsed Cells | Tumor Cell |
| CEA.605V9 | YLSGANLNV (2) | 73³ | 13 | 13 | 80 | 1600 | 4 | + | + |
| CEA.691 | IMIGVLVGV (16) | 69 | 62 | 13 | 106 | 89 | 5 | + | + |
| | | | | | | | | | |
| p53.139L2B3 | KLBPVQLWV (6) | 34 | 8.7 | 20 | 11 | --⁴ | 4 | + | + |
| p53.149M2 | SMPPPGTRV (8) | 172 | 215 | 13 | 425 | 667 | 4 | + | + |
| | | | | | | | | | |
| MAGE3.112 | KVAELVHFL (18) | 69 | 29 | 14 | 168 | 17 | 5 | + | + |
| MAGE2.157 | YLQLVFGIEV (24) | 50 | 165 | 345 | 370 | 9302 | 4 | + | + |
| | | | | | | | | | |
| HER2/neu.689 | RLLQETELV (22) | 21 | -- | 625 | 34 | -- | 2 | + | + |
| HER2/neu.665 | WLGVVFGI (23) | 14 | -- | 2500 | 430 | 2000 | 2 | N.D. | + |

| | | | | | | | | | |
|---|---|---|---|---|---|---|---|---|---|
| 1) The peptide designations are derived from the target antigen (e.g. CEA) and the numeral relates to the first amino acid in the protein (*e.g.* 691). Analogs are noted by the amino acid inserted by substitution and the peptide position substituted (*e.g.* V9). 2) HLA binding was measured by a competitive binding assay where lower values indicate greater binding affinity. 3) Standard errors corresponding to HLA binding were presented in previous figures. 4) (--) indicates binding affinity > 10,000 nM. | | | | | | | | | |

**Table 10: Identified CTL Epitopes for an A2 Vaccine**

| Source | Sequence (SEQ ID NO:) | HLA-A2 Binding Affinity (IC50 nM) | | | | | | CTL¹ | | |
|---|---|---|---|---|---|---|---|---|---|---|
| | | A*0201 | A*0202 | A*0203 | A*0206 | A*6802 | No. A2 Alleles Bound | Sequence | Wild-type Sequence⁴ | Tumor |
| CEA.24V9 | LLTFWNPPV (19) | 16 | 307 | 26 | 56 | 952 | 4 | 1/1 | TBD² | 1/1 |
| CEA.233V10 | VLYGPDAPTV (1) | 26 | 430 | 16 | 206 | 952 | 4 | 3/4 | 2/2 | 1/4 |
| CEA.687 | ATVGIMIGV (3) | 36 | 8.8 | 20 | 11 | 0.80 | 5 | 1/1 | 1/1 | 1/1 |
| P53.25V11 | LLPENNVLSPV (4) | 38 | 4 | 4 | 9 | 30 | 5 | 2/3 | 1/3 | 1/3 |
| P53.139L2 | KLCPVQLWV (5) | 122 | 239 | 29 | 23 | --³ | 4 | 2/5 | 2/3 | 1/3 |
| Her2/neu.369 | KIFGSLAFL (17) | 36 | 9 | 19 | 23 | 3333 | 4 | 10/11 | 10/11 | 7/11 |
| Her2/neu. 369V2V9 | KVFGSLAFV (10) | 20 | 19 | 769 | 15 | 29 | 4 | 4/4 | 3/4 | 2/4 |
| Her2/neu.952 | YMIMVKCWMI (25) | 20 | 307 | 83 | 116 | 267 | 5 | 2/3 | 2/3 | 2/3 |
| MAGE3.159 | QLVFGIELMEV (21) | 7.9 | 74 | 217 | 185 | 267 | 5 | 3/3 | 3/3 | 1/3 |
| MAGE3.160 | LVFGIELMEV (20) | 29 | 20 | 7.7 | 28 | 14 | 5 | 4/4 | 4/4 | 1/4 |

| | | | | | | | | | | |
|---|---|---|---|---|---|---|---|---|---|---|
| 1) Number of donors yielding a positive response/total tested. 2) To be determined 3) - indicates binding affinity ≤ 10,000 nM. 4) For peptides that are not analogs, "Sequence" and "Wild-type Sequence" provide the same information | | | | | | | | | | |

**Table 11. Population coverage by HLA class I supertype epitopes.**

| | | Minimal Allelic Frequency | | | | | |
|---|---|---|---|---|---|---|---|
| Supertype | Representative HLA Molecules* | Caucasian | Black | Japanese | Chinese | Hispanic | Average |
| A2 | 2.1, 2.2, 2.3, 2.5, 2.6, 2.7, 68.02 | 45.8 | 39.0 | 42.4 | 45.9 | 43.0 | 43.2 |
| A3 | 3, 11, 31, 33, 68.01 | 37.5 | 42.1 | 45.8 | 52.7 | 43.1 | 44.2 |
| B7 | 7, 51, 53, 35, 54 | 43.2 | 55.1 | 57.1 | 43.0 | 49.3 | 49.5 |
| Total Population Coverage | | 84.3 | 86.8 | 89.5 | 89.8 | 86.8 | 87.4 |

| | | | | | | | |
|---|---|---|---|---|---|---|---|
| For A2, all A2 subtypes were included; for A3, the five listed allotypes were used; for B7, several additional allotypes were included based on binding pocket analysis. | | | | | | | |

**Table 12: Tumor Associated Antigens and Genes (TAA)**

| **ANTIGEN** | **REFERENCE** |
|---|---|
| MAGE 1 | (Traversari C., Boon T, J.Ex. Med 176:1453, 1992) |
| MAGE 2 | (De Smet C., Boon T, Immunogenetics, 39(2)121-9, 1994) |
| MAGE 3 | (Gaugler B., Boon T, J.Ex. Med 179: 921, 1994) |
| MAGE-11 | (Jurk M., Winnacker L, Int.J.Cancer 75, 762-766, 1998) |
| MAGE-A10 | (Huang L., Van Pel A, J.Immunology, 162:6849-6854) |
| BAGE | (Boel P., Bruggen V, Immunity 2:167, 1995) |
| GAGE | (Eynde V., Boon T, J.Exp. Med 182:689, 1995) |
| RAGE | (Gaugler B., Eynde V, Immunogenetics, 44:325, 1996) |
| MAGE-C1 | (Lucas S., Boon T, Cancer Research, 58, 743-752, 1998) |
| LAGE-1 | (Lethel B., Boon T, Int J cancer, 10; 76(6) 903-908 |
| CAG-3 | (Wang R--Rosenberg S, J.Immunology, 161:3591-3596, 1998) |
| DAM | (Fleischhauer K., Traversari C, Cancer Research, 58,14,2969, 1998) |
| MUC1 | (Karanikas V., McKenzie IF, J.clnical investigation, 100:11, 1-10, 1997) |
| MUC2 | (Bohm C., Hanski, Int.J.Cancer 75, 688-693, 1998) |
| MUC18 | (Putz E., Pantel K, Cancer Res 59(1):241-248, 1999) |
| NY-ESO-1 | (Chen Y., Old LJ PNAS, 94, 1914-18, 1997) |
| MUM-1 | (Coulie P., Boon T, PNAS 92:7976, 1995) |
| CDK4 | (Wolfel T., Beach D, Science 269:1281, 1995) |
| BRCA2 | (Wooster R---Stratton M, Nature, 378, 789-791, 1995) |
| NY-LU-1 | (Gure A., Chen, Cancer Research, 58, 1034-41, 1998) |
| NY-LU-7 | (Gure A., Chen, Cancer Research, 58, 1034-41, 1998) |
| NY-LU-12 | (Gure A., Chen, Cancer Research, 58, 1034-41, 1998) |
| CASP8 | (Mandruzzato S., Bruggen P, J.Ex.Med 186, 5, 785-793, 1997) |
| RAS | (Sidransky D., Vogelstein B, Science, 256:102) |
| KIAA0205 | (Gueguen M., Eynde, J.Immunology, 160:6188-94, 1998) |
| SCCs | (Molina R., Ballesta AM, Tumor Biol, 17(2):81-9, 1996) |
| p53 | (Hollstein M., Harris CC, Science, 253, 49-53, 1991) |
| p73 | (Kaghad M., Caput D, Cell; 90(4):809-19, 1997) |
| CEA | (Muraro R., Schlom J, Cancer Research, 45:5769-55780, 1985) |
| Her 2/neu | (Disis M., Cheever M, Cancer Res 54:1071, 1994) |
| Melan-A | (Coulie P., Boon T, J.Ex.Med, 180:35, 1994) |
| gp100 | (Bakker A., Figdor, J.Ex.Med 179:1005, 1994) |
| Tyrosinase | (Wolfel T., Boon T, E.J.I 24:759, 1994) |
| TRP2 | (Wang R., Rosenberg S.A, J.Ex.Med 184:2207, 1996) |
| gp75/TRP1 | (Wang R., Rosenberg S.A, J.Ex.Med 183:1131, 1996) |
| PSM | (Pinto J. T., Heston W.D.W., Clin Cancer Res 2(9); 1445-1451, 1996) |
| PSA | (Correale P., Tsang K, J. Natl cancer institute, 89:293-300, 1997) |
| PT1-1 | (Sun Y., Fisher PB, Cancer Research, 57(1):18-23, 1997) |
| B-catenin | (Robbins P., Rosenberg SA, J.Ex. Med 183:1185, 1996) |
| PRAME | (Neumann E., Seliger B, Cancer Research, 58, 4090-4095, 1998) |
| Telomearse | (Kishimoto K., Okamoto E, J Surg Oncol, 69(3):119-124, 1998) |
| FAK | (Kornberg LJ, Head Neck, 20(8):745-52, 1998) |
| Tn antigen | (Wang B1, J Submicrosc Cytol Path, 30(4):503-509, 1998) |
| cyclin D1 protein (Linggui K., Yaowu Z, Cancer Lett 130(1-2), 93-101, 1998) | |
| NOEY2 | (Yu Y., Bat RC, PNAS, 96(1):214-219, 1999) |
| EGF-R | (Biesterfeld S.---- Cancer Weekly, Feb15, 1999) |
| SART-1 | (Matsumoto H., Itoh K, Japanese Journal of Cancer Research, 59, iss12,1292-1295,1998) |
| CAPB | (Cancer Weekly, March 29,4-5,1999) |
| HPVE7 | (Rosenberg S.A.Immunity, 10, 282-287, 1999) |
| p15 | (Rosenberg S.A., Immunity, 10, 282-287, 1999) |
| Folate receptor | (Gruner B.A., Weitman S.D., Investigational New Drugs, Voll6, iss3, 205-219, 1998) |
| CDC27 | (Wang R.F., Rosenberg SA, Science, vol 284, 1351-1354, 1999) |
| PAGE-1 | (Chen, J. Biol. Chem: 273:17618-17625,1998) |
| PAGE-4 | (Brinkmann: PNAS, 95:10757,1998) |
| Kallikrein 2 | (Darson:Urology, 49:857-862, 1997) |
| PSCA | (Reiter R., PNAS, 95:1735-1740, 1998) |
| DD3 | (Bussemakers M.J.G, European Urology, 35:408-412, 1999) |
| RBP-1 | (Takahashi T., British Journal of Cancer, 81(2):342-349, 1999) |
| RU2 | (Eybde V.D., J.Exp.Med, 190 (12):1793-1799, 1999) |
| Folate binding | (Kim D., Anticancer Research, 19:2907-2916, 1999) |
| protein | |
| EGP-2 | (Heidenreich R., Human Gene Therapy, 11:9-19, 2000) |

**Table 13a. Phenotypic frequencies by supertype family.**

| | Minimal Population Coverage | | | | | |
|---|---|---|---|---|---|---|
| Allelle | Caucasian | North American Black | Japanese | Chinese | Hispanic | Average |
| A2 supertype | 45.8 | 39.0 | 42.4 | 45.9 | 43.0 | 43.2 |
| A3 supertype | 37.5 | 42.1 | 45.8 | 52.7 | 43.1 | 44.2 |
| B7 supertype | 38.6 | 52.7 | 48.8 | 35.5 | 47.1 | 44.7 |
| A1 supertype | 47.1 | 16.1 | 21.8 | 14.7 | 26.3 | 25.2 |
| A24 supertype | 23.9 | 38.9 | 58.6 | 40.1 | 38.3 | 40.0 |
| Total | 99.0 | 97.3 | 100.0 | 99.1 | 98.8 | 98.8 |

**Table 13b. Phenotypic frequencies by motif type in current study.**

| | Minimal Population Coverage | | | | | |
|---|---|---|---|---|---|---|
| Allelle | Caucasian | North American Black | Japanese | Chinese | Hispanic | Average |
| A2 supertype | 45.8 | 39.0 | 42.4 | 45.9 | 43.0 | 43.2 |
| A3 supertype | 37.5 | 42.1 | 45.8 | 52.7 | 43.1 | 44.2 |
| B7 supertype | 38.6 | 52.7 | 48.8 | 35.5 | 47.1 | 44.7 |
| A1 | 28.6 | 10.1 | 1.4 | 9.2 | 10.1 | 11.9 |
| A24 | 16.8 | 8.8 | 58.1 | 32.9 | 26.7 | 28.7 |
| Total | 95.1 | 90.6 | 99.1 | 97.5 | 94.8 | 95.4 |

**Table 14 Sequence motifs associated with binding-MHC specificity analyzed in the current study**

| | Anchor position | | |
|---|---|---|---|
| Allelle | p2 | p3 | C terminus |
| A3 supertype | LMIVAST | -- | KR |
| B7 supertype | P | -- | LMIVFWYA |
| A1 | TSM or | DEAS | YA |
| A24 | YFWM | -- | FLIW |

| | | | |
|---|---|---|---|
| -- indicates any naturally occurring residue accepted. | | | |

**Table 15 Motif-positive peptides derived from target TAA**

| | | A3 supertype | | B7 supertype | | A1 | | A24 | | |
|---|---|---|---|---|---|---|---|---|---|---|
| Antigen | | Wildtype | Analog ¹ | Wildtype | Analog | Wildtype | Analog | Wildtype | Analog | Total |
| CEA | Motif-positive | 60 | 113 | 79 | 205 | 50 | 81 | 46 | 61 | 695 |
| | Tested | 52 | 12 | 79 | 92 | 48 | 17 | 15 | 13 | 358 |
| Her2/neu | Motif-positive | 107 | 194 | 85 | 237 | 32 | 55 | 43 | 69 | 822 |
| | Tested | 53 | 11 | 85 | 128 | 26 | 55 | 41 | 6 | 364 |
| Mage2/3 | Motif-positive | 63 | 113 | 61 | 166 | 27 | 49 | 51 | 81 | 611 |
| | Tested | 63 | 17 | 61 | 85 | 27 | 13 | 46 | 8 | 321 |
| p53 | Motif-positive | 78 | 147 | 64 | 188 | 15 | 24 | 20 | 30 | 566 |
| | Tested | 78 | 24 | 62 | 103 | 14 | 5 | 12 | 2 | 300 |
| Total | Motif-positive | 308 | 567 | 289 | 796 | 124 | 209 | 160 | 241 | 2694 |
| | Tested | 247 | 64 | 287 | 408 | 115 | 49 | 144 | 29 | 1343 |

| | | | | | | | | | | |
|---|---|---|---|---|---|---|---|---|---|---|
| 1) Numbers refer to maximum analogs possible substituting at the p2 or C-terminus anchor position. Substitution at both anchors concurrently not considered. For the B7 supertype, analog numbers presented also include F1 substitutions, a non-anchor substitution. | | | | | | | | | | |

**Table 16a. CEA-derived A3 supertype crossbinders**

| | | | HLA Binding (IC₅₀ nM) | | | | | | Number of CTL inductions yielding ¹⁾ positive responses / total tested | |
|---|---|---|---|---|---|---|---|---|---|---|
| SEQ ID NO | Sequence | Source | A*0301 | A*1101 | A*3101 | A*3301 | A*6801 | Number of A3 alleles crossbound | Wildtype-pulsed EHM target cell line | Tumor target ²⁾ cell line |
| 42 | HLFGYSWYK | CEA.61 | 22 | 24 | 20 | 18 | 3.5 | 5 | 3/4 | 2/4 |
| 43 | TISPLNTSYR | CEA241 | 1594 | 158 | 207 | 569 | 44 | 3 | | |
| 44 | TISPLNTSYK | CEA.241.K10 | 61 | 182 | -³⁾ | - | 116 | 3 | 1/1 | 0/1 |
| 45 | TVSPLNTSYR | CEA.241.V2 | 458 | 55 | 188 | 558 | 8.7 | 4 | | |
| 46 | RTLTLLSVTR | CEA.376 | 524 | 55 | 6.0 | 1036 | 160 | 3 | | |
| 47 | RVLTLLSVTR | CEA376.V2 | 344 | 222 | 11 | 6042 | 667 | 3 | | |
| 48 | PTISPSYTYYR | CEA418 | - | 46 | 44 | 784 | 57 | 3 | | |
| 49 | TISPSYTYYR | CEA419 | 3438 | 21 | 72 | 171 | 3.1 | 4 | | |
| 50 | ISPSYTYYR | CEA420 | 1342 | 143 | 21 | 518 | 11 | 3 | | |
| 51 | IVPSYTYYR | CEA.420.V2 | 92 | 13 | 26 | 58 | 2.6 | 5 | | |
| 52 | RTLTLFNVTR | CEA.554 | 111 | 13 | 5.0 | 1611 | 99 | 4 | 1/1 | nt |
| 53 | RVLTLFNVTR | CEA.554.V2 | 297 | 94 | 9.0 | 7632 | 42 | 4 | | |
| 54 | HTQVLFIAK | CEA.636 | 1183 | 35 | 106 | 132 | 160 | 4 | | |
| 55 | FVSNLATGR | CEA.656 | 5790 | 122 | 333 | 104 | 8.2 | 4 | | |

| | | | | | | | | | | |
|---|---|---|---|---|---|---|---|---|---|---|
| 1) A positive response is defined as 10% specific lysis above background in the ⁵¹Cr release assay or twice background and at least 50pg IFNγ/well above background in at least one of 48 wells tested. 2) The tumor target cell lines used are SW480 (A3 ; CEA+) and SW403 (A3+, CEA+). Only tumor target data from wells positive for recognition of peptide-pulsed target cells is shown. 3) -indicates binding affinity = 10,000nM. | | | | | | | | | | |

**Table 16b. Her2/neu-derived A3 supertype crossbinders**

| | | | HLA Binding (IC₅₀ nM) | | | | | | Number of CTL inductions yielding ¹⁾ positive responses / total tested | |
|---|---|---|---|---|---|---|---|---|---|---|
| SEQ ID NO | Sequence | Source | A*0301 | A*1101 | A*3101 | A*3301 | A*6801 | Number of A3 alleles crossbound | Wildtype-pulsed EHM target cell line | Tumor target²⁾ cell line |
| 56 | GVVFGILIKR | Her2/neu.668 | 611 | 182 | 305 | 2071 | 19 | 3 | | |
| 57 | VVFGILIKR | Her2/neu.669 | 100 | 8.3 | 13 | 78 | 4.0 | 5 | 1/3 | 0/1 |
| 58 | VVFGILIKRR | Her2/neu.669 | 3667 | 375 | 290 | 193 | 15 | 4 | | |
| 59 | VVFGILIKK | Her2/neu.669.K9 | 22 | 19 | 3750 | - ³⁾ | 35 | 3 | | |
| 60 | KERKYTMRR | Het2/neu.681 | 15 | 3333 | 16 | 4028 | - | 2 | 3/3 | 2/3 |
| 61 | KIRKYTMRK | Her2/neu.681.K9 | 177 | 1091 | 6429 | - | - | 1 | | |
| 62 | VLRENTSPK | Her2/neu.754 | 28 | 462 | 129 | 290 | - | 4 | 4/7 | 3/6 |
| 63 | VVRENTSPR | Her2/neu.754.V2R9 | 200 | 5455 | 375 | 126 | 178 | 4 | | |
| 64 | LLDHVRENR | Her2/neu.806 | 297 | - | 500 | 326 | 5714 | 3 | | |
| 65 | LAARNVLVK | Her2/neu.846 | 190 | 211 | - | - | 500 | 3 | | |
| 66 | LVAENVLVK | Her2/neu.846.V2 | 42 | 214 | 9000 | - | 205 | 3 | | |
| 67 | LVKSPNHVK | Her2/neu.852 | 23 | 86 | 182 | 784 | 73 | 4 | 0/3 | 0/1 |
| 68 | KTTDFGLAR | Her2/neu.860 | 65 | 25 | 100 | - | 1633 | 3 | 0/1 | nt |
| 69 | KVTDFGLAR | Her2/neu.860.V2 | 201 | 76 | 106 | - | 133 | 4 | | |
| 70 | MALESILRR | Her2/neu.889 | 3235 | 253 | 191 | 132 | 127 | 4 | | |
| 71 | MVLESILRR | Hei2/neu.889.V2 | 216 | 273 | 207 | 153 | 22 | 5 | | |
| 72 | MVLESILRK | Her2/neu.889.V2K9 | 61 | 16 | - | 2636 | 381 | 3 | | |
| 73 | LVSEFSRMAR | Her2/neu.972 | 1528 | 182 | 49 | 126 | 36 | 4 | | |
| 74 | LVSEFSRMAK | Het2/neu.972.K10 | 250 | 71 | 2250 | 5273 | 62 | 3 | | |
| 75 | ASPLDSTFYR | Her2/neu.997 | - | 90 | 150 | 2071 | 154 | 3 | | |

| | | | | | | | | | | |
|---|---|---|---|---|---|---|---|---|---|---|
| 1) A positive response is defined as 10% specific lysis above background in the ⁵¹Cr release assay or twice background and at least 50pg IFNγ/well above background in at least one or 48 wells tested. 2) The tumor target cell lines used are SW480 (A3-, CEA+) and SW403 (A3+, CEA+). Only tumor target data from wells positive for recognition of peptide-pulsed target cells is shown. 3)-indicates binding affinity =10,000nM. | | | | | | | | | | |

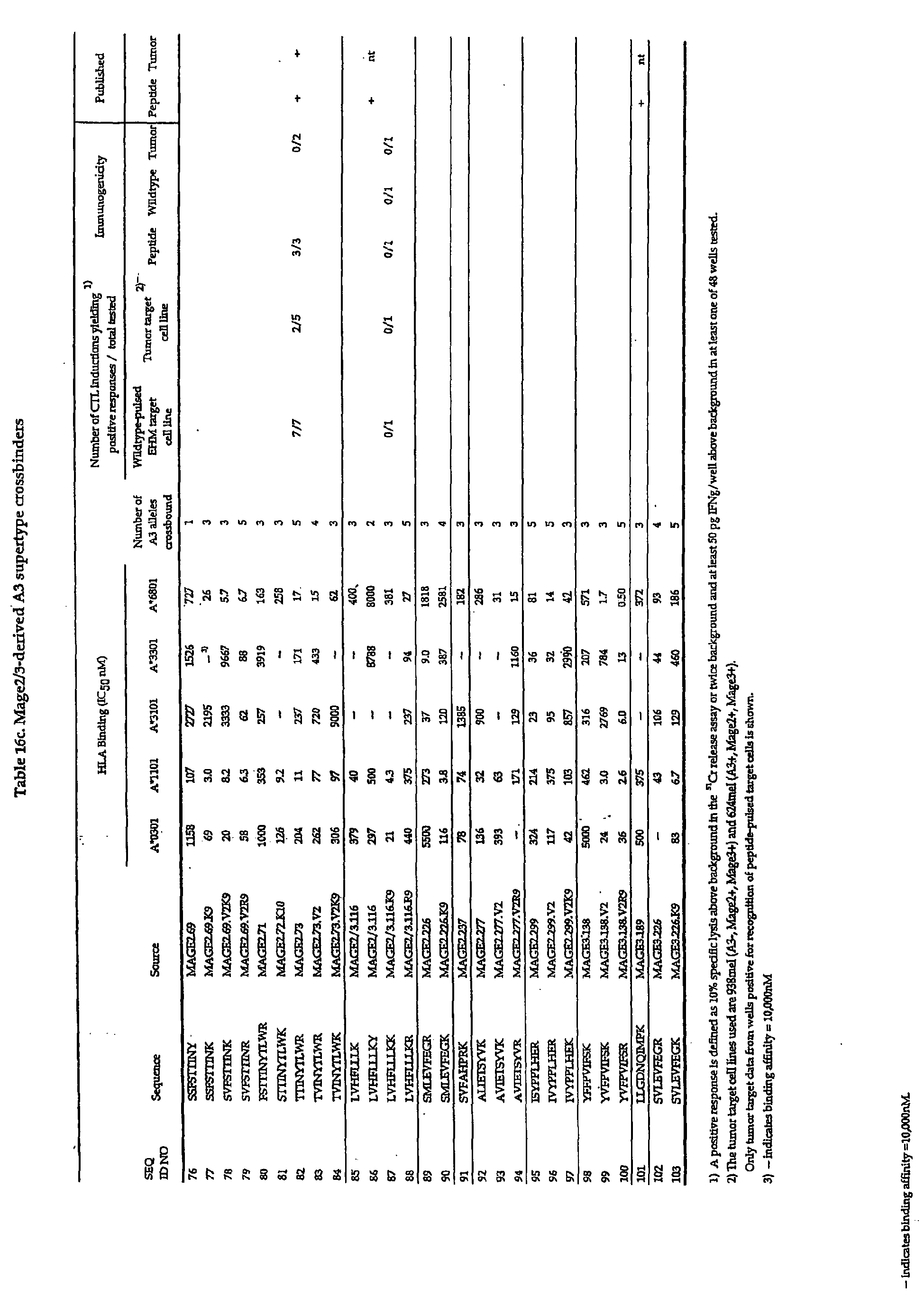

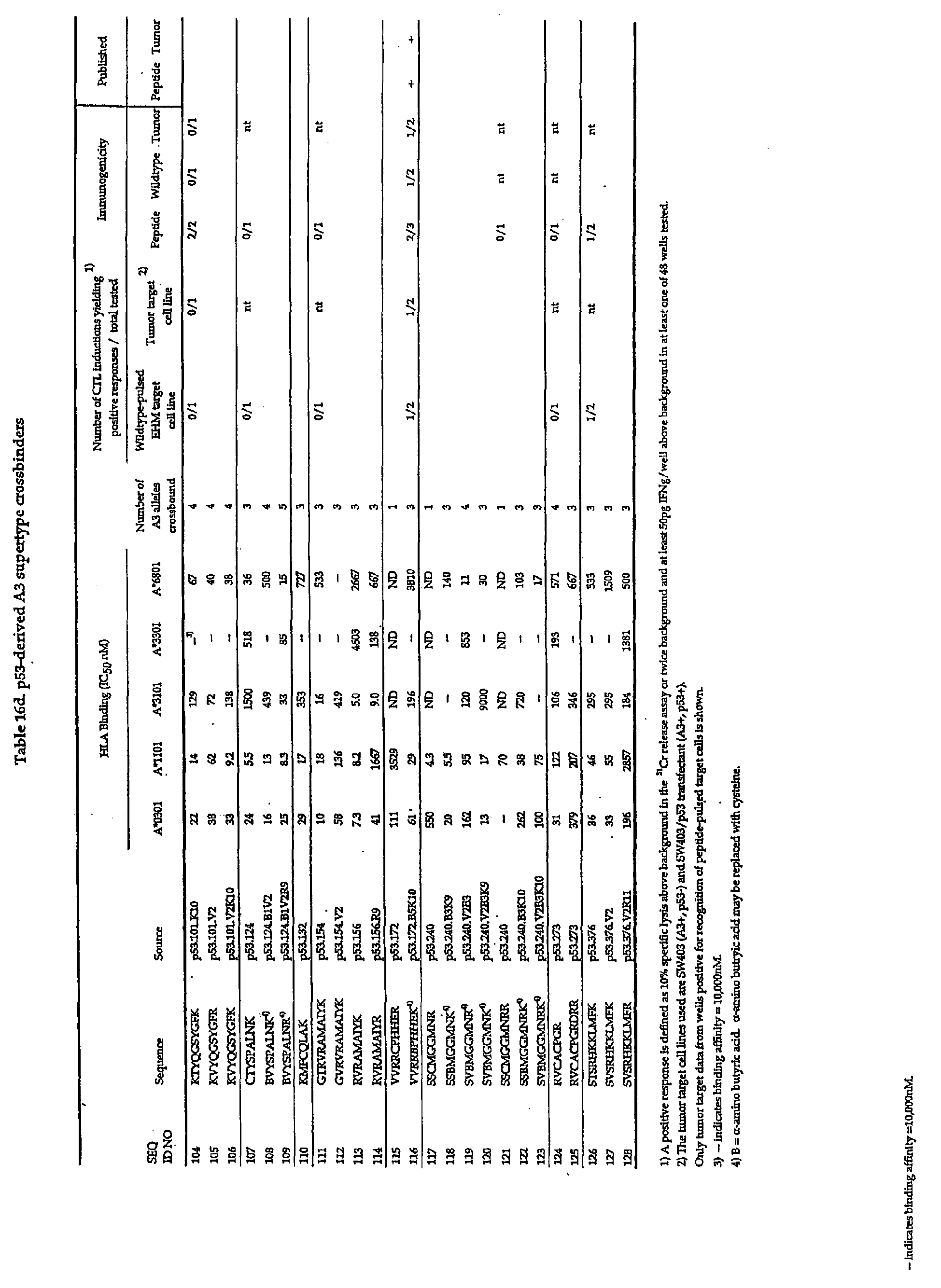

**Table 17a. CEA-derived B7 supertype crossbinders**

| | | | | | | | | | Immunogenicity | | |
|---|---|---|---|---|---|---|---|---|---|---|---|
| SEQ ID NO | Sequence | Source | B*0702 nM | B*3501 nM | B*5101 nM | B*5301 nM | B*5401 nM | Number of B7 alleles crossbound | Peptide | Wildtype | Tumor |
| 129 | IPWQRLLLTA | CEA.13 | 125 | - | 2115 | 2657 | 32 | 2 | | | |
| 130 | IPWQRLLLTI | CEA.13I10 | 39 | - | 19 | 291 | 270 | 4 | | 0/1 | |
| 131 | LPQHLFGYSW | CEA.58 | - | 9000 | - | 22 | 4348 | 1 | | | |
| 132 | LPQHLFGYSI | CEA.58I10 | 212 | - | 262 | 930 | 172 | 3 | | | |
| 133 | YPNASLLI | CFA.102 | 196 | 514 | 8.1 | 40 | 137 | 4 | | | |

| | | | | | | | | | | | |
|---|---|---|---|---|---|---|---|---|---|---|---|
| - indicates binding affinity =10,000nM. | | | | | | | | | | | |

**Table 17b. Her2/neu-derived B7 supertype crossbinders**

| | | | | | | | | | Immunogenicity | | |
|---|---|---|---|---|---|---|---|---|---|---|---|
| SEQ ID NO | Sequence | Source | B*0702 nM | B*3501 nM | B*5101 nM | B*5301 nM | B*5401 nM | Number of B7 alleles crossbound | Peptide | Wildtype | Tumor |
| 134 | KPYDGIPA | Her2/neu.921 | 367 | - | - | 490 | 29 | 3 | | | |
| 135 | KPYDGIPI | Her2/neu.92118 | 115 | - | 74 | 5167 | 303 | 3 | | | |
| 136 | LPQPPICTI | Hex2/neu.941 | 196 | - | 4.2 | 28 | 19 | 4 | | | |
| 137 | PPSPREGPLPA | Her2/neu.1149 | 12 | - | - | - | 83 | 2 | | | |
| 138 | PPSPREGPLPI | Her2/neu.1149.I11 | 22 | - | 423 | 85 | - | 3 | 1/2 | | 0/2 |
| 139 | SPAFDNLYI | Her2/neu.1214 | 290 | - | 229 | 155 | - | 3 | | | |

| | | | | | | | | | | | |
|---|---|---|---|---|---|---|---|---|---|---|---|
| - indicates binding affinity =10,00nM. | | | | | | | | | | | |

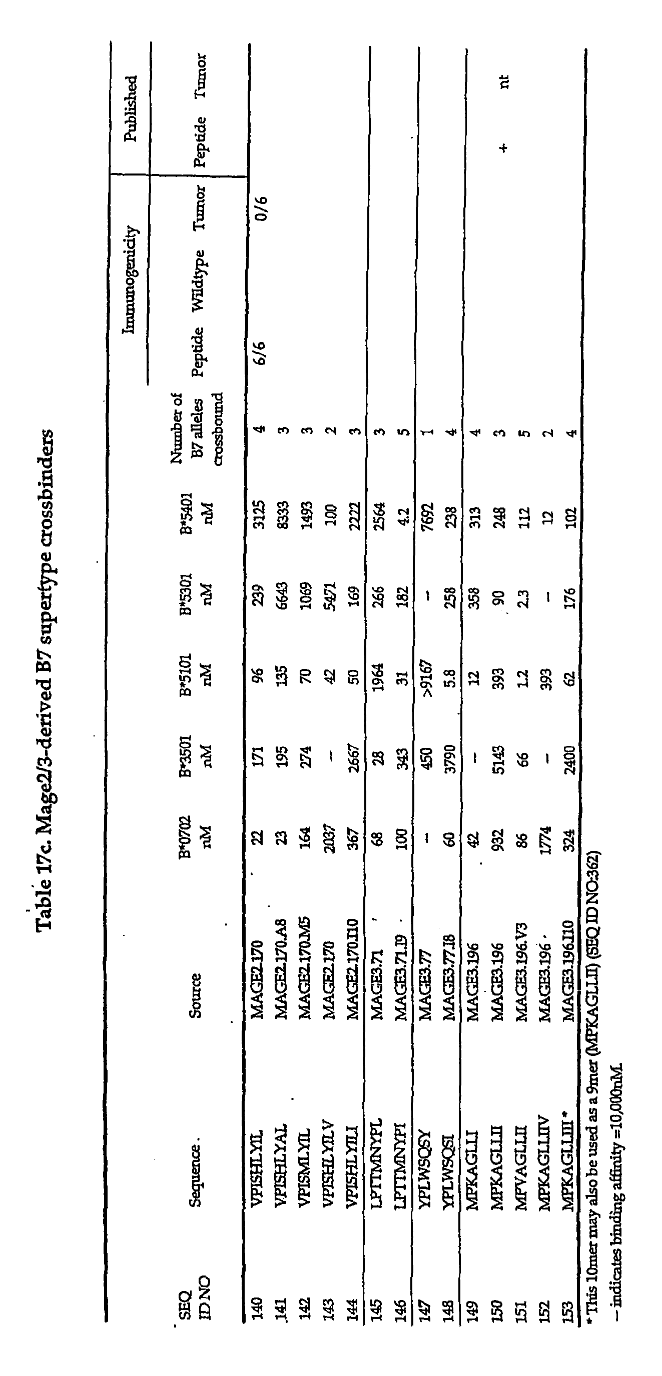

**Table 17d. p53-derived B7 supertype crossbinders**

| | | | | | | | | | Immunogenicity | | |
|---|---|---|---|---|---|---|---|---|---|---|---|
| SEQ ID NO | Sequence | Source | B*0702 nM | B*3501 nM | B*5101 nM | B*5301 nM | B*5401 nM | Number of B7 alleles crossbound | Peptide | Wildtype | Tumor |
| 154 | APAAPTPAA | p53.76 | 18 | 360 | - | - | 1.9 | 3 | | | |
| 155 | APAAPTPAAPA | p53.76 | 14 | 3273 | - | 93 | 53 | 3 | 0/1 | | nt |
| 156 | APAPAPSW | p53.84 | 500 | 3273 | - | 135 | 91 | 3 | | | |
| 157 | APAPAPSI | p53.84.I8 | 275 | - | 500 | 291 | - | 3 | | | |

| | | | | | | | | | | | |
|---|---|---|---|---|---|---|---|---|---|---|---|
| - indicates binding affinity =10,000nM. | | | | | | | | | | | |

**Table 18a. CEA-derived A1 binders**

| SEQ ID NO | AA | Sequence | Source | A*0101 nM |
|---|---|---|---|---|
| 158 | 11 | RVDGNRQIIGY | CEA.72 | 294 |
| 159 | 9 | QQATPGPAY | CEA.87 | -- |
| 160 | 9 | QQDTPGPAY | CEA.87.D3 | 57 |
| 161 | 11 | RSDSVILNVLY | CEA.225 | 47 |
| 162 | 10 | PTISPLNTSY | CEA.240 | 1000 |
| 163 | 10 | PTDSPLNTSY | CEA.240.D3 | 266 |
| 164 | 9 | AASNPPAQY | CEA.261 | -- |
| 165 | 9 | AADNPPAQY | CEA.261.D3 | 46 |
| 166 | 9 | ITVNNSGSY | CEA.289 | 2500 |
| 167 | 9 | ITDNNSGSY | CEA.289.D3 | 96 |
| 168 | 9 | VTRNDVGPY | CEA.383 | -- |
| 169 | 9 | VTDNDVGPY | CEA.383.D3 | 4.1 |
| 170 | 11 | HSDPVILNVLY | CEA.403 | 26 |
| 171 | 10 | PTISPSYTYY | CEA.418 | 325 |
| 172 | 10 | PTDSPSYTYY | CEA.418.D3 | 1.1 |
| 173 | 9 | PTISPSYTY | CEA.418 | 7143 |
| 174 | 9 | PTDSPSYTY | CEA.418.D3 | 38 |
| 175 | 9 | TISPSYTYY | CEA.419 | 1042 |
| 176 | 9 | TIDPSYTYY | CEA.419.D3 | 3.1 |
| 177 | 10 | HAASNPPAQY | CEA.438 | 2688 |
| 178 | 9 | AADNPPAQY | CEA.439.D3 | 45 |
| 179 | 9 | ITEKNSGLY | CEA.467 | 641 |
| 180 | 9 | ITDKNSGLY | CEA.467.D3 | 12 |
| 181 | 11 | RSDPVTLDVLY | CEA.581 | 7.8 |
| 182 | 10 | HSASNPSPQY | CEA.616 | 74 |
| 183 | 10 | HTASNPSPQY | CEA.616.T2 | 132 |
| 184 | 10 | HSDSNPSPQY | CEA.616.D3 | 45 |

| | | | | |
|---|---|---|---|---|
| -- indicates binding affinity =10,000nM. | | | | |

**Table 18b. Her2/neu-derived A1 binders**

| SEQ ID NO | AA | Sequence | Source | A*0101 nM |
|---|---|---|---|---|
| 185 | 9 | VMAGVGSPY | Her2/neu.773 | 625 |
| 186 | 9 | VMDGVGSPY | Her2/neu.773.D3 | 40 |
| 187 | 10 | CMQIAKGMSY | Her2/neu.826 | 83 |
| 188 | 10 | CTQIAKGMSY | Her2/neu.826.T2 | 19 |
| 189 | 9 | LLDIDETEY | Her2/neu.869 | 3.3 |
| 190 | 9 | LTDIDETEY | Her2/neu,869.T2 | 5.7 |
| 191 | 10 | FTHQSDVWSY | Her2/neu.899 | 9.3 |
| 192 | 10 | FTDQSDVWSY | Her2/neu.899.D3 | 0.60 |
| 193 | 10 | PASPLDSTFY | Her2/neu.996 | 1667 |
| 194 | 10 | PADPLDSTFY | Her2/neu.996.D3 | 19 |
| 195 | 9 | ASPLDSTFY | Her2/neu.997 | 862 |
| 196 | 9 | ATPLDSTFY . | Her2/neu.997.T2 | 36 |
| 197 | 10 | MGDLVDAEEY | Her2/neu.1014 | 2083 |
| 198 | 10 | MTDLVDAEEY | Her2/neu.1014.T2 | 2.3 |
| 199 | 9 | LTCSPQPEY | Her2/neu.1131 | 192 |
| 200 | 9 | LTDSPQPEY | Her2/neu.1131.D3 | 32 |
| 201 | 10 | FSPAFDNLYY | Her2/neu.1213 | 4.5 |
| 212 | 10 | FTPAFDNLYY | Her2/neu.1213.T2 | 0.80 |
| 203 | 9 | FSPAFDNLY | Her2/neu.1213 | 581 |
| 204 | 9 | FTPAFDNLY | Her2/neu.1213.T2 | 7.8 |
| 205 | 9 | SPAFDNLYY | Her2/neu.1214 | NT |
| 206 | 9 | SPDFDNLYY | Her2/neu.1214.D3 | 74 |
| 207 | 10 | GTPTAENPEY | Her2/neu.1239 | 397 |
| 208 | 10 | GTDTAENPEY | Her2/neu.1239.D3 | 26 |

| | | | | |
|---|---|---|---|---|
| -- indicates binding affinity =10,000nM.. | | | | |

**Table 18c. Mage2/3-derived Al binders**

| | | | | | Published Immunogenicity | |
|---|---|---|---|---|---|---|
| SEQ ID NO | AA | Sequence | Source | A*0101 nM | Peptide | Tumor |
| 209 | 10 | ASSFSITINY | MAGE2.68 | 1563 | | |
| 210 | 10 | ATSFSTTINY | MAGE2.68.T2 | 455 | | |
| 211 | 10 | ASDFSTITNY | MAGE2.68.D3 | 25 | | |
| 212 | 9 | SSFSTTINY | MAGE2.69 | 1563 | | |
| 213 | 9 | STFSTTINY | MAGE2.69.T2 | 490 | | |
| 214 | 11 | VVEVVPISHLY | MAGE2.166 | 125 | | |
| 215 | 8 | VTCLGLSY | MAGE2.179 | 1136 | | |
| 216 | 8 | VTDLGLSY | MAGE2.179.D3 | 2.7 | | |
| 217 | 10 | LMQDLVQENY | MAGE2.246 | 556 | | |
| 218 | 10 | LTQDLVQENY | MAGE2.246.T2 | 58 | | |
| 219 | 9 | MQDLVQENY | MAGE2.247 | 17 | | |
| 220 | 9 | MTDLVQENY | MAGE2.247.T2 | 0.80 | | |
| 221 | 10 | ASSLPTTMNY | MAGE3.68 | 11 | | |
| 222 | 10 | ATSLPTTMNY | MAGE3.68.T2 | 208 | | |
| 223 | 10 | ASDLPTTMNY | MAGE3.68.D3 | 2.6 | | |
| 224 | 9 | SSLPTTMNY | MAGE3.69 | 676 | | |
| 225 | 9 | STLPTTMNY | MAGE3.69.T2 | 58 | | |
| 226 | 11 | TMNYPLWSQSY | MAGE3.74 | 301 | | |
| 227 | 9 | GSVVGNWQY | MAGE3.137 | 4237 | | |
| 228 | 9 | GTWGNWQY | MAGE3.137.T2 | 36 | | |
| 229 | 11 | LMEVDPIGHLY | MAGE3.166 | 3.3 | | |
| 230 | 9 | EVDPIGHLY | MAGE3.168 | 6.8 | + | + |
| 231 | 9 | ETDPIGHLY | MAGE3.168.T2 | 0.70 | | |
| 232 | 8 | ATCLGLSY | MAGE3.179 | 227 | | |
| 233 | 10 | LTQHFVQENY | MAGE3.246 | 96 | | |
| 234 | 10 | LTDHFVQENY | MAGE3.246.D3 | 2.3 | | |
| 235 | 9 | ISGGPHISY | MAGE3.293 | 676 | | |
| 236 | 9 | ITGGPHISY | MAGE3.293.T2 | 36 | | |

**Table 18d. p53-derived A1 binders**

| SEQ ID NO | AA | Sequence | Source | A*0101 nM |
|---|---|---|---|---|
| 237 | 10 | PSQKTYQGSY | p53.98 | 1786 |
| 238 | 10 | PTQKTYQGSY | p53.98.T2 | 36 |
| 239 | 10 | GTAKSVTCTY | p53.117 | 76 |
| 240 | 10 | GTDKSVTCTY | p53.117.D3 | 42 |
| 241 | 10 | RVEGNLRVEY | p53.196 | 1136 |
| 242 | 10 | RVDGNLRVEY | p53.196.D3 | 46 |
| 243 | 10 | VGSDCTTIHY | p53.225 | 96 |
| 244 | 9 | GSDCTTIHY | p53.226 | 0.80 |
| 245 | 9 | GTDCTTIHY | p53.226.T2 | 0.90 |
| 246 | 11 | GSDCTTIHYNY | p53.226 | 68 |

**Table 19a. CEA-derived A24 binders**

| | | | | | Published Immunogenicity | |
|---|---|---|---|---|---|---|
| SEQ ID NO | AA | Sequence | Source | A*0101 nM | Peptide | Tumor |
| 247 | 9 | RWCIPWQRL | CEA.10 | 923 | | |
| 248 | 9 | RYCIPWQRF | CEA.10.Y2F9 | 191 | | |
| 249 | 10 | RWCIPWQRLL | CEA.10 | 308 | | |
| 250 | 10 | RYCIPWQRLF | CEA.10.Y2F10 | 26 | | |
| 251 | 11 | RWCIPWQRLLL | CEA.10 | 152 | | |
| 252 | 11 | PWQRLLLTASL | CEA.14 | 324 | | |
| 253 | 10 | FWNPPTTAKL | CEA.27 | 400 | | |
| 254 | 10 | FYNPPTTAKF | CEA.27.Y2F10 | 182 | | |
| 255 | 8 | IYPNASLL | CEA.101 | 177 | | |
| 256 | 9 | IYPNASLLI | CEA.101 | 1.7 | | |
| 257 | 9 | IYPNASLLF | CEA.101.F9 | 2.2 | | |
| 258 | 11 | PYTLHVIKSDL | CEA.119 | 480 | | |
| 259 | 10 | VYPELPKPSI | CEA.140 | 1519 | | |
| 260 | 10 | VYPELPKPSF | CEA.140.F10 | 106 | | |
| 261 | 9 | LWWVNNQSL | CEA.177 | 546 | | |
| 262 | 9 | LYWVNNQSF | CEA.177.Y2F9 | 63 | | |
| 263 | 9 | LYGPDAPTI | CEA.234 | 57 | | |
| 264 | 9 | LYGPDAPTF | CEA.234.F9 | 63 | | |
| 265 | 10 | QYSWFVNGTF | CEA.268 | 3.5 | | |
| 266 | 8 | SWFVNGTF | CEA.270 | 480 | | |
| 267 | 10 | TFQQSTQELF | CEA.276 | 750 | | |
| 268 | 10 | TYQQSTQELF | CEA.276.Y2 | 308 | | |
| 269 | 9 | VYAEPPKPF | CEA.318 | 41 | | |
| 270 | 10 | VYAEPPKPFI | CEA.318 | 667 | | |
| 271 | 10 | VYAEPPKPFF | CEA.318.F10 | 27 | | |
| 272 | 11 | TYLWWVNNQSL | CEA.353 | 46 | | |
| 273 | 9 | LYGPDDPTI | CEA.412 | 353 | | |
| 274 | 11 | SYTYYRPGVNL | CEA.423 | 218 | | |
| 275 | 9 | TYYRPGVIVL | CEA.425 | 185 | | |
| 276 | 9 | TYYRPGVNF | CEA.425.F9 | 52 | | |
| 277 | 11 | TYYRPGVNLSL | CEA.425 | 132 | | |
| 278 | 10 | YYRPGVNLSL | CEA.426 | 86 | | |
| 279 | 10 | YYRPGVNLSF | CEA.426.F10 | 10 | | |
| 280 | 10 | QYSWLIDGNI | CEA.446 | 800 | | |
| 281 | 10 | QYSWLIDGNF | CEA.446.F10 | 60 | | |
| 282 | 11 | TYLWWVNGQSL | CEA.531 | 92 | | |
| 283 | 9 | LWWVNGQSL | CEA.533 | 1463 | | |
| 284 | 9 | LYWVNGQSF | CEA.533.Y2F9 | 16 | | |
| 285 | 9 | LYGPDTPII | CEA.590 | 46 | | |
| 286 | 10 | SYLSGANLNL | CRA.604 | 207 | | |
| 287 | 10 | SYLSGANLNF | CEA.604.F10 | 10 | | |
| 288 | 9 | QYSWRINGI | CEA.624 | 444 | | |
| 289 | 9 | QYSWRINGF | CEA.624.F9 | 109 | | |
| 290 | 9 | TYACFVSNL | CEA.652 | 10 | + | + |
| 291 | 9 | TYACFVSNF | CEA.652.F9 | 8.6 | | |

**Table 19b. Her2/neu-derived A24 binders**

| SEQ ID NO | AA | Sequence | Source | A*2401 |
|---|---|---|---|---|
| 292 | 9 | PYVSRLLGI | Her2/neu.780 | 71 |
| 293 | 9 | PYVSRLLGF | Her2/neu.780.F9 | 9.2 |
| 294 | 11 | PYVSRLLGICL | Her2/neu.780 | 375 |
| 295 | 10 | GMSYLEDVRL | Her2/neu.832 | NT |
| 296 | 10 | GYSYLEDVRF | Her2/neu.832.Y2F10 | 235 |
| 297 | 9 | KWMALESIL | Her2/neu.887 | 800 |
| 298 | 9 | KYMALESIF | Her2/neu.887.Y2F9 | 19 |
| 299 | 9 | RFTHQSDVW | Her2/neu.898 | 1091 |
| 300 | 9 | RYTHQSDVF | Her2/neu.898.Y2F9 | 60 |
| 301 | 9 | VWSYGVTVW | Her2/neu.905 | 150 |
| 302 | 9 | VYSYGVTVF | Her2/neu.905.Y2F9 | 16 |
| 303 | 11 | VWSYGVTVWEL | Her2/neu.905 | 130 |
| 304 | 9 | SYGVTVWEL | Her2/neu.907 | 100 |
| 305 | 9 | SYGVTVWEF | Her2/neu.907.F9 | 26 |
| 306 | 9 | VYMIMVKCW | Her2/neu.951 | 75 |
| 307 | 9 | VYMIMVKCF | Her2/neu.951.F9 | 19 |
| 308 | 11 | VYMIMVKCWMI | Her2/neu.951 | 6.7 |
| 309 | 9 | RFRELVSEF | Her2/neu.968 | 667 |
| 310 | 9 | RYRELVSEF | Her2/neu.968.Y2 | 36 |
| 311 | 9 | RMARDPQRF | Her2/neu.978 | 3750 |
| 312 | 9 | RYARDPQRF | Her2/neu.978.Y2 | 120 |

**Table 19c. Mage2/3-derived A24 binders**

| | | | | | Published Immunogenicity | |
|---|---|---|---|---|---|---|
| SEQ ID NO | AA | Sequence | Source | A*2401 nM | Peptide | Tumor |
| 313 | 11 | SFSTTINYTLW | MAGE2.70 | 429 | | |
| 314 | 10 | SYSTTINYTF | MAGE2.70.Y2F10 | 15 | | |
| 315 | 9 | MFPDLESEF | MAGE2.97 | 857 | | |
| 316 | 9 | MYPDLESEF | MAGE2.97.Y2 | 52 | | |
| 317 | 9 | KMVELVHFL | MAGE2.112 | 750 | | |
| 318 | 9 | KYVELVHFF | MAGE2.112.Y2F9 | 7,1 | | |
| 319 | 11 | IFSKASEYLQL | MAGE2.150 | 126 | | |
| 320 | 9 | IYSKASEYF | MAGE2.150.Y2F9 | 15 | | |
| 321 | 9 | EYLQLVFGI | MAGE2.156 | 3.4 | + | + |
| 322 | 9 | EYLQLVFGF | MAGE2.156.F9 | 4.0 | | |
| 323 | 10 | LYILVTCLGL | MAGE2.175 | 857 | | |
| 324 | 10 | LYILVTCLGF | MAGE2.175.F10 | 18 | | |
| 325 | 9 | VMPKTGLLI | MAGE2.195 | 52 | | |
| 326 | 9 | VYPKTGLLF | MAGE2.195.Y2F9 | 5.5 | | |
| 327 | 10 | VMPKTGLLII | MAGE2.195 | 207 | | |
| 328 | 10 | VYPKTGLLIF | MAGE2.195.Y2F10 | 2.9 | | |
| 329 | 10 | EFLWGPRALI | MAGE2.270 | 1237 | | |
| 330 | 10 | EYLWGPRALF | MAGE2.270.Y2F10 | 10 | | |
| 331 | 8 | LWGPRALI | MAGE2.272 | 100 | | |
| 332 | 10 | SYVKVLHHTL | MAGE2.282 | 75 | | |
| 333 | 10 | SYVKVLHHTF | MAGE2.282.F10 | 34 | | |
| 334 | 9 | TFPDLESEF | MAGE3.97 | 2449 | + | + |
| 335 | 9 | TYPDLESEF | MAGE3.97.Y2 | 218 | | |
| 336 | 9 | NWQYFFPVI | MAGE3.142 | 23 | | |
| 337 | 9 | NYQYFFPVF | MAGE3.142.Y2F9 | 3.4 | | |
| 338 | 10 | NYQYFFPVIF | MAGE3.142.Y2 | 23 | | |
| 339 | 8 | QYFFPVIF | MAGE3.144 | 100 | | |
| 340 | 9 | IFSKASSSL . | MAGE3.150 | 750 | | |
| 341 | 9 | IYSKASSSF | MAGE3.150.Y2F9 | 375 | | |
| 342 | 11 | IFSKASSSLQL | MAGE3.150 | 132 | | |
| 343 | 10 | LYIFATCLGL | MAGE3.175 | 3429 | | |
| 344 | 10 | LYIFATCLGF | MAGE3.175.F1Q | 10 | | |
| 345 | 9 | IMPKAGLLI | MAGE3.195 | 29 | + | + |
| 346 | 9 | IYPKAGLLP | MAGE3.195.Y2F9 | 9.2 | | |
| 347 | 10 | IMPKAGLLII | MAGE3.195 | 240 | | |
| 348 | 10 | IYPKAGLLIF | MAGE3.195.Y2P10 | 1.2 | | |
| 349 | 11 | IWEELSVLEVF | MAGE3.221 | 462 | | |
| 350 | 8 | SYPPLHEW | MAGE3.300 | 286 | | |
| 351 | 10 | SYPPLHEWVL | MAGE3.300 | 20 | | |
| 352 | 10 | SYPPLHEWVF | MAGE3.300.P10 | 5.5 | | |

**Table 19d. p53-derived A24 binders**

| SEQ ID NO | AA | Sequence | Source | A*2401 nM |
|---|---|---|---|---|
| 353 | 9 | TFSDLWKLL | p53.18 | -- |
| 354 | 9 | TYSDLWKLF | p53.18.Y2F9 | 5.5 |
| 355 | 8 | TYQGSYGF | p53.102 | 109 |
| 356 | 10 | TYQGSYGFRL | p53.102 | 100 |
| 357 | 10 | TYQGSYGFRF | p53.102.F10 | 30 |
| 358 | 8 | SYGFRLGF | p53.106 | 429 |
| 359 | 9 | SYGFRLGFL | p53.106 | 600 |
| 360 | 9 | SYGFRLGFF | p53.106.F9 | 121 |
| 361 | 10 | TYSPALNKMF | p53.125 | 2.4 |

| | | | | |
|---|---|---|---|---|
| indicates binding affinity =10,000 nM | | | | |

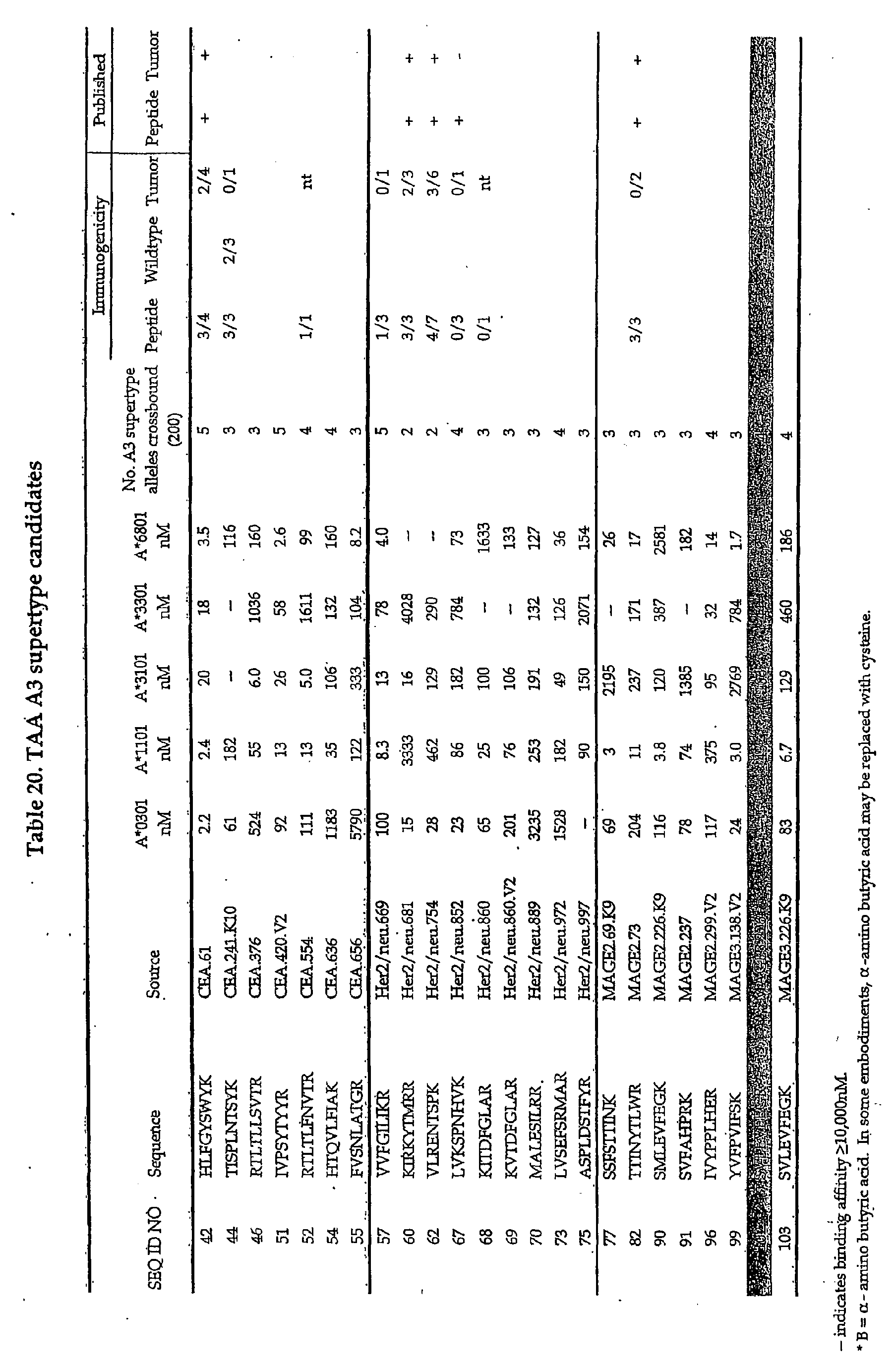

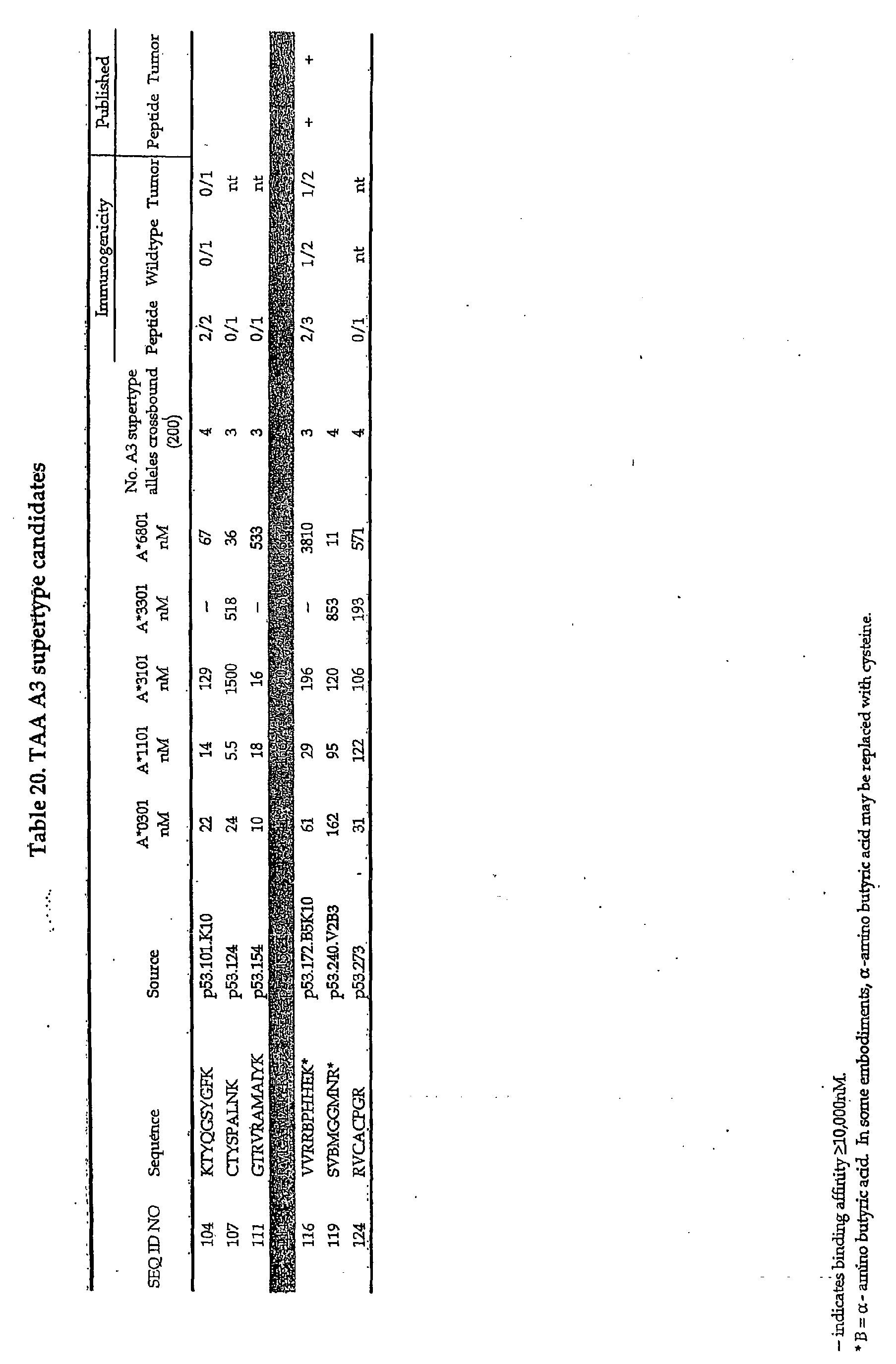

**Table 21. TAA-derived B7 supertype candidates**

| | | | | | | | | | Immunogenicity | | | Published | |
|---|---|---|---|---|---|---|---|---|---|---|---|---|---|
| SEQ ID NO | Sequence | Source | B*0702 nM | B*3501 nM | B*5101 nM | B*5301 nM | B*5401 nM | No. B7 supertype alleles crossbound (200) | Peptide | Wildtype | Tumor | Peptide | Tumor |
| 133 | YPNASLLI | CEA.102 | 196 | 514 | 8.1 | 40 | 137 | 4 | | | | | |
| 136 | LPQPPICTI | Hei2/neu.941¹ | 196 | - ²⁾ | 4.2 | 28 | 19 | 4 | | | | | |
| 140 | VPISHLYIL | MAGE2.170 | 22 | 171 | 96 | 239 | 3125 | 3 | 6/6 | | 0/6 | | |
| 146 | LPTTMNYPI | MAGE3.71.19 | 100 | 343 | 31 | 182 | 4.2 | 4 | | | | | |
| 153 | MPKAGLLIII 1) | MAGE3.196.I10 | 324 | 2400 | 62 | 176 | 102 | 3 | | | | + | |
| 155 | APAAPTPAAPA | p53.76 | 14 | 3273 | - | 93 | 53 | 3 | 0/1 | | nt | | |

| | | | | | | | | | | | | | |
|---|---|---|---|---|---|---|---|---|---|---|---|---|---|
| 1) This 10mer may also be used as a 9mer (MPKAGLLII) (SEQ ID NO:362) 2) -indicates binding affinity=10,000nM. | | | | | | | | | | | | | |

**Table 22. TAA-derived A1 candidates**

| | | | | | Published Immunogenicity | |
|---|---|---|---|---|---|---|
| SEQ ID NO | AA | Sequence | Source | A*0101 nM | Peptide | Tumor |
| 161 | 11 | RSDSVILNVLY | CEA.225 | 47 | | |
| 167 | 9 | ITDNNSGSY | CEA.289.D3 | 96 | | |
| 170 | 11 | HSDPVILNVLY | CEA.403 | 26 | | |
| 172 | 10 | PTDSPSYTYY | CEA.418.D3 | 1.1 | | |
| 178 | 9 | AADNPPAQY | CEA.439.D3 | 45 | | |
| 180 | 9 | ITDKNSGLY | CEA.467.D3 | 12 | | |
| 181 | 11 | RSDPVTLDVLY | CEA.581 | 7.8 | | |
| 182 | 10 | HSASNPSPQY | CEA.616 | 74 | | |
| 186 | 9 | VMDGVGSPY | Her2/neu.773.D3 | 40 | | |
| 188 | 10 | CTQIAKGMSY | Her2/neu.826.T2 | 19 | | |
| 189 | 9 | LLDIDETEY | Her2/neu.869 | 3.3 | | |
| 191 | 10 | FTHQSDVWSY | Her2/neu.899 | 9.3 | | |
| 194 | 10 | PADPLDSTFY | Her2/neu.996.D3 | 19 | | |
| 198 | 10 | MTDLVDAEEY | Her2/neu.1014.T2 | 2.3 | | |
| 200 | 9 | LTDSPQPEY | Her2/neu.1131.D3 | 32 | | |
| 201 | 10 | FSPAFDNLYY | Her2/neu.1213 | 4.5 | | |
| 208 | 10 | GTDTAENPEY | Her2/neu.1239.D3 | 26 | | |
| 211 | 10 | ASDFSTTINY | MAGE2.68.D3 | 25 | | |
| 216 | 8 | VTDLGLSY | MAGE2.179.D3 | 2.7 | | |
| 219 | 9 | MQDLVQENY | MAGE2.247 | 17 | | |
| 221 | 10 | ASSLPTTMNY | MAGE3.68 | 11 | | |
| 228 | 9 | GTWGNWQY | MAGE3.137.T2 | 36 | | |
| 230 | 9 | EVDPIGHLY | MAGE3.168 | 6.8 | + | + |
| 234 | 10 | LTDHFVQENY | MAGE3.246.D3 | 2.3 | | |
| 236 | 9 | ITGGPHISY | MAGE3.293.T2 | 36 | | |
| 238 | 10 | PTQKTYQGSY | p53.98.T2 | 36 | | |
| 239 | 10 | GTAKSVTCTY | p53.117 | 76 | | |
| 240 | 10 | GTDKSVTCTY | p53.117.D3 | 42 | | |
| 242 | 10 | RVDGNLRVEY | p53.196.D3 | 46 | | |
| 246 | 11 | GSDCTTIHYNY | p53.226 | 68 | | |

**Table 23. TAA-derived A24 candidates**

| | | | | | Published Immunogenicity | |
|---|---|---|---|---|---|---|
| SEQ ID NO | AA | Sequence | Source | A*0101 nM | Peptide | Tumor |
| 256 | 9 | IYPNASLLI | CEA.101 | 1.7 | | |
| 263 | 9 | LYGPDAPTI | CEA.234 | 57 | | |
| 265 | 10 | QYSWFVNGTF | CEA.268 | 3.5 | | |
| 269 | 9 | VYAEPPKPF | CEA.318 | 41 | | |
| 272 | 11 | TYLWWVNNQSL | CEA.353 | 46 | | |
| 278 | 10 | YYRPGVNLSL | CEA.426 | 86 | | |
| 279 | 10 | YYRPGVNLSF | CEA.426.F10 | 10 | | |
| 281 | 10 | QYSWLIDGNF | CEA.446.F10 | 60 | | |
| 282 | 11 | TYLWWVNGQSL | CEA.531 | 92 | | |
| 285 | 9 | LYGPDTPII | CEA.590 | 46 | | |
| 287 | 10 | SYLSGANLNF | CEA.604.F10 | 10 | | |
| 290 | 9 | TYACFVSNL | CEA.652 | 10 | | |
| 292 | 9 | PYVSRLLGI | Her2/neu.780 | 71 | | |
| 293 | 9 | PYVSRLLGF | Her2/neu.780.F9 | 9.2 | | |
| 304 | 9 | SYGVTVWEL | Her2/neu.907 | 100 | | |
| 305 | 9 | SYGVTVWEF | Her2/neu.907.F9 | 26 | | |
| 308 | 11 | VYMIMVKCWMI | Her2/neu.951 | 6.7 | | |
| 310 | 9 | RYRELVSEF | Her2/neu.968.Y2 | 36 | | |
| 316 | 9 | MYPDLESEF | MAGE2.97.Y2 | 52 | | |
| 321 | 9 | EYLQLVFGI | MAGE2.156 | 3.4 | | |
| 324 | 10 | LYILVTCLGF | MAGE2.175.F10 | 18 | | |
| 325 | 9 | VMPKTGLLI | MAGE2.195 | 52 | | |
| 331 | 8 | LWGPRALI | MAGE2.272 | 100 | | |
| 332 | 10 | SYVKVLHHTL | MAGE2.282 | 75 | | |
| 333 | 10 | SYVKVLHHTF | MAGE2.282.F10 | 34 | | |
| 334 | 9 | TFPDLESEF | MAGE3.97 | 2449 | | |
| 335 | 9 | TYPDLESEF | MAGE3.97.Y2 | 218 | | |
| 336 | 9 | NWQYFFPVI | MAGE3.142 | 23 | | |
| 344 | 10 | LYIFATCLGF | MAGE3.175.F10 | 10 | | |
| 345 | 9 | IMPKAGLLI | MAGE3.195 | 29 | | |
| 351 | 10 | SYPPLHEWVL | MAGE3.300 | 20 | | |
| 356 | 10 | TYQGSYGFRL | p53.102 | 100 | | |
| 361 | 10 | TYSPALNKMF | p53.125 | 2.4 | | |

**Table 24. TAA Candidate Summary**

| | A3 supertype | | B7 supertype | | A1 | | A24 | | |
|---|---|---|---|---|---|---|---|---|---|
| Antigen | Wildtype | Analog | Wildtype | Analog | Wildtype | Analog | Wildtype | Analog | Total |
| CEA | 5 | 2 | 1 | 0 | 4 | 3 | 9 | 2 | 26 |
| Her2/neu | 7 | 1 | 1 | 0 | 3 | 6 | 3 | 1 | 22 |
| Mage2/3 | 3 | 3 | 1 | 2 | 4 | 4 | 7 | 3 | 27 |
| p53 | 2 | 4 | 1 | 0 | 2 | 2 | 2 | 0 | 13 |
| Total | 17 | 10 | 4 | 2 | 13 | 15 | 21 | 6 | 88 |

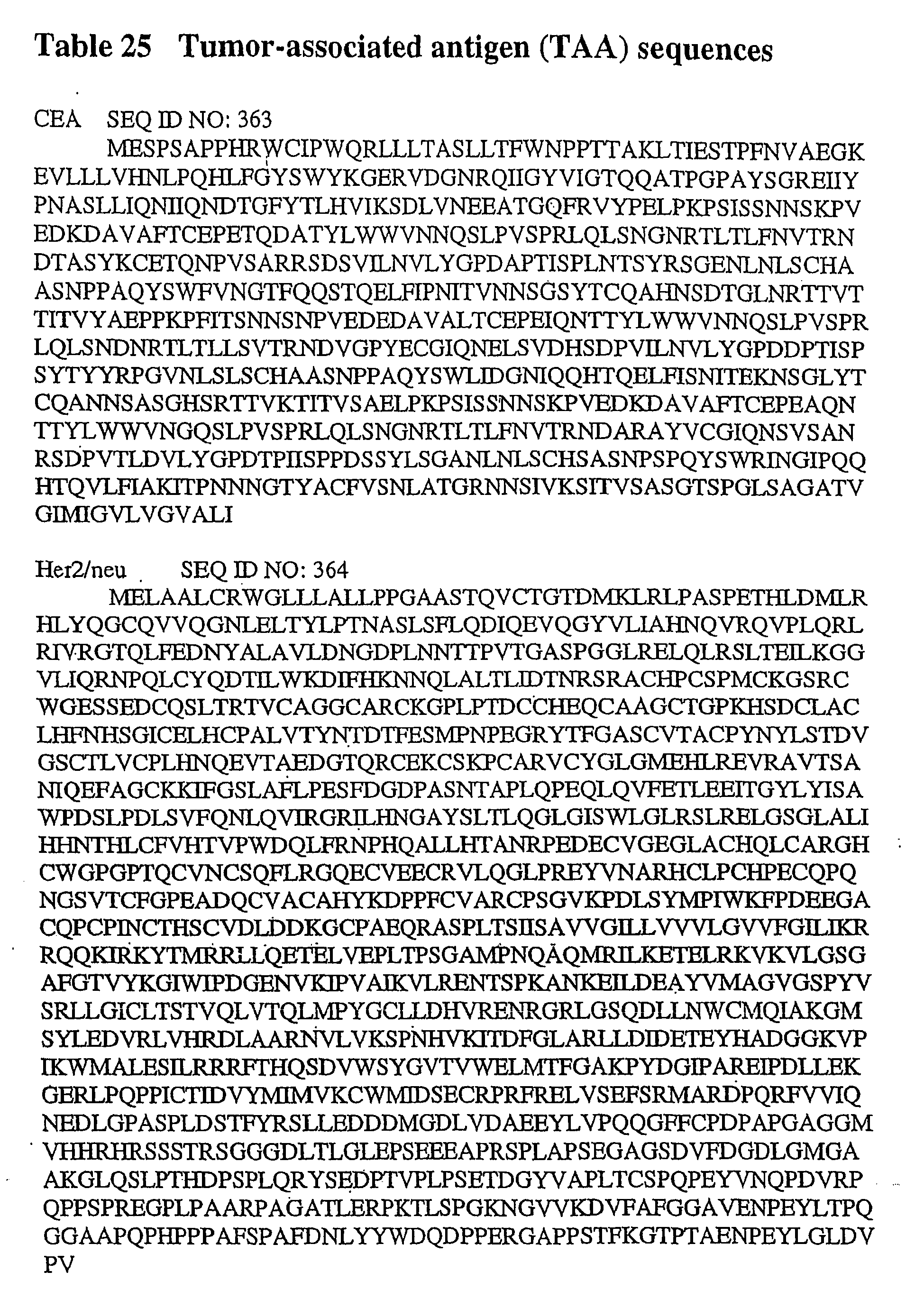

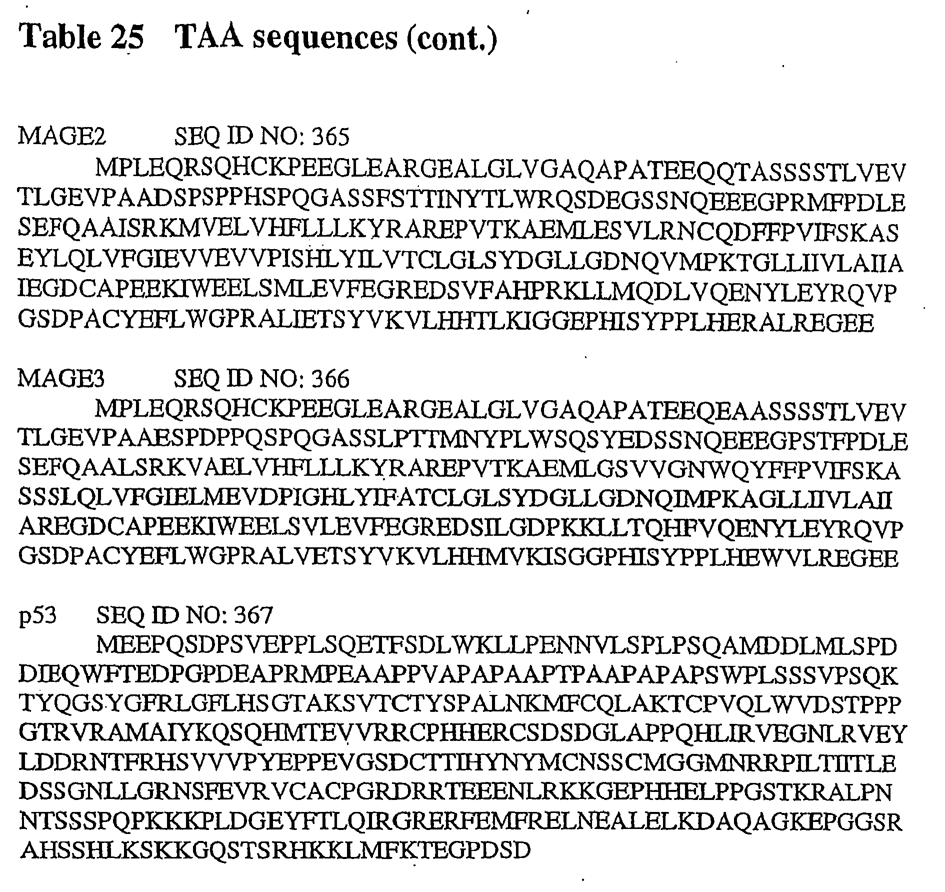

**Table 26: CEA-derived B44 peptides**

| Seq ID No. | Sequence | AA | Protein | Position | B* 1801 | B* 4001 | B* 4002 | B* 4402 | B* 4403 | B* 4501 | Degeneracy |
|---|---|---|---|---|---|---|---|---|---|---|---|
| 368 | AEGKEVLLL | 9 | CEA | 46 | 359 | 34 | 60 | 179 | 1.5 | 161 | 6 |
| 369 | QELFIPNITV | 10 | CEA | 282 | 81 | 121 | 27 | 48 | 2.6 | 14 | 6 |
| 370 | IESTPFNVAEG | 11 | CEA | 38 | 87 | 1074 | 352 | 89 | 8.7 | 84 | 5 |
| 371 | YECGIQNEL | 9 | CEA | 391 | 82 | 71 | 53 | 452 | 5.3 | 855 | 5 |
| 372 | YECGIQNELSV | 11 | CEA | 391 | 9.2 | 28 | 26 | 1714 | 0.46 | 155 | 5 |
| 373 | MESPSAPPHRW | 11 | CEA | 1 | 12 | 943 | 1915 | 5.3 | 41 | 359 | 4 |
| 374 | IBSTPFNVA | 9 | CEA | 38 | 14 | 2542 | 36 | 19,157 | 1.2 | 13 | 4 |
| 375 | AFGKEVLLLV | 10 | CEA | 46 | 5135 | 69 | 408 | 223 | 8.6 | 994 | 4 |
| 376 | KEVLLLVHNL | 10 | CEA | 49 | 893 | 1.0 | 4.4 | 326 | 2.3 | 2512 | 4 |
| 377 | REIIYPNASL | 10 | CEA | 98 | 4340 | 0.57 | 7.5 | 412 | 1.7 | 954 | 4 |
| 378 | REIIYPNASLL | 11 | CEA | 98 | 1788 | 2.4 | 12 | 57 | 0,38 | 1777 | 4 |
| 379 | CETQNPVSA | 9 | CEA | 215 | 73 | 7016 | 261 | -- | 10 | 15 | 4 |
| 380 | QELFIPNIT | 9 | CEA | 282 | 125 | 4361 | 172 | 1217 | 3.0 | 18 | 4 |
| 381 | PBAQNTTYLWWV | 12 | CEA | 525 | 205 | 3802 | 1097 | 414 | 183 | 25 | 4 |
| 382 | GERVDGNRQII | 11 | CEA | 70 | 764 | 278 | 18 | 871 | 1.3 | -- | 3 |
| 383 | NEEATGQFRVY | 11 | CEA | 131 | 7.7 | 3252 | 999 | 9.6 | 69 | 3986 | 3 |
| 384 | CEPETQDAT | 9 | CEA | 167 | 4009 | 3646 | 410 | 5450 | 50 | 97 | 3 |
| 385 | GENLNLSCHA | 10 | CEA | 252 | 14,373 | 1341 | 357 | 8610 | 5.3 | 271 | 3 |
| 386 | GENLNLSCHAA | 11 | CEA | 252 | 7838 | 4557 | 63 | 1907 | 9.0 | 32 | 3 |
| 387 | QELFIPNI | 8 | CEA | 282 | 127 | 5815 | 147 | 1339 | 8.5 | 1319 | 3 |
| 388 | CEPEIQNTTYL | 11 | CEA | 345 | 129 | 287 | 1603 | 1245 | 60 | 11,981 | 3 |
| 389 | PEIQNTTYLWW | 11 | CEA | 347 | 172 | 749 | 1045 | 17 | 227 | 1365 | 3 |
| 390 | PEIQNTTYLWWV | 12 | CEA | 347 | 517 | 511 | 291 | 167 | 66 | 932 | 3 |
| 391 | NELSVDHSDPV | 11 | CEA | 397 | 49 | 1704 | 1128 | 1615 | 38 | 78 | 3 |
| 392 | QELFISNIT | 9 | CEA | 460 | 530 | 6571 | 58 | 2334 | 3.9 | 80 | 3 |
| 393 | PEAQNTTYLWW | 11 | CEA | 525 | 147 | 2096 | 3090 | 121 | 79 | 2005 | 3 |
| 394 | GERVDGNRQI | 10 | CEA | 70 | 9395 | 1933 | 49 | 2544 | 13 | 19,464 | 2 |
| 395 | NEEATGQFRV | 10 | CEA | 131 | 998 | -- | -- | 4536 | 471 | 405 | 2 |
| 396 | BBATGQFRV | 9 | CEA | 132 | 611 | 803 | 1025 | 1602 | 82 | 13 | 2 |
| 397 | EEATGQFRVY | 10 | CEA | 132 | 64 | -- | 1532 | 26 | 1041 | 1374 | 2 |
| 398 | VEDKDAVAF | 9 | CEA | 157 | 94 | 121 | 1583 | 963 | 1443 | -- | 2 |
| 399 | CEPMQDATYL | 11 | CEA | 167 | 831 | 311 | 3388 | 398 | 807 | -- | 2 |
| 400 | VEDEDAVAL | 9 | CEA | 335 | 840 | 11 | 2665 | 9691 | 51 | -- | 2 |
| 401 | CEPEIQNTTYLWW | 13 | CEA | 345 | 204 | 1027 | 3589 | 12 | 508 | 865 | 2 |
| 402 | PEIQNTTYL | 9 | CEA | 347 | 923 | 138 | 2786 | 16,816 | 231 | 1825 | 2 |
| 403 | AELPKPSI | 8 | CEA | 498 | 7423 | 6697 | 131 | 959 | 19 | 2608 | 2 |
| 404 | PEAQNTTY | 8 | CEA | 525 | 149 | 2594 | 2437 | -- | 76 | 3255 | 2 |
| 405 | AEGKEVLL | 8 | CEA | 46 | 11,455 | 1311 | 5303 | 17,268 | 129 | 14,165 | 1 |
| 406 | ABPPKPFTT | 9 | CEA | 320 | 14,614 | 7067 | 3438 | -- | 214 | 1838 | 1 |
| 407 | CEPEIQNTT | 9 | CEA | 345 | 8575 | 10,080 | 1453 | 19,027 | 119 | 2401 | 1 |
| 408 | CEPEIQNTTY | 10 | CBA | 345 | 1459 | -- | -- | 49 | 14,596 | -- | 1 |
| 409 | PEIQNTTYLW | 10 | CEA | 347 | 819 | 3301 | 9423 | 13 | 6173 | 10,011 | 1 |
| 410 | QBLFISNI | 8 | CEA | 460 | 889 | 6396 | 1175 | 2282 | 70 | 1172 | 1 |
| 411 | TBKNSGLY | 8 | CEA | 468 | 211 | 9851 | 7117 | 1868 | 605 | 10,248 | 1 |
| 412 | TEKNSGLYT | 9 | CEA | 468 | 713 | 7522 | 1724 | 6134 | 99 | 1850 | 1 |
| 413 | CEPEAQNTTY | 10 | CEA | 523 | 9525 | -- | -- | 61 | -- | 17,330 | 1 |
| 414 | CEPEAQNTTYL | 11 | CEA | 523 | 962 | 2184 | 11,723 | 3419 | 131 | 2450 | 1 |
| 415 | PEAQNTTYLW | 10 | CEA | 525 | 17,082 | -- | -- | 27 | -- | -- | 1 |
| 416 | NEEATGQF | 8 | CEA | 131 | 7326 | -- | -- | 8366 | 17,054 | 17,737 | 0 |
| 417 | PELPKPSI | 8 | CEA | 142 | 9106 | -- | -- | -- | 5143 | -- | 0 |
| 418 | VEDKDAVAFT | 10 | CEA | 157 | 1434 | 11,648 | 2077 | 16,919 | 803 | -- | 0 |
| 419 | CEPETQDATY | 10 | CEA | 167 | 1353 | -- | -- | 787 | 536 | -- | 0 |
| 420 | PETQDATY | 8 | CEA | 169 | 10,803 | -- | -- | 6343 | 19,466 | -- | 0 |
| 421 | PETQDATYL | 9 | CEA | 169 | 13,219 | 1374 | -- | -- | 2488 | 13,430 | 0 |
| 422 | PETQDATYLW | 10 | CEA | 169 | 9346 | -- | -- | 850 | 915 | -- | 0 |
| 423 | PETQDATYLWW | 11 | CEA | 169 | 5308 | -- | -- | 2819 | 600 | 11,631 | 0 |
| 424 | CETQNPV | 7 | CEA | 215 | 5798 | 18,808 | 7450 | 13,463 | 819 | -- | 0 |
| 425 | AEPPKPFI | 8 | CEA | 320 | 12,800 | -- | -- | 13,720 | 904 | -- | 0 |
| 426 | VEDEDAVALT | 10 | CEA | 335 | 7275 | 895 | 2359 | 6668 | 2682 | -- | 0 |
| 427 | PEIQNTTY | 8 | CEA | 347 | 1023 | 7274 | -- | -- | 1618 | -- | 0 |
| 428 | CEPEAQNTT | 9 | CEA | 523 | 15,594 | 11,134 | -- | -- | 1212 | 2579 | 0 |
| 429 | PEAQNTTYL | 9 | CEA | 525 | 9500 | 3092 | 9280 | -- | 1840 | -- | 0 |

**Table 27: HER 2/new-derived B44 peptides**

| SEQ ID NO | Sequence | AA | Protein | Position | B* 1801 | B* 4001 | B* 4002 | B* 4402 | B* 4403 | B* 4501 | Degeneracy |
|---|---|---|---|---|---|---|---|---|---|---|---|
| 430 | MBLAALCRWGL | 11 | Her2/neu | 1 | 6.4 | 24 | 30 | 17 | 0.92 | 116 | 6 |
| 431 | LELTYLPTNASL | 12 | Her2/neu | 60 | 88 | 38 | 31 | 420 | 3.7 | 37 | 6 |
| 432 | QEVQGYVLI | 9 | Her2/neu | 78 | 42 | 36 | 28 | 45 | 1.7 | 92 | 6 |
| 433 | GBGLACHQLCA | 11 | Her2/neu | 506 | 62 | 39 | 97 | 159 | 2.7 | 196 | 6 |
| 434 | ABQRASPL | 8 | Her2/neu | 644 | 16 | 73 | 13 | 243 | 0.38 | 120 | 6 |
| 435 | AEQRASPLTSI | 11 | Her2/neu | 644 | 467 | 19 | 58 | 5.1 | 2.5 | 11 | 6 |
| 436 | KEILDBAYVM | 10 | Her2/neu | 765 | 13 | 5.5 | 4.0 | 35 | 3.3 | 234 | 6 |
| 437 | GERLPQPPI | 9 | Her2/neu | 938 | 62 | 71 | 3.3 | 27 | 1.1 | 15 | 6 |
| 438 | SECRPRFREL | 10 | Her2/neu | 963 | 80 | 307 | 18 | 11 | 0.20 | 25 | 6 |
| 439 | AENPEYLGL | 9 | Her2/neu | 1243 | 17 | 2.2 | 271 | 45 | 2.5 | 155 | 6 |
| 440 | RELQLRSLTEI | 11 | Her2/neu | 138 | 261 | 2.8 | 3.7 | 125 | 0.99 | 269 | 6 |
| 441 | QEFAGCKKIFG | 11 | Her2/neu | 362 | 211 | 423 | 477 | 37 | 2.1 | 138 | 6 |
| 442 | LEBITGYLYISA | 12 | Her2/neu | 403 | 35 | 177 | 78 | 323 | 4.6 | 110 | 6 |
| 443 | SEFSRMARDPQRF | 13 | Her2/neu | 974 | 44 | 454 | 45 | 54 | 11 | 361 | 6 |
| 444 | NEDLGPASPL | 10 | Her2/neu | 991 | 107 | 281 | 150 | 40 | 6.0 | 231 | 6 |
| 445 | SEEEAPRSPL | 10 | Her2/neu | 1066 | 151 | 155 | 217 | 37 | 8.4 | 84 | 6 |
| 446 | SBTDGYVAPL | 10 | Her2/neu | 1122 | 94. | 214 | 184 | 386 | 2.4 | 302 | 6 |
| 447 | LELTYLPTNA | 10 | Her2/neu | 60 | 332 | 325 | 10 | 6428 | 3.1 | 24 | 5 |
| 448 | QEVQGYVL | 8 | Her2/neu | 78 | 3.4 | 28 | 5.0 | 1307 | 0.92 | 33 | 5 |
| 449 | FEDNYALAV | 9 | Her2/neu | 108 | 9.5 | 11 | 6.2 | 9942 | 0.42 | 154 | 5 |
| 450 | RELQIRSLT | 9 | Her2/neu | 138 | 638 | 316 | 13 | 465 | 0.20 | 162 | 5 |
| 451 | MEHLREVRA | 9 | Her2/neu | 347 | 233 | -- | 386 | 38 | 3.2 | 19 | 5 |
| 452 | MEHLREVRAV | 10 | Her2/neu | 347 | 72 | -- | 160 | 180 | 13 | 140 | 5 |
| 453 | MEHLREVRAVTSA | 13 | Her2/neu | 347 | 77 | 5662 | 120 | 281 | 21 | 16 | 5 |
| 454 | QEPAGCKKIF | 10 | Her2/neu | 362 | 53 | 3686 | 12 | 4.0 | 3.6 | 115 | 5 |
| 455 | EEITGYLYISA | 11 | Her2/neu | 404 | 0.94 | 1440 | 52 | 4.5 | 2.1 | 0.93 | 5 |
| 456 | RELGSGLAL | 9 | Her2/neu | 459 | 359 | 3.7 | 0.9 | 473 | 0.97 | 2262 | 5 |
| 457 | RELGSGLALI | 10 | Her2/neu | 459 | 4810 | 22 | 4.4 | 32 | 0.78 | 324 | 5 |
| 458 | GBGLACHQL | 9 | Her2/neu | 506 | 13,766 | 14 | 88 | 66 | 11 | 340 | 5 |
| 459 | RBYVNARHCL | 10 Hec2/neu | | 552 | 1327 | 39 | 4,8 | 106 | 0.97 | 126 | 5 |
| 460 | BBGACQPCPI | 10 | Her2/neu | 619 | 119 | -- | 340 | 52 | 80 | 401 | 5 |
| 461 | AEQRASPLT | 9 | Her2/neu | 644 | 346 | 874 | 183 | 103 | 1.8 | 10 | 5 |
| 462 | QBTELVEPL | 9 | Her2/neu | 692 | 12 | 9.1 | 36 | 310 | 3.5 | 1232 | 5 |
| 463 | QETELVEPLT | 10 | Her2/neu | 692 | 15 | 293 | 338 | 1619 | 13 | 288 | 5 |
| 464 | GENVKIPVAI | 10 | Her2/neu | 743 | 563 | 314 | 3.7 | 230 | 2.8 | 198 | 5 |
| 465 | RELVSEFSRM | 10 | Her2/neu | 970 | 9.1 | 28 | 4.3 | 33 | 0.12 | 1726 | 5 |
| 466 | RELVSEFSRMA | 11 | Her2/neu | 970 | 168 | 191 | 143 | 2613 | 3.5 | 32 | 5 |
| 467 | ABEYLVPQQGFF | 12 | Her2/neu | 1020 | 124 | 262 | 589 | 24 | 49 | 172 | 5 |
| 468 | SEDPTVPL | 8 | Her2/neu | 1113 | 103 | 71 | 161 | 9450 | 2.0 | 308 | 5 |
| 469 | SETDGYVAPLT | 11 | Her2/neu | 1122 | 66 | 125 | 224 | 1225 | 2.2 | 45 | 5 |
| 470 | AENPEYLGLDV | 11 | Her2/neu | 1243 | 11,934 | 28 | 139 | 69 | 3.0 | 24 | 5 |
| 471 | MELAALCRW | 9 | Her2/neu | 1 | 7.5 | 4301 | 141 | 26 | 15 | 1140 | 4 |
| 472 | MELAALCRWG | 10 | Her2/neu | 1 | 102 | 8684 | 1840 | 5.7 | 135 | 408 | 4 |
| 473 | QEVQGYVLIA | 10 | Her2/neu | 78 | 61 | 772 | 64 | 1871 | 15 | 11 | 4 |
| 474 | FEDNYALAVL | 10 | Her2/neu | 108 | 321 | 6.2 | 48 | 2844 | 3.8 | 3095 | 4 |
| 475 | RELQLRSL | 8 | Her2/neu | 138 | 42 | 49 | 5.9 | 2248 | 0.62 | 1372 | 4 |
| 476 | TEILKGGVL | 9 | Her2/neu | 146 | 125 | 30 | 14 | 697 | 0.28 | 2480 | 4 |
| 477 | TBILKGGVLI | 10 | Her2/neu | 146 | 1021 | 241 | 294 | 24 | 21 | 7600 | 4 |
| 478 | GESSEDCQSL | 10 | Her2/neu | 206 | -- | 8.1 | 23 | 427 | 5.1 | 2491 | 4 |
| 479 | SEDCQSLTRTV | 11 | Her2/neu | 209 | 101 | 4322 | 311 | 943 | 21 | 10 | 4 |
| 480 | PEGRYTFGASCV | 12 | Her2/neu | 285 | 1366 | 2.6 | 6.1 | 1410 | 348 | 356 | 4 |
| 481 | REVRAVTSA | 9 | Her2/neu | 351 | 626 | 427 | 0.7 | 3160 | 0.18 | 9.3 | 4 |
| 482 | REVRAVTSANI | 11 | Her2/neu | 351 | 4491 | 17 | 30 | 1680 | 1.8 | d21 | 4 |
| 483 | EEITGYLY | 8 | Her2/neu | 404 | 20 | 5713 | 1223 | 38 | 83 | 238 | 4 |
| 484 | BEFTOYLYI | 9 | Her2/neu | 404 | 86 | 906 | 916 | 14 | 121 | 94 | 4 |
| 485 | EEITGYLYISAW | 12 | Her2/neu | 404 | 36 | 837 | 1966 | 37 | 87 | 57 | 4 |
| 486 | QECVEECRVL | 10 | Her2/neu | 538 | 315 | 444 | 399 | 606 | 22 | 2863 | 4 |
| 487 | VEECRVLQGL | 10 | Her2/neu | 541 | 270 | 227 | 5815 | 237 | 189 | 16,094 | 4 |
| 488 | GENVKIPVA | 9 | Her2/neu | 743 | 1508 | 293 | 3.0 | -- | 1.7 | 13 | 4 |
| 489 | KEILDEAYV | 9 | Her2/neu | 765 | 1358 | 62 | 43 | 6466 | 8.4 | 42 | 4 |
| 490 | KEILDEAYVMA | 11 | Her2/neu | 765 | 731 | 252 | 95 | 11,514 | 64 | 123 | 4 |
| 491 | DEAYVMAGVG | 10 | Her2/neu | 769 | 122 | 203 | 154 | 4033 | 5609 | 218 | 4 |
| 492 | TEYHADGGKVPI | 12 | Her2/neu | 875 | 632 | 195 | 7.1 | 3342 | 1.4 | 361 | 4 |
| 493 | GBRLPQPPICTI | 12 | Her2/neu | 938 | 8538 | 398 | 95 | 935 | 0.60 | 40 | 4 |
| 494 | ABBYLVPQQGF | 11 | Her2/neu | 1020 | 125 | 584 | 1831 | 21 | 99 | 268 | 4 |
| 495 | EEYLVPQQG | 9 | Her2/neu | 1021 | 66 | 10,344 | 176 | 2200 | 126 | 131 | 4 |
| 496 | EBYLVPQQGF | 10 | Her2/neu | 1021 | 12 | -- | 2551 | 21 | 11 | 73 | 4 |
| 497 | EBYLVPQQGFF | 11 | Her2/neu | 1021 | 94 | 4291 | 1695 | 78 | 168 | 154 | 4 |
| 498 | EEEAPRSPL | 9 | Her2/neu | 1067 | 902 | 4490 | 316 | 177 | 362 | 307 | 4 |
| 499 | EEAPRSPLA | 9 | Her2/neu | 1068 | 486 | 10,707 | 4900 | 200 | 294 | 4.5 | 4 |
| 500 | REGPLPAARPA | 11 | Her2/neu | 1153 | 157 | 543 | 78 | -- | 4.2 | 347 | 4 |
| 501 | RELQLRSLTEIL | 12 | Her2/neu | 138 | 1252 | 15 | 26 | 2286 | 0.50 | 865 | 3 |
| 502 | GESSEDCQSLT | 11 | Her2/neu | 206 | 742 | 48 | 180 | 14,396 | 40 | 2158 | 3 |
| 503 | CELHCPAL | 8 | Her2/neu | 264 | 150 | 871 | 259 | 4361 | 39 | -- | 3 |
| 504 | CELHCPALV | 9 | Her2/neu | 264 | 136 | 4805 | 319 | 2308 | 52 | 1110 | 3 |
| 505 | CELHCPALVT | 10 | Her2/neu | 264 | 80 | -- | 65 | 933 | 18 | 1275 | 3 |
| 506 | CELHCPALVTY | 11 | Her2/neu | 264 | 12 | 3469 | 3198 | 140 | 89 | 2779 | 3 |
| 507 | FESMPNPEG | 9 | Her2/neu | 279 | 6068 | -- | 59 | 14,846 | 20 | 155 | 3 |
| 508 | FESMPNPEGRY | 11 | Hec2/neu | 279 | 74 | 3666 | 3533 | 59 | 70 | 1394 | 3 |
| 509 | QEFAGCKKI | 9 | Her2/neu | 362 | 1120 | 736 | 131 | 85 | 44 | 2684 | 3 |
| 510 | FETLEEITGYL | 11 | Her2/neu | 400 | 133 | 78 | 649 | 7490 | 42 | 2200 | 3 |
| 511 | LEEITGYLYI | 10 | Her2/neu | 403 | 143 | 914 | 2996 | 222 | 143 | 1488 | 3 |
| 512 | PBDECVGEGL | 10 | Her2/neu | 500 | 1257 | 278 | 257 | 6331 | 49 | -- | 3 |
| 513 | DECVGEGL | 8 | Her2/neu | 502 | 49 | 4864 | 481 | 938 | 34 | 14,244 | 3 |
| 514 | TBLVEPLTPSGA | 12 | Her2/neu | 694 | 167 | 4104 | 103 | 2118 | 28 | 2739 | 3 |
| 515 | RBNTSPKANKEIL | 13 | Her2/neu | 756 | 11,950 | 5.8 | 64 | -- | 8.6 | 9105 | 3 |
| 516 | KEILDEAY | 8 | Her2/neu | 765 | 82 | 921 | 430 | 7485 | 74 | 2646 | 3 |
| 517 | DEAYVMAGV | 9 | Her2/neu | 769 | 58 | 5327 | 1245 | 8006 | 138 | 161 | 3 |
| 518 | LESILRRRF | 9 | Her2/neu | 891 | 29 | -- | 3475 | 5.8 | 101 | 12,918 | 3 |
| 519 | WBLMTFGAKPY | 11 | Her2/neu | 913 | 13 | 509 | 778 | 24 | 75 | 1216 | 3 |
| 520 | REIPDLLEKGERL | 13 | Her2/neu | 929 | 3212 | 34 | 226 | 2914 | 31 | 14,043 | 3 |
| 521 | GERLPQPPICT | 11 | Her2/neu | 938 | 12,486 | -- | 23 | 9094 | 3.9 | 15 | 3 |
| 522 | SECRPRFRELV | 11 | Her2/neu | 963 | 1996 | 3673 | 121 | 927 | 18 | 118 | 3 |
| 523 | SEGAGSDVF | 9 | Her2/neu | 1078 | 74 | 5627 | 6525 | 33 | 192 | 6960 | 3 |
| 524 | PEYLGLDVPV | 10 | Her2/neu | 1246 | 613 | 352 | 35 | 1371 | 1.7 | 610 | 3 |
| 525 | CBKCSKPCARVCY | 13 | Her2/neu | 331 | 763 | 16,796 | 1292 | 340 | 117 | 1815 | 2 |
| 526 | MEHLREVRAVT | 11 | Her2/neu | 347 | 1064 | 1963 | 2207 | 795 | 111 | 74 | 2 |
| 527 | LEEITGYL | 8 | Her2/neu | 403 | 242 | 830 | 1805 | 8038 | 403 | -- | 2 |
| 528 | DEEGACQPCPI | 11 | Her2/neu | 618 | 451 | 5517 | 7293 | 968 | 438 | 1323 | 2 |
| 529 | TELVEPL | 7 | Her2/neu | 694 | 162 | 14,164 | 1258 | 8854 | 66 | -- | 2 |
| 530 | VEPLTPSGA | 9 | Her2/neu | 697 | 7321 | -- | 96 | 8516 | 191 | 17,037 | 2 |
| 531 | KETELRKVKV | 10 | Her2/neu | 716 | 11,925 | -- | 68 | 2936 | 15 | 1603 | 2 |
| 532 | TELRKVKVL | 9 | Her2/neu | 718 | 1514 | 4698 | 11 | 1844 | 2.5 | 14,147 | 2 |
| 533 | LBDVRLVHRDL | 11 | Her2/neu | 836 | 729 | 325 | 641 | 818 | 59 | 2382 | 2 |
| 534 | TEYHADGGKV | 10 | Her2/neu | 875 | 239 | 5246 | 2003 | 2911 | 15 | 1571 | 2 |
| 535 | LESILRRRFT | 10 | Her2/neu | 891 | 82 | -- | 1189 | 34 | 657 | 2251 | 2 |
| 536 | LBDDDMGDL | 9 | Her2/neu | 1009 | 191 | 556 | 351 | 722 | 900 | 6251 | 2 |
| 537 | ABBYLVPQQG | 10 | Her2/neu | 1020 | 723 | -- | -- | 1549 | 479 | 127 | 2 |
| 538 | SBEBAPRSPLA | 11 | Her2/neu | 1066 | 1318 | 3604 | 5110 | 8550 | 158 | 27 | 2 |
| 539 | SEGAGSDVFDG | 11 | Her2/neu | 1078 | 928 | 3751 | 5695 | 374 | 286 | 3008 | 2 |
| 540 | PEYLTPQGGAA | 11 | Her2/neu | 1194 | 1724 | -- | 200 | -- | 354 | 4011 | 2 |
| 541 | PERGAPPST | 9 | Her2/neu | 1228 | 390 | 4744 | 7679 | 1116 | 178 | 7767 | 2 |
| 542 | PETHLDML | 8 | Her2/neu | 39 | 1954 | 8387 | 6118 | -- | 83 | -- | 1 |
| 543 | PETHLDMLRHL | 11 | Her2/neu | 39 | 1322 | 700 | 2971 | 11,534 | 70 | 4329 | 1 |
| 544 | SEDCQSL | 7 | Her2/neu | 209 | 18,245 | 2691 | 14,258 | 8248 | 431 | 19,225 | 1 |
| 545 | HEQCAAGCT | 9 | Her2/neu | 237 | 1995 | -- | 7377 | 14,068 | 178 | 2974 | 1 |
| 546 | PEGRYTPGASCVT | 13 | Her2/neu | 285 | 6602 | 4411 | 3286 | 1560 | 456 | 1198 | 1 |
| 547 | QEVTAEDGT | 9 | Her2/neu | 320 | 5207 | -- | 3122 | 7886 86 | 66 | 1843 | 1 |
| 548 | CEKCSKPCA | 9 | Her2/neu | 331 | 3740 | -- | 2703 | 12,538 | 342 | 8007 | 1 |
| 549 | CBKCSKPCARV | 11 | Her2/neu | 331 | 1167 | 4103 | 2079 | 9594 | 101 | 1561 | 1 |
| 550 | RBVRAVT | 7 | Her2/neu | 351 | 8564 | 3136 | 725 | -- | 29 | -- | 1 |
| 551 | FETLEEI | 7 | Her2/neu | 400 | 1518 | 7621 | 2110 | -- | 69 | -- | 1 |
| 552 | FETLEEITGY | 10 | Her2/neu | 400 | 671 | -- | -- | 262 | 1679 | -- | 1 |
| 553 | DBCVGEGLACHQL | 13 | Her2/neu | 502 | 586 | 4421 | 3965 | 3093 | 468 | 5888 | 1 |
| 554 | QECVEECRV | 9 | Her2/neu | 538 | 15,799 | 8755 | 1664 | 4348 | 210 | 4542 | 1 |
| 555 | VEECRVLQG | 9 | Her2/neu | 541 | 1528 | 8947 | 7622 | 12,736 | 305 | -- | 1 |
| 556 | BECRVLQGL | 9 | Her2/neu | 542 | 890 | 7076 | 2029 | 717 | 434 | 1185 | 1 |
| 557 | PBCQPQNGSV | 10 | Her2/neu | 565 | 7962 | -- | -- | 12,964 | 472 | -- | 1 |
| 558 | TELVEPLTPSO | 11 | Her2/neu | 694 | 601 | 2978 | 3703 | | 2,69 | 14,079 1 | 1 |
| 559 | VBPLTPSGAM , | 10 | Her2/neu | 697 | 4649 | 1667 | 584 | 4368 | 108 | -- | 1 |
| 560 | KBTEIRKVKVL | 11 | Her2/neu | 716 | 9529 | 2973 | 1868 | 7136 | 71 | 12,237 | 1 |
| 561 | TBLRKVKVLG | 10 | Her2/neu | 718 | 721 | -- | 601 | 3650 | 14 | 12,816 | 1 |
| 562 | DBTEYHADG | 9 | Her2/nou | 873 | 159 | -- | -- | -- | 1397 | 13,353 | 1 |
| 563 | DBTBYHADGG | 10 | Her2/neu | 873 | 613 | -- | 16,801 | 3891 | 269 | -- | 1 |
| 564 | REEPDLLEKO | 10 | Her2/neu | 929 | 649 | 4493 | 814 | 1270 | 13 | 1977 | 1 |
| 565 | SECRPRF | 7 | Her2/neu | 963 | 926 | 18,181 | 1157 | 852 | 48 | 8856 | 1 |
| 566 | EEEAPRSPLA | 10 | Her2/neu | 1067 | 6611 | -- | -- | 3128 | 960 | 14 | 1 |
| 567 | EEAPRSPL | 8 | Her2lneu | 1068 | 1191 | 3489 | 1611 | 3020 | 171 | 1926 | 1 |
| 568 | SBGAGSDVPDGDL | 13 | Her2/neu | 1078 | 1563 | 52 | 2097 | 1595 | 749 | 3001 | 1 |
| 569 | PBYVNQPDV | 9 | Her2/neu | 1137 | 831 | 3437 | 1581 | 823 | 48 | 2536 | 1 |
| 570 | VBNPBYLTPQG | 11 | Her2/neu | 1191 | 8386 | -- | -- | 17,337 | 11 | 4188 | 1 |
| 571 | PBYLTPQGG | 9 | Her2/neu | 1194 | 1456 | 18,951 | 13,860 | 6532 | 284 | 18,990 | 1 |
| 572 | PERGAPPSTF | 10 | Her2/neu | 1228 | 1062 | 14,884 | 3437 | 6871 | 208 | 15,700 | 1 |
| 573 | PBTHLDM | 7 | Her2/neu | 39 | -- | 15,506 | -- | -- | 5081 | -- | 0 |
| 574 | SEDCQSLTRT | 10 | Her2/neu | 209 | 7257 | 8550 | 11,529 | 518 | 2857 | 5178 | 0 |
| 575 | HEQCAAGCTG | 10 | Her2/neu | 237 | 3805 | 6126 | 8285 | 3168 | 806 | 2072 | 0 |
| 576 | PEGRYTFGA | 9 | Her2/neu | 285 | 5252 | -- | -- | -- | 658 | 15,119 | 0 |
| 577 | PESFDGDPA | 9 | Her2/neu | 378 | 14,489 | 11,550 | -- | -- | 1170 | 4418 | 0 |
| 578 | PEQLQVFET | 9 | Her2/neu | 394 | 1798 | 11,635 | -- | 5814 | 4659 | 9322 | 0 |
| 579 | PBQLQVFBTL | 10 | Her2/neu | 394 | 1314 | -- | 4890 | 6107 | 754 | -- | 0 |
| 580 | PEQLQVFETLEEI | 13 | Her2/neu | 394 | 9.147 | 5393 | -- | -- | 3232 | -- | 0 |
| 581 | FETLEEITG | 9 | Her2/neu | 400 | 1750 | 14,182 | 8715 | 10,330 | 753 | -- | 0 |
| 582 | LEEITGY | 7 | Her2/neu | 403 | 8594 | 17,584 | 11,496 | -- | 989 | -- | 0 |
| 583 | LEEITGYLY | 9 | Her2/neu | 403 | 1048 | 13,469 | -- | 870 | 5335 | -- | 0 |
| 584 | RILHNGAYSL | 10 | Her2/neu | 434 | 2345 | -- | 726 | -- | -- | -- | 0 |
| 585 | PEDECVGEG | 9 | Her2/neu | 500 | 11,794 | 931 | 647 | 7327 | 559 | -- | 0 |
| 586 | PEDECVGEGLA | 11 | Her2/neu | 500 | 6685 | -- | -- | 4217 | 1933 | -- | 0 |
| 587 | DECVGEGLA | 9 | Her2/neu | 502 | 1006 | 4742 | 3131 | 14,445 | 506 | 1114 | 0 |
| 588 | PECQPQNGSVT | 11 | Her2/neu | 565 | 8882 | -- | -- | 5093 | 2353 | -- | 0 |
| 589 | PECQPQNGSVTCF | 13 | Her2/neu | 565 | 4787 | 1772 | 8115 | 3564 | 2161 | -- | 0 |
| 590 | PEADQCVACA | 10 | Her2/neu | 579 | 10,271 | -- | 16,262 | 6242 | 2155 | 1486 | 0 |
| 591 | PBADQCVACAHY | 12 | Her2/neu | 579 | 2819 | 12,352 | 11,420 | 1163 | 3516 | 3057 | 0 |
| 592 | LEDVRLV | 7 | Her2/neu | 836 | 16,473 | 17,438 | -- | -- | 2658 | -- | 0 |
| 593 | DETEYHA | 7 | Her2/neu | 873 | 6105 | -- | 18,324 | -- | 10,523 | -- | 0 |
| 594 | LEDDDMGDLV | 10 | Her2/neu | 1009 | 9176 | 2963 | 3596 | 6746 | 3641 | 10,475 | 0 |
| 595 | LERPKTLSPG | 10 | Her2/neu | 1167 | 10,194 | -- | -- | 2333 | 1400 | 7162 | 0 |
| 596 | VENPEYL | 7 | Her2/neu | 1191 | -- | 14,701 | -- | 14,253 | 12,085 | -- | 0 |
| 597 | PEYLTPQGGA | 10 | Her2/neu | 1194 | 7436 | -- | -- | 9016 | 5779 | 15,093 | 0 |

**Table 28: p53-derived B44 peptides**

| SEQ ID NO | Sequence | AA | Protein | Position | B* 1801 | B* 4001 | B* 4002 | B* 4402 | B* 4403 | B* 4501 | Degeneracy |
|---|---|---|---|---|---|---|---|---|---|---|---|
| 598 | RERFEMFREL | 10 | p53 | 335 | 83 | 29 | 17 | 17 | 0.34 | 422 | 6 |
| 599 | GEYFRLQIRG | 10 | p53 | 325 | 108 | 88 | 19 | 2452 | 3.9 | 157 | 5 |
| 600 | QBTFSDLWKL | 10 | p53 | 16 | 736 | 199 | 255 | 39 | 14 | 901 | 4 |
| 601 | DEAPRMPEA | 9 | p53 | 61 | 84 | 297 | 4577 | 6448 | 98 | 10 | 4 |
| 602 | HERCSDSDGL | 10 | p53 | 179 | 139 | 171 | 61 | 1468 | 6.0 | 1723 | 4 |
| 603 | VEYLDDRNTF | 10 | p53 | 203 | 0.94 | 501 | 37 | 32 | 1.4 | 3601 | 4 |
| 604 | FEVRVCACPG | 10 | p53 | 270 | 64 | 2043 | 4.9 | 180 | 0.76 | 1872 | 4 |
| 605 | GEYFRLQI | 8 | p53 | 325 | 7774 | 112 | 60 | 3511 | 1.0 | 261 | 4 |
| 606 | FEMPRELNEA | 10 | p53 | 338 | 127 | 3207 | 223 | 952 | 2.0 | 208 | 4 |
| 607 | FEMFRELNEAL | 11 | p53 | 338 | 475 | 17 | 8.8 | 748 | 1.1 | 1352 | 4 |
| 608 | RELNEALEL | 9 | p53 | 342 | 3000 | 15 | 30 | 256 | 1.1 | 3337 | 4 |
| 609 | IBQWFTBDPG | 10 | p53 | 50 | 151 | 1250 | 2114 | 5595 | 142 | 197 | 3 |
| 610 | VEGNLRVBYL | 10 | p53 | 197 | 104 | 481 | 2565 | 1963 | 22 | 15,189 | 3 |
| 611 | GEPHHELPPG | 10 | p53 | 293 | 108 | 3323 | 1888 | 11,728 | 4.4 | 20 | 3 |
| 612 | TEDPGPDBAPRM | 12 | p53 | 55 | 570 | 361 | 1326 | 2791 | 141 | 1702 | 2 |
| 613 | DEAPRMPEAA | 10 | p53 | 61 | 121 | 1497 | 8444 | 2594 | 1037 | 100 | 2 |
| 614 | HERCSDSDG | 9 | p53 | 179 | 1118 | 67 | -- | 2032 | 208 | 13,390 | 2 |
| 615 | HERCSDSDGLA | 11 | p53 | 179 | 1408 | 4879 | 1915 | -- | 96 | 186 | 2 |
| 616 | LEDSSGNL | 8 | p53 | 257 | 17,736 | 782 | 108 | -- | 211 | 15,946 | 2 |
| 617 | LEDSSGNLL | 9 | p53 | 257 | 1140 | 2.2 | 2771 | 1865 | 43 | -- | 2 |
| 618 | GEPHHELPPGST | 12 | p53 | 293 | 3814 | -- | 5418 | 4477 | 413 | 132 | 2 |
| 619 | RERFEMF | 7 | p53 | 335 | 180 | 4079 | 1907 | -- | 108 | -- | 2 |
| 620 | LELKDAQAG | 9 | p53 | 348 | 170 | 18,706 | 3659 | 5126 | 30 | 1989 | 2 |
| 621 | MEEPQSDPSV | 10 | p53 | 1 | 8970 | 3802 | 16,536 | 1927 | 816 | 175 | 1 |
| 622 | VEPPLSQET | 9 | p53 | 10 | 8302 | 17,052 | -- | 3186 | 236 | -- | 1 |
| 623 | VEPPLSQETF | 10 | p53 | 10 | 814 | -- | -- | 406 | 525 | -- | 1 |
| 624 | QETFSDLWKLL | 11 | p53 | 16 | 4158 | 3366 | 740 | 631 | 168 | 1218 | 1 |
| 625 | PENNVLSPL | 9 | p53 | 27 | 1150 | 1261 | 718 | 11,174 | 8.8 | -- | 1 |
| 626 | DEAPRMPEAAPPV | 13 | p53 | 61 | 583 | -- | 2715 | -- | 1727 | 87 | 1 |
| 627 | VEGNLRVEY | 9 | p53 | 197 | 832 | 12,752 | -- | 61 | 2583 | -- | 1 |
| 628 | VEYLDDRNT | 9 | p53 | 203 | 1442 | -- | -- | 10,071 | 157 | 13,503 | 1 |
| 629 | YEPPEVGSDCT | 11 | p53 | 220 | 16,872 | -- | 125 | 13,349 | 12,712 | 19,199 | 1 |
| 630 | YEPPEVGSDCTTI | 13 | p53 | 220 | 9330 | 3530 | 689 | 3009 | 351 | -- | 1 |
| 631 | PEVGSDCTTI | 10 | p53 | 223 | 611 | 4552 | 248 | 2293 | 2046 | -- | 1 |
| 632 | LEDSSGNLLG | 10 | p53 | 257 | 1062 | 531 | 697 | 7905 | 153 | 19,256 | 1 |
| 633 | TEEENLRKKG | 10 | p53 | 284 | -- | -- | -- | -- | 315 | -- | 1 |
| 634 | HELPPGSTKRA | 11 | p53 | 297 | 6034 | 3974 | 3255 | -- | 189 | 1472 | 1 |
| 635 | NEALELKDA | 9 | p53 | 345 | 1925 | 3887 | 6640 | 4270 | 1582 | 129 | 1 |
| 636 | NEALELKDAQA | 11 | p53 | 345 | 742 | 6235 | 5071 | -- | 949 | 53 | 1 |
| 637 | EEPQSDPSV | 9 | p53 | 2 | 9454 | -- | -- | 5972 | 1507 | 1620 | 0 |
| 638 | QBTFSDL | 7 | p53 | 16 | 8514 | 18,350 | 12,210 | -- | 1612 | 18,051 | 0 |
| 639 | TEDPGPDEA | 9 | p53 | 55 | 15,049 | 1217 | -- | 8035 | 2763 | 1789 | 0 |
| 640 | PEAAPPV | 7 | p53 | 67 | 14,223 | -- | -- | -- | 5429 | -- | 0 |
| 641 | PEAAPPVAPA | 10 | p53 | 67 | 2638 | 5800 | 14,593 | 2192 | 653 | 901 | 0 |
| 642 | PEVGSDCTT | 9 | p53 | 223 | 18,612 | 1189 | -- | 7109 | 12,997 | -- | 0 |
| 643 | PEVGSDCTTIHY | 12 | p53 | 223 | 1182 | 10,188 | 7994 | 4093 | 1939 | 2251 | 0 |
| 644 | EEENLRKKG | 9 | p53 | 285 | 1464 | 687 | -- | 3662 | 706 | 18,421 | 0 |

**Table 29: MAGE2-derived B44 peptides**

| Seq ID NO | Sequence | AA | Protein | Position | B* 1801 | B* 4001 | B* 4002 | B* 4402 | B* 4403 | B* 4501 | Degeneracy |
|---|---|---|---|---|---|---|---|---|---|---|---|
| 645 | LESEFQAAI | 9 | MAGE2 | 101 | 14 | 41 | 39 | 43 | 1.0 | 78 | 6 |
| 646 | LESEFQAAISRKM | 13 | MAGE2 | 101 | 26 | 264 | 46 | 427 | 14 | 102 | 6 |
| 647 | SEFQAAISRKM | 11 | MAGE2 | 103 | 7.0 | 345 | 107 | 88 | 1.2 | 161 | 6 |
| 648 | SEFQAAISRKMV | 12 | MAGE2 | 103 | 47 | 300 | 25 | 111 | 3.2 | 256 | 6 |
| 649 | SEYLQLVFG | 9 | MAGE2 | 155 | 18 | 235 | 421 | 348 | 19 | 113 | 6 |
| 650 | SEYLQLVFGI | 10 | MAGE2 | 155 | 5.2 | 20 | 6.1 | 3.7 | 0.84 | 4.4 | 6 |
| 651 | SBYLQLVFGIEVV | 13 | MAGE2 | 155 | 12 | 44 | 17 | 229 | 7.6 | 22 | 6 |
| 652 | WEBLSMLEVF | 10 | MAGE2 | 222 | 4.0 | 463 | 30 | 15 | 22 | 290 | 6 |
| 653 | QENYLEYRQV | 10 | MAGE2 | 252 | 210 | 493 | 102 | 17 | 16 | 27 | 6 |
| 654 | YEFLWGPRALI | 11 | MAGE2 | 269 | 5.2 | 4.1 | 2.8 | 92 | 0.59 | 450 | 6 |
| 655 | LEARGEALGLVGA | 13 | MAGE2 | 16 | 228 | 29 | 50 | 3886 | 3.7 | 135 | 5 |
| 656 | VEVTLGEVPA | 10 | MAGE2 | 46 | 14 | 371 | 31 | 3801 | 0.52 | 15 | 5 |
| 657 | EEGPRMFPDL | 10 | MAGE2 | 92 | 128 | 4438 | 486 | 291 | 13 | 42 | 5 |
| 658 | AEMLESVL | 8 | MAGE2 | 133 | 968 | 14 | 31 | 327 | 0.88 | 302 | 5 |
| 659 | LESVLRNCQDFF | 12 | MAGE2 | 136 | 56 | 246 | 370 | 264 | 54 | 1466 | 5 |
| 660 | SEYLQLVF | 8 | MAGE2 | 155 | 0.97 | 765 | 6.0 | 284 | 0.70 | 122 | 5 |
| 661 | VEVVPISHLYI | 11 | MAGE2 | 167 | 97 | 135 | 146 | 335 | 7.2 | 3788 | 5 |
| 662 | EEKRWEELSM | 10 | MAGE2 | 218 | 86 | -- | 477 | 46 | 28 | 107 | 5 |
| 663 | EELSMLEVFEG | 11 | MAGE2 | 223 | 1.5 | -- | 294 | 4.6 | 23 | 163 | 5 |
| 664 | YEFLWGPRA | 9 | MAGE2 | 269 | 5.3 | 30 | 5.2 | 4246 | 1.1 | 241 | 5 |
| 665 | YEFLWGPRAL | 10 | MAGB2 | 269 | 17 | 8.5 | 1.6 | 130 | 0.72 | 753 | 5 |
| 667 | IETSYVKVL | 9 | MAGE2 | 279 | 72 | 7.2 | 23 | 33 | 2.6 | 11,902 | 5 |
| 668 | LEARGEAL | 8 | MAGE2 | 16 | 163 | 99 | 26 | -- | 2.9 | -- | 4 |
| 669 | LEARGEALGL | 10 | MAGE2 | 16 | 81 | 184 | 277 | 2275 | 4.1 | 3046 | 4 |
| 670 | LEARGBALGLV | 11 | MAGE2 | 16 | 158 | 198 | 345 | -- | 13 | 1912 | 4 |
| 671 | VELVHFLL | 8 | MAGB2 | 114 | 5.0 | 69 | 31 | 3322 | 1.2 | 2427 | 4 |
| 672 | VELVHFLLL | 9 | MAGE2 | 114 | 71 | 79 | 31 | 559 | 3.1 | 1129 | 4 |
| 673 | REPVTKAEM | 9 | MAGE2 | 127 | 60 | 40 | 284 | 6577 | 4.5 | 832 | 4 |
| 674 | REPVTKAEML | 10 | MAGE2 | 127 | 88 | 23 | 264 | 763 | 21 | 917 | 4 |
| 675 | IEVVEVVPI | 9 | MAGE2 | 164 | 11 | 4.7 | 60 | 11,313 | 1.3 | 6423 | 4 |
| 676 | VEVVPISHL | 9 | MAGE2 | 167 | 149 | 2.8 | 66 | 9082 | 2.3 | 13,803 | 4 |
| 678 | VEVVPISHLYIL | 12 | MAGE2 | 167 | 191 | 20 | 17 | 935 | 3.2 | 1926 | 4 |
| 679 | VEVVPISHLYILV | 13 | MAGE2 | 167 | 197 | 373 | 110 | 562 | 25 | 839 | 4 |
| 680 | WEELSMLEV | 9 | MAGE2 | 222 | 70 | 2174 | 37 | 657 | 2.5 | 134 | 4 |
| 681 | EELSMLEVF | 9 | MAGB2 | 223 | 1.4 | 16,436 | 252 | 22 | 2.8 | 1013 | 4 |
| 682 | GEPHISYPPL | 10 | MAGE2 | 295 | 7254 | 7.0 | 2.9 | 1200 | 0.71 | 380 | 4 |
| 683 | EEGLEARGEAL | 11 | MAGE2 | 13 | 179 | 300 | 578 | 2630 | 19 | 1812 | 3 |
| 684 | GBALGLVGA | 9 | MAGE2 | 20 | 9529 | 510 | 34 | 6134 | 2.2 | 17 | 3 |
| 685 | GBALGLVGAQA | 11 | MAGE2 | 20 | 877 | 4293 | 52 | 3575 | 1.4 | 28 | 3 |
| 686 | EEQQTASSSSTL | 12 | MAGE2 | 34 | 8999 | 301 | 2287 | 160 | 570 | 205 | 3 |
| 687 | QBEEGPRM | 8 | MAGE2 | 90 | 298 | 11,598 | 431 | 19,255 | 118 | 6730 | 3 |
| 688 | QEEBGPRMF | 9 | MAGE2 | 90 | 414 | 626 | 7747 | 237 | 409 | 2171 | 3 |
| 689 | VELVHFLLLKY | 11 | MAGE2 | 114 | 52 | 550 | 294 | 1551 | 49 | 1790 | 3 |
| 690 | LBSVLRNCQDF | 11 | MAGE2 | 136 | 64 | 5409 | 3458 | 209 | 76 | 15,241 | 3 |
| 691 | IEVVBVVPISHLY | 13 | MAGB2 | 164 | 108 | 1191 | 610 | 214 | 123 | 2639 | 3 |
| 692 | VEVVPISHLY | 10 | MAGE2 | 167 | 99 | 11,522 | 4385 | 13 | 346 | 6776 | 3 |
| 693 | FEGREDSVF | 9 | MAGE2 | 231 | 9.8 | 2366 | 348 | 4434 | 13 | 3339 | 3 |
| 694 | EEGLEARGEA | 10 | MAGE2 | 13 | 1077 | 3434 | 3227 | 216 | 684 | 30 | 2 |
| 695 | LEARGBALG | 9 | MAGE2 | 16 | 155 | 1161 | 3006 | -- | 24 | 2688 | 2 |
| 696 | VEVTLGEVPAA | 11 | MAGE2 | 46 | 124 | -- | 919 | -- | 44 | 1583 | 2 |
| 697 | EEEGPRMFPDL | 11 | MAGE2 | 91 | 1011 | 2646 | 3470 | 3273 | 131 | 209 | 2 |
| 698 | SEFQAAI | 7 | MAGE2 | 103 | 181 | 6830 | 779 | 2660 | 33 | 9597 | 2 |
| 699 | REPVTKAEMLESV | 13 | MAGE2 | 127 | 2495 | 253 | 605 | 4546 | 40 | 4579 | 2 |
| 700 | IEGDCAPEEKI | 11 | MAGE2 | 211 | 844 | -- | -- | 2627 | 486 | 183 | 2 |
| 701 | EEKIWEEL | 8 | MAGE2 | 218 | 753 | 9084 | 2599 | 12,420 | 104 | 171 | 2 |
| 702 | EEKIWEELSML | 11 | MAGE2 | 218 | 1641 | 4978 | -- | 1862 | 375 | 181 | 2 |
| 703 | LEVFEGRBDSV | 11 | MAGE2 | 228 | 639 | 2624 | 295 | -- | 46 | -- | 2 |
| 704 | FEGREDSVFA | 10 | MAGE2 | 231 | 242 | -- | 4775 | 6879 | 192 | 503 | 2 |
| 705 | PEEGLEARG | 9 | MAGE2 | 12 | 1252 | 292 | -- | -- | 2094 | 1967 | 1 |
| 706 | EEQQTASSSST | 11 | MAGE2 | 34 | 752 | 2306 | -- | 5910 | 1552 | 134 | 1 |
| 707 | QEEEGPRMFPDL | 12 | MAGE2 | 90 | 4178 | 1769 | 2931 | 2186 | 394 | 1200 | 1 |
| 708 | EEEGPRMF | 8 | MAGE2 | 91 | 723 | 12,281 | -- | 2406 | 213 | 943 | 1 |
| 709 | SEYLQLV | 7 | MAGE2 | 155 | 1375 | 7777 | 658 | 733 | 21 | 930 | 1 |
| 710 | PEEKIWEEL | 9 | MAGE2 | 217 | 577 | 19,449 | 3908 | 2893 | 235 | 17,345 | 1 |
| 711 | GEPHISY | 7 | MAGE2 | 295 | 8833 | 12,272 | 6716 | -- | 272 | -- | 1 |
| 712 | PEEGLEARGEA | 11 | MAGE2 | 12 | 15,209 | -- | 18,624 | -- | 950 | 3193 | 0 |
| 713 | EEGLEARGEALGL | 13 | MAGE2 | 13 | 8199 | 3694 | 18,543 | 2515 | 4468 | 5856 | 0 |
| 714 | DEGSSNQEEEG | 11 | MAGE2 | 84 | 10,836 | -- | -- | -- | 2125 | -- | 0 |
| 715 | PEEKTWEELSM | 11 | MAGE2 | 217 | 6039 | -- | 18,680 | -- | 985 | 15,500 | 0 |
| 716 | WEBLSML | 7 | MAGE2 | 222 | 1288 | 781 | 740 | -- | 610 | -- | 0 |
| 717 | WEELSML | 7 | MAGE2 | 234 | 17,670 | 10,408 | -- | -- | 2010 | 15,074 | 0 |
| 718 | WEELSML | 7 | MAGE2 | 252 | 8292 | 6581 | -- | 2999 | 10,009 | 2724 | 0 |

**Table 30: MAGE3-defived B44**

| SEQ ID NO | Sequence | AA | Protein | Position | B* 1801 | B* 4001 | B* 4002 | B* 4402 | B* 4403 | B* 4501 | Degeneracy |
|---|---|---|---|---|---|---|---|---|---|---|---|
| 719 | QEAASSSSTL | 10 | MACB3 | 36 | 144 | 49 | 47 | 56 | 13 | 287 | 6 |
| 720 | LESEFQAAL | 9 | MACB3 | 101 | 5.4 | 19 | 16 | 95 | 1.0 | 113 | 6 |
| 721 | AELVHFLLL | 9 | MAGE3 | 114 | 160 | 25 | 3.1 | 18 | 0.94 | 141 | 6 |
| 722 | MEVDPIGHL | 9 | MAGE3 | 167 | 14 | 1.6 | 21 | 165 | 1.7 | 247 | 6 |
| 723 | MEVDPIGHLY1 | 11 | MAGE3 | 167 | 9.8 | 34 | 16 | 64 | 0.91 | 95 | 6 |
| 724 | AELVHFLL | 8 | MAGE3 | 114 | 120 | 71 | 6.8 | 1186 | 0.16 | 452 | 5 |
| 725 | ABLVHFLLLKY | 11 | MAGE3 | 114 | 153 | 32 | 39 | 178 | 1.6 | 670 | 5 |
| 726 | ABMLGSVVG | 9 | MAGE3 | 133 | 96 | 1899 | 109 | 19 | 1.6 | 11 | 5 |
| 727 | REKIWEELSV | 10 | MAGE3 | 218 | 449 | 8947 | 79 | 396 | 17 | 17 | 5 |
| 728 | WEELSVLEVF | 10 | MAGE3 | 222 | 14 | 75 | 37 | 14 | 13 | 1701 | 5 |
| 729 | VETSYVKVL | 9 | MAGE3 | 279 | 87 | 7.8 | 57 | 80 | 1.1 | 2687 | 5 |
| 730 | BBGPSTPPDL | 10 | MAGE3 | 92 | 165 | 655 | 591 | 198 | 127 | 128 | 4 |
| 731 | IELMEVDPI | 9 | MAGE3 | 164 | 78 | 296 | 252 | 4042 | 3.1 | 11,937 | 4 |
| 732 | MEVDPIGHLY | 10 | MAGE3 | 167 | 14 | 617 | 625 | 11 | 99 | 169 | 4 |
| 733 | EEKIWEELSVL | 11 | MAGE3 | 218 | 133 | 25 | 1255 | 1416 | 58 | 218 | 4 |
| 734 | EELSVLEVF | 9 | MAGE3 | 223 | 7.3 | 75 | 3314 | 99 | 12 | 2120 | 4 |
| 735 | EEEGPSTF | 8 | MAGE3 | 91 | 201 | 1008 | 435 | 3933 | 27 | 1819 | 3 |
| 736 | BBBGPSTFPDL | 11 | MAGE3 | 91 | 935 | 431 | 2120 | 2685 | 102 | 158 | 3 |
| 737 | WEELSVLEV | 9 | MAGE3 | 222 | 8.0 | 2479 | 158 | -- | 2.6 | 538 | 3 |
| 738 | FEGREDSIL | 9 | MAGE3 | 231 | 1091 | 4.9 | 439 | 1925 | 11 | -- | 3 |
| 739 | FEGREDSILG | 10 | MAGE3 | 231 | 229 | 460 | 4361 | 8534 | 172 | -- | 3 |
| 740 | QEAASSSST | 9 | MAGE3 | 36 | 1422 | -- | 1480 | 3823 | 41 | 110 | 2 |
| 741 | REGDCAPEEKI | 11 | MAGE3 | 211 | 973 | 2418 | 830 | 4038 | 42 | 146 | 2 |
| 742 | LEVFEGREDSI | 11 | MAGE3 | 228 | 4745 | 206 | 512 | -- | 69 | -- | 2 |
| 743 | FEGREDSI | 8 | MAGE3 | 231 | 5763 | 718 | 127 | 14,181 | 13 | 2291 | 2 |
| 744 | QEEEGPSTF | 9 | MAGE3 | 90 | 841 | -- | 16,118 | 324 | 529 | 2450 | 1 |
| 745 | IELMEVDPIG | 10 | MAGB3 | 164 | 506 | 6592 | 5325 | 222 | -- | 7604 | 1 |

**Table 31. Hepatitis B Virus Core Protein (SEQ ID NO: 754)**

| |
|---|
| MQLFHLCLIISCSCPTVQASKLCLGWLWGMDIDPYKEFGATVELLSFLPSDFFPSV RDLLDTASALYREALESPEHCSPHHTALRQAILCWGELMTLATWVGVNLEDPASR DLVVSYVNTNMGLKFRQLLWFHISCLTFGRETVIEYLVSFGVWIRTPPAYRPPNAPIL STLPETTVVRRRGRSPRRRTPSPRRRRSQSPRRRRSQSRESQC |

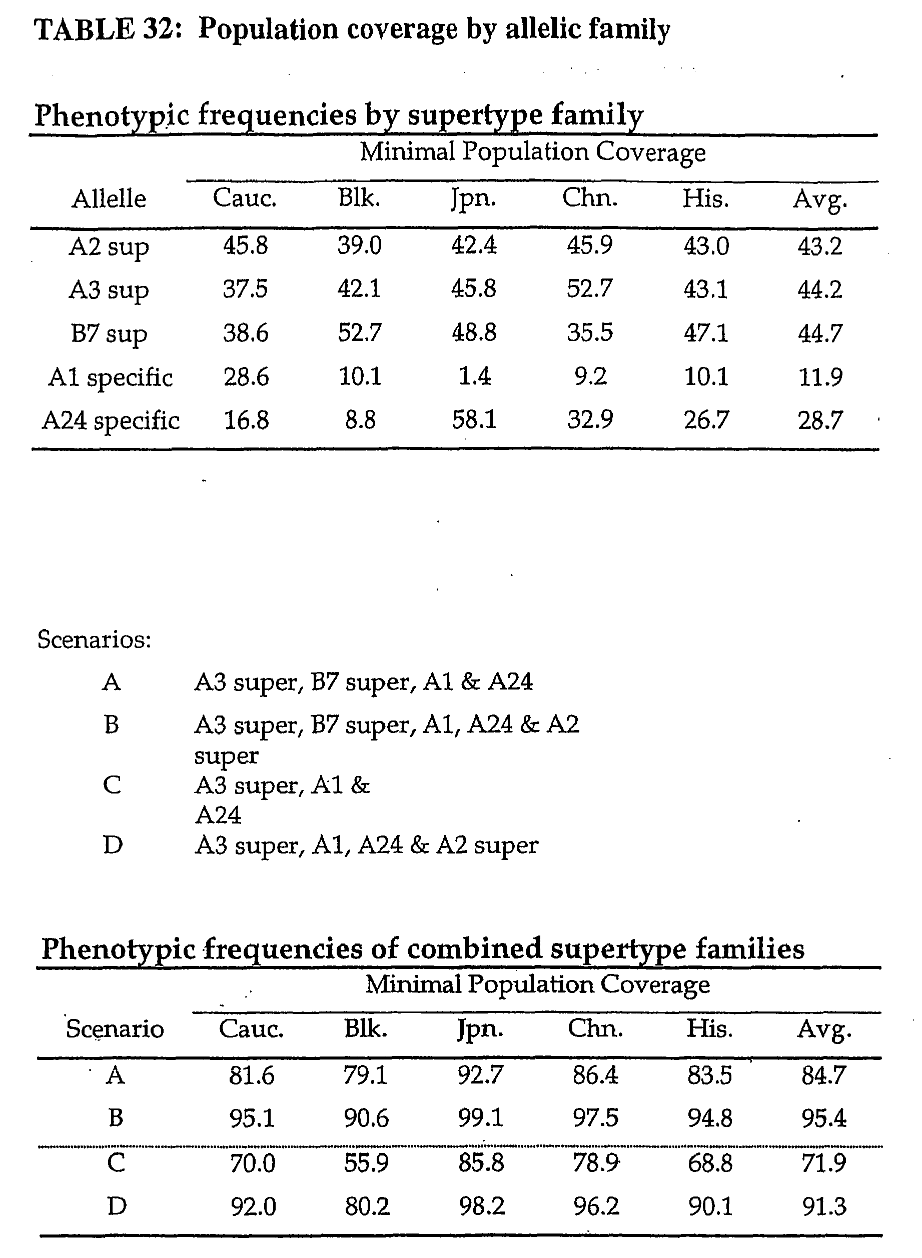

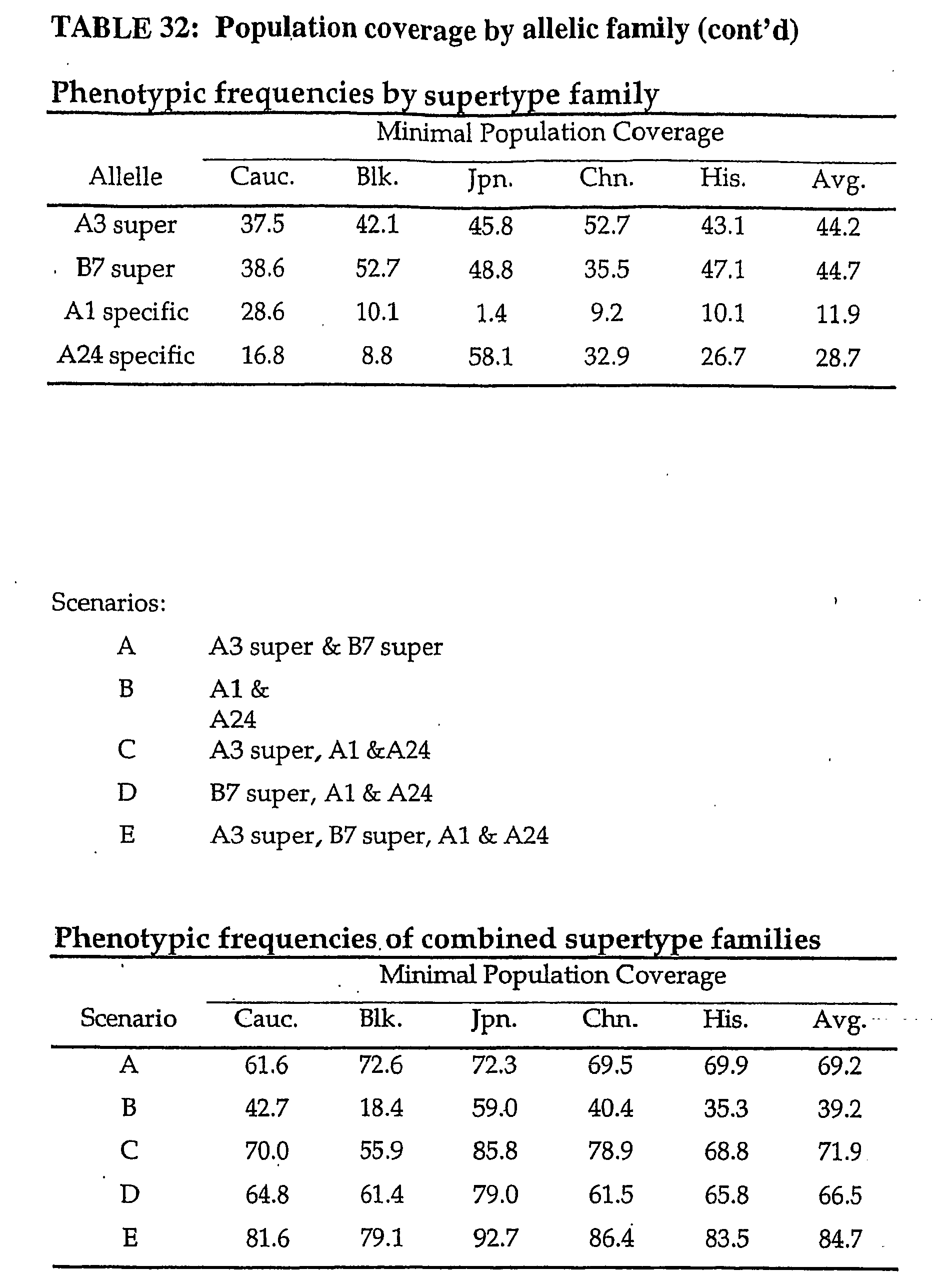

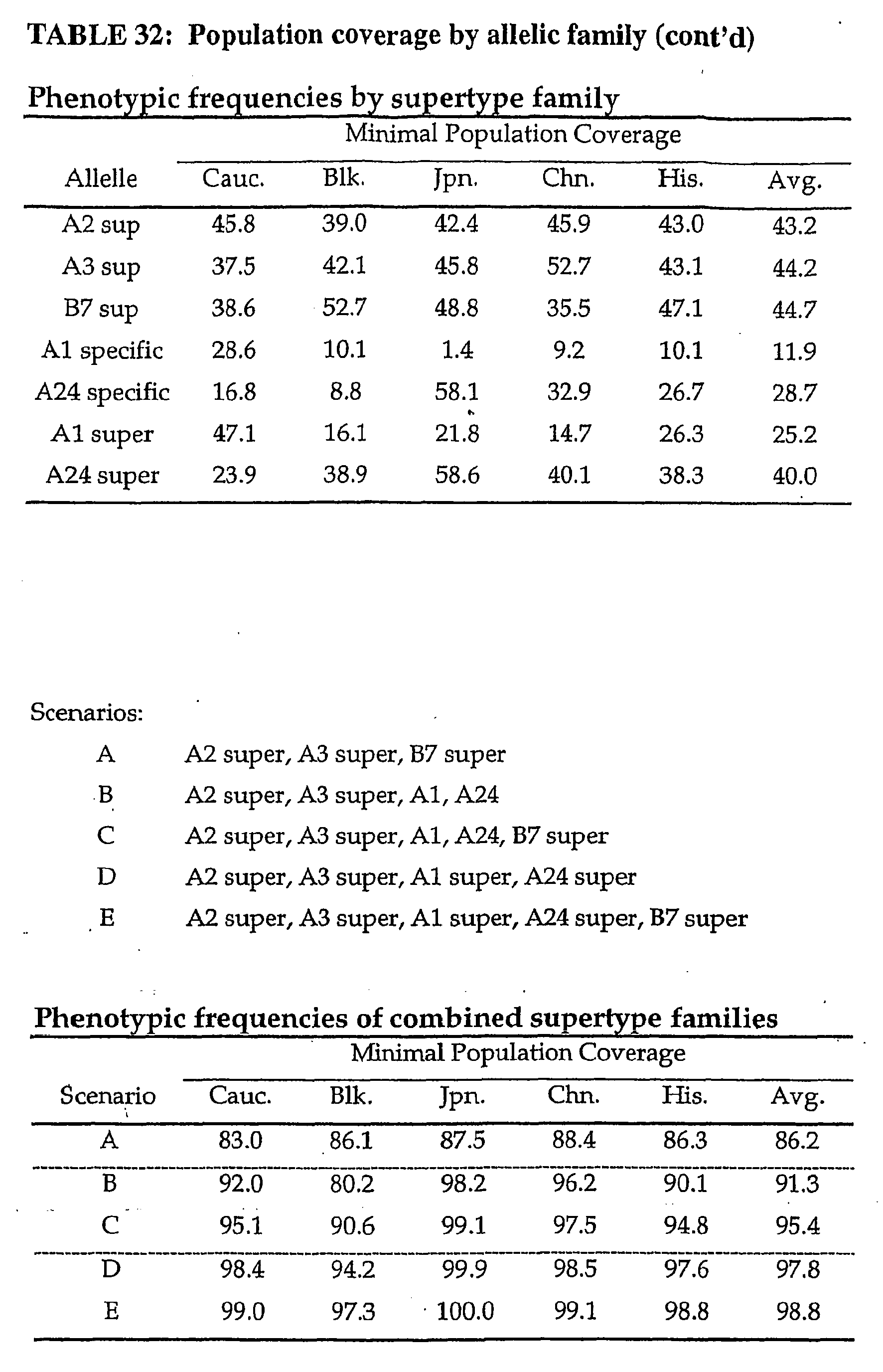

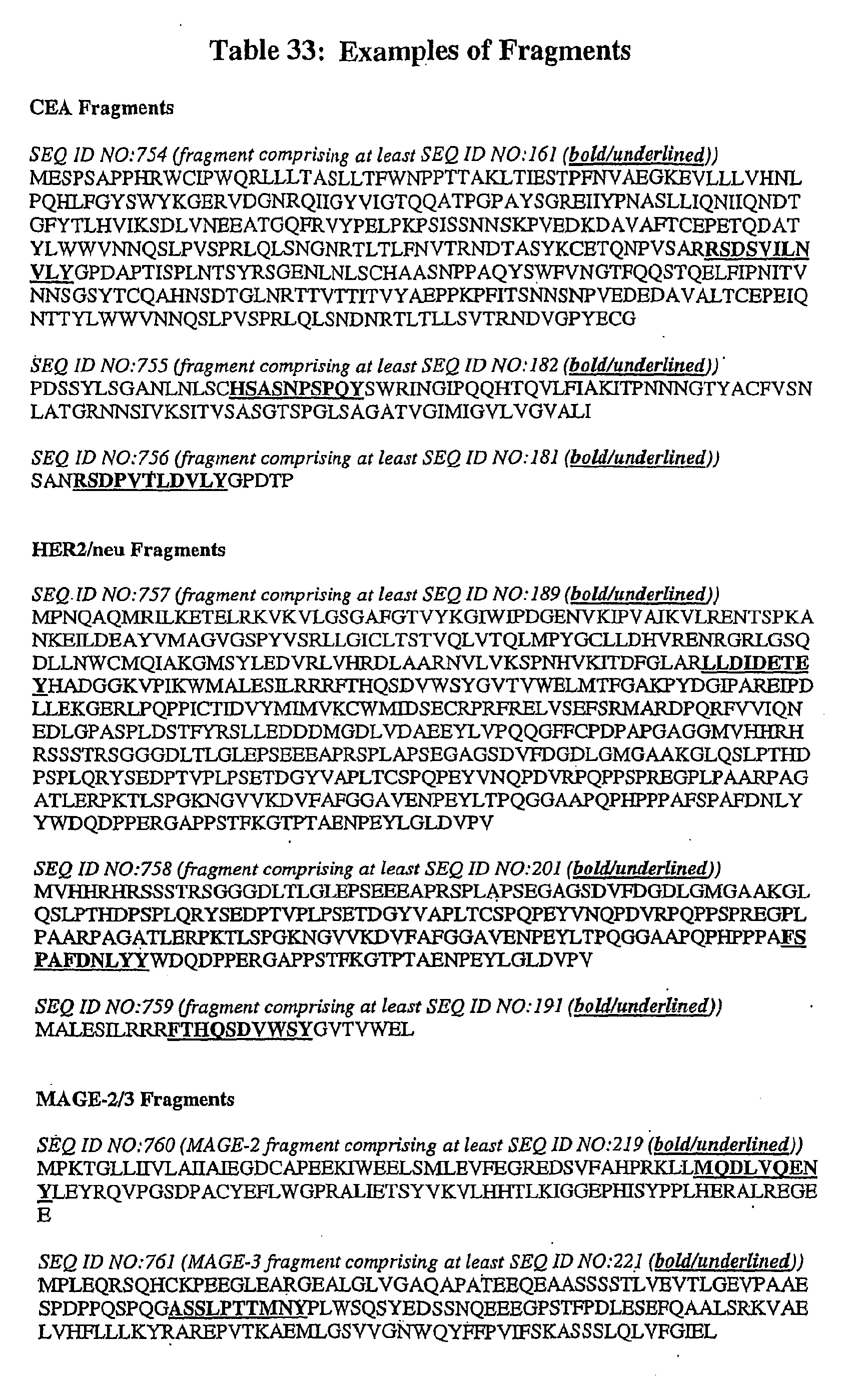

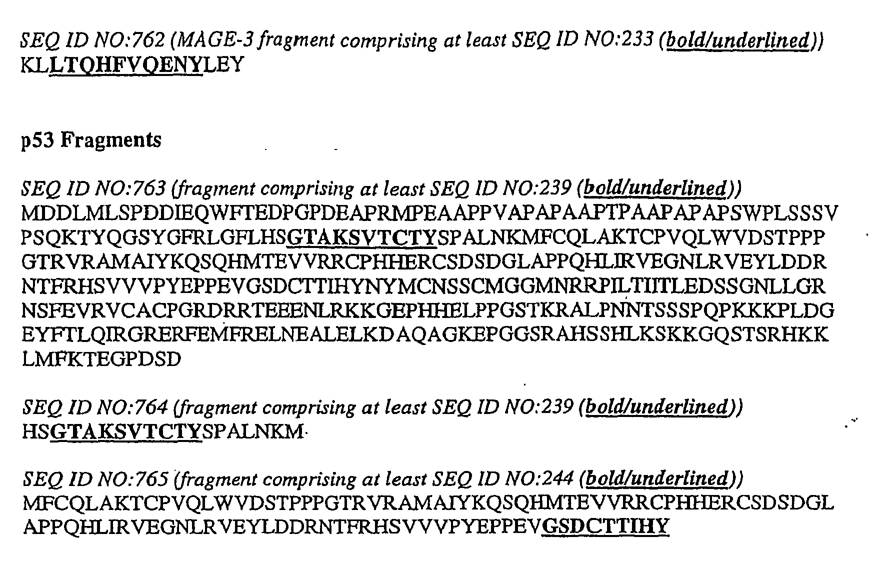

Some additional embodiments of the invention are described in the following paragraphs.
1. An isolated peptide selected from the group consisting of:
   (a) a peptide comprising at least 1 epitope or analog selected from the group consisting of SEQ ID Nos : 42-361, but excluding SEQ ID NO : 42,60, 62,67, 82,86, 101,116, 153, 362,230, 265,290, 321,334, and 345;
   (b) a peptide comprising at least 2 epitopes or analogs selected from the group consisting of the epitopes and analogs in Table 16a;
   (c) a peptide comprising at least 3 epitopes or analogs selected from the group consisting of the epitopes and analogs in Table 16b;
   (d) a peptide comprising at least 4 epitopes or analogs selected from the group consisting of the epitopes and analogs in Table 16c;
   (e) a peptide comprising at least 2 epitopes or analogs selected from the group consisting of the epitopes and analogs in Table 16d;
   (f) a peptide comprising at least 1 epitope or analog selected from the group consisting of the epitopes and analogs in Table 17a;
   (g) a peptide comprising at least 1 epitope or analog selected from the group consisting of the epitopes and analogs in Table 17b;
   (h) a peptide comprising at least 3 epitopes or analogs selected from the group consisting of the epitopes and analogs in Table 17c;
   (i) a peptide comprising at least 1 epitope or analog selected from the group consisting of the epitopes and analogs in Table 17d;
   (j) a peptide comprising at least 1 epitope or analog selected from the group consisting of the epitopes and analogs in Table 18a;
   (k) a peptide comprising at least 1 epitope or analog selected from the group consisting of the epitopes and analogs in Table 18b;
   (l) a peptide comprising at least 2 epitopes or analogs selected from the group consisting of the epitopes and analogs in Table 18c;
   (m) a peptide comprising at least 1 epitope or analog selected from the group consisting of the epitopes and analogs in Table 18d;
   (n) a peptide comprising at least 3 epitopes or analogs selected from the group consisting of the epitopes and analogs in Table 19a;
   (o) a peptide comprising at least 1 epitope or analog selected from the group consisting of the epitopes and analogs in Table 19b;
   (p) a peptide comprising at least 4 epitopes or analogs selected from the group consisting of the epitopes and analogs in Table 19c;
   (q) a peptide comprising at least 1 epitope or analog selected from the group consisting of the epitopes and analogs in Table 19d;
   (r) a peptide comprising at least 7 epitopes or analogs selected from the group consisting of the epitopes and analogs in Table 20;
   (s) a peptide comprising at least 2 epitopes or analogs selected from the group consisting of the epitopes and analogs in Table 21;
   (t) a peptide comprising at least 2 epitopes or analogs selected from the group consisting of the epitopes and analogs in Table 22;
   (u) a peptide comprising at least 6 epitopes or analogs selected from the group consisting of the epitopes and analogs in Table 23;
   (v) a peptide comprising at least 1,2, 3,4, 5, or 6 epitopes or analogs selected from the group consisting of the epitopes and analogs in Table 26;
   (w) a peptide comprising at least 1,2, 3,4, 5, or 6 epitopes or analogs selected from the group consisting of the epitopes and analogs in Table 27;
   (x) a peptide comprising at least 1,2, 3,4, 5, or 6 epitopes or analogs selected from the group consisting of the epitopes and analogs in Table 28;
   (y) a peptide comprising at least 1,2, 3,4, 5, or 6 epitopes or analogs selected from the group consisting of the epitopes and analogs in Table 29; and
   (z) a peptide comprising at least 1,2, 3,4, 5, or 6 epitopes or analogs selected from the group consisting of the epitopes and analogs in Table 30.
2. The peptide of paragrah 1, which comprises at least 2 of said epitopes or analogs.
3. The peptide of paragraph 1, which comprises at least 3 of said epitopes or analogs.
4. The peptide of paragraph 1, which comprises at least 4 of said epitopes or analogs.
5. The peptide of paragraph 1, which comprises at least 5 of said epitopes or analogs.
6. The peptide of paragraph 1, which comprises at least 6 of said epitopes or analogs.
7. The peptide of paragraph 1, which comprises at least 7 of said epitopes or analogs.
8. The peptide of paragraph 1, which comprises at least 8 of said epitopes or analogs.
9. The peptide of paragraph 1, which comprises at least 9 of said epitopes or analogs.
10. An isolated peptide comprising (a) an epitope or analog selected from the group consisting of SEQ ID NO : 42,60, 62, 67,82, 86,101, 116,153, 362,230, 265,290, 321,334, and 345; and (b) an epitope or analog selected from the group consisting of SEQ ID NO : 41-59,61, 63-66,68-81, 83-85, 87- 100,102-115, 117-152,154-229, 231-264,266-289, 291-320, 322-333,335-344, and 346-361.
11. An isolated peptide comprising (a) an epitope or analog selected from the group consisting of: SEQ ID NO : 42,60, 62, 67,82, 86,101, 116,153, 362,230, 265,290, 321,334, and 345; and (b) an epitope or analog selected from the group consisting of: SEQ ID NO: 368-745.
12. An isolated peptide comprising (a) an epitope or analog selected from the group consisting of SEQ ID NO: 1-25; and (b) an epitope or analog selected from the group consisting of: SEQ ID NO: 41-59,61, 63-66,68-81, 83-85,87-100, 102-115, 117-152,154-229, 231-264, 266-289, 291-320,322-333, 335- 344, and 346-361.
13. An isolated peptide comprising (a) an epitope or analog selected from the group consisting of: SEQ ID NO: 1-25; and (b) an epitope or analog selected from the group consisting of: SEQ ID NO: 368-745.
14. An isolated peptide selected from the group consisting of :
   (a) a peptide comprising at least 2 epitopes or analogs selected from the group consisting of: SEQ ID NO : 42,44, 46, 51, 52,54, and 55;
   (b) a peptide comprising at least 4 epitopes or analogs selected from the group consisting of: SEQ ID NO: 57,60, 62,67, 68,69, 70,73, and 75;
   (c) a peptide comprising at least 2 epitopes or analogs selected from the group consisting of: SEQ ID NO : 77,82, 90, 91, 96,99, 102, and 103;
   (d) a peptide comprising at least 2 epitopes or analogs selected from the group consisting of : SEQ ID NO : 104,107, 111, 114,116, 119, and 124;
   (e) a peptide comprising at least 1 epitope or analog selected from the group consisting of SEQ ID NO : 133, 136, and 155;
   (f) a peptide comprising at least 2 epitopes or analogs selected from the group consisting of SEQ ID NO : 140,146, and 153;
   (g) a peptide comprising at least 1 epitope or analog selected from the group consisting of: SEQ ID NO : 161,167, 170, 172, 178, 180,181, and 182;
   (h) a peptide comprising at least 1 epitope or analog selected from the group consisting of: SEQ ID NO : 186,188, 189, 191,194, 198,200, 201, and 208;
   (i) a peptide comprising at least 2 epitopes or analogs selected from the group consisting of SEQ ID NO : 211, 216,219, 221, 228, 230,234, and 236;
   (j) a peptide comprising at least 1 epitope or analog selected from the group consisting of SEQ ID NO : 238, 239,240, 242, and 246;
   (k) a peptide comprising at least 3 epitopes or analogs selected from the group consisting of : SEQ ID NO : 256,263, 265, 269,272, 278, 279,281, 282,285, 287, and 290;
   (l) a peptide comprising at least 1 epitope or analog selected from the group consisting of: SEQ ID NO: 292,293, 304, 305, 308, and 310;
   (m) a peptide comprising at least 4 epitopes or analogs selected from the group consisting of: SEQ ID NO: 316,321, 324, 345,331-336, 344,345, and 351;
   (n) a peptide comprising at least 1 epitope or analog selected from the group consisting of: SEQ ID NO: 356 and 361.
15. The peptide of any one of paragraphs 1-14, which comprises an HTL peptide.
16. A composition comprising the peptide of any of paragraphs 1-15, and a diluent.
17. A composition comprising:
   (aa) at least 1 epitope or analog selected from the group consisting of : SEQ ID Nos: 42-361, but excluding SEQ ID NO : 42,60, 62,67, 82,86, 101,116, 153,362, 230,265, 290,321, 334, and 345;
   (bb) at least 2 epitopes or analogs selected from the group consisting of the epitopes and analogs in Table 16a;
   (cc) at least 3 epitopes or analogs selected from the group consisting of the epitopes and analogs in Table 16b;
   (dd) at least 4 epitopes or analogs selected from the group consisting of the epitopes and analogs in Table 16c;
   (ee) at least 2 epitopes or analogs selected from the group consisting of the epitopes and analogs in Table 16d;
   (ff) at least 1 epitope or analog selected from the group consisting of the epitopes and analogs in Table 17a;
   (gg) at least 1 epitope or analog selected from the group consisting of the epitopes and analogs in Table 17b;
   (hh) at least 3 epitopes or analogs selected from the group consisting of the epitopes and analogs in Table 17c;
   (ii) at least 1 epitope or analog selected from the group consisting of the epitopes and analogs in Table 17d;
   (jj) at least 1 epitope or analog selected from the group consisting of the epitopes and analogs in Table 18a;
   (kk) at least 1 epitope or analog selected from the group consisting of the epitopes and analogs in Table 18b;
   (ll) at least 2 epitopes or analogs selected from the group consisting of the epitopes and analogs in Table 18c;
   (mm) at least 1 epitope or analog selected from the group consisting of the epitopes and analogs in Table 18d;
   (nn) at least 3 epitopes or analogs selected from the group consisting of the epitopes and analogs in Table 19a;
   (oo) at least 1 epitope or analog selected from the group consisting of the epitopes and analogs in Table 19b;
   (pp) at least 4 epitopes or analogs selected from the group consisting of the epitopes and analogs in Table 19c;
   (qq) at least 1 epitope or analog selected from the group consisting of the epitopes and analogs in Table 19d;
   (rr) at least 7 epitopes or analogs selected from the group consisting of the epitopes and analogs in Table 20;
   (ss) at least 2 epitopes or analogs selected from the group consisting of the epitopes and analogs in Table 21;
   (tt) at least 2 epitopes or analogs selected from the group consisting of the epitopes and analogs in Table 22;
   (uu) at least 6 epitopes or analogs selected from the group consisting of the epitopes and analogs in Table 23;
   (vv) at least 1,2, 3,4, 5, or 6 epitopes or analogs selected from the group consisting of the epitopes and analogs in Table 26;
   (ww) at least 1,2, 3,4, 5, or 6 epitopes or analogs selected from the group consisting of the epitopes and analogs in Table 27;
   (xx) at least 1, 2,3, 4,5, or 6 epitopes or analogs selected from the group consisting of the epitopes and analogs in Table 28;
   (yy) at least 1,2, 3,4, 5, or 6 epitopes or analogs selected from the group consisting of the epitopes and analogs in Table 29; and
   (zz) at least 1,2, 3,4, 5, or 6 epitopes or analogs selected from the group consisting of the epitopes and analogs in Table 30.
18. The composition of paragraph 17, which comprises at least 2 of said epitopes or analogs.
19. The composition of paragraph 17, which comprises at least 3 of said epitopes or analogs.
20. The composition of paragraph 17, which comprises at least 4 of said epitopes or analogs.
21. The composition of paragraph 17, which comprises at least 5 of said epitopes or analogs.
22. The composition of paragraph 17, which comprises at least 6 of said epitopes or analogs.
23. The composition of paragraph 17, which comprises at least 7 of said epitopes or analogs.
24. The composition of paragraph 17, which comprises at least 8 of said epitopes or analogs.
25. The composition of paragraph 17, which comprises at least 9 of said epitopes or analogs.
26. A composition comprising (a) an epitope or analog selected from the group consisting of SEQ ID NO: 42,60, 62,67, 82, 86,101, 116,153, 362,230, 265,290, 321,334, and 345; and (b) an epitope or analog selected from the group consisting of: SEQ ID NO: 41-59,61, 63-66, 68-81, 83-85, 87-100,102-115, 117-152,154-229, 231-264, 266-289, 291-320,322-333, 335- 344, and 346-361.
27. A composition comprising (a) an epitope or analog selected from the group consisting of: SEQ ID NO: 42, 60, 62, 67, 82, 86, 101, 116, 153,362, 230,265, 290,321, 334, and 345; and (b) an epitope or analog selected from the group consisting of : SEQ ID NO: 368-745.
28. A composition comprising (a) an epitope or analog selected from the group consisting of: SEQ ID NO: 1-25; and (b) an epitope or analog selected from the group consisting of: SEQ ID NO: 41-59,61, 63-66,68-81, 83-85, 87-100, 102-115,117-152,154-229, 231-264, 266-289, 291-320,322-333, 335-344, and 346-361.
29. A composition comprising (a) an epitope or analog selected from the group consisting of: SEQ ID NO: 1-25; and (b) an epitope or analog selected from the group consisting of: SEQ ID NO: 368-745.
30. A composition selected from the group consisting of:
   (o) at least 2 epitopes or analogs selected from the group consisting of SEQ ID NO: 42,44, 46,51, 52,54, and 55;
   (p) at least 4 epitopes or analogs selected from the group consisting of : SEQ ID NO: 57,60, 62,67, 68,69, 70,73, and 75;
   (q) at least 2 epitopes or analogs selected from the group consisting of SEQ ID NO: 77,82, 90,91, 96,99, 102, and 103;
   (r) at least 2 epitopes or analogs selected from the group consisting of : SEQ ID NO: 104,107, 111, 114, 116, 119, and 124;
   (s) at least 1 epitope or analog selected from the group consisting of SEQ ID NO: 133,136, and 155;
   (t) at least 2 epitopes or analogs selected from the group consisting of: SEQ ID NO: 140,146, and 153;
   (u) at least 1 epitope or analog selected from the group consisting of: SEQ ID NO : 161,167, 170,172, 178, 180, 181, and 182;
   (v) at least 1 epitope or analog selected from the group consisting of: SEQ ID NO: 186, 188, 189, 191,194, 198, 200,201, and 208;
   (w) at least 2 epitopes or analogs selected from the group consisting of: SEQ ID NO: 211,216, 219,221, 228, 230, 234, and 236;
   (x) at least 1 epitope or analog selected from the group consisting of : SEQ ID NO: 238,239, 240,242, and 246;
   (y) at least 3 epitopes or analogs selected from the group consisting of SEQ ID NO: 256,263, 265,269, 272,278, 279,281, 282, 285, 287, and 290;
   (z) at least 1 epitope or analog selected from the group consisting of: SEQ ID NO: 292,293, 304,305, 308, and 310;
   (aa) at least 4 epitopes or analogs selected from the group consisting of: SEQ ID NO: 316,321, 324,345, 331-336, 344,345, and 351;
   (bb) at least 1 epitope or analog selected from the group consisting of SEQ ID NO: 356 and 361.
31. The composition of any of paragraphs 17-30, which further comprises an HTL peptide.

## Claims

1. A composition comprising a combination of epitopes or analogs wherein at least one epitope or analog is derived from each of the tumour associated antigens:
i) carcinoembryonic antigen (CEA);
ii) p53;
iii) HER2/neu; and
iv) MAGE2/3,
and wherein said epitopes or analogs are present on one or more isolated peptides, each peptide being less than about 50 amino acids in length.

2. A composition according to claim 1 wherein each peptide is a homopolymer of one epitope or analog derived from carcinoembryonic antigen (CEA), p53, HER2/neu or MAGE 2/3.

3. A composition according to claim 1 wherein each peptide is a heteropolymer of two or more different epitopes or analogs derived from carcinoembryonic antigen (CEA), p53, HER2/neu or MAGE 2/3.

4. A composition according to claim 1 wherein each peptide comprises two or more different epitopes or analogs from different tumour associated antigens selected from the group set out in claim 1.

5. A composition according to any one of the preceding claims further comprising a carrier.

6. The composition of claim 5, further comprising a CTL-inducing peptide.

7. The composition of claim 6, wherein said CTL-inducing peptide also comprises an epitope or analog derived from a tumor associated antigen selected from the group set out in claim 1.

8. The composition according to any one of claims 1 to 4, further comprising a T helper peptide.

9. The composition of claim 8, wherein said T helper peptide is a pan-DR binding epitope.

10. The composition according to claim 5, wherein said carrier is a liposome, and wherein said one or more peptides is on or within said liposome.

11. The composition according to claim 5, wherein said carrier is a lipid.

12. The composition according to claim 5 wherein in said one or more peptides is linked to a spacer molecule.

13. The composition according to claim 5, wherein said carrier is an antigen presenting cell, and wherein said one or more peptide is on or within said antigen presenting cell.

14. The composition of claim 13, wherein said antigen presenting cell is a dendritic cell.

15. A polypeptide comprising at least a first epitope and a second epitope, wherein said first epitope comprises an epitope or analog derived from a first tumor associated antigen and said second epitope comprises an epitope or analog derived from a second tumour associated antigen wherein said first and second tumour associated antigens are selected from the group set out in claim 1.

16. A composition comprising the polypeptide of claim 15, wherein said polypeptide is a homopolymer.

17. A composition comprising the polypeptide of claim 15, wherein said polypeptide is a heteropolymer.

18. An isolated nucleic acid encoding the polypeptide of claim 15.

19. A composition or polypeptides according to any one of claims 1 to 17 for use in a method of treating or preventing cancer in a mammal comprising administering the composition or polypeptide to a mammal in need thereof.

20. An isolated nucleic acid according to claim 18 for use in a method of treating or preventing cancer in a mammal comprising administering the nucleic acid to a mammal in need thereof.


SEQUENCE LISTING

<110>  Epimmune Inc.

<120>  HLA-A1, -A2, -A3, -A24, -B7, and -B44 Tumor Associated Antigen
       Peptides and Compositions

<130>  JEC/FP6515449

<140>  Divisional of EP 03812851.8
<141>  2003-12-10

<140>  EP 03812851.8
<141>  2003-12-10

<140>  PCT/US03/038949
<141>  2003-12-10

<150>  US 60/432,017
<151>  2002-12-10

<160>  767

<170>  PatentIn version 3.2

<210>  1
<211>  10
<212>  PRT
<213>  Homo sapiens

<400>  1

Val Leu Tyr Gly Pro Asp Ala Pro Thr Val
1               5                   10

<210>  2
<211>  9
<212>  PRT
<213>  Homo sapiens

<400>  2

Tyr Leu Ser Gly Ala Asn Leu Asn Val
1               5

<210>  3
<211>  9
<212>  PRT
<213>  Homo sapiens

<400>  3

Ala Thr Val Gly Ile Met Ile Gly Val
1               5

<210>  4
<211>  11
<212>  PRT

<213>  Homo sapiens

<400>  4
Leu Leu Pro Glu Asn Asn Val Leu Ser Pro Val
1               5                   10


<210>  5
<211>  9
<212>  PRT
<213>  Homo sapiens

<400>  5

Lys Leu Cys Pro Val Gln Leu Trp Val
1               5


<210>  6
<211>  9
<212>  PRT
<213>  Homo sapiens

<220>
<221>  misc_feature
<222>  (3)..(3)
<223>  Xaa = alpha-amino butyric acid

<400>  6

Lys Leu Xaa Pro Val Gln Leu Trp Val
1               5


<210>  7
<211>  9
<212>  PRT
<213>  Homo sapiens

<400>  7

Ser Leu Pro Pro Pro Gly Thr Arg Val
1               5


<210>  8
<211>  9
<212>  PRT
<213>  Homo sapiens

<400>  8

Ser Met Pro Pro Pro Gly Thr Arg Val
1               5


<210>  9
<211>  9
<212>  PRT

<213>  Homo sapiens

<400>  9

Lys Leu Phe Gly Ser Leu Ala Phe Val
1               5


<210>  10
<211>  9
<212>  PRT
<213>  Homo sapiens

<400>  10

Lys Val Phe Gly Ser Leu Ala Phe Val
1               5


<210>  11
<211>  10
<212>  PRT
<213>  Homo sapiens

<400>  11

Val Met Ala Gly Val Gly Ser Pro Tyr Val
1               5                   10


<210>  12
<211>  9
<212>  PRT
<213>  Homo sapiens

<400>  12

Ala Leu Cys Arg Trp Gly Leu Leu Leu
1               5


<210>  13
<211>  9
<212>  PRT
<213>  Homo sapiens

<400>  13

Phe Leu Trp Gly Pro Arg Ala Leu Val
1               5


<210>  14
<211>  9
<212>  PRT
<213>  Homo sapiens

<400>  14

His Leu Tyr Gln Gly Cys Gln Val Val
1               5

<210>  15
<211>  9
<212>  PRT
<213>  Homo sapiens

<400>  15

Ile Leu His Asn Gly Ala Tyr Ser Leu
1               5

<210>  16
<211>  9
<212>  PRT
<213>  Homo sapiens

<400>  16

Ile Met Ile Gly Val Leu Val Gly Val
1               5

<210>  17
<211>  9
<212>  PRT
<213>  Homo sapiens

<400>  17

Lys Ile Phe Gly Ser Leu Ala Phe Leu
1               5

<210>  18
<211>  9
<212>  PRT
<213>  Homo sapiens

<400>  18

Lys Val Ala Glu Leu Val His Phe Leu
1               5

<210>  19
<211>  9
<212>  PRT
<213>  Homo sapiens

<400>  19

Leu Leu Thr Phe Trp Asn Pro Pro Val
1               5

<210>  20
<211>  10
<212>  PRT
<213>  Homo sapiens

<400>  20

Leu Val Phe Gly Ile Glu Leu Met Glu Val
1               5                   10


<210>  21
<211>  11
<212>  PRT
<213>  Homo sapiens

<400>  21

Gln Leu Val Phe Gly Ile Glu Leu Met Glu Val
1               5                   10


<210>  22
<211>  9
<212>  PRT
<213>  Homo sapiens

<400>  22

Arg Leu Leu Gln Glu Thr Glu Leu Val
1               5


<210>  23
<211>  9
<212>  PRT
<213>  Homo sapiens

<400>  23

Val Val Leu Gly Val Val Phe Gly Ile
1               5


<210>  24
<211>  10
<212>  PRT
<213>  Homo sapiens

<400>  24

Tyr Leu Gln Leu Val Phe Gly Ile Glu Val
1               5                   10


<210>  25
<211>  10
<212>  PRT
<213>  Homo sapiens

<400>  25

Tyr Met Ile Met Val Lys Cys Trp Met Ile
1               5                   10

<210>  26
<211>  14
<212>  PRT
<213>  Clostridium tetani

<400>  26

Gln Tyr Ile Lys Ala Asn Ser Lys Phe Ile Gly Ile Thr Glu
1               5                   10

<210>  27
<211>  21
<212>  PRT
<213>  Plasmodium falciparum

<400>  27

Asp Ile Glu Lys Lys Ile Ala Lys Met Glu Lys Ala Ser Ser Val Phe
1               5                   10                  15

Asn Val Val Asn Ser
            20

<210>  28
<211>  16
<212>  PRT
<213>  Streptococcus sp.

<400>  28

Gly Ala Val Asp Ser Ile Leu Gly Gly Val Ala Thr Tyr Gly Ala Ala
1               5                   10                  15

<210>  29
<211>  13
<212>  PRT
<213>  Unknown

<220>
<223>  PADRE peptide

<220>
<221>  misc_feature
<222>  (1)..(1)
<223>  May be L- or D-Alanine

<220>

<221>  misc_feature
<222>  (3)..(3)
<223>  Xaa may be Cyclohexylalanine, Phe or Tyr

<220>
<221>  misc_feature
<222>  (7)..(7)
<223>  Xaa may be Trp, Tyr, His or Asn

<220>
<221>  misc_feature
<222>  (13)..(13)
<223>  May be L- or D-Alanine

<220>
<221>  misc_feature
<222>  (13)..(13)
<223>  May be amidated

<400>  29

Ala Lys Xaa Val Ala Ala Xaa Thr Leu Lys Ala Ala Ala
1               5                   10


<210>  30
<211>  13
<212>  PRT
<213>  Unknown

<220>
<223>  PADRE Peptide


<220>
<221>  misc_feature
<222>  (3)..(3)
<223>  Xaa may be cyclohexylalanine

<400>  30

Ala Lys Xaa Val Ala Ala Trp Thr Leu Lys Ala Ala Ala
1               5                   10


<210>  31
<211>  13
<212>  PRT
<213>  Unknown

<220>
<223>  PADRE Peptide

<400>  31

Ala Lys Phe Val Ala Ala Trp Thr Leu Lys Ala Ala Ala
1               5                   10

<210>  32
<211>  13
<212>  PRT
<213>  Unknown

<220>
<223>  PADRE Peptide

<400>  32

Ala Lys Tyr Val Ala Ala Trp Thr Leu Lys Ala Ala Ala
1               5                   10


<210>  33
<211>  13
<212>  PRT
<213>  Unknown

<220>
<223>  PADRE Peptide

<400>  33

Ala Lys Phe Val Ala Ala Tyr Thr Leu Lys Ala Ala Ala
1               5                   10


<210>  34
<211>  13
<212>  PRT
<213>  Unknown

<220>
<223>  PADRE Peptide


<220>
<221>  misc_feature
<222>  (3)..(3)
<223>  Xaa may be cyclohexylalanine

<400>  34

Ala Lys Xaa Val Ala Ala Tyr Thr Leu Lys Ala Ala Ala
1               5                   10


<210>  35
<211>  13
<212>  PRT
<213>  Unknown

<220>
<223>  PADRE Peptide

<400>  35

Ala Lys Tyr Val Ala Ala Tyr Thr Leu Lys Ala Ala Ala

1                   5                        10


<210>  36
<211>  13
<212>  PRT
<213>  Unknown

<220>
<223>  PADRE Peptide

<400>  36

Ala Lys Phe Val Ala Ala His Thr Leu Lys Ala Ala Ala
1               5                   10


<210>  37
<211>  13
<212>  PRT
<213>  Unknown

<220>
<223>  PADRE Peptide


<220>
<221>  misc_feature
<222>  (3)..(3)
<223>  Xaa may be cyclohexylalanine

<400>  37

Ala Lys Xaa Val Ala Ala His Thr Leu Lys Ala Ala Ala
1               5                   10


<210>  38
<211>  13
<212>  PRT
<213>  Unknown

<220>
<223>  PADRE Peptide

<400>  38

Ala Lys Tyr Val Ala Ala His Thr Leu Lys Ala Ala Ala
1               5                   10


<210>  39
<211>  13
<212>  PRT
<213>  Unknown

<220>
<223>  PADRE Peptide

<400>  39

Ala Lys Phe Val Ala Ala Asn Thr Leu Lys Ala Ala Ala
1               5                   10


<210>  40
<211>  13
<212>  PRT
<213>  Unknown

<220>
<223>  PADRE Peptide


<220>
<221>  misc_feature
<222>  (3)..(3)
<223>  Xaa may be cyclohexylalanine

<400>  40

Ala Lys Xaa Val Ala Ala Asn Thr Leu Lys Ala Ala Ala
1               5                   10


<210>  41
<211>  13
<212>  PRT
<213>  Unknown

<220>
<223>  PADRE Peptide

<400>  41

Ala Lys Tyr Val Ala Ala Asn Thr Leu Lys Ala Ala Ala
1               5                   10


<210>  42
<211>  9
<212>  PRT
<213>  Homo sapiens

<400>  42

His Leu Phe Gly Tyr Ser Trp Tyr Lys
1               5


<210>  43
<211>  10
<212>  PRT
<213>  Homo sapiens

<400>  43

Thr Ile Ser Pro Leu Asn Thr Ser Tyr Arg

1                   5                       10


<210>  44
<211>  10
<212>  PRT
<213>  Homo sapiens

<400>  44

Thr Ile Ser Pro Leu Asn Thr Ser Tyr Lys
1               5                   10


<210>  45
<211>  10
<212>  PRT
<213>  Homo sapiens

<400>  45

Thr Val Ser Pro Leu Asn Thr Ser Tyr Arg
1               5                   10


<210>  46
<211>  10
<212>  PRT
<213>  Homo sapiens

<400>  46

Arg Thr Leu Thr Leu Leu Ser Val Thr Arg
1               5                   10


<210>  47
<211>  10
<212>  PRT
<213>  Homo sapiens

<400>  47

Arg Val Leu Thr Leu Leu Ser Val Thr Arg
1               5                   10


<210>  48
<211>  11
<212>  PRT
<213>  Homo sapiens

<400>  48

Pro Thr Ile Ser Pro Ser Tyr Thr Tyr Tyr Arg
1               5                   10


<210>  49

<211>  10
<212>  PRT
<213>  Homo sapiens

<400>  49

Thr Ile Ser Pro Ser Tyr Thr Tyr Tyr Arg
1               5                   10


<210>  50
<211>  9
<212>  PRT
<213>  Homo sapiens

<400>  50

Ile Ser Pro Ser Tyr Thr Tyr Tyr Arg
1               5


<210>  51
<211>  9
<212>  PRT
<213>  Homo sapiens

<400>  51

Ile Val Pro Ser Tyr Thr Tyr Tyr Arg
1               5


<210>  52
<211>  10
<212>  PRT
<213>  Homo sapiens

<400>  52

Arg Thr Leu Thr Leu Phe Asn Val Thr Arg
1               5                   10


<210>  53
<211>  10
<212>  PRT
<213>  Homo sapiens

<400>  53

Arg Val Leu Thr Leu Phe Asn Val Thr Arg
1               5                   10


<210>  54
<211>  9
<212>  PRT
<213>  Homo sapiens

<400>  54

His Thr Gln Val Leu Phe Ile Ala Lys
1               5


<210>  55
<211>  9
<212>  PRT
<213>  Homo sapiens

<400>  55

Phe Val Ser Asn Leu Ala Thr Gly Arg
1               5


<210>  56
<211>  10
<212>  PRT
<213>  Homo sapiens

<400>  56

Gly Val Val Phe Gly Ile Leu Ile Lys Arg
1               5                   10


<210>  57
<211>  9
<212>  PRT
<213>  Homo sapiens

<400>  57

Val Val Phe Gly Ile Leu Ile Lys Arg
1               5


<210>  58
<211>  10
<212>  PRT
<213>  Homo sapiens

<400>  58

Val Val Phe Gly Ile Leu Ile Lys Arg Arg
1               5                   10


<210>  59
<211>  9
<212>  PRT
<213>  Homo sapiens

<400>  59

Val Val Phe Gly Ile Leu Ile Lys Lys
1               5

<210>  60
<211>  9
<212>  PRT
<213>  Homo sapiens

<400>  60

Lys Ile Arg Lys Tyr Thr Met Arg Arg
1               5


<210>  61
<211>  9
<212>  PRT
<213>  Homo sapiens

<400>  61

Lys Ile Arg Lys Tyr Thr Met Arg Lys
1               5


<210>  62
<211>  9
<212>  PRT
<213>  Homo sapiens

<400>  62

Val Leu Arg Glu Asn Thr Ser Pro Lys
1               5


<210>  63
<211>  9
<212>  PRT
<213>  Homo sapiens

<400>  63

Val Val Arg Glu Asn Thr Ser Pro Arg
1               5


<210>  64
<211>  9
<212>  PRT
<213>  Homo sapiens

<400>  64

Leu Leu Asp His Val Arg Glu Asn Arg
1               5


<210>  65
<211>  9

<212>  PRT
<213>  Homo sapiens

<400>  65

Leu Ala Ala Arg Asn Val Leu Val Lys
1               5


<210>  66
<211>  9
<212>  PRT
<213>  Homo sapiens

<400>  66

Leu Val Ala Arg Asn Val Leu Val Lys
1               5


<210>  67
<211>  9
<212>  PRT
<213>  Homo sapiens

<400>  67

Leu Val Lys Ser Pro Asn His Val Lys
1               5


<210>  68
<211>  9
<212>  PRT
<213>  Homo sapiens

<400>  68

Lys Ile Thr Asp Phe Gly Leu Ala Arg
1               5


<210>  69
<211>  9
<212>  PRT
<213>  Homo sapiens

<400>  69

Lys Val Thr Asp Phe Gly Leu Ala Arg
1               5


<210>  70
<211>  9
<212>  PRT
<213>  Homo sapiens

<400>  70

Met Ala Leu Glu Ser Ile Leu Arg Arg
1               5


<210>  71
<211>  9
<212>  PRT
<213>  Homo sapiens

<400>  71

Met Val Leu Glu Ser Ile Leu Arg Arg
1               5


<210>  72
<211>  9
<212>  PRT
<213>  Homo sapiens

<400>  72

Met Val Leu Glu Ser Ile Leu Arg Lys
1               5


<210>  73
<211>  10
<212>  PRT
<213>  Homo sapiens

<400>  73

Leu Val Ser Glu Phe Ser Arg Met Ala Arg
1               5                   10


<210>  74
<211>  10
<212>  PRT
<213>  Homo sapiens

<400>  74

Leu Val Ser Glu Phe Ser Arg Met Ala Lys
1               5                   10


<210>  75
<211>  10
<212>  PRT
<213>  Homo sapiens

<400>  75

Ala Ser Pro Leu Asp Ser Thr Phe Tyr Arg
1               5                   10

<210>  76
<211>  9
<212>  PRT
<213>  Homo sapiens

<400>  76

Ser Ser Phe Ser Thr Thr Ile Asn Tyr
1               5


<210>  77
<211>  9
<212>  PRT
<213>  Homo sapiens

<400>  77

Ser Ser Phe Ser Thr Thr Ile Asn Lys
1               5


<210>  78
<211>  9
<212>  PRT
<213>  Homo sapiens

<400>  78

Ser Val Phe Ser Thr Thr Ile Asn Lys
1               5


<210>  79
<211>  9
<212>  PRT
<213>  Homo sapiens

<400>  79

Ser Val Phe Ser Thr Thr Ile Asn Arg
1               5


<210>  80
<211>  11
<212>  PRT
<213>  Homo sapiens

<400>  80

Phe Ser Thr Thr Ile Asn Tyr Thr Leu Trp Arg
1               5                   10


<210>  81
<211>  10
<212>  PRT

<213>  Homo sapiens

<400>  81

Ser Thr Thr Ile Asn Tyr Thr Leu Trp Lys
1               5                   10


<210>  82
<211>  9
<212>  PRT
<213>  Homo sapiens

<400>  82

Thr Thr Ile Asn Tyr Thr Leu Trp Arg
1               5


<210>  83
<211>  9
<212>  PRT
<213>  Homo sapiens

<400>  83

Thr Val Ile Asn Tyr Thr Leu Trp Arg
1               5


<210>  84
<211>  9
<212>  PRT
<213>  Homo sapiens

<400>  84

Thr Val Ile Asn Tyr Thr Leu Trp Lys
1               5


<210>  85
<211>  8
<212>  PRT
<213>  Homo sapiens

<400>  85

Leu Val His Phe Leu Leu Leu Lys
1               5


<210>  86
<211>  9
<212>  PRT
<213>  Homo sapiens

<400>  86

Leu Val His Phe Leu Leu Leu Lys Tyr
1               5

<210>  87
<211>  9
<212>  PRT
<213>  Homo sapiens

<400>  87

Leu Val His Phe Leu Leu Leu Lys Lys
1               5

<210>  88
<211>  9
<212>  PRT
<213>  Homo sapiens

<400>  88

Leu Val His Phe Leu Leu Leu Lys Arg
1               5

<210>  89
<211>  9
<212>  PRT
<213>  Homo sapiens

<400>  89

Ser Met Leu Glu Val Phe Glu Gly Arg
1               5

<210>  90
<211>  9
<212>  PRT
<213>  Homo sapiens

<400>  90

Ser Met Leu Glu Val Phe Glu Gly Lys
1               5

<210>  91
<211>  8
<212>  PRT
<213>  Homo sapiens

<400>  91

Ser Val Phe Ala His Pro Arg Lys
1               5

<210> 92
<211> 9
<212> PRT
<213> Homo sapiens

<400> 92

Ala Leu Ile Glu Thr Ser Tyr Val Lys
1               5


<210> 93
<211> 9
<212> PRT
<213> Homo sapiens

<400> 93

Ala Val Ile Glu Thr Ser Tyr Val Lys
1               5


<210> 94
<211> 9
<212> PRT
<213> Homo sapiens

<400> 94

Ala Val Ile Glu Thr Ser Tyr Val Arg
1               5


<210> 95
<211> 9
<212> PRT
<213> Homo sapiens

<400> 95

Ile Ser Tyr Pro Pro Leu His Glu Arg
1               5


<210> 96
<211> 9
<212> PRT
<213> Homo sapiens

<400> 96

Ile Val Tyr Pro Pro Leu His Glu Arg
1               5


<210> 97
<211> 9
<212> PRT
<213> Homo sapiens

<400>  97

Ile Val Tyr Pro Pro Leu His Glu Lys
1               5


<210>  98
<211>  9
<212>  PRT
<213>  Homo sapiens

<400>  98

Tyr Phe Phe Pro Val Ile Phe Ser Lys
1               5


<210>  99
<211>  9
<212>  PRT
<213>  Homo sapiens

<400>  99

Tyr Val Phe Pro Val Ile Phe Ser Lys
1               5


<210>  100
<211>  9
<212>  PRT
<213>  Homo sapiens

<400>  100

Tyr Val Phe Pro Val Ile Phe Ser Arg
1               5


<210>  101
<211>  10
<212>  PRT
<213>  Homo sapiens

<400>  101

Leu Leu Gly Asp Asn Gln Ile Met Pro Lys
1               5                   10


<210>  102
<211>  9
<212>  PRT
<213>  Homo sapiens

<400>  102

Ser Val Leu Glu Val Phe Glu Gly Arg

1               5


<210>  103
<211>  9
<212>  PRT
<213>  Homo sapiens

<400>  103

Ser Val Leu Glu Val Phe Glu Gly Lys
1               5


<210>  104
<211>  10
<212>  PRT
<213>  Homo sapiens

<400>  104

Lys Thr Tyr Gln Gly Ser Tyr Gly Phe Lys
1               5                   10


<210>  105
<211>  10
<212>  PRT
<213>  Homo sapiens

<400>  105

Lys Val Tyr Gln Gly Ser Tyr Gly Phe Arg
1               5                   10


<210>  106
<211>  10
<212>  PRT
<213>  Homo sapiens

<400>  106

Lys Val Tyr Gln Gly Ser Tyr Gly Phe Lys
1               5                   10


<210>  107
<211>  9
<212>  PRT
<213>  Homo sapiens

<400>  107

Cys Thr Tyr Ser Pro Ala Leu Asn Lys
1               5


<210>  108

<211>  9
<212>  PRT
<213>  Homo sapiens

<220>
<221>  misc_feature
<222>  (1)..(1)
<223>  Xaa = alpha-amino butyric acid

<400>  108

Xaa Val Tyr Ser Pro Ala Leu Asn Lys
1               5


<210>  109
<211>  9
<212>  PRT
<213>  Homo sapiens

<220>
<221>  misc_feature
<222>  (1)..(1)
<223>  Xaa = alpha-amino butyric acid

<400>  109

Xaa Val Tyr Ser Pro Ala Leu Asn Arg
1               5


<210>  110
<211>  8
<212>  PRT
<213>  Homo sapiens

<400>  110

Lys Met Phe Cys Gln Leu Ala Lys
1               5


<210>  111
<211>  11
<212>  PRT
<213>  Homo sapiens

<400>  111

Gly Thr Arg Val Arg Ala Met Ala Ile Tyr Lys
1               5                   10


<210>  112
<211>  11
<212>  PRT
<213>  Homo sapiens

<400>  112

Gly Val Arg Val Arg Ala Met Ala Ile Tyr Lys
1               5                   10


<210>  113
<211>  9
<212>  PRT
<213>  Homo sapiens

<400>  113

Arg Val Arg Ala Met Ala Ile Tyr Lys
1               5


<210>  114
<211>  9
<212>  PRT
<213>  Homo sapiens

<400>  114

Arg Val Arg Ala Met Ala Ile Tyr Arg
1               5


<210>  115
<211>  10
<212>  PRT
<213>  Homo sapiens

<400>  115

Val Val Arg Arg Cys Pro His His Glu Arg
1               5                   10


<210>  116
<211>  10
<212>  PRT
<213>  Homo sapiens

<220>
<221>  misc_feature
<222>  (5)..(5)
<223>  Xaa = alpha-amino butyric acid

<400>  116

Val Val Arg Arg Xaa Pro His His Glu Lys
1               5                   10


<210>  117
<211>  9
<212>  PRT
<213>  Homo sapiens

<400>  117

Ser Ser Cys Met Gly Gly Met Asn Arg
1               5


<210>  118
<211>  9
<212>  PRT
<213>  Homo sapiens

<220>
<221>  misc_feature
<222>  (3)..(3)
<223>  Xaa = alpha-amino butyric acid

<400>  118

Ser Ser Xaa Met Gly Gly Met Asn Lys
1               5


<210>  119
<211>  9
<212>  PRT
<213>  Homo sapiens

<220>
<221>  misc_feature
<222>  (3)..(3)
<223>  Xaa = alpha-amino butyric acid

<400>  119

Ser Val Xaa Met Gly Gly Met Asn Arg
1               5


<210>  120
<211>  9
<212>  PRT
<213>  Homo sapiens

<220>
<221>  misc_feature
<222>  (3)..(3)
<223>  Xaa = alpha-amino butyric acid

<400>  120

Ser Val Xaa Met Gly Gly Met Asn Lys
1               5


<210>  121
<211>  10
<212>  PRT
<213>  Homo sapiens

<400>  121

Ser Ser Cys Met Gly Gly Met Asn Arg Arg
1               5                   10


<210>  122
<211>  10
<212>  PRT
<213>  Homo sapiens

<220>
<221>  misc_feature
<222>  (3)..(3)
<223>  Xaa = alpha-amino butyric acid

<400>  122

Ser Ser Xaa Met Gly Gly Met Asn Arg Lys
1               5                   10


<210>  123
<211>  10
<212>  PRT
<213>  Homo sapiens

<220>
<221>  misc_feature
<222>  (3)..(3)
<223>  Xaa = alpha-amino butyric acid

<400>  123

Ser Val Xaa Met Gly Gly Met Asn Arg Lys
1               5                   10


<210>  124
<211>  8
<212>  PRT
<213>  Homo sapiens

<400>  124

Arg Val Cys Ala Cys Pro Gly Arg
1               5


<210>  125
<211>  11
<212>  PRT
<213>  Homo sapiens

<400>  125

Arg Val Cys Ala Cys Pro Gly Arg Asp Arg Arg
1               5                   10

<210> 126
<211> 11
<212> PRT
<213> Homo sapiens

<400> 126

Ser Thr Ser Arg His Lys Lys Leu Met Phe Lys
1               5                   10


<210> 127
<211> 11
<212> PRT
<213> Homo sapiens

<400> 127

Ser Val Ser Arg His Lys Lys Leu Met Phe Lys
1               5                   10


<210> 128
<211> 11
<212> PRT
<213> Homo sapiens

<400> 128

Ser Val Ser Arg His Lys Lys Leu Met Phe Arg
1               5                   10


<210> 129
<211> 10
<212> PRT
<213> Homo sapiens

<400> 129

Ile Pro Trp Gln Arg Leu Leu Leu Thr Ala
1               5                   10


<210> 130
<211> 10
<212> PRT
<213> Homo sapiens

<400> 130

Ile Pro Trp Gln Arg Leu Leu Leu Thr Ile
1               5                   10


<210> 131
<211> 10
<212> PRT

<213>  Homo sapiens

<400>  131

Leu Pro Gln His Leu Phe Gly Tyr Ser Trp
1               5                   10


<210>  132
<211>  10
<212>  PRT
<213>  Homo sapiens

<400>  132

Leu Pro Gln His Leu Phe Gly Tyr Ser Ile
1               5                   10


<210>  133
<211>  8
<212>  PRT
<213>  Homo sapiens

<400>  133

Tyr Pro Asn Ala Ser Leu Leu Ile
1               5


<210>  134
<211>  8
<212>  PRT
<213>  Homo sapiens

<400>  134

Lys Pro Tyr Asp Gly Ile Pro Ala
1               5


<210>  135
<211>  8
<212>  PRT
<213>  Homo sapiens

<400>  135

Lys Pro Tyr Asp Gly Ile Pro Ile
1               5


<210>  136
<211>  9
<212>  PRT
<213>  Homo sapiens

<400>  136

Leu Pro Gln Pro Pro Ile Cys Thr Ile
1               5

<210>  137
<211>  11
<212>  PRT
<213>  Homo sapiens

<400>  137

Pro Pro Ser Pro Arg Glu Gly Pro Leu Pro Ala
1               5                   10

<210>  138
<211>  11
<212>  PRT
<213>  Homo sapiens

<400>  138

Pro Pro Ser Pro Arg Glu Gly Pro Leu Pro Ile
1               5                   10

<210>  139
<211>  9
<212>  PRT
<213>  Homo sapiens

<400>  139

Ser Pro Ala Phe Asp Asn Leu Tyr Ile
1               5

<210>  140
<211>  9
<212>  PRT
<213>  Homo sapiens

<400>  140

Val Pro Ile Ser His Leu Tyr Ile Leu
1               5

<210>  141
<211>  9
<212>  PRT
<213>  Homo sapiens

<400>  141

Val Pro Ile Ser His Leu Tyr Ala Leu
1               5

<210>  142
<211>  9
<212>  PRT
<213>  Homo sapiens

<400>  142

Val Pro Ile Ser Met Leu Tyr Ile Leu
1               5


<210>  143
<211>  10
<212>  PRT
<213>  Homo sapiens

<400>  143

Val Pro Ile Ser His Leu Tyr Ile Leu Val
1               5                   10


<210>  144
<211>  10
<212>  PRT
<213>  Homo sapiens

<400>  144

Val Pro Ile Ser His Leu Tyr Ile Leu Ile
1               5                   10


<210>  145
<211>  9
<212>  PRT
<213>  Homo sapiens

<400>  145

Leu Pro Thr Thr Met Asn Tyr Pro Leu
1               5


<210>  146
<211>  9
<212>  PRT
<213>  Homo sapiens

<400>  146

Leu Pro Thr Thr Met Asn Tyr Pro Ile
1               5


<210>  147
<211>  8
<212>  PRT
<213>  Homo sapiens

<400>  147

Tyr Pro Leu Trp Ser Gln Ser Tyr
1               5


<210>  148
<211>  8
<212>  PRT
<213>  Homo sapiens

<400>  148

Tyr Pro Leu Trp Ser Gln Ser Ile
1               5


<210>  149
<211>  8
<212>  PRT
<213>  Homo sapiens

<400>  149

Met Pro Lys Ala Gly Leu Leu Ile
1               5


<210>  150
<211>  9
<212>  PRT
<213>  Homo sapiens

<400>  150

Met Pro Lys Ala Gly Leu Leu Ile Ile
1               5


<210>  151
<211>  9
<212>  PRT
<213>  Homo sapiens

<400>  151

Met Pro Val Ala Gly Leu Leu Ile Ile
1               5


<210>  152
<211>  10
<212>  PRT
<213>  Homo sapiens

<400>  152

Met Pro Lys Ala Gly Leu Leu Ile Ile Val

1                   5                       10


<210>  153
<211>  10
<212>  PRT
<213>  Homo sapiens

<400>  153

Met Pro Lys Ala Gly Leu Leu Ile Ile Ile
1               5                   10


<210>  154
<211>  9
<212>  PRT
<213>  Homo sapiens

<400>  154

Ala Pro Ala Ala Pro Thr Pro Ala Ala
1               5


<210>  155
<211>  11
<212>  PRT
<213>  Homo sapiens

<400>  155

Ala Pro Ala Ala Pro Thr Pro Ala Ala Pro Ala
1               5                   10


<210>  156
<211>  8
<212>  PRT
<213>  Homo sapiens

<400>  156

Ala Pro Ala Pro Ala Pro Ser Trp
1               5


<210>  157
<211>  8
<212>  PRT
<213>  Homo sapiens

<400>  157

Ala Pro Ala Pro Ala Pro Ser Ile
1               5


<210>  158

<211>  11
<212>  PRT
<213>  Homo sapiens

<400>  158

Arg Val Asp Gly Asn Arg Gln Ile Ile Gly Tyr
1               5                   10


<210>  159
<211>  9
<212>  PRT
<213>  Homo sapiens

<400>  159

Gln Gln Ala Thr Pro Gly Pro Ala Tyr
1               5


<210>  160
<211>  9
<212>  PRT
<213>  Homo sapiens

<400>  160

Gln Gln Asp Thr Pro Gly Pro Ala Tyr
1               5


<210>  161
<211>  11
<212>  PRT
<213>  Homo sapiens

<400>  161

Arg Ser Asp Ser Val Ile Leu Asn Val Leu Tyr
1               5                   10


<210>  162
<211>  10
<212>  PRT
<213>  Homo sapiens

<400>  162

Pro Thr Ile Ser Pro Leu Asn Thr Ser Tyr
1               5                   10


<210>  163
<211>  10
<212>  PRT
<213>  Homo sapiens

<400>  163

Pro Thr Asp Ser Pro Leu Asn Thr Ser Tyr
1               5                   10


<210>  164
<211>  9
<212>  PRT
<213>  Homo sapiens

<400>  164

Ala Ala Ser Asn Pro Pro Ala Gln Tyr
1               5


<210>  165
<211>  9
<212>  PRT
<213>  Homo sapiens

<400>  165

Ala Ala Asp Asn Pro Pro Ala Gln Tyr
1               5


<210>  166
<211>  9
<212>  PRT
<213>  Homo sapiens

<400>  166

Ile Thr Val Asn Asn Ser Gly Ser Tyr
1               5


<210>  167
<211>  9
<212>  PRT
<213>  Homo sapiens

<400>  167

Ile Thr Asp Asn Asn Ser Gly Ser Tyr
1               5


<210>  168
<211>  9
<212>  PRT
<213>  Homo sapiens

<400>  168

Val Thr Arg Asn Asp Val Gly Pro Tyr
1               5

<210>  169
<211>  9
<212>  PRT
<213>  Homo sapiens

<400>  169

Val Thr Asp Asn Asp Val Gly Pro Tyr
1               5


<210>  170
<211>  11
<212>  PRT
<213>  Homo sapiens

<400>  170

His Ser Asp Pro Val Ile Leu Asn Val Leu Tyr
1               5                   10


<210>  171
<211>  10
<212>  PRT
<213>  Homo sapiens

<400>  171

Pro Thr Ile Ser Pro Ser Tyr Thr Tyr Tyr
1               5                   10


<210>  172
<211>  10
<212>  PRT
<213>  Homo sapiens

<400>  172

Pro Thr Asp Ser Pro Ser Tyr Thr Tyr Tyr
1               5                   10


<210>  173
<211>  9
<212>  PRT
<213>  Homo sapiens

<400>  173

Pro Thr Ile Ser Pro Ser Tyr Thr Tyr
1               5


<210>  174
<211>  9

<212>  PRT
<213>  Homo sapiens

<400>  174

Pro Thr Asp Ser Pro Ser Tyr Thr Tyr
1               5


<210>  175
<211>  9
<212>  PRT
<213>  Homo sapiens

<400>  175

Thr Ile Ser Pro Ser Tyr Thr Tyr Tyr
1               5


<210>  176
<211>  9
<212>  PRT
<213>  Homo sapiens

<400>  176

Thr Ile Asp Pro Ser Tyr Thr Tyr Tyr
1               5


<210>  177
<211>  10
<212>  PRT
<213>  Homo sapiens

<400>  177

His Ala Ala Ser Asn Pro Pro Ala Gln Tyr
1               5                   10


<210>  178
<211>  9
<212>  PRT
<213>  Homo sapiens

<400>  178

Ala Ala Asp Asn Pro Pro Ala Gln Tyr
1               5


<210>  179
<211>  9
<212>  PRT
<213>  Homo sapiens

<400>  179

Ile Thr Glu Lys Asn Ser Gly Leu Tyr
1               5

<210>  180
<211>  9
<212>  PRT
<213>  Homo sapiens

<400>  180

Ile Thr Asp Lys Asn Ser Gly Leu Tyr
1               5

<210>  181
<211>  11
<212>  PRT
<213>  Homo sapiens

<400>  181

Arg Ser Asp Pro Val Thr Leu Asp Val Leu Tyr
1               5                   10

<210>  182
<211>  10
<212>  PRT
<213>  Homo sapiens

<400>  182

His Ser Ala Ser Asn Pro Ser Pro Gln Tyr
1               5                   10

<210>  183
<211>  10
<212>  PRT
<213>  Homo sapiens

<400>  183

His Thr Ala Ser Asn Pro Ser Pro Gln Tyr
1               5                   10

<210>  184
<211>  10
<212>  PRT
<213>  Homo sapiens

<400>  184

His Ser Asp Ser Asn Pro Ser Pro Gln Tyr
1               5                   10

<210>  185
<211>  9
<212>  PRT
<213>  Homo sapiens

<400>  185

Val Met Ala Gly Val Gly Ser Pro Tyr
1               5


<210>  186
<211>  9
<212>  PRT
<213>  Homo sapiens

<400>  186

Val Met Asp Gly Val Gly Ser Pro Tyr
1               5


<210>  187
<211>  10
<212>  PRT
<213>  Homo sapiens

<400>  187

Cys Met Gln Ile Ala Lys Gly Met Ser Tyr
1               5                   10


<210>  188
<211>  10
<212>  PRT
<213>  Homo sapiens

<400>  188

Cys Thr Gln Ile Ala Lys Gly Met Ser Tyr
1               5                   10


<210>  189
<211>  9
<212>  PRT
<213>  Homo sapiens

<400>  189

Leu Leu Asp Ile Asp Glu Thr Glu Tyr
1               5


<210>  190
<211>  9
<212>  PRT

<213>  Homo sapiens

<400>  190

Leu Thr Asp Ile Asp Glu Thr Glu Tyr
1               5


<210>  191
<211>  10
<212>  PRT
<213>  Homo sapiens

<400>  191

Phe Thr His Gln Ser Asp Val Trp Ser Tyr
1               5                   10


<210>  192
<211>  10
<212>  PRT
<213>  Homo sapiens

<400>  192

Phe Thr Asp Gln Ser Asp Val Trp Ser Tyr
1               5                   10


<210>  193
<211>  10
<212>  PRT
<213>  Homo sapiens

<400>  193

Pro Ala Ser Pro Leu Asp Ser Thr Phe Tyr
1               5                   10


<210>  194
<211>  10
<212>  PRT
<213>  Homo sapiens

<400>  194

Pro Ala Asp Pro Leu Asp Ser Thr Phe Tyr
1               5                   10


<210>  195
<211>  9
<212>  PRT
<213>  Homo sapiens

<400>  195

Ala Ser Pro Leu Asp Ser Thr Phe Tyr
1               5

<210>  196
<211>  9
<212>  PRT
<213>  Homo sapiens

<400>  196

Ala Thr Pro Leu Asp Ser Thr Phe Tyr
1               5

<210>  197
<211>  10
<212>  PRT
<213>  Homo sapiens

<400>  197

Met Gly Asp Leu Val Asp Ala Glu Glu Tyr
1               5                   10

<210>  198
<211>  10
<212>  PRT
<213>  Homo sapiens

<400>  198

Met Thr Asp Leu Val Asp Ala Glu Glu Tyr
1               5                   10

<210>  199
<211>  9
<212>  PRT
<213>  Homo sapiens

<400>  199

Leu Thr Cys Ser Pro Gln Pro Glu Tyr
1               5

<210>  200
<211>  9
<212>  PRT
<213>  Homo sapiens

<400>  200

Leu Thr Asp Ser Pro Gln Pro Glu Tyr
1               5

<210>  201
<211>  10
<212>  PRT
<213>  Homo sapiens

<400>  201

Phe Ser Pro Ala Phe Asp Asn Leu Tyr Tyr
1               5                   10


<210>  202
<211>  10
<212>  PRT
<213>  Homo sapiens

<400>  202

Phe Thr Pro Ala Phe Asp Asn Leu Tyr Tyr
1               5                   10


<210>  203
<211>  9
<212>  PRT
<213>  Homo sapiens

<400>  203

Phe Ser Pro Ala Phe Asp Asn Leu Tyr
1               5


<210>  204
<211>  9
<212>  PRT
<213>  Homo sapiens

<400>  204

Phe Thr Pro Ala Phe Asp Asn Leu Tyr
1               5


<210>  205
<211>  9
<212>  PRT
<213>  Homo sapiens

<400>  205

Ser Pro Ala Phe Asp Asn Leu Tyr Tyr
1               5


<210>  206
<211>  9
<212>  PRT
<213>  Homo sapiens

<400>  206

Ser Pro Asp Phe Asp Asn Leu Tyr Tyr
1               5


<210>  207
<211>  10
<212>  PRT
<213>  Homo sapiens

<400>  207

Gly Thr Pro Thr Ala Glu Asn Pro Glu Tyr
1               5                   10


<210>  208
<211>  10
<212>  PRT
<213>  Homo sapiens

<400>  208

Gly Thr Asp Thr Ala Glu Asn Pro Glu Tyr
1               5                   10


<210>  209
<211>  10
<212>  PRT
<213>  Homo sapiens

<400>  209

Ala Ser Ser Phe Ser Thr Thr Ile Asn Tyr
1               5                   10


<210>  210
<211>  10
<212>  PRT
<213>  Homo sapiens

<400>  210

Ala Thr Ser Phe Ser Thr Thr Ile Asn Tyr
1               5                   10


<210>  211
<211>  10
<212>  PRT
<213>  Homo sapiens

<400>  211

Ala Ser Asp Phe Ser Thr Thr Ile Asn Tyr

1                   5                       10


<210>  212
<211>  9
<212>  PRT
<213>  Homo sapiens

<400>  212

Ser Ser Phe Ser Thr Thr Ile Asn Tyr
1               5


<210>  213
<211>  9
<212>  PRT
<213>  Homo sapiens

<400>  213

Ser Thr Phe Ser Thr Thr Ile Asn Tyr
1               5


<210>  214
<211>  11
<212>  PRT
<213>  Homo sapiens

<400>  214

Val Val Glu Val Val Pro Ile Ser His Leu Tyr
1               5                   10


<210>  215
<211>  8
<212>  PRT
<213>  Homo sapiens

<400>  215

Val Thr Cys Leu Gly Leu Ser Tyr
1               5


<210>  216
<211>  8
<212>  PRT
<213>  Homo sapiens

<400>  216

Val Thr Asp Leu Gly Leu Ser Tyr
1               5


<210>  217

<211>  10
<212>  PRT
<213>  Homo sapiens

<400>  217

Leu Met Gln Asp Leu Val Gln Glu Asn Tyr
1               5                   10


<210>  218
<211>  10
<212>  PRT
<213>  Homo sapiens

<400>  218

Leu Thr Gln Asp Leu Val Gln Glu Asn Tyr
1               5                   10


<210>  219
<211>  9
<212>  PRT
<213>  Homo sapiens

<400>  219

Met Gln Asp Leu Val Gln Glu Asn Tyr
1               5


<210>  220
<211>  9
<212>  PRT
<213>  Homo sapiens

<400>  220

Met Thr Asp Leu Val Gln Glu Asn Tyr
1               5


<210>  221
<211>  10
<212>  PRT
<213>  Homo sapiens

<400>  221

Ala Ser Ser Leu Pro Thr Thr Met Asn Tyr
1               5                   10


<210>  222
<211>  10
<212>  PRT
<213>  Homo sapiens

<400>  222

Ala Thr Ser Leu Pro Thr Thr Met Asn Tyr
1               5                   10


<210>  223
<211>  10
<212>  PRT
<213>  Homo sapiens

<400>  223

Ala Ser Asp Leu Pro Thr Thr Met Asn Tyr
1               5                   10


<210>  224
<211>  9
<212>  PRT
<213>  Homo sapiens

<400>  224

Ser Ser Leu Pro Thr Thr Met Asn Tyr
1               5


<210>  225
<211>  9
<212>  PRT
<213>  Homo sapiens

<400>  225

Ser Thr Leu Pro Thr Thr Met Asn Tyr
1               5


<210>  226
<211>  11
<212>  PRT
<213>  Homo sapiens

<400>  226

Thr Met Asn Tyr Pro Leu Trp Ser Gln Ser Tyr
1               5                   10


<210>  227
<211>  9
<212>  PRT
<213>  Homo sapiens

<400>  227

Gly Ser Val Val Gly Asn Trp Gln Tyr
1               5

<210>  228
<211>  9
<212>  PRT
<213>  Homo sapiens

<400>  228

Gly Thr Val Val Gly Asn Trp Gln Tyr
1               5


<210>  229
<211>  11
<212>  PRT
<213>  Homo sapiens

<400>  229

Leu Met Glu Val Asp Pro Ile Gly His Leu Tyr
1               5                   10


<210>  230
<211>  9
<212>  PRT
<213>  Homo sapiens

<400>  230

Glu Val Asp Pro Ile Gly His Leu Tyr
1               5


<210>  231
<211>  9
<212>  PRT
<213>  Homo sapiens

<400>  231

Glu Thr Asp Pro Ile Gly His Leu Tyr
1               5


<210>  232
<211>  8
<212>  PRT
<213>  Homo sapiens

<400>  232

Ala Thr Cys Leu Gly Leu Ser Tyr
1               5


<210>  233
<211>  10

<212>  PRT
<213>  Homo sapiens

<400>  233

Leu Thr Gln His Phe Val Gln Glu Asn Tyr
1               5                   10


<210>  234
<211>  10
<212>  PRT
<213>  Homo sapiens

<400>  234

Leu Thr Asp His Phe Val Gln Glu Asn Tyr
1               5                   10


<210>  235
<211>  9
<212>  PRT
<213>  Homo sapiens

<400>  235

Ile Ser Gly Gly Pro His Ile Ser Tyr
1               5


<210>  236
<211>  9
<212>  PRT
<213>  Homo sapiens

<400>  236

Ile Thr Gly Gly Pro His Ile Ser Tyr
1               5


<210>  237
<211>  10
<212>  PRT
<213>  Homo sapiens

<400>  237

Pro Ser Gln Lys Thr Tyr Gln Gly Ser Tyr
1               5                   10


<210>  238
<211>  10
<212>  PRT
<213>  Homo sapiens

<400>  238

Pro Thr Gln Lys Thr Tyr Gln Gly Ser Tyr
1               5                   10

<210>  239
<211>  10
<212>  PRT
<213>  Homo sapiens

<400>  239

Gly Thr Ala Lys Ser Val Thr Cys Thr Tyr
1               5                   10

<210>  240
<211>  10
<212>  PRT
<213>  Homo sapiens

<400>  240

Gly Thr Asp Lys Ser Val Thr Cys Thr Tyr
1               5                   10

<210>  241
<211>  10
<212>  PRT
<213>  Homo sapiens

<400>  241

Arg Val Glu Gly Asn Leu Arg Val Glu Tyr
1               5                   10

<210>  242
<211>  10
<212>  PRT
<213>  Homo sapiens

<400>  242

Arg Val Asp Gly Asn Leu Arg Val Glu Tyr
1               5                   10

<210>  243
<211>  10
<212>  PRT
<213>  Homo sapiens

<400>  243

Val Gly Ser Asp Cys Thr Thr Ile His Tyr
1               5                   10

<210> 244
<211> 9
<212> PRT
<213> Homo sapiens

<400> 244

Gly Ser Asp Cys Thr Thr Ile His Tyr
1               5


<210> 245
<211> 9
<212> PRT
<213> Homo sapiens

<400> 245

Gly Thr Asp Cys Thr Thr Ile His Tyr
1               5


<210> 246
<211> 11
<212> PRT
<213> Homo sapiens

<400> 246

Gly Ser Asp Cys Thr Thr Ile His Tyr Asn Tyr
1               5                   10


<210> 247
<211> 9
<212> PRT
<213> Homo sapiens

<400> 247

Arg Trp Cys Ile Pro Trp Gln Arg Leu
1               5


<210> 248
<211> 9
<212> PRT
<213> Homo sapiens

<400> 248

Arg Tyr Cys Ile Pro Trp Gln Arg Phe
1               5


<210> 249
<211> 10
<212> PRT

<213>  Homo sapiens

<400>  249

Arg Trp Cys Ile Pro Trp Gln Arg Leu Leu
1               5                   10


<210>  250
<211>  10
<212>  PRT
<213>  Homo sapiens

<400>  250

Arg Tyr Cys Ile Pro Trp Gln Arg Leu Phe
1               5                   10


<210>  251
<211>  11
<212>  PRT
<213>  Homo sapiens

<400>  251

Arg Trp Cys Ile Pro Trp Gln Arg Leu Leu Leu
1               5                   10


<210>  252
<211>  11
<212>  PRT
<213>  Homo sapiens

<400>  252

Pro Trp Gln Arg Leu Leu Leu Thr Ala Ser Leu
1               5                   10


<210>  253
<211>  10
<212>  PRT
<213>  Homo sapiens

<400>  253

Phe Trp Asn Pro Pro Thr Thr Ala Lys Leu
1               5                   10


<210>  254
<211>  10
<212>  PRT
<213>  Homo sapiens

<400>  254

Phe Tyr Asn Pro Pro Thr Thr Ala Lys Phe
1               5                   10


<210>  255
<211>  8
<212>  PRT
<213>  Homo sapiens

<400>  255

Ile Tyr Pro Asn Ala Ser Leu Leu
1               5


<210>  256
<211>  9
<212>  PRT
<213>  Homo sapiens

<400>  256

Ile Tyr Pro Asn Ala Ser Leu Leu Ile
1               5


<210>  257
<211>  9
<212>  PRT
<213>  Homo sapiens

<400>  257

Ile Tyr Pro Asn Ala Ser Leu Leu Phe
1               5


<210>  258
<211>  11
<212>  PRT
<213>  Homo sapiens

<400>  258

Phe Tyr Thr Leu His Val Ile Lys Ser Asp Leu
1               5                   10


<210>  259
<211>  10
<212>  PRT
<213>  Homo sapiens

<400>  259

Val Tyr Pro Glu Leu Pro Lys Pro Ser Ile
1               5                   10

<210> 260
<211> 10
<212> PRT
<213> Homo sapiens

<400> 260

Val Tyr Pro Glu Leu Pro Lys Pro Ser Phe
1               5                   10


<210> 261
<211> 9
<212> PRT
<213> Homo sapiens

<400> 261

Leu Trp Trp Val Asn Asn Gln Ser Leu
1               5


<210> 262
<211> 9
<212> PRT
<213> Homo sapiens

<400> 262

Leu Tyr Trp Val Asn Asn Gln Ser Phe
1               5


<210> 263
<211> 9
<212> PRT
<213> Homo sapiens

<400> 263

Leu Tyr Gly Pro Asp Ala Pro Thr Ile
1               5


<210> 264
<211> 9
<212> PRT
<213> Homo sapiens

<400> 264

Leu Tyr Gly Pro Asp Ala Pro Thr Phe
1               5


<210> 265
<211> 10
<212> PRT
<213> Homo sapiens

<400>  265

Gln Tyr Ser Trp Phe Val Asn Gly Thr Phe
1               5                   10


<210>  266
<211>  8
<212>  PRT
<213>  Homo sapiens

<400>  266

Ser Trp Phe Val Asn Gly Thr Phe
1               5


<210>  267
<211>  10
<212>  PRT
<213>  Homo sapiens

<400>  267

Thr Phe Gln Gln Ser Thr Gln Glu Leu Phe
1               5                   10


<210>  268
<211>  10
<212>  PRT
<213>  Homo sapiens

<400>  268

Thr Tyr Gln Gln Ser Thr Gln Glu Leu Phe
1               5                   10


<210>  269
<211>  9
<212>  PRT
<213>  Homo sapiens

<400>  269

Val Tyr Ala Glu Pro Pro Lys Pro Phe
1               5


<210>  270
<211>  10
<212>  PRT
<213>  Homo sapiens

<400>  270

Val Tyr Ala Glu Pro Pro Lys Pro Phe Ile

1                   5                       10


<210>  271
<211>  10
<212>  PRT
<213>  Homo sapiens

<400>  271

Val Tyr Ala Glu Pro Pro Lys Pro Phe Phe
1               5                   10


<210>  272
<211>  11
<212>  PRT
<213>  Homo sapiens

<400>  272

Thr Tyr Leu Trp Trp Val Asn Asn Gln Ser Leu
1               5                   10


<210>  273
<211>  9
<212>  PRT
<213>  Homo sapiens

<400>  273

Leu Tyr Gly Pro Asp Asp Pro Thr Ile
1               5


<210>  274
<211>  11
<212>  PRT
<213>  Homo sapiens

<400>  274

Ser Tyr Thr Tyr Tyr Arg Pro Gly Val Asn Leu
1               5                   10


<210>  275
<211>  9
<212>  PRT
<213>  Homo sapiens

<400>  275

Thr Tyr Tyr Arg Pro Gly Val Asn Leu
1               5


<210>  276

<211>  9
<212>  PRT
<213>  Homo sapiens

<400>  276

Thr Tyr Tyr Arg Pro Gly Val Asn Phe
1               5


<210>  277
<211>  11
<212>  PRT
<213>  Homo sapiens

<400>  277

Thr Tyr Tyr Arg Pro Gly Val Asn Leu Ser Leu
1               5                   10


<210>  278
<211>  10
<212>  PRT
<213>  Homo sapiens

<400>  278

Tyr Tyr Arg Pro Gly Val Asn Leu Ser Leu
1               5                   10


<210>  279
<211>  10
<212>  PRT
<213>  Homo sapiens

<400>  279

Tyr Tyr Arg Pro Gly Val Asn Leu Ser Phe
1               5                   10


<210>  280
<211>  10
<212>  PRT
<213>  Homo sapiens

<400>  280

Gln Tyr Ser Trp Leu Ile Asp Gly Asn Ile
1               5                   10


<210>  281
<211>  10
<212>  PRT
<213>  Homo sapiens

<400>  281

Gln Tyr Ser Trp Leu Ile Asp Gly Asn Phe
1               5                   10


<210>  282
<211>  11
<212>  PRT
<213>  Homo sapiens

<400>  282

Thr Tyr Leu Trp Trp Val Asn Gly Gln Ser Leu
1               5                   10


<210>  283
<211>  9
<212>  PRT
<213>  Homo sapiens

<400>  283

Leu Trp Trp Val Asn Gly Gln Ser Leu
1               5


<210>  284
<211>  9
<212>  PRT
<213>  Homo sapiens

<400>  284

Leu Tyr Trp Val Asn Gly Gln Ser Phe
1               5


<210>  285
<211>  9
<212>  PRT
<213>  Homo sapiens

<400>  285

Leu Tyr Gly Pro Asp Thr Pro Ile Ile
1               5


<210>  286
<211>  10
<212>  PRT
<213>  Homo sapiens

<400>  286

Ser Tyr Leu Ser Gly Ala Asn Leu Asn Leu
1               5                   10

<210>  287
<211>  10
<212>  PRT
<213>  Homo sapiens

<400>  287

Ser Tyr Leu Ser Gly Ala Asn Leu Asn Phe
1               5                   10


<210>  288
<211>  9
<212>  PRT
<213>  Homo sapiens

<400>  288

Gln Tyr Ser Trp Arg Ile Asn Gly Ile
1               5


<210>  289
<211>  9
<212>  PRT
<213>  Homo sapiens

<400>  289

Gln Tyr Ser Trp Arg Ile Asn Gly Phe
1               5


<210>  290
<211>  9
<212>  PRT
<213>  Homo sapiens

<400>  290

Thr Tyr Ala Cys Phe Val Ser Asn Leu
1               5


<210>  291
<211>  9
<212>  PRT
<213>  Homo sapiens

<400>  291

Thr Tyr Ala Cys Phe Val Ser Asn Phe
1               5


<210>  292
<211>  9

<212>  PRT
<213>  Homo sapiens

<400>  292

Pro Tyr Val Ser Arg Leu Leu Gly Ile
1               5


<210>  293
<211>  9
<212>  PRT
<213>  Homo sapiens

<400>  293

Pro Tyr Val Ser Arg Leu Leu Gly Phe
1               5


<210>  294
<211>  11
<212>  PRT
<213>  Homo sapiens

<400>  294

Pro Tyr Val Ser Arg Leu Leu Gly Ile Cys Leu
1               5                   10


<210>  295
<211>  10
<212>  PRT
<213>  Homo sapiens

<400>  295

Gly Met Ser Tyr Leu Glu Asp Val Arg Leu
1               5                   10


<210>  296
<211>  10
<212>  PRT
<213>  Homo sapiens

<400>  296

Gly Tyr Ser Tyr Leu Glu Asp Val Arg Phe
1               5                   10


<210>  297
<211>  9
<212>  PRT
<213>  Homo sapiens

<400>  297

Lys Trp Met Ala Leu Glu Ser Ile Leu
1               5

<210>  298
<211>  9
<212>  PRT
<213>  Homo sapiens

<400>  298

Lys Tyr Met Ala Leu Glu Ser Ile Phe
1               5

<210>  299
<211>  9
<212>  PRT
<213>  Homo sapiens

<400>  299

Arg Phe Thr His Gln Ser Asp Val Trp
1               5

<210>  300
<211>  9
<212>  PRT
<213>  Homo sapiens

<400>  300

Arg Tyr Thr His Gln Ser Asp Val Phe
1               5

<210>  301
<211>  9
<212>  PRT
<213>  Homo sapiens

<400>  301

Val Trp Ser Tyr Gly Val Thr Val Trp
1               5

<210>  302
<211>  9
<212>  PRT
<213>  Homo sapiens

<400>  302

Val Tyr Ser Tyr Gly Val Thr Val Phe
1               5

<210> 303
<211> 11
<212> PRT
<213> Homo sapiens

<400> 303

Val Trp Ser Tyr Gly Val Thr Val Trp Glu Leu
1               5                   10


<210> 304
<211> 9
<212> PRT
<213> Homo sapiens

<400> 304

Ser Tyr Gly Val Thr Val Trp Glu Leu
1               5


<210> 305
<211> 9
<212> PRT
<213> Homo sapiens

<400> 305

Ser Tyr Gly Val Thr Val Trp Glu Phe
1               5


<210> 306
<211> 9
<212> PRT
<213> Homo sapiens

<400> 306

Val Tyr Met Ile Met Val Lys Cys Trp
1               5


<210> 307
<211> 9
<212> PRT
<213> Homo sapiens

<400> 307

Val Tyr Met Ile Met Val Lys Cys Phe
1               5


<210> 308
<211> 11
<212> PRT

<213>  Homo sapiens

<400>  308

Val Tyr Met Ile Met Val Lys Cys Trp Met Ile
1               5                   10


<210>  309
<211>  9
<212>  PRT
<213>  Homo sapiens

<400>  309

Arg Phe Arg Glu Leu Val Ser Glu Phe
1               5


<210>  310
<211>  9
<212>  PRT
<213>  Homo sapiens

<400>  310

Arg Tyr Arg Glu Leu Val Ser Glu Phe
1               5


<210>  311
<211>  9
<212>  PRT
<213>  Homo sapiens

<400>  311

Arg Met Ala Arg Asp Pro Gln Arg Phe
1               5


<210>  312
<211>  9
<212>  PRT
<213>  Homo sapiens

<400>  312

Arg Tyr Ala Arg Asp Pro Gln Arg Phe
1               5


<210>  313
<211>  11
<212>  PRT
<213>  Homo sapiens

<400>  313

Ser Phe Ser Thr Thr Ile Asn Tyr Thr Leu Trp
1               5                   10


<210>  314
<211>  10
<212>  PRT
<213>  Homo sapiens

<400>  314

Ser Tyr Ser Thr Thr Ile Asn Tyr Thr Phe
1               5                   10


<210>  315
<211>  9
<212>  PRT
<213>  Homo sapiens

<400>  315

Met Phe Pro Asp Leu Glu Ser Glu Phe
1               5


<210>  316
<211>  9
<212>  PRT
<213>  Homo sapiens

<400>  316

Met Tyr Pro Asp Leu Glu Ser Glu Phe
1               5


<210>  317
<211>  9
<212>  PRT
<213>  Homo sapiens

<400>  317

Lys Met Val Glu Leu Val His Phe Leu
1               5


<210>  318
<211>  9
<212>  PRT
<213>  Homo sapiens

<400>  318

Lys Tyr Val Glu Leu Val His Phe Phe
1               5

<210>  319
<211>  11
<212>  PRT
<213>  Homo sapiens

<400>  319

Ile Phe Ser Lys Ala Ser Glu Tyr Leu Gln Leu
1               5                   10


<210>  320
<211>  9
<212>  PRT
<213>  Homo sapiens

<400>  320

Ile Tyr Ser Lys Ala Ser Glu Tyr Phe
1               5


<210>  321
<211>  9
<212>  PRT
<213>  Homo sapiens

<400>  321

Glu Tyr Leu Gln Leu Val Phe Gly Ile
1               5


<210>  322
<211>  9
<212>  PRT
<213>  Homo sapiens

<400>  322

Glu Tyr Leu Gln Leu Val Phe Gly Phe
1               5


<210>  323
<211>  10
<212>  PRT
<213>  Homo sapiens

<400>  323

Leu Tyr Ile Leu Val Thr Cys Leu Gly Leu
1               5                   10


<210>  324
<211>  10
<212>  PRT
<213>  Homo sapiens

<400>  324

Leu Tyr Ile Leu Val Thr Cys Leu Gly Phe
1               5                   10


<210>  325
<211>  9
<212>  PRT
<213>  Homo sapiens

<400>  325

Val Met Pro Lys Thr Gly Leu Leu Ile
1               5


<210>  326
<211>  9
<212>  PRT
<213>  Homo sapiens

<400>  326

Val Tyr Pro Lys Thr Gly Leu Leu Phe
1               5


<210>  327
<211>  10
<212>  PRT
<213>  Homo sapiens

<400>  327

Val Met Pro Lys Thr Gly Leu Leu Ile Ile
1               5                   10


<210>  328
<211>  10
<212>  PRT
<213>  Homo sapiens

<400>  328

Val Tyr Pro Lys Thr Gly Leu Leu Ile Phe
1               5                   10


<210>  329
<211>  10
<212>  PRT
<213>  Homo sapiens

<400>  329

Glu Phe Leu Trp Gly Pro Arg Ala Leu Ile

1                   5                       10


<210>  330
<211>  10
<212>  PRT
<213>  Homo sapiens

<400>  330

Glu Tyr Leu Trp Gly Pro Arg Ala Leu Phe
1               5                   10


<210>  331
<211>  8
<212>  PRT
<213>  Homo sapiens

<400>  331

Leu Trp Gly Pro Arg Ala Leu Ile
1               5


<210>  332
<211>  10
<212>  PRT
<213>  Homo sapiens

<400>  332

Ser Tyr Val Lys Val Leu His His Thr Leu
1               5                   10


<210>  333
<211>  10
<212>  PRT
<213>  Homo sapiens

<400>  333

Ser Tyr Val Lys Val Leu His His Thr Phe
1               5                   10


<210>  334
<211>  9
<212>  PRT
<213>  Homo sapiens

<400>  334

Thr Phe Pro Asp Leu Glu Ser Glu Phe
1               5


<210>  335

<211>  9
<212>  PRT
<213>  Homo sapiens

<400>  335

Thr Tyr Pro Asp Leu Glu Ser Glu Phe
1               5


<210>  336
<211>  9
<212>  PRT
<213>  Homo sapiens

<400>  336

Asn Trp Gln Tyr Phe Phe Pro Val Ile
1               5


<210>  337
<211>  9
<212>  PRT
<213>  Homo sapiens

<400>  337

Asn Tyr Gln Tyr Phe Phe Pro Val Phe
1               5


<210>  338
<211>  10
<212>  PRT
<213>  Homo sapiens

<400>  338

Asn Tyr Gln Tyr Phe Phe Pro Val Ile Phe
1               5                   10


<210>  339
<211>  8
<212>  PRT
<213>  Homo sapiens

<400>  339

Gln Tyr Phe Phe Pro Val Ile Phe
1               5


<210>  340
<211>  9
<212>  PRT
<213>  Homo sapiens

<400>  340

Ile Phe Ser Lys Ala Ser Ser Ser Leu
1               5


<210>  341
<211>  9
<212>  PRT
<213>  Homo sapiens

<400>  341

Ile Tyr Ser Lys Ala Ser Ser Ser Phe
1               5


<210>  342
<211>  11
<212>  PRT
<213>  Homo sapiens

<400>  342

Ile Phe Ser Lys Ala Ser Ser Ser Leu Gln Leu
1               5                   10


<210>  343
<211>  10
<212>  PRT
<213>  Homo sapiens

<400>  343

Leu Tyr Ile Phe Ala Thr Cys Leu Gly Leu
1               5                   10


<210>  344
<211>  10
<212>  PRT
<213>  Homo sapiens

<400>  344

Leu Tyr Ile Phe Ala Thr Cys Leu Gly Phe
1               5                   10


<210>  345
<211>  9
<212>  PRT
<213>  Homo sapiens

<400>  345

Ile Met Pro Lys Ala Gly Leu Leu Ile
1               5

<210> 346
<211> 9
<212> PRT
<213> Homo sapiens

<400> 346

Ile Tyr Pro Lys Ala Gly Leu Leu Phe
1               5


<210> 347
<211> 10
<212> PRT
<213> Homo sapiens

<400> 347

Ile Met Pro Lys Ala Gly Leu Leu Ile Ile
1               5                   10


<210> 348
<211> 10
<212> PRT
<213> Homo sapiens

<400> 348

Ile Tyr Pro Lys Ala Gly Leu Leu Ile Phe
1               5                   10


<210> 349
<211> 11
<212> PRT
<213> Homo sapiens

<400> 349

Ile Trp Glu Glu Leu Ser Val Leu Glu Val Phe
1               5                   10


<210> 350
<211> 8
<212> PRT
<213> Homo sapiens

<400> 350

Ser Tyr Pro Pro Leu His Glu Trp
1               5


<210> 351
<211> 10

<212>  PRT
<213>  Homo sapiens

<400>  351

Ser Tyr Pro Pro Leu His Glu Trp Val Leu
1               5                   10


<210>  352
<211>  10
<212>  PRT
<213>  Homo sapiens

<400>  352

Ser Tyr Pro Pro Leu His Glu Trp Val Phe
1               5                   10


<210>  353
<211>  9
<212>  PRT
<213>  Homo sapiens

<400>  353

Thr Phe Ser Asp Leu Trp Lys Leu Leu
1               5


<210>  354
<211>  9
<212>  PRT
<213>  Homo sapiens

<400>  354

Thr Tyr Ser Asp Leu Trp Lys Leu Phe
1               5


<210>  355
<211>  8
<212>  PRT
<213>  Homo sapiens

<400>  355

Thr Tyr Gln Gly Ser Tyr Gly Phe
1               5


<210>  356
<211>  10
<212>  PRT
<213>  Homo sapiens

<400>  356

Thr Tyr Gln Gly Ser Tyr Gly Phe Arg Leu
1               5                   10

<210>  357
<211>  10
<212>  PRT
<213>  Homo sapiens

<400>  357

Thr Tyr Gln Gly Ser Tyr Gly Phe Arg Phe
1               5                   10

<210>  358
<211>  8
<212>  PRT
<213>  Homo sapiens

<400>  358

Ser Tyr Gly Phe Arg Leu Gly Phe
1               5

<210>  359
<211>  9
<212>  PRT
<213>  Homo sapiens

<400>  359

Ser Tyr Gly Phe Arg Leu Gly Phe Leu
1               5

<210>  360
<211>  9
<212>  PRT
<213>  Homo sapiens

<400>  360

Ser Tyr Gly Phe Arg Leu Gly Phe Phe
1               5

<210>  361
<211>  10
<212>  PRT
<213>  Homo sapiens

<400>  361

Thr Tyr Ser Pro Ala Leu Asn Lys Met Phe
1               5                   10

<210>  362

<400>  362

000


<210>  363
<211>  702
<212>  PRT
<213>  Homo sapiens

<400>  363

Met Glu Ser Pro Ser Ala Pro Pro His Arg Trp Cys Ile Pro Trp Gln
1               5                   10                  15

Arg Leu Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Val His Asn Leu Pro Gln His Leu Phe Gly
    50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300

Asp Thr Gly Leu Asn Arg Thr Thr Val Thr Thr Ile Thr Val Tyr Ala
305                 310                 315                 320

Glu Pro Pro Lys Pro Phe Ile Thr Ser Asn Asn Ser Asn Pro Val Glu
                325                 330                 335

Asp Glu Asp Ala Val Ala Leu Thr Cys Glu Pro Glu Ile Gln Asn Thr
            340                 345                 350

Thr Tyr Leu Trp Trp Val Asn Asn Gln Ser Leu Pro Val Ser Pro Arg
        355                 360                 365

Leu Gln Leu Ser Asn Asp Asn Arg Thr Leu Thr Leu Leu Ser Val Thr
    370                 375                 380

Arg Asn Asp Val Gly Pro Tyr Glu Cys Gly Ile Gln Asn Glu Leu Ser
385                 390                 395                 400

Val Asp His Ser Asp Pro Val Ile Leu Asn Val Leu Tyr Gly Pro Asp
                405                 410                 415

Asp Pro Thr Ile Ser Pro Ser Tyr Thr Tyr Tyr Arg Pro Gly Val Asn
            420                 425                 430

Leu Ser Leu Ser Cys His Ala Ala Ser Asn Pro Pro Ala Gln Tyr Ser
        435                 440                 445

Trp Leu Ile Asp Gly Asn Ile Gln Gln His Thr Gln Glu Leu Phe Ile
    450                 455                 460

Ser Asn Ile Thr Glu Lys Asn Ser Gly Leu Tyr Thr Cys Gln Ala Asn
465                 470                 475                 480

Asn Ser Ala Ser Gly His Ser Arg Thr Thr Val Lys Thr Ile Thr Val
                485                 490                 495

Ser Ala Glu Leu Pro Lys Pro Ser Ile Ser Ser Asn Asn Ser Lys Pro
            500                 505                 510

Val Glu Asp Lys Asp Ala Val Ala Phe Thr Cys Glu Pro Glu Ala Gln
        515                 520                 525

Asn Thr Thr Tyr Leu Trp Trp Val Asn Gly Gln Ser Leu Pro Val Ser
    530                 535                 540

Pro Arg Leu Gln Leu Ser Asn Gly Asn Arg Thr Leu Thr Leu Phe Asn
545                 550                 555                 560

Val Thr Arg Asn Asp Ala Arg Ala Tyr Val Cys Gly Ile Gln Asn Ser
                565                 570                 575

Val Ser Ala Asn Arg Ser Asp Pro Val Thr Leu Asp Val Leu Tyr Gly
            580                 585                 590

Pro Asp Thr Pro Ile Ile Ser Pro Pro Asp Ser Ser Tyr Leu Ser Gly
        595                 600                 605

Ala Asn Leu Asn Leu Ser Cys His Ser Ala Ser Asn Pro Ser Pro Gln
    610                 615                 620

Tyr Ser Trp Arg Ile Asn Gly Ile Pro Gln Gln His Thr Gln Val Leu
625                 630                 635                 640

Phe Ile Ala Lys Ile Thr Pro Asn Asn Asn Gly Thr Tyr Ala Cys Phe
                645                 650                 655

Val Ser Asn Leu Ala Thr Gly Arg Asn Asn Ser Ile Val Lys Ser Ile
            660                 665                 670

Thr Val Ser Ala Ser Gly Thr Ser Pro Gly Leu Ser Ala Gly Ala Thr
        675                 680                 685

Val Gly Ile Met Ile Gly Val Leu Val Gly Val Ala Leu Ile
    690                 695                 700

<210> 364
<211> 1255
<212> PRT
<213> Homo sapiens

<400> 364

<210>  365
<211>  314
<212>  PRT
<213>  Homo sapiens

<400>  365

Met Pro Leu Glu Gln Arg Ser Gln His Cys Lys Pro Glu Glu Gly Leu
1               5                   10                  15

Glu Ala Arg Gly Glu Ala Leu Gly Leu Val Gly Ala Gln Ala Pro Ala
            20                  25                  30

Thr Glu Glu Gln Gln Thr Ala Ser Ser Ser Ser Thr Leu Val Glu Val
        35                  40                  45

Thr Leu Gly Glu Val Pro Ala Ala Asp Ser Pro Ser Pro Pro His Ser
    50                  55                  60

Pro Gln Gly Ala Ser Ser Phe Ser Thr Thr Ile Asn Tyr Thr Leu Trp
65                  70                  75                  80

Arg Gln Ser Asp Glu Gly Ser Ser Asn Gln Glu Glu Glu Gly Pro Arg
                85                  90                  95

Met Phe Pro Asp Leu Glu Ser Glu Phe Gln Ala Ala Ile Ser Arg Lys
            100                 105                 110

Met Val Glu Leu Val His Phe Leu Leu Leu Lys Tyr Arg Ala Arg Glu
        115                 120                 125

Pro Val Thr Lys Ala Glu Met Leu Glu Ser Val Leu Arg Asn Cys Gln
    130                 135                 140

Asp Phe Phe Pro Val Ile Phe Ser Lys Ala Ser Glu Tyr Leu Gln Leu
145                 150                 155                 160

Val Phe Gly Ile Glu Val Val Glu Val Val Pro Ile Ser His Leu Tyr
                165                 170                 175

Ile Leu Val Thr Cys Leu Gly Leu Ser Tyr Asp Gly Leu Leu Gly Asp
            180                 185                 190

Asn Gln Val Met Pro Lys Thr Gly Leu Leu Ile Ile Val Leu Ala Ile
        195                 200                 205

Ile Ala Ile Glu Gly Asp Cys Ala Pro Glu Glu Lys Ile Trp Glu Glu
    210                 215                 220

Leu Ser Met Leu Glu Val Phe Glu Gly Arg Glu Asp Ser Val Phe Ala
225                 230                 235                 240

His Pro Arg Lys Leu Leu Met Gln Asp Leu Val Gln Glu Asn Tyr Leu
                245                 250                 255

Glu Tyr Arg Gln Val Pro Gly Ser Asp Pro Ala Cys Tyr Glu Phe Leu

260                 265                 270

Trp Gly Pro Arg Ala Leu Ile Glu Thr Ser Tyr Val Lys Val Leu His
        275                 280                 285

His Thr Leu Lys Ile Gly Gly Glu Pro His Ile Ser Tyr Pro Pro Leu
    290                 295                 300

His Glu Arg Ala Leu Arg Glu Gly Glu Glu
305                 310

<210>  366
<211>  314
<212>  PRT
<213>  Homo sapiens

<400>  366

Met Pro Leu Glu Gln Arg Ser Gln His Cys Lys Pro Glu Glu Gly Leu
1               5                   10                  15

Glu Ala Arg Gly Glu Ala Leu Gly Leu Val Gly Ala Gln Ala Pro Ala
            20                  25                  30

Thr Glu Glu Gln Glu Ala Ala Ser Ser Ser Ser Thr Leu Val Glu Val
        35                  40                  45

Thr Leu Gly Glu Val Pro Ala Ala Glu Ser Pro Asp Pro Pro Gln Ser
    50                  55                  60

Pro Gln Gly Ala Ser Ser Leu Pro Thr Thr Met Asn Tyr Pro Leu Trp
65                  70                  75                  80

Ser Gln Ser Tyr Glu Asp Ser Ser Asn Gln Glu Glu Glu Gly Pro Ser
                85                  90                  95

Thr Phe Pro Asp Leu Glu Ser Glu Phe Gln Ala Ala Leu Ser Arg Lys
            100                 105                 110

Val Ala Glu Leu Val His Phe Leu Leu Leu Lys Tyr Arg Ala Arg Glu
        115                 120                 125

Pro Val Thr Lys Ala Glu Met Leu Gly Ser Val Val Gly Asn Trp Gln
    130                 135                 140

Tyr Phe Phe Pro Val Ile Phe Ser Lys Ala Ser Ser Ser Leu Gln Leu

145                 150                 155                 160

Val Phe Gly Ile Glu Leu Met Glu Val Asp Pro Ile Gly His Leu Tyr
                165                 170                 175

Ile Phe Ala Thr Cys Leu Gly Leu Ser Tyr Asp Gly Leu Leu Gly Asp
            180                 185                 190

Asn Gln Ile Met Pro Lys Ala Gly Leu Leu Ile Ile Val Leu Ala Ile
        195                 200                 205

Ile Ala Arg Glu Gly Asp Cys Ala Pro Glu Glu Lys Ile Trp Glu Glu
    210                 215                 220

Leu Ser Val Leu Glu Val Phe Glu Gly Arg Glu Asp Ser Ile Leu Gly
225                 230                 235                 240

Asp Pro Lys Lys Leu Leu Thr Gln His Phe Val Gln Glu Asn Tyr Leu
                245                 250                 255

Glu Tyr Arg Gln Val Pro Gly Ser Asp Pro Ala Cys Tyr Glu Phe Leu
            260                 265                 270

Trp Gly Pro Arg Ala Leu Val Glu Thr Ser Tyr Val Lys Val Leu His
        275                 280                 285

His Met Val Lys Ile Ser Gly Gly Pro His Ile Ser Tyr Pro Pro Leu
    290                 295                 300

His Glu Trp Val Leu Arg Glu Gly Glu Glu
305                 310

<210>  367
<211>  393
<212>  PRT
<213>  Homo sapiens

<400>  367

<210>  368
<211>  9
<212>  PRT
<213>  Homo sapiens

<400>  368

Ala Glu Gly Lys Glu Val Leu Leu Leu
1               5

<210>  369
<211>  10
<212>  PRT
<213>  Homo sapiens

<400>  369

Gln Glu Leu Phe Ile Pro Asn Ile Thr Val
1               5                   10

<210>  370
<211>  11
<212>  PRT
<213>  Homo sapiens

<400>  370

Ile Glu Ser Thr Pro Phe Asn Val Ala Glu Gly
1               5                   10


<210>  371
<211>  9
<212>  PRT
<213>  Homo sapiens

<400>  371

Tyr Glu Cys Gly Ile Gln Asn Glu Leu
1               5


<210>  372
<211>  11
<212>  PRT
<213>  Homo sapiens

<400>  372

Tyr Glu Cys Gly Ile Gln Asn Glu Leu Ser Val
1               5                   10


<210>  373
<211>  11
<212>  PRT
<213>  Homo sapiens

<400>  373

Met Glu Ser Pro Ser Ala Pro Pro His Arg Trp
1               5                   10


<210>  374
<211>  9
<212>  PRT
<213>  Homo sapiens

<400>  374

Ile Glu Ser Thr Pro Phe Asn Val Ala
1               5


<210>  375
<211>  10
<212>  PRT
<213>  Homo sapiens

<400>  375

Ala Glu Gly Lys Glu Val Leu Leu Leu Val
1               5                   10

<210>  376
<211>  10
<212>  PRT
<213>  Homo sapiens

<400>  376

Lys Glu Val Leu Leu Leu Val His Asn Leu
1               5                   10


<210>  377
<211>  10
<212>  PRT
<213>  Homo sapiens

<400>  377

Arg Glu Ile Ile Tyr Pro Asn Ala Ser Leu
1               5                   10


<210>  378
<211>  11
<212>  PRT
<213>  Homo sapiens

<400>  378

Arg Glu Ile Ile Tyr Pro Asn Ala Ser Leu Leu
1               5                   10


<210>  379
<211>  9
<212>  PRT
<213>  Homo sapiens

<400>  379

Cys Glu Thr Gln Asn Pro Val Ser Ala
1               5


<210>  380
<211>  9
<212>  PRT
<213>  Homo sapiens

<400>  380

Gln Glu Leu Phe Ile Pro Asn Ile Thr
1               5


<210>  381
<211>  12

<212>  PRT
<213>  Homo sapiens

<400>  381

Pro Glu Ala Gln Asn Thr Thr Tyr Leu Trp Trp Val
1               5                   10


<210>  382
<211>  11
<212>  PRT
<213>  Homo sapiens

<400>  382

Gly Glu Arg Val Asp Gly Asn Arg Gln Ile Ile
1               5                   10


<210>  383
<211>  11
<212>  PRT
<213>  Homo sapiens

<400>  383

Asn Glu Glu Ala Thr Gly Gln Phe Arg Val Tyr
1               5                   10


<210>  384
<211>  9
<212>  PRT
<213>  Homo sapiens

<400>  384

Cys Glu Pro Glu Thr Gln Asp Ala Thr
1               5


<210>  385
<211>  10
<212>  PRT
<213>  Homo sapiens

<400>  385

Gly Glu Asn Leu Asn Leu Ser Cys His Ala
1               5                   10


<210>  386
<211>  11
<212>  PRT
<213>  Homo sapiens

<400>  386

Gly Glu Asn Leu Asn Leu Ser Cys His Ala Ala
1               5                   10

<210>  387
<211>  8
<212>  PRT
<213>  Homo sapiens

<400>  387

Gln Glu Leu Phe Ile Pro Asn Ile
1               5

<210>  388
<211>  11
<212>  PRT
<213>  Homo sapiens

<400>  388

Cys Glu Pro Glu Ile Gln Asn Thr Thr Tyr Leu
1               5                   10

<210>  389
<211>  11
<212>  PRT
<213>  Homo sapiens

<400>  389

Pro Glu Ile Gln Asn Thr Thr Tyr Leu Trp Trp
1               5                   10

<210>  390
<211>  12
<212>  PRT
<213>  Homo sapiens

<400>  390

Pro Glu Ile Gln Asn Thr Thr Tyr Leu Trp Trp Val
1               5                   10

<210>  391
<211>  11
<212>  PRT
<213>  Homo sapiens

<400>  391

Asn Glu Leu Ser Val Asp His Ser Asp Pro Val
1               5                   10

<210>  392
<211>  9
<212>  PRT
<213>  Homo sapiens

<400>  392

Gln Glu Leu Phe Ile Ser Asn Ile Thr
1               5


<210>  393
<211>  11
<212>  PRT
<213>  Homo sapiens

<400>  393

Pro Glu Ala Gln Asn Thr Thr Tyr Leu Trp Trp
1               5                   10


<210>  394
<211>  10
<212>  PRT
<213>  Homo sapiens

<400>  394

Gly Glu Arg Val Asp Gly Asn Arg Gln Ile
1               5                   10


<210>  395
<211>  10
<212>  PRT
<213>  Homo sapiens

<400>  395

Asn Glu Glu Ala Thr Gly Gln Phe Arg Val
1               5                   10


<210>  396
<211>  9
<212>  PRT
<213>  Homo sapiens

<400>  396

Glu Glu Ala Thr Gly Gln Phe Arg Val
1               5


<210>  397
<211>  10
<212>  PRT

<213>  Homo sapiens

<400>  397

Glu Glu Ala Thr Gly Gln Phe Arg Val Tyr
1               5                   10


<210>  398
<211>  9
<212>  PRT
<213>  Homo sapiens

<400>  398

Val Glu Asp Lys Asp Ala Val Ala Phe
1               5


<210>  399
<211>  11
<212>  PRT
<213>  Homo sapiens

<400>  399

Cys Glu Pro Glu Thr Gln Asp Ala Thr Tyr Leu
1               5                   10


<210>  400
<211>  9
<212>  PRT
<213>  Homo sapiens

<400>  400

Val Glu Asp Glu Asp Ala Val Ala Leu
1               5


<210>  401
<211>  13
<212>  PRT
<213>  Homo sapiens

<400>  401

Cys Glu Pro Glu Ile Gln Asn Thr Thr Tyr Leu Trp Trp
1               5                   10


<210>  402
<211>  9
<212>  PRT
<213>  Homo sapiens

<400>  402

Pro Glu Ile Gln Asn Thr Thr Tyr Leu
1               5

<210>  403
<211>  8
<212>  PRT
<213>  Homo sapiens

<400>  403

Ala Glu Leu Pro Lys Pro Ser Ile
1               5

<210>  404
<211>  8
<212>  PRT
<213>  Homo sapiens

<400>  404

Pro Glu Ala Gln Asn Thr Thr Tyr
1               5

<210>  405
<211>  8
<212>  PRT
<213>  Homo sapiens

<400>  405

Ala Glu Gly Lys Glu Val Leu Leu
1               5

<210>  406
<211>  9
<212>  PRT
<213>  Homo sapiens

<400>  406

Ala Glu Pro Pro Lys Pro Phe Ile Thr
1               5

<210>  407
<211>  9
<212>  PRT
<213>  Homo sapiens

<400>  407

Cys Glu Pro Glu Ile Gln Asn Thr Thr
1               5

<210>  408
<211>  10
<212>  PRT
<213>  Homo sapiens

<400>  408

Cys Glu Pro Glu Ile Gln Asn Thr Thr Tyr
1               5                   10


<210>  409
<211>  10
<212>  PRT
<213>  Homo sapiens

<400>  409

Pro Glu Ile Gln Asn Thr Thr Tyr Leu Trp
1               5                   10


<210>  410
<211>  8
<212>  PRT
<213>  Homo sapiens

<400>  410

Gln Glu Leu Phe Ile Ser Asn Ile
1               5


<210>  411
<211>  8
<212>  PRT
<213>  Homo sapiens

<400>  411

Thr Glu Lys Asn Ser Gly Leu Tyr
1               5


<210>  412
<211>  9
<212>  PRT
<213>  Homo sapiens

<400>  412

Thr Glu Lys Asn Ser Gly Leu Tyr Thr
1               5


<210>  413
<211>  10
<212>  PRT
<213>  Homo sapiens

<400>  413

Cys Glu Pro Glu Ala Gln Asn Thr Thr Tyr
1               5                   10


<210>  414
<211>  11
<212>  PRT
<213>  Homo sapiens

<400>  414

Cys Glu Pro Glu Ala Gln Asn Thr Thr Tyr Leu
1               5                   10


<210>  415
<211>  10
<212>  PRT
<213>  Homo sapiens

<400>  415

Pro Glu Ala Gln Asn Thr Thr Tyr Leu Trp
1               5                   10


<210>  416
<211>  8
<212>  PRT
<213>  Homo sapiens

<400>  416

Asn Glu Glu Ala Thr Gly Gln Phe
1               5


<210>  417
<211>  8
<212>  PRT
<213>  Homo sapiens

<400>  417

Pro Glu Leu Pro Lys Pro Ser Ile
1               5


<210>  418
<211>  10
<212>  PRT
<213>  Homo sapiens

<400>  418

Val Glu Asp Lys Asp Ala Val Ala Phe Thr

1                   5                   10


<210>  419
<211>  10
<212>  PRT
<213>  Homo sapiens

<400>  419

Cys Glu Pro Glu Thr Gln Asp Ala Thr Tyr
1               5                   10


<210>  420
<211>  8
<212>  PRT
<213>  Homo sapiens

<400>  420

Pro Glu Thr Gln Asp Ala Thr Tyr
1               5


<210>  421
<211>  9
<212>  PRT
<213>  Homo sapiens

<400>  421

Pro Glu Thr Gln Asp Ala Thr Tyr Leu
1               5


<210>  422
<211>  10
<212>  PRT
<213>  Homo sapiens

<400>  422

Pro Glu Thr Gln Asp Ala Thr Tyr Leu Trp
1               5                   10


<210>  423
<211>  11
<212>  PRT
<213>  Homo sapiens

<400>  423

Pro Glu Thr Gln Asp Ala Thr Tyr Leu Trp Trp
1               5                   10


<210>  424

<211>  7
<212>  PRT
<213>  Homo sapiens

<400>  424

Cys Glu Thr Gln Asn Pro Val
1               5


<210>  425
<211>  8
<212>  PRT
<213>  Homo sapiens

<400>  425

Ala Glu Pro Pro Lys Pro Phe Ile
1               5


<210>  426
<211>  10
<212>  PRT
<213>  Homo sapiens

<400>  426

Val Glu Asp Glu Asp Ala Val Ala Leu Thr
1               5                   10


<210>  427
<211>  8
<212>  PRT
<213>  Homo sapiens

<400>  427

Pro Glu Ile Gln Asn Thr Thr Tyr
1               5


<210>  428
<211>  9
<212>  PRT
<213>  Homo sapiens

<400>  428

Cys Glu Pro Glu Ala Gln Asn Thr Thr
1               5


<210>  429
<211>  9
<212>  PRT
<213>  Homo sapiens

<400>  429

Pro Glu Ala Gln Asn Thr Thr Tyr Leu
1               5


<210>  430
<211>  11
<212>  PRT
<213>  Homo sapiens

<400>  430

Met Glu Leu Ala Ala Leu Cys Arg Trp Gly Leu
1               5                   10


<210>  431
<211>  12
<212>  PRT
<213>  Homo sapiens

<400>  431

Leu Glu Leu Thr Tyr Leu Pro Thr Asn Ala Ser Leu
1               5                   10


<210>  432
<211>  9
<212>  PRT
<213>  Homo sapiens

<400>  432

Gln Glu Val Gln Gly Tyr Val Leu Ile
1               5


<210>  433
<211>  11
<212>  PRT
<213>  Homo sapiens

<400>  433

Gly Glu Gly Leu Ala Cys His Gln Leu Cys Ala
1               5                   10


<210>  434
<211>  8
<212>  PRT
<213>  Homo sapiens

<400>  434

Ala Glu Gln Arg Ala Ser Pro Leu
1               5

<210>  435
<211>  11
<212>  PRT
<213>  Homo sapiens

<400>  435

Ala Glu Gln Arg Ala Ser Pro Leu Thr Ser Ile
1               5                   10


<210>  436
<211>  10
<212>  PRT
<213>  Homo sapiens

<400>  436

Lys Glu Ile Leu Asp Glu Ala Tyr Val Met
1               5                   10


<210>  437
<211>  9
<212>  PRT
<213>  Homo sapiens

<400>  437

Gly Glu Arg Leu Pro Gln Pro Pro Ile
1               5


<210>  438
<211>  10
<212>  PRT
<213>  Homo sapiens

<400>  438

Ser Glu Cys Arg Pro Arg Phe Arg Glu Leu
1               5                   10


<210>  439
<211>  9
<212>  PRT
<213>  Homo sapiens

<400>  439

Ala Glu Asn Pro Glu Tyr Leu Gly Leu
1               5


<210>  440
<211>  11

<212>  PRT
<213>  Homo sapiens

<400>  440

Arg Glu Leu Gln Leu Arg Ser Leu Thr Glu Ile
1               5                   10


<210>  441
<211>  11
<212>  PRT
<213>  Homo sapiens

<400>  441

Gln Glu Phe Ala Gly Cys Lys Lys Ile Phe Gly
1               5                   10


<210>  442
<211>  12
<212>  PRT
<213>  Homo sapiens

<400>  442

Leu Glu Glu Ile Thr Gly Tyr Leu Tyr Ile Ser Ala
1               5                   10


<210>  443
<211>  13
<212>  PRT
<213>  Homo sapiens

<400>  443

Ser Glu Phe Ser Arg Met Ala Arg Asp Pro Gln Arg Phe
1               5                   10


<210>  444
<211>  10
<212>  PRT
<213>  Homo sapiens

<400>  444

Asn Glu Asp Leu Gly Pro Ala Ser Pro Leu
1               5                   10


<210>  445
<211>  10
<212>  PRT
<213>  Homo sapiens

<400>  445

Ser Glu Glu Glu Ala Pro Arg Ser Pro Leu
1               5                   10


<210>  446
<211>  10
<212>  PRT
<213>  Homo sapiens

<400>  446

Ser Glu Thr Asp Gly Tyr Val Ala Pro Leu
1               5                   10


<210>  447
<211>  10
<212>  PRT
<213>  Homo sapiens

<400>  447

Leu Glu Leu Thr Tyr Leu Pro Thr Asn Ala
1               5                   10


<210>  448
<211>  8
<212>  PRT
<213>  Homo sapiens

<400>  448

Gln Glu Val Gln Gly Tyr Val Leu
1               5


<210>  449
<211>  9
<212>  PRT
<213>  Homo sapiens

<400>  449

Phe Glu Asp Asn Tyr Ala Leu Ala Val
1               5


<210>  450
<211>  9
<212>  PRT
<213>  Homo sapiens

<400>  450

Arg Glu Leu Gln Leu Arg Ser Leu Thr
1               5

<210> 451
<211> 9
<212> PRT
<213> Homo sapiens

<400> 451

Met Glu His Leu Arg Glu Val Arg Ala
1               5


<210> 452
<211> 10
<212> PRT
<213> Homo sapiens

<400> 452

Met Glu His Leu Arg Glu Val Arg Ala Val
1               5                   10


<210> 453
<211> 13
<212> PRT
<213> Homo sapiens

<400> 453

Met Glu His Leu Arg Glu Val Arg Ala Val Thr Ser Ala
1               5                   10


<210> 454
<211> 10
<212> PRT
<213> Homo sapiens

<400> 454

Gln Glu Phe Ala Gly Cys Lys Lys Ile Phe
1               5                   10


<210> 455
<211> 11
<212> PRT
<213> Homo sapiens

<400> 455

Glu Glu Ile Thr Gly Tyr Leu Tyr Ile Ser Ala
1               5                   10


<210> 456
<211> 9
<212> PRT

<213>  Homo sapiens

<400>  456

Arg Glu Leu Gly Ser Gly Leu Ala Leu
1               5


<210>  457
<211>  10
<212>  PRT
<213>  Homo sapiens

<400>  457

Arg Glu Leu Gly Ser Gly Leu Ala Leu Ile
1               5                   10


<210>  458
<211>  9
<212>  PRT
<213>  Homo sapiens

<400>  458

Gly Glu Gly Leu Ala Cys His Gln Leu
1               5


<210>  459
<211>  10
<212>  PRT
<213>  Homo sapiens

<400>  459

Arg Glu Tyr Val Asn Ala Arg His Cys Leu
1               5                   10


<210>  460
<211>  10
<212>  PRT
<213>  Homo sapiens

<400>  460

Glu Glu Gly Ala Cys Gln Pro Cys Pro Ile
1               5                   10


<210>  461
<211>  9
<212>  PRT
<213>  Homo sapiens

<400>  461

Ala Glu Gln Arg Ala Ser Pro Leu Thr
1               5

<210>  462
<211>  9
<212>  PRT
<213>  Homo sapiens

<400>  462

Gln Glu Thr Glu Leu Val Glu Pro Leu
1               5

<210>  463
<211>  10
<212>  PRT
<213>  Homo sapiens

<400>  463

Gln Glu Thr Glu Leu Val Glu Pro Leu Thr
1               5                   10

<210>  464
<211>  10
<212>  PRT
<213>  Homo sapiens

<400>  464

Gly Glu Asn Val Lys Ile Pro Val Ala Ile
1               5                   10

<210>  465
<211>  10
<212>  PRT
<213>  Homo sapiens

<400>  465

Arg Glu Leu Val Ser Glu Phe Ser Arg Met
1               5                   10

<210>  466
<211>  11
<212>  PRT
<213>  Homo sapiens

<400>  466

Arg Glu Leu Val Ser Glu Phe Ser Arg Met Ala
1               5                   10

<210>  467
<211>  12
<212>  PRT
<213>  Homo sapiens

<400>  467

Ala Glu Glu Tyr Leu Val Pro Gln Gln Gly Phe Phe
1               5                   10


<210>  468
<211>  8
<212>  PRT
<213>  Homo sapiens

<400>  468

Ser Glu Asp Pro Thr Val Pro Leu
1               5


<210>  469
<211>  11
<212>  PRT
<213>  Homo sapiens

<400>  469

Ser Glu Thr Asp Gly Tyr Val Ala Pro Leu Thr
1               5                   10


<210>  470
<211>  11
<212>  PRT
<213>  Homo sapiens

<400>  470

Ala Glu Asn Pro Glu Tyr Leu Gly Leu Asp Val
1               5                   10


<210>  471
<211>  9
<212>  PRT
<213>  Homo sapiens

<400>  471

Met Glu Leu Ala Ala Leu Cys Arg Trp
1               5


<210>  472
<211>  10
<212>  PRT
<213>  Homo sapiens

<400>  472

Met Glu Leu Ala Ala Leu Cys Arg Trp Gly
1               5                   10


<210>  473
<211>  10
<212>  PRT
<213>  Homo sapiens

<400>  473

Gln Glu Val Gln Gly Tyr Val Leu Ile Ala
1               5                   10


<210>  474
<211>  10
<212>  PRT
<213>  Homo sapiens

<400>  474

Phe Glu Asp Asn Tyr Ala Leu Ala Val Leu
1               5                   10


<210>  475
<211>  8
<212>  PRT
<213>  Homo sapiens

<400>  475

Arg Glu Leu Gln Leu Arg Ser Leu
1               5


<210>  476
<211>  9
<212>  PRT
<213>  Homo sapiens

<400>  476

Thr Glu Ile Leu Lys Gly Gly Val Leu
1               5


<210>  477
<211>  10
<212>  PRT
<213>  Homo sapiens

<400>  477

Thr Glu Ile Leu Lys Gly Gly Val Leu Ile

1               5                   10


<210>  478
<211>  10
<212>  PRT
<213>  Homo sapiens

<400>  478

Gly Glu Ser Ser Glu Asp Cys Gln Ser Leu
1               5                   10


<210>  479
<211>  11
<212>  PRT
<213>  Homo sapiens

<400>  479

Ser Glu Asp Cys Gln Ser Leu Thr Arg Thr Val
1               5                   10


<210>  480
<211>  12
<212>  PRT
<213>  Homo sapiens

<400>  480

Pro Glu Gly Arg Tyr Thr Phe Gly Ala Ser Cys Val
1               5                   10


<210>  481
<211>  9
<212>  PRT
<213>  Homo sapiens

<400>  481

Arg Glu Val Arg Ala Val Thr Ser Ala
1               5


<210>  482
<211>  11
<212>  PRT
<213>  Homo sapiens

<400>  482

Arg Glu Val Arg Ala Val Thr Ser Ala Asn Ile
1               5                   10


<210>  483

<211>  8
<212>  PRT
<213>  Homo sapiens

<400>  483

Glu Glu Ile Thr Gly Tyr Leu Tyr
1               5


<210>  484
<211>  9
<212>  PRT
<213>  Homo sapiens

<400>  484

Glu Glu Ile Thr Gly Tyr Leu Tyr Ile
1               5


<210>  485
<211>  12
<212>  PRT
<213>  Homo sapiens

<400>  485

Glu Glu Ile Thr Gly Tyr Leu Tyr Ile Ser Ala Trp
1               5                   10


<210>  486
<211>  10
<212>  PRT
<213>  Homo sapiens

<400>  486

Gln Glu Cys Val Glu Glu Cys Arg Val Leu
1               5                   10


<210>  487
<211>  10
<212>  PRT
<213>  Homo sapiens

<400>  487

Val Glu Glu Cys Arg Val Leu Gln Gly Leu
1               5                   10


<210>  488
<211>  9
<212>  PRT
<213>  Homo sapiens

<400>  488

Gly Glu Asn Val Lys Ile Pro Val Ala
1               5


<210>  489
<211>  9
<212>  PRT
<213>  Homo sapiens

<400>  489

Lys Glu Ile Leu Asp Glu Ala Tyr Val
1               5


<210>  490
<211>  11
<212>  PRT
<213>  Homo sapiens

<400>  490

Lys Glu Ile Leu Asp Glu Ala Tyr Val Met Ala
1               5                   10


<210>  491
<211>  10
<212>  PRT
<213>  Homo sapiens

<400>  491

Asp Glu Ala Tyr Val Met Ala Gly Val Gly
1               5                   10


<210>  492
<211>  12
<212>  PRT
<213>  Homo sapiens

<400>  492

Thr Glu Tyr His Ala Asp Gly Gly Lys Val Pro Ile
1               5                   10


<210>  493
<211>  12
<212>  PRT
<213>  Homo sapiens

<400>  493

Gly Glu Arg Leu Pro Gln Pro Pro Ile Cys Thr Ile
1               5                   10

<210>  494
<211>  11
<212>  PRT
<213>  Homo sapiens

<400>  494

Ala Glu Glu Tyr Leu Val Pro Gln Gln Gly Phe
1               5                   10


<210>  495
<211>  9
<212>  PRT
<213>  Homo sapiens

<400>  495

Glu Glu Tyr Leu Val Pro Gln Gln Gly
1               5


<210>  496
<211>  10
<212>  PRT
<213>  Homo sapiens

<400>  496

Glu Glu Tyr Leu Val Pro Gln Gln Gly Phe
1               5                   10


<210>  497
<211>  11
<212>  PRT
<213>  Homo sapiens

<400>  497

Glu Glu Tyr Leu Val Pro Gln Gln Gly Phe Phe
1               5                   10


<210>  498
<211>  9
<212>  PRT
<213>  Homo sapiens

<400>  498

Glu Glu Glu Ala Pro Arg Ser Pro Leu
1               5


<210>  499
<211>  9

<212>  PRT
<213>  Homo sapiens

<400>  499

Glu Glu Ala Pro Arg Ser Pro Leu Ala
1               5


<210>  500
<211>  11
<212>  PRT
<213>  Homo sapiens

<400>  500

Arg Glu Gly Pro Leu Pro Ala Ala Arg Pro Ala
1               5                   10


<210>  501
<211>  12
<212>  PRT
<213>  Homo sapiens

<400>  501

Arg Glu Leu Gln Leu Arg Ser Leu Thr Glu Ile Leu
1               5                   10


<210>  502
<211>  11
<212>  PRT
<213>  Homo sapiens

<400>  502

Gly Glu Ser Ser Glu Asp Cys Gln Ser Leu Thr
1               5                   10


<210>  503
<211>  8
<212>  PRT
<213>  Homo sapiens

<400>  503

Cys Glu Leu His Cys Pro Ala Leu
1               5


<210>  504
<211>  9
<212>  PRT
<213>  Homo sapiens

<400>  504

Cys Glu Leu His Cys Pro Ala Leu Val
1               5

<210>  505
<211>  10
<212>  PRT
<213>  Homo sapiens

<400>  505

Cys Glu Leu His Cys Pro Ala Leu Val Thr
1               5                   10

<210>  506
<211>  11
<212>  PRT
<213>  Homo sapiens

<400>  506

Cys Glu Leu His Cys Pro Ala Leu Val Thr Tyr
1               5                   10

<210>  507
<211>  9
<212>  PRT
<213>  Homo sapiens

<400>  507

Phe Glu Ser Met Pro Asn Pro Glu Gly
1               5

<210>  508
<211>  11
<212>  PRT
<213>  Homo sapiens

<400>  508

Phe Glu Ser Met Pro Asn Pro Glu Gly Arg Tyr
1               5                   10

<210>  509
<211>  9
<212>  PRT
<213>  Homo sapiens

<400>  509

Gln Glu Phe Ala Gly Cys Lys Lys Ile
1               5

<210> 510
<211> 11
<212> PRT
<213> Homo sapiens

<400> 510

Phe Glu Thr Leu Glu Glu Ile Thr Gly Tyr Leu
1               5                   10


<210> 511
<211> 10
<212> PRT
<213> Homo sapiens

<400> 511

Leu Glu Glu Ile Thr Gly Tyr Leu Tyr Ile
1               5                   10


<210> 512
<211> 10
<212> PRT
<213> Homo sapiens

<400> 512

Pro Glu Asp Glu Cys Val Gly Glu Gly Leu
1               5                   10


<210> 513
<211> 8
<212> PRT
<213> Homo sapiens

<400> 513

Asp Glu Cys Val Gly Glu Gly Leu
1               5


<210> 514
<211> 12
<212> PRT
<213> Homo sapiens

<400> 514

Thr Glu Leu Val Glu Pro Leu Thr Pro Ser Gly Ala
1               5                   10


<210> 515
<211> 13
<212> PRT

<213>  Homo sapiens

<400>  515

Arg Glu Asn Thr Ser Pro Lys Ala Asn Lys Glu Ile Leu
1               5                   10


<210>  516
<211>  8
<212>  PRT
<213>  Homo sapiens

<400>  516

Lys Glu Ile Leu Asp Glu Ala Tyr
1               5


<210>  517
<211>  9
<212>  PRT
<213>  Homo sapiens

<400>  517

Asp Glu Ala Tyr Val Met Ala Gly Val
1               5


<210>  518
<211>  9
<212>  PRT
<213>  Homo sapiens

<400>  518

Leu Glu Ser Ile Leu Arg Arg Arg Phe
1               5


<210>  519
<211>  11
<212>  PRT
<213>  Homo sapiens

<400>  519

Trp Glu Leu Met Thr Phe Gly Ala Lys Pro Tyr
1               5                   10


<210>  520
<211>  13
<212>  PRT
<213>  Homo sapiens

<400>  520

Arg Glu Ile Pro Asp Leu Leu Glu Lys Gly Glu Arg Leu
1               5                   10

<210>  521
<211>  11
<212>  PRT
<213>  Homo sapiens

<400>  521

Gly Glu Arg Leu Pro Gln Pro Pro Ile Cys Thr
1               5                   10

<210>  522
<211>  11
<212>  PRT
<213>  Homo sapiens

<400>  522

Ser Glu Cys Arg Pro Arg Phe Arg Glu Leu Val
1               5                   10

<210>  523
<211>  9
<212>  PRT
<213>  Homo sapiens

<400>  523

Ser Glu Gly Ala Gly Ser Asp Val Phe
1               5

<210>  524
<211>  10
<212>  PRT
<213>  Homo sapiens

<400>  524

Pro Glu Tyr Leu Gly Leu Asp Val Pro Val
1               5                   10

<210>  525
<211>  13
<212>  PRT
<213>  Homo sapiens

<400>  525

Cys Glu Lys Cys Ser Lys Pro Cys Ala Arg Val Cys Tyr
1               5                   10

<210>  526
<211>  11
<212>  PRT
<213>  Homo sapiens

<400>  526

Met Glu His Leu Arg Glu Val Arg Ala Val Thr
1               5                   10


<210>  527
<211>  8
<212>  PRT
<213>  Homo sapiens

<400>  527

Leu Glu Glu Ile Thr Gly Tyr Leu
1               5


<210>  528
<211>  11
<212>  PRT
<213>  Homo sapiens

<400>  528

Asp Glu Glu Gly Ala Cys Gln Pro Cys Pro Ile
1               5                   10


<210>  529
<211>  7
<212>  PRT
<213>  Homo sapiens

<400>  529

Thr Glu Leu Val Glu Pro Leu
1               5


<210>  530
<211>  9
<212>  PRT
<213>  Homo sapiens

<400>  530

Val Glu Pro Leu Thr Pro Ser Gly Ala
1               5


<210>  531
<211>  10
<212>  PRT
<213>  Homo sapiens

<400>  531

Lys Glu Thr Glu Leu Arg Lys Val Lys Val
1               5                   10


<210>  532
<211>  9
<212>  PRT
<213>  Homo sapiens

<400>  532

Thr Glu Leu Arg Lys Val Lys Val Leu
1               5


<210>  533
<211>  11
<212>  PRT
<213>  Homo sapiens

<400>  533

Leu Glu Asp Val Arg Leu Val His Arg Asp Leu
1               5                   10


<210>  534
<211>  10
<212>  PRT
<213>  Homo sapiens

<400>  534

Thr Glu Tyr His Ala Asp Gly Gly Lys Val
1               5                   10


<210>  535
<211>  10
<212>  PRT
<213>  Homo sapiens

<400>  535

Leu Glu Ser Ile Leu Arg Arg Arg Phe Thr
1               5                   10


<210>  536
<211>  9
<212>  PRT
<213>  Homo sapiens

<400>  536

Leu Glu Asp Asp Asp Met Gly Asp Leu

1                   5


<210>  537
<211>  10
<212>  PRT
<213>  Homo sapiens

<400>  537

Ala Glu Glu Tyr Leu Val Pro Gln Gln Gly
1               5                   10


<210>  538
<211>  11
<212>  PRT
<213>  Homo sapiens

<400>  538

Ser Glu Glu Glu Ala Pro Arg Ser Pro Leu Ala
1               5                   10


<210>  539
<211>  11
<212>  PRT
<213>  Homo sapiens

<400>  539

Ser Glu Gly Ala Gly Ser Asp Val Phe Asp Gly
1               5                   10


<210>  540
<211>  11
<212>  PRT
<213>  Homo sapiens

<400>  540

Pro Glu Tyr Leu Thr Pro Gln Gly Gly Ala Ala
1               5                   10


<210>  541
<211>  9
<212>  PRT
<213>  Homo sapiens

<400>  541

Pro Glu Arg Gly Ala Pro Pro Ser Thr
1               5


<210>  542

<211>  8
<212>  PRT
<213>  Homo sapiens

<400>  542

Pro Glu Thr His Leu Asp Met Leu
1               5


<210>  543
<211>  11
<212>  PRT
<213>  Homo sapiens

<400>  543

Pro Glu Thr His Leu Asp Met Leu Arg His Leu
1               5                   10


<210>  544
<211>  7
<212>  PRT
<213>  Homo sapiens

<400>  544

Ser Glu Asp Cys Gln Ser Leu
1               5


<210>  545
<211>  9
<212>  PRT
<213>  Homo sapiens

<400>  545

His Glu Gln Cys Ala Ala Gly Cys Thr
1               5


<210>  546
<211>  13
<212>  PRT
<213>  Homo sapiens

<400>  546

Pro Glu Gly Arg Tyr Thr Phe Gly Ala Ser Cys Val Thr
1               5                   10


<210>  547
<211>  9
<212>  PRT
<213>  Homo sapiens

<400>  547

Gln Glu Val Thr Ala Glu Asp Gly Thr
1               5


<210>  548
<211>  9
<212>  PRT
<213>  Homo sapiens

<400>  548

Cys Glu Lys Cys Ser Lys Pro Cys Ala
1               5


<210>  549
<211>  11
<212>  PRT
<213>  Homo sapiens

<400>  549

Cys Glu Lys Cys Ser Lys Pro Cys Ala Arg Val
1               5                   10


<210>  550
<211>  7
<212>  PRT
<213>  Homo sapiens

<400>  550

Arg Glu Val Arg Ala Val Thr
1               5


<210>  551
<211>  7
<212>  PRT
<213>  Homo sapiens

<400>  551

Phe Glu Thr Leu Glu Glu Ile
1               5


<210>  552
<211>  10
<212>  PRT
<213>  Homo sapiens

<400>  552

Phe Glu Thr Leu Glu Glu Ile Thr Gly Tyr
1               5                   10

<210> 553
<211> 13
<212> PRT
<213> Homo sapiens

<400> 553

Asp Glu Cys Val Gly Glu Gly Leu Ala Cys His Gln Leu
1               5                   10


<210> 554
<211> 9
<212> PRT
<213> Homo sapiens

<400> 554

Gln Glu Cys Val Glu Glu Cys Arg Val
1               5


<210> 555
<211> 9
<212> PRT
<213> Homo sapiens

<400> 555

Val Glu Glu Cys Arg Val Leu Gln Gly
1               5


<210> 556
<211> 9
<212> PRT
<213> Homo sapiens

<400> 556

Glu Glu Cys Arg Val Leu Gln Gly Leu
1               5


<210> 557
<211> 10
<212> PRT
<213> Homo sapiens

<400> 557

Pro Glu Cys Gln Pro Gln Asn Gly Ser Val
1               5                   10


<210> 558
<211> 11

<212>  PRT
<213>  Homo sapiens

<400>  558

Thr Glu Leu Val Glu Pro Leu Thr Pro Ser Gly
1               5                   10


<210>  559
<211>  10
<212>  PRT
<213>  Homo sapiens

<400>  559

Val Glu Pro Leu Thr Pro Ser Gly Ala Met
1               5                   10


<210>  560
<211>  11
<212>  PRT
<213>  Homo sapiens

<400>  560

Lys Glu Thr Glu Leu Arg Lys Val Lys Val Leu
1               5                   10


<210>  561
<211>  10
<212>  PRT
<213>  Homo sapiens

<400>  561

Thr Glu Leu Arg Lys Val Lys Val Leu Gly
1               5                   10


<210>  562
<211>  9
<212>  PRT
<213>  Homo sapiens

<400>  562

Asp Glu Thr Glu Tyr His Ala Asp Gly
1               5


<210>  563
<211>  10
<212>  PRT
<213>  Homo sapiens

<400>  563

Asp Glu Thr Glu Tyr His Ala Asp Gly Gly
1               5                   10

<210>  564
<211>  10
<212>  PRT
<213>  Homo sapiens

<400>  564

Arg Glu Ile Pro Asp Leu Leu Glu Lys Gly
1               5                   10

<210>  565
<211>  7
<212>  PRT
<213>  Homo sapiens

<400>  565

Ser Glu Cys Arg Pro Arg Phe
1               5

<210>  566
<211>  10
<212>  PRT
<213>  Homo sapiens

<400>  566

Glu Glu Glu Ala Pro Arg Ser Pro Leu Ala
1               5                   10

<210>  567
<211>  8
<212>  PRT
<213>  Homo sapiens

<400>  567

Glu Glu Ala Pro Arg Ser Pro Leu
1               5

<210>  568
<211>  13
<212>  PRT
<213>  Homo sapiens

<400>  568

Ser Glu Gly Ala Gly Ser Asp Val Phe Asp Gly Asp Leu
1               5                   10

<210>  569
<211>  9
<212>  PRT
<213>  Homo sapiens

<400>  569

Pro Glu Tyr Val Asn Gln Pro Asp Val
1               5


<210>  570
<211>  11
<212>  PRT
<213>  Homo sapiens

<400>  570

Val Glu Asn Pro Glu Tyr Leu Thr Pro Gln Gly
1               5                   10


<210>  571
<211>  9
<212>  PRT
<213>  Homo sapiens

<400>  571

Pro Glu Tyr Leu Thr Pro Gln Gly Gly
1               5


<210>  572
<211>  10
<212>  PRT
<213>  Homo sapiens

<400>  572

Pro Glu Arg Gly Ala Pro Pro Ser Thr Phe
1               5                   10


<210>  573
<211>  7
<212>  PRT
<213>  Homo sapiens

<400>  573

Pro Glu Thr His Leu Asp Met
1               5


<210>  574
<211>  10
<212>  PRT

<213>  Homo sapiens

<400>  574

Ser Glu Asp Cys Gln Ser Leu Thr Arg Thr
1               5                   10


<210>  575
<211>  10
<212>  PRT
<213>  Homo sapiens

<400>  575

His Glu Gln Cys Ala Ala Gly Cys Thr Gly
1               5                   10


<210>  576
<211>  9
<212>  PRT
<213>  Homo sapiens

<400>  576

Pro Glu Gly Arg Tyr Thr Phe Gly Ala
1               5


<210>  577
<211>  9
<212>  PRT
<213>  Homo sapiens

<400>  577

Pro Glu Ser Phe Asp Gly Asp Pro Ala
1               5


<210>  578
<211>  9
<212>  PRT
<213>  Homo sapiens

<400>  578

Pro Glu Gln Leu Gln Val Phe Glu Thr
1               5


<210>  579
<211>  10
<212>  PRT
<213>  Homo sapiens

<400>  579

Pro Glu Gln Leu Gln Val Phe Glu Thr Leu
1               5                   10

<210>  580
<211>  13
<212>  PRT
<213>  Homo sapiens

<400>  580

Pro Glu Gln Leu Gln Val Phe Glu Thr Leu Glu Glu Ile
1               5                   10

<210>  581
<211>  9
<212>  PRT
<213>  Homo sapiens

<400>  581

Phe Glu Thr Leu Glu Glu Ile Thr Gly
1               5

<210>  582
<211>  7
<212>  PRT
<213>  Homo sapiens

<400>  582

Leu Glu Glu Ile Thr Gly Tyr
1               5

<210>  583
<211>  9
<212>  PRT
<213>  Homo sapiens

<400>  583

Leu Glu Glu Ile Thr Gly Tyr Leu Tyr
1               5

<210>  584
<211>  10
<212>  PRT
<213>  Homo sapiens

<400>  584

Arg Ile Leu His Asn Gly Ala Tyr Ser Leu
1               5                   10

<210>  585
<211>  9
<212>  PRT
<213>  Homo sapiens

<400>  585

Pro Glu Asp Glu Cys Val Gly Glu Gly
1               5


<210>  586
<211>  11
<212>  PRT
<213>  Homo sapiens

<400>  586

Pro Glu Asp Glu Cys Val Gly Glu Gly Leu Ala
1               5                   10


<210>  587
<211>  9
<212>  PRT
<213>  Homo sapiens

<400>  587

Asp Glu Cys Val Gly Glu Gly Leu Ala
1               5


<210>  588
<211>  11
<212>  PRT
<213>  Homo sapiens

<400>  588

Pro Glu Cys Gln Pro Gln Asn Gly Ser Val Thr
1               5                   10


<210>  589
<211>  13
<212>  PRT
<213>  Homo sapiens

<400>  589

Pro Glu Cys Gln Pro Gln Asn Gly Ser Val Thr Cys Phe
1               5                   10


<210>  590
<211>  10
<212>  PRT
<213>  Homo sapiens

<400>  590

Pro Glu Ala Asp Gln Cys Val Ala Cys Ala
1               5                   10


<210>  591
<211>  12
<212>  PRT
<213>  Homo sapiens

<400>  591

Pro Glu Ala Asp Gln Cys Val Ala Cys Ala His Tyr
1               5                   10


<210>  592
<211>  7
<212>  PRT
<213>  Homo sapiens

<400>  592

Leu Glu Asp Val Arg Leu Val
1               5


<210>  593
<211>  7
<212>  PRT
<213>  Homo sapiens

<400>  593

Asp Glu Thr Glu Tyr His Ala
1               5


<210>  594
<211>  10
<212>  PRT
<213>  Homo sapiens

<400>  594

Leu Glu Asp Asp Asp Met Gly Asp Leu Val
1               5                   10


<210>  595
<211>  10
<212>  PRT
<213>  Homo sapiens

<400>  595

Leu Glu Arg Pro Lys Thr Leu Ser Pro Gly

1               5                   10


<210>  596
<211>  7
<212>  PRT
<213>  Homo sapiens

<400>  596

Val Glu Asn Pro Glu Tyr Leu
1               5


<210>  597
<211>  10
<212>  PRT
<213>  Homo sapiens

<400>  597

Pro Glu Tyr Leu Thr Pro Gln Gly Gly Ala
1               5                   10


<210>  598
<211>  10
<212>  PRT
<213>  Homo sapiens

<400>  598

Arg Glu Arg Phe Glu Met Phe Arg Glu Leu
1               5                   10


<210>  599
<211>  10
<212>  PRT
<213>  Homo sapiens

<400>  599

Gly Glu Tyr Phe Thr Leu Gln Ile Arg Gly
1               5                   10


<210>  600
<211>  10
<212>  PRT
<213>  Homo sapiens

<400>  600

Gln Glu Thr Phe Ser Asp Leu Trp Lys Leu
1               5                   10


<210>  601

<211>  9
<212>  PRT
<213>  Homo sapiens

<400>  601

Asp Glu Ala Pro Arg Met Pro Glu Ala
1               5


<210>  602
<211>  10
<212>  PRT
<213>  Homo sapiens

<400>  602

His Glu Arg Cys Ser Asp Ser Asp Gly Leu
1               5                   10


<210>  603
<211>  10
<212>  PRT
<213>  Homo sapiens

<400>  603

Val Glu Tyr Leu Asp Asp Arg Asn Thr Phe
1               5                   10


<210>  604
<211>  10
<212>  PRT
<213>  Homo sapiens

<400>  604

Phe Glu Val Arg Val Cys Ala Cys Pro Gly
1               5                   10


<210>  605
<211>  8
<212>  PRT
<213>  Homo sapiens

<400>  605

Gly Glu Tyr Phe Thr Leu Gln Ile
1               5


<210>  606
<211>  10
<212>  PRT
<213>  Homo sapiens

<400>  606

Phe Glu Met Phe Arg Glu Leu Asn Glu Ala
1               5                   10


<210>  607
<211>  11
<212>  PRT
<213>  Homo sapiens

<400>  607

Phe Glu Met Phe Arg Glu Leu Asn Glu Ala Leu
1               5                   10


<210>  608
<211>  9
<212>  PRT
<213>  Homo sapiens

<400>  608

Arg Glu Leu Asn Glu Ala Leu Glu Leu
1               5


<210>  609
<211>  10
<212>  PRT
<213>  Homo sapiens

<400>  609

Ile Glu Gln Trp Phe Thr Glu Asp Pro Gly
1               5                   10


<210>  610
<211>  10
<212>  PRT
<213>  Homo sapiens

<400>  610

Val Glu Gly Asn Leu Arg Val Glu Tyr Leu
1               5                   10


<210>  611
<211>  10
<212>  PRT
<213>  Homo sapiens

<400>  611

Gly Glu Pro His His Glu Leu Pro Pro Gly
1               5                   10

<210>  612
<211>  12
<212>  PRT
<213>  Homo sapiens

<400>  612

Thr Glu Asp Pro Gly Pro Asp Glu Ala Pro Arg Met
1               5                   10


<210>  613
<211>  10
<212>  PRT
<213>  Homo sapiens

<400>  613

Asp Glu Ala Pro Arg Met Pro Glu Ala Ala
1               5                   10


<210>  614
<211>  9
<212>  PRT
<213>  Homo sapiens

<400>  614

His Glu Arg Cys Ser Asp Ser Asp Gly
1               5


<210>  615
<211>  11
<212>  PRT
<213>  Homo sapiens

<400>  615

His Glu Arg Cys Ser Asp Ser Asp Gly Leu Ala
1               5                   10


<210>  616
<211>  8
<212>  PRT
<213>  Homo sapiens

<400>  616

Leu Glu Asp Ser Ser Gly Asn Leu
1               5


<210>  617
<211>  9

<212>  PRT
<213>  Homo sapiens

<400>  617

Leu Glu Asp Ser Ser Gly Asn Leu Leu
1               5


<210>  618
<211>  12
<212>  PRT
<213>  Homo sapiens

<400>  618

Gly Glu Pro His His Glu Leu Pro Pro Gly Ser Thr
1               5                   10


<210>  619
<211>  7
<212>  PRT
<213>  Homo sapiens

<400>  619

Arg Glu Arg Phe Glu Met Phe
1               5


<210>  620
<211>  9
<212>  PRT
<213>  Homo sapiens

<400>  620

Leu Glu Leu Lys Asp Ala Gln Ala Gly
1               5


<210>  621
<211>  10
<212>  PRT
<213>  Homo sapiens

<400>  621

Met Glu Glu Pro Gln Ser Asp Pro Ser Val
1               5                   10


<210>  622
<211>  9
<212>  PRT
<213>  Homo sapiens

<400>  622

Val Glu Pro Pro Leu Ser Gln Glu Thr
1               5


<210>  623
<211>  10
<212>  PRT
<213>  Homo sapiens

<400>  623

Val Glu Pro Pro Leu Ser Gln Glu Thr Phe
1               5                   10


<210>  624
<211>  11
<212>  PRT
<213>  Homo sapiens

<400>  624

Gln Glu Thr Phe Ser Asp Leu Trp Lys Leu Leu
1               5                   10


<210>  625
<211>  9
<212>  PRT
<213>  Homo sapiens

<400>  625

Pro Glu Asn Asn Val Leu Ser Pro Leu
1               5


<210>  626
<211>  13
<212>  PRT
<213>  Homo sapiens

<400>  626

Asp Glu Ala Pro Arg Met Pro Glu Ala Ala Pro Pro Val
1               5                   10


<210>  627
<211>  9
<212>  PRT
<213>  Homo sapiens

<400>  627

Val Glu Gly Asn Leu Arg Val Glu Tyr
1               5

<210>  628
<211>  9
<212>  PRT
<213>  Homo sapiens

<400>  628

Val Glu Tyr Leu Asp Asp Arg Asn Thr
1               5


<210>  629
<211>  11
<212>  PRT
<213>  Homo sapiens

<400>  629

Tyr Glu Pro Pro Glu Val Gly Ser Asp Cys Thr
1               5                   10


<210>  630
<211>  13
<212>  PRT
<213>  Homo sapiens

<400>  630

Tyr Glu Pro Pro Glu Val Gly Ser Asp Cys Thr Thr Ile
1               5                   10


<210>  631
<211>  10
<212>  PRT
<213>  Homo sapiens

<400>  631

Pro Glu Val Gly Ser Asp Cys Thr Thr Ile
1               5                   10


<210>  632
<211>  10
<212>  PRT
<213>  Homo sapiens

<400>  632

Leu Glu Asp Ser Ser Gly Asn Leu Leu Gly
1               5                   10


<210>  633
<211>  10
<212>  PRT

<213>  Homo sapiens

<400>  633

Thr Glu Glu Glu Asn Leu Arg Lys Lys Gly
1               5                   10


<210>  634
<211>  11
<212>  PRT
<213>  Homo sapiens

<400>  634

His Glu Leu Pro Pro Gly Ser Thr Lys Arg Ala
1               5                   10


<210>  635
<211>  9
<212>  PRT
<213>  Homo sapiens

<400>  635

Asn Glu Ala Leu Glu Leu Lys Asp Ala
1               5


<210>  636
<211>  11
<212>  PRT
<213>  Homo sapiens

<400>  636

Asn Glu Ala Leu Glu Leu Lys Asp Ala Gln Ala
1               5                   10


<210>  637
<211>  9
<212>  PRT
<213>  Homo sapiens

<400>  637

Glu Glu Pro Gln Ser Asp Pro Ser Val
1               5


<210>  638
<211>  7
<212>  PRT
<213>  Homo sapiens

<400>  638

Gln Glu Thr Phe Ser Asp Leu
1               5


<210>  639
<211>  9
<212>  PRT
<213>  Homo sapiens

<400>  639

Thr Glu Asp Pro Gly Pro Asp Glu Ala
1               5


<210>  640
<211>  7
<212>  PRT
<213>  Homo sapiens

<400>  640

Pro Glu Ala Ala Pro Pro Val
1               5


<210>  641
<211>  10
<212>  PRT
<213>  Homo sapiens

<400>  641

Pro Glu Ala Ala Pro Pro Val Ala Pro Ala
1               5                   10


<210>  642
<211>  9
<212>  PRT
<213>  Homo sapiens

<400>  642

Pro Glu Val Gly Ser Asp Cys Thr Thr
1               5


<210>  643
<211>  12
<212>  PRT
<213>  Homo sapiens

<400>  643

Pro Glu Val Gly Ser Asp Cys Thr Thr Ile His Tyr
1               5                   10

<210>  644
<211>  9
<212>  PRT
<213>  Homo sapiens

<400>  644

Glu Glu Glu Asn Leu Arg Lys Lys Gly
1               5


<210>  645
<211>  9
<212>  PRT
<213>  Homo sapiens

<400>  645

Leu Glu Ser Glu Phe Gln Ala Ala Ile
1               5


<210>  646
<211>  13
<212>  PRT
<213>  Homo sapiens

<400>  646

Leu Glu Ser Glu Phe Gln Ala Ala Ile Ser Arg Lys Met
1               5                   10


<210>  647
<211>  11
<212>  PRT
<213>  Homo sapiens

<400>  647

Ser Glu Phe Gln Ala Ala Ile Ser Arg Lys Met
1               5                   10


<210>  648
<211>  12
<212>  PRT
<213>  Homo sapiens

<400>  648

Ser Glu Phe Gln Ala Ala Ile Ser Arg Lys Met Val
1               5                   10


<210>  649
<211>  9
<212>  PRT
<213>  Homo sapiens

<400>  649

Ser Glu Tyr Leu Gln Leu Val Phe Gly
1               5


<210>  650
<211>  10
<212>  PRT
<213>  Homo sapiens

<400>  650

Ser Glu Tyr Leu Gln Leu Val Phe Gly Ile
1               5                   10


<210>  651
<211>  13
<212>  PRT
<213>  Homo sapiens

<400>  651

Ser Glu Tyr Leu Gln Leu Val Phe Gly Ile Glu Val Val
1               5                   10


<210>  652
<211>  10
<212>  PRT
<213>  Homo sapiens

<400>  652

Trp Glu Glu Leu Ser Met Leu Glu Val Phe
1               5                   10


<210>  653
<211>  10
<212>  PRT
<213>  Homo sapiens

<400>  653

Gln Glu Asn Tyr Leu Glu Tyr Arg Gln Val
1               5                   10


<210>  654
<211>  11
<212>  PRT
<213>  Homo sapiens

<400>  654

Tyr Glu Phe Leu Trp Gly Pro Arg Ala Leu Ile

1               5                   10


<210>  655
<211>  13
<212>  PRT
<213>  Homo sapiens

<400>  655

Leu Glu Ala Arg Gly Glu Ala Leu Gly Leu Val Gly Ala
1               5                   10


<210>  656
<211>  10
<212>  PRT
<213>  Homo sapiens

<400>  656

Val Glu Val Thr Leu Gly Glu Val Pro Ala
1               5                   10


<210>  657
<211>  10
<212>  PRT
<213>  Homo sapiens

<400>  657

Glu Glu Gly Pro Arg Met Phe Pro Asp Leu
1               5                   10


<210>  658
<211>  8
<212>  PRT
<213>  Homo sapiens

<400>  658

Ala Glu Met Leu Glu Ser Val Leu
1               5


<210>  659
<211>  12
<212>  PRT
<213>  Homo sapiens

<400>  659

Leu Glu Ser Val Leu Arg Asn Cys Gln Asp Phe Phe
1               5                   10


<210>  660

<211>  8
<212>  PRT
<213>  Homo sapiens

<400>  660

Ser Glu Tyr Leu Gln Leu Val Phe
1               5


<210>  661
<211>  11
<212>  PRT
<213>  Homo sapiens

<400>  661

Val Glu Val Val Pro Ile Ser His Leu Tyr Ile
1               5                   10


<210>  662
<211>  10
<212>  PRT
<213>  Homo sapiens

<400>  662

Glu Glu Lys Ile Trp Glu Glu Leu Ser Met
1               5                   10


<210>  663
<211>  11
<212>  PRT
<213>  Homo sapiens

<400>  663

Glu Glu Leu Ser Met Leu Glu Val Phe Glu Gly
1               5                   10


<210>  664
<211>  9
<212>  PRT
<213>  Homo sapiens

<400>  664

Tyr Glu Phe Leu Trp Gly Pro Arg Ala
1               5


<210>  665
<211>  10
<212>  PRT
<213>  Homo sapiens

<400>  665

Tyr Glu Phe Leu Trp Gly Pro Arg Ala Leu
1               5                   10


<210>  666

<400>  666

000


<210>  667
<211>  9
<212>  PRT
<213>  Homo sapiens

<400>  667

Ile Glu Thr Ser Tyr Val Lys Val Leu
1               5


<210>  668
<211>  8
<212>  PRT
<213>  Homo sapiens

<400>  668

Leu Glu Ala Arg Gly Glu Ala Leu
1               5


<210>  669
<211>  10
<212>  PRT
<213>  Homo sapiens

<400>  669

Leu Glu Ala Arg Gly Glu Ala Leu Gly Leu
1               5                   10


<210>  670
<211>  11
<212>  PRT
<213>  Homo sapiens

<400>  670

Leu Glu Ala Arg Gly Glu Ala Leu Gly Leu Val
1               5                   10


<210>  671
<211>  8

<212>  PRT
<213>  Homo sapiens

<400>  671

Val Glu Leu Val His Phe Leu Leu
1               5


<210>  672
<211>  9
<212>  PRT
<213>  Homo sapiens

<400>  672

Val Glu Leu Val His Phe Leu Leu Leu
1               5


<210>  673
<211>  9
<212>  PRT
<213>  Homo sapiens

<400>  673

Arg Glu Pro Val Thr Lys Ala Glu Met
1               5


<210>  674
<211>  10
<212>  PRT
<213>  Homo sapiens

<400>  674

Arg Glu Pro Val Thr Lys Ala Glu Met Leu
1               5                   10


<210>  675
<211>  9
<212>  PRT
<213>  Homo sapiens

<400>  675

Ile Glu Val Val Glu Val Val Pro Ile
1               5


<210>  676
<211>  9
<212>  PRT
<213>  Homo sapiens

<400>  676

Val Glu Val Val Pro Ile Ser His Leu
1               5


<210>  677

<400>  677

000


<210>  678
<211>  12
<212>  PRT
<213>  Homo sapiens

<400>  678

Val Glu Val Val Pro Ile Ser His Leu Tyr Ile Leu
1               5                   10


<210>  679
<211>  13
<212>  PRT
<213>  Homo sapiens

<400>  679

Val Glu Val Val Pro Ile Ser His Leu Tyr Ile Leu Val
1               5                   10


<210>  680
<211>  9
<212>  PRT
<213>  Homo sapiens

<400>  680

Trp Glu Glu Leu Ser Met Leu Glu Val
1               5


<210>  681
<211>  9
<212>  PRT
<213>  Homo sapiens

<400>  681

Glu Glu Leu Ser Met Leu Glu Val Phe
1               5


<210>  682
<211>  10
<212>  PRT

<213>  Homo sapiens

<400>  682

Gly Glu Pro His Ile Ser Tyr Pro Pro Leu
1               5                   10


<210>  683
<211>  11
<212>  PRT
<213>  Homo sapiens

<400>  683

Glu Glu Gly Leu Glu Ala Arg Gly Glu Ala Leu
1               5                   10


<210>  684
<211>  9
<212>  PRT
<213>  Homo sapiens

<400>  684

Gly Glu Ala Leu Gly Leu Val Gly Ala
1               5


<210>  685
<211>  11
<212>  PRT
<213>  Homo sapiens

<400>  685

Gly Glu Ala Leu Gly Leu Val Gly Ala Gln Ala
1               5                   10


<210>  686
<211>  12
<212>  PRT
<213>  Homo sapiens

<400>  686

Glu Glu Gln Gln Thr Ala Ser Ser Ser Ser Thr Leu
1               5                   10


<210>  687
<211>  8
<212>  PRT
<213>  Homo sapiens

<400>  687

Gln Glu Glu Glu Gly Pro Arg Met
1               5

<210>  688
<211>  9
<212>  PRT
<213>  Homo sapiens

<400>  688

Gln Glu Glu Glu Gly Pro Arg Met Phe
1               5

<210>  689
<211>  11
<212>  PRT
<213>  Homo sapiens

<400>  689

Val Glu Leu Val His Phe Leu Leu Leu Lys Tyr
1               5                   10

<210>  690
<211>  11
<212>  PRT
<213>  Homo sapiens

<400>  690

Leu Glu Ser Val Leu Arg Asn Cys Gln Asp Phe
1               5                   10

<210>  691
<211>  13
<212>  PRT
<213>  Homo sapiens

<400>  691

Ile Glu Val Val Glu Val Val Pro Ile Ser His Leu Tyr
1               5                   10

<210>  692
<211>  10
<212>  PRT
<213>  Homo sapiens

<400>  692

Val Glu Val Val Pro Ile Ser His Leu Tyr
1               5                   10

<210>  693
<211>  9
<212>  PRT
<213>  Homo sapiens

<400>  693

Phe Glu Gly Arg Glu Asp Ser Val Phe
1               5


<210>  694
<211>  10
<212>  PRT
<213>  Homo sapiens

<400>  694

Glu Glu Gly Leu Glu Ala Arg Gly Glu Ala
1               5                   10


<210>  695
<211>  9
<212>  PRT
<213>  Homo sapiens

<400>  695

Leu Glu Ala Arg Gly Glu Ala Leu Gly
1               5


<210>  696
<211>  11
<212>  PRT
<213>  Homo sapiens

<400>  696

Val Glu Val Thr Leu Gly Glu Val Pro Ala Ala
1               5                   10


<210>  697
<211>  11
<212>  PRT
<213>  Homo sapiens

<400>  697

Glu Glu Glu Gly Pro Arg Met Phe Pro Asp Leu
1               5                   10


<210>  698
<211>  7
<212>  PRT
<213>  Homo sapiens

<400>  698

Ser Glu Phe Gln Ala Ala Ile
1               5


<210>  699
<211>  13
<212>  PRT
<213>  Homo sapiens

<400>  699

Arg Glu Pro Val Thr Lys Ala Glu Met Leu Glu Ser Val
1               5                   10


<210>  700
<211>  11
<212>  PRT
<213>  Homo sapiens

<400>  700

Ile Glu Gly Asp Cys Ala Pro Glu Glu Lys Ile
1               5                   10


<210>  701
<211>  8
<212>  PRT
<213>  Homo sapiens

<400>  701

Glu Glu Lys Ile Trp Glu Glu Leu
1               5


<210>  702
<211>  11
<212>  PRT
<213>  Homo sapiens

<400>  702

Glu Glu Lys Ile Trp Glu Glu Leu Ser Met Leu
1               5                   10


<210>  703
<211>  11
<212>  PRT
<213>  Homo sapiens

<400>  703

Leu Glu Val Phe Glu Gly Arg Glu Asp Ser Val

1               5                   10


<210>  704
<211>  10
<212>  PRT
<213>  Homo sapiens

<400>  704

Phe Glu Gly Arg Glu Asp Ser Val Phe Ala
1               5                   10


<210>  705
<211>  9
<212>  PRT
<213>  Homo sapiens

<400>  705

Pro Glu Glu Gly Leu Glu Ala Arg Gly
1               5


<210>  706
<211>  11
<212>  PRT
<213>  Homo sapiens

<400>  706

Glu Glu Gln Gln Thr Ala Ser Ser Ser Ser Thr
1               5                   10


<210>  707
<211>  12
<212>  PRT
<213>  Homo sapiens

<400>  707

Gln Glu Glu Glu Gly Pro Arg Met Phe Pro Asp Leu
1               5                   10


<210>  708
<211>  8
<212>  PRT
<213>  Homo sapiens

<400>  708

Glu Glu Glu Gly Pro Arg Met Phe
1               5


<210>  709

<211>  7
<212>  PRT
<213>  Homo sapiens

<400>  709

Ser Glu Tyr Leu Gln Leu Val
1               5


<210>  710
<211>  9
<212>  PRT
<213>  Homo sapiens

<400>  710

Pro Glu Glu Lys Ile Trp Glu Glu Leu
1               5


<210>  711
<211>  7
<212>  PRT
<213>  Homo sapiens

<400>  711

Gly Glu Pro His Ile Ser Tyr
1               5


<210>  712
<211>  11
<212>  PRT
<213>  Homo sapiens

<400>  712

Pro Glu Glu Gly Leu Glu Ala Arg Gly Glu Ala
1               5                   10


<210>  713
<211>  13
<212>  PRT
<213>  Homo sapiens

<400>  713

Glu Glu Gly Leu Glu Ala Arg Gly Glu Ala Leu Gly Leu
1               5                   10


<210>  714
<211>  11
<212>  PRT
<213>  Homo sapiens

<400>  714

Asp Glu Gly Ser Ser Asn Gln Glu Glu Glu Gly
1               5                   10


<210>  715
<211>  11
<212>  PRT
<213>  Homo sapiens

<400>  715

Pro Glu Glu Lys Ile Trp Glu Glu Leu Ser Met
1               5                   10


<210>  716
<211>  7
<212>  PRT
<213>  Homo sapiens

<400>  716

Trp Glu Glu Leu Ser Met Leu
1               5


<210>  717
<211>  7
<212>  PRT
<213>  Homo sapiens

<400>  717

Trp Glu Glu Leu Ser Met Leu
1               5


<210>  718
<211>  7
<212>  PRT
<213>  Homo sapiens

<400>  718

Trp Glu Glu Leu Ser Met Leu
1               5


<210>  719
<211>  10
<212>  PRT
<213>  Homo sapiens

<400>  719

Gln Glu Ala Ala Ser Ser Ser Ser Thr Leu
1               5                   10

<210>  720
<211>  9
<212>  PRT
<213>  Homo sapiens

<400>  720

Leu Glu Ser Glu Phe Gln Ala Ala Leu
1               5


<210>  721
<211>  9
<212>  PRT
<213>  Homo sapiens

<400>  721

Ala Glu Leu Val His Phe Leu Leu Leu
1               5


<210>  722
<211>  9
<212>  PRT
<213>  Homo sapiens

<400>  722

Met Glu Val Asp Pro Ile Gly His Leu
1               5


<210>  723
<211>  11
<212>  PRT
<213>  Homo sapiens

<400>  723

Met Glu Val Asp Pro Ile Gly His Leu Tyr Ile
1               5                   10


<210>  724
<211>  8
<212>  PRT
<213>  Homo sapiens

<400>  724

Ala Glu Leu Val His Phe Leu Leu
1               5


<210>  725
<211>  11

<212>  PRT
<213>  Homo sapiens

<400>  725

Ala Glu Leu Val His Phe Leu Leu Leu Lys Tyr
1               5                   10


<210>  726
<211>  9
<212>  PRT
<213>  Homo sapiens

<400>  726

Ala Glu Met Leu Gly Ser Val Val Gly
1               5


<210>  727
<211>  10
<212>  PRT
<213>  Homo sapiens

<400>  727

Glu Glu Lys Ile Trp Glu Glu Leu Ser Val
1               5                   10


<210>  728
<211>  10
<212>  PRT
<213>  Homo sapiens

<400>  728

Trp Glu Glu Leu Ser Val Leu Glu Val Phe
1               5                   10


<210>  729
<211>  9
<212>  PRT
<213>  Homo sapiens

<400>  729

Val Glu Thr Ser Tyr Val Lys Val Leu
1               5


<210>  730
<211>  10
<212>  PRT
<213>  Homo sapiens

<400>  730

Glu Glu Gly Pro Ser Thr Phe Pro Asp Leu
1               5                   10


<210>  731
<211>  9
<212>  PRT
<213>  Homo sapiens

<400>  731

Ile Glu Leu Met Glu Val Asp Pro Ile
1               5


<210>  732
<211>  10
<212>  PRT
<213>  Homo sapiens

<400>  732

Met Glu Val Asp Pro Ile Gly His Leu Tyr
1               5                   10


<210>  733
<211>  11
<212>  PRT
<213>  Homo sapiens

<400>  733

Glu Glu Lys Ile Trp Glu Glu Leu Ser Val Leu
1               5                   10


<210>  734
<211>  9
<212>  PRT
<213>  Homo sapiens

<400>  734

Glu Glu Leu Ser Val Leu Glu Val Phe
1               5


<210>  735
<211>  8
<212>  PRT
<213>  Homo sapiens

<400>  735

Glu Glu Glu Gly Pro Ser Thr Phe
1               5

<210>  736
<211>  11
<212>  PRT
<213>  Homo sapiens

<400>  736

Glu Glu Glu Gly Pro Ser Thr Phe Pro Asp Leu
1               5                   10


<210>  737
<211>  9
<212>  PRT
<213>  Homo sapiens

<400>  737

Trp Glu Glu Leu Ser Val Leu Glu Val
1               5


<210>  738
<211>  9
<212>  PRT
<213>  Homo sapiens

<400>  738

Phe Glu Gly Arg Glu Asp Ser Ile Leu
1               5


<210>  739
<211>  10
<212>  PRT
<213>  Homo sapiens

<400>  739

Phe Glu Gly Arg Glu Asp Ser Ile Leu Gly
1               5                   10


<210>  740
<211>  9
<212>  PRT
<213>  Homo sapiens

<400>  740

Gln Glu Ala Ala Ser Ser Ser Ser Thr
1               5


<210>  741
<211>  11
<212>  PRT

<213>  Homo sapiens

<400>  741

Arg Glu Gly Asp Cys Ala Pro Glu Glu Lys Ile
1               5                   10


<210>  742
<211>  11
<212>  PRT
<213>  Homo sapiens

<400>  742

Leu Glu Val Phe Glu Gly Arg Glu Asp Ser Ile
1               5                   10


<210>  743
<211>  8
<212>  PRT
<213>  Homo sapiens

<400>  743

Phe Glu Gly Arg Glu Asp Ser Ile
1               5


<210>  744
<211>  9
<212>  PRT
<213>  Homo sapiens

<400>  744

Gln Glu Glu Glu Gly Pro Ser Thr Phe
1               5


<210>  745
<211>  10
<212>  PRT
<213>  Homo sapiens

<400>  745

Ile Glu Leu Met Glu Val Asp Pro Ile Gly
1               5                   10


<210>  746
<211>  212
<212>  PRT
<213>  Hepatitis B virus

<400>  746

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210>  747
<211>  10
<212>  PRT

<213>  Unknown

<220>
<223>  A3 non-natural consensus peptide

<400>  747

Lys Val Phe Pro Tyr Ala Leu Ile Asn Lys
1               5                   10


<210>  748
<211>  11
<212>  PRT
<213>  Hepatitis B virus

<400>  748

Ser Thr Leu Pro Glu Thr Tyr Val Val Arg Arg
1               5                   10


<210>  749
<211>  9
<212>  PRT
<213>  Unknown

<220>
<223>  B35con2 peptide

<400>  749

Phe Pro Phe Lys Tyr Ala Ala Ala Phe
1               5


<210>  750
<211>  9
<212>  PRT
<213>  Unknown

<220>
<223>  A0201 Signal Sequence 5-13a.Y7

<400>  750

Ala Pro Arg Thr Leu Val Tyr Leu Leu
1               5


<210>  751
<211>  9
<212>  PRT
<213>  Homo sapiens

<400>  751

Tyr Thr Ala Val Val Pro Leu Val Tyr
1               5

<210>  752
<211>  9
<212>  PRT
<213>  Unknown

<220>
<223>  A1con peptide

<400>  752

Tyr Leu Glu Pro Ala Ile Ala Lys Tyr
1               5


<210>  753
<211>  9
<212>  PRT
<213>  Unknown

<220>
<223>  A24con peptide

<400>  753

Ala Tyr Ile Asp Asn Tyr Asn Lys Phe
1               5


<210>  754
<211>  394
<212>  PRT
<213>  Homo sapiens

<400>  754

Met Glu Ser Pro Ser Ala Pro Pro His Arg Trp Cys Ile Pro Trp Gln
1               5                   10                  15


Arg Leu Leu Leu Thr Ala Ser Leu Leu Thr Phe Trp Asn Pro Pro Thr
            20                  25                  30


Thr Ala Lys Leu Thr Ile Glu Ser Thr Pro Phe Asn Val Ala Glu Gly
        35                  40                  45


Lys Glu Val Leu Leu Leu Val His Asn Leu Pro Gln His Leu Phe Gly
    50                  55                  60


Tyr Ser Trp Tyr Lys Gly Glu Arg Val Asp Gly Asn Arg Gln Ile Ile
65                  70                  75                  80


Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300

Asp Thr Gly Leu Asn Arg Thr Thr Val Thr Thr Ile Thr Val Tyr Ala
305                 310                 315                 320

Glu Pro Pro Lys Pro Phe Ile Thr Ser Asn Asn Ser Asn Pro Val Glu

325                 330                 335

Asp Glu Asp Ala Val Ala Leu Thr Cys Glu Pro Glu Ile Gln Asn Thr
            340                 345                 350

Thr Tyr Leu Trp Trp Val Asn Asn Gln Ser Leu Pro Val Ser Pro Arg
        355                 360                 365

Leu Gln Leu Ser Asn Asp Asn Arg Thr Leu Thr Leu Leu Ser Val Thr
    370                 375                 380

Arg Asn Asp Val Gly Pro Tyr Glu Cys Gly
385                 390

<210>  755
<211>  102
<212>  PRT
<213>  Homo sapiens

<400>  755

Pro Asp Ser Ser Tyr Leu Ser Gly Ala Asn Leu Asn Leu Ser Cys His
1               5                   10                  15

Ser Ala Ser Asn Pro Ser Pro Gln Tyr Ser Trp Arg Ile Asn Gly Ile
            20                  25                  30

Pro Gln Gln His Thr Gln Val Leu Phe Ile Ala Lys Ile Thr Pro Asn
        35                  40                  45

Asn Asn Gly Thr Tyr Ala Cys Phe Val Ser Asn Leu Ala Thr Gly Arg
    50                  55                  60

Asn Asn Ser Ile Val Lys Ser Ile Thr Val Ser Ala Ser Gly Thr Ser
65                  70                  75                  80

Pro Gly Leu Ser Ala Gly Ala Thr Val Gly Ile Met Ile Gly Val Leu
                85                  90                  95

Val Gly Val Ala Leu Ile
            100

<210>  756
<211>  19
<212>  PRT
<213>  Homo sapiens

<400> 756

Ser Ala Asn Arg Ser Asp Pro Val Thr Leu Asp Val Leu Tyr Gly Pro
1               5                   10                  15

Asp Thr Pro

<210> 757
<211> 550
<212> PRT
<213> Homo sapiens

<400> 757

Met Pro Asn Gln Ala Gln Met Arg Ile Leu Lys Glu Thr Glu Leu Arg
1               5                   10                  15

Lys Val Lys Val Leu Gly Ser Gly Ala Phe Gly Thr Val Tyr Lys Gly
            20                  25                  30

Ile Trp Ile Pro Asp Gly Glu Asn Val Lys Ile Pro Val Ala Ile Lys
        35                  40                  45

Val Leu Arg Glu Asn Thr Ser Pro Lys Ala Asn Lys Glu Ile Leu Asp
    50                  55                  60

Glu Ala Tyr Val Met Ala Gly Val Gly Ser Pro Tyr Val Ser Arg Leu
65                  70                  75                  80

Leu Gly Ile Cys Leu Thr Ser Thr Val Gln Leu Val Thr Gln Leu Met
                85                  90                  95

Pro Tyr Gly Cys Leu Leu Asp His Val Arg Glu Asn Arg Gly Arg Leu
            100                 105                 110

Gly Ser Gln Asp Leu Leu Asn Trp Cys Met Gln Ile Ala Lys Gly Met
        115                 120                 125

Ser Tyr Leu Glu Asp Val Arg Leu Val His Arg Asp Leu Ala Ala Arg
    130                 135                 140

Asn Val Leu Val Lys Ser Pro Asn His Val Lys Ile Thr Asp Phe Gly
145                 150                 155                 160

Leu Ala Arg Leu Leu Asp Ile Asp Glu Thr Glu Tyr His Ala Asp Gly
                165                 170                 175

Gly Lys Val Pro Ile Lys Trp Met Ala Leu Glu Ser Ile Leu Arg Arg
            180                 185                 190

Arg Phe Thr His Gln Ser Asp Val Trp Ser Tyr Gly Val Thr Val Trp
        195                 200                 205

Glu Leu Met Thr Phe Gly Ala Lys Pro Tyr Asp Gly Ile Pro Ala Arg
    210                 215                 220

Glu Ile Pro Asp Leu Leu Glu Lys Gly Glu Arg Leu Pro Gln Pro Pro
225                 230                 235                 240

Ile Cys Thr Ile Asp Val Tyr Met Ile Met Val Lys Cys Trp Met Ile
                245                 250                 255

Asp Ser Glu Cys Arg Pro Arg Phe Arg Glu Leu Val Ser Glu Phe Ser
            260                 265                 270

Arg Met Ala Arg Asp Pro Gln Arg Phe Val Val Ile Gln Asn Glu Asp
        275                 280                 285

Leu Gly Pro Ala Ser Pro Leu Asp Ser Thr Phe Tyr Arg Ser Leu Leu
    290                 295                 300

Glu Asp Asp Asp Met Gly Asp Leu Val Asp Ala Glu Glu Tyr Leu Val
305                 310                 315                 320

Pro Gln Gln Gly Phe Phe Cys Pro Asp Pro Ala Pro Gly Ala Gly Gly
                325                 330                 335

Met Val His His Arg His Arg Ser Ser Ser Thr Arg Ser Gly Gly Gly
            340                 345                 350

Asp Leu Thr Leu Gly Leu Glu Pro Ser Glu Glu Glu Ala Pro Arg Ser
        355                 360                 365

Pro Leu Ala Pro Ser Glu Gly Ala Gly Ser Asp Val Phe Asp Gly Asp
    370                 375                 380

Leu Gly Met Gly Ala Ala Lys Gly Leu Gln Ser Leu Pro Thr His Asp
385                 390                 395                 400

Pro Ser Pro Leu Gln Arg Tyr Ser Glu Asp Pro Thr Val Pro Leu Pro

405                 410                 415

Ser Glu Thr Asp Gly Tyr Val Ala Pro Leu Thr Cys Ser Pro Gln Pro
            420                 425                 430

Glu Tyr Val Asn Gln Pro Asp Val Arg Pro Gln Pro Pro Ser Pro Arg
        435                 440                 445

Glu Gly Pro Leu Pro Ala Ala Arg Pro Ala Gly Ala Thr Leu Glu Arg
    450                 455                 460

Pro Lys Thr Leu Ser Pro Gly Lys Asn Gly Val Val Lys Asp Val Phe
465                 470                 475                 480

Ala Phe Gly Gly Ala Val Glu Asn Pro Glu Tyr Leu Thr Pro Gln Gly
                485                 490                 495

Gly Ala Ala Pro Gln Pro His Pro Pro Pro Ala Phe Ser Pro Ala Phe
            500                 505                 510

Asp Asn Leu Tyr Tyr Trp Asp Gln Asp Pro Pro Glu Arg Gly Ala Pro
        515                 520                 525

Pro Ser Thr Phe Lys Gly Thr Pro Thr Ala Glu Asn Pro Glu Tyr Leu
    530                 535                 540

Gly Leu Asp Val Pro Val
545                 550

<210>  758
<211>  214
<212>  PRT
<213>  Homo sapiens

<400>  758

Met Val His His Arg His Arg Ser Ser Ser Thr Arg Ser Gly Gly Gly
1               5                   10                  15

Asp Leu Thr Leu Gly Leu Glu Pro Ser Glu Glu Glu Ala Pro Arg Ser
            20                  25                  30

Pro Leu Ala Pro Ser Glu Gly Ala Gly Ser Asp Val Phe Asp Gly Asp
        35                  40                  45

Leu Gly Met Gly Ala Ala Lys Gly Leu Gln Ser Leu Pro Thr His Asp

50                  55                  60

Pro Ser Pro Leu Gln Arg Tyr Ser Glu Asp Pro Thr Val Pro Leu Pro
65                  70                  75                  80

Ser Glu Thr Asp Gly Tyr Val Ala Pro Leu Thr Cys Ser Pro Gln Pro
                85                  90                  95

Glu Tyr Val Asn Gln Pro Asp Val Arg Pro Gln Pro Pro Ser Pro Arg
            100                 105                 110

Glu Gly Pro Leu Pro Ala Ala Arg Pro Ala Gly Ala Thr Leu Glu Arg
        115                 120                 125

Pro Lys Thr Leu Ser Pro Gly Lys Asn Gly Val Val Lys Asp Val Phe
    130                 135                 140

Ala Phe Gly Gly Ala Val Glu Asn Pro Glu Tyr Leu Thr Pro Gln Gly
145                 150                 155                 160

Gly Ala Ala Pro Gln Pro His Pro Pro Pro Ala Phe Ser Pro Ala Phe
                165                 170                 175

Asp Asn Leu Tyr Tyr Trp Asp Gln Asp Pro Pro Glu Arg Gly Ala Pro
            180                 185                 190

Pro Ser Thr Phe Lys Gly Thr Pro Thr Ala Glu Asn Pro Glu Tyr Leu
        195                 200                 205

Gly Leu Asp Val Pro Val
    210

<210>  759
<211>  27
<212>  PRT
<213>  Homo sapiens

<400>  759

Met Ala Leu Glu Ser Ile Leu Arg Arg Arg Phe Thr His Gln Ser Asp
1               5                   10                  15

Val Trp Ser Tyr Gly Val Thr Val Trp Glu Leu
            20                  25

<210>  760

<211>  119
<212>  PRT
<213>  Homo sapiens

<400>  760

Met Pro Lys Thr Gly Leu Leu Ile Ile Val Leu Ala Ile Ile Ala Ile
1               5                   10                  15

Glu Gly Asp Cys Ala Pro Glu Glu Lys Ile Trp Glu Glu Leu Ser Met
            20                  25                  30

Leu Glu Val Phe Glu Gly Arg Glu Asp Ser Val Phe Ala His Pro Arg
        35                  40                  45

Lys Leu Leu Met Gln Asp Leu Val Gln Glu Asn Tyr Leu Glu Tyr Arg
    50                  55                  60

Gln Val Pro Gly Ser Asp Pro Ala Cys Tyr Glu Phe Leu Trp Gly Pro
65                  70                  75                  80

Arg Ala Leu Ile Glu Thr Ser Tyr Val Lys Val Leu His His Thr Leu
                85                  90                  95

Lys Ile Gly Gly Glu Pro His Ile Ser Tyr Pro Pro Leu His Glu Arg
            100                 105                 110

Ala Leu Arg Glu Gly Glu Glu
        115


<210>  761
<211>  166
<212>  PRT
<213>  Homo sapiens

<400>  761
   Met Pro Leu Glu Gln Arg Ser Gln His Cys Lys Pro Glu Glu Gly Leu
   1               5                   10                  15
   Glu Ala Arg Gly Glu Ala Leu Gly Leu Val Gly Ala Gln Ala Pro Ala
               20                  25                  30
   Thr Glu Glu Gln Glu Ala Ala Ser Ser Ser Ser Thr Leu Val Glu Val
           35                  40                  45
   Thr Leu Gly Glu Val Pro Ala Ala Glu Ser Pro Asp Pro Pro Gln Ser
       50                  55                  60
   Pro Gln Gly Ala Ser Ser Leu Pro Thr Thr Met Asn Tyr Pro Leu Trp
   65                  70                  75                  80
   Ser Gln Ser Tyr Glu Asp Ser Ser Asn Gln Glu Glu Glu Gly Pro Ser
                   85                  90                  95
   Thr Phe Pro Asp Leu Glu Ser Glu Phe Gln Ala Ala Leu Ser Arg Lys
               100                 105                 110
   Val Ala Glu Leu Val His Phe Leu Leu Leu Lys Tyr Arg Ala Arg Glu
           115                 120                 125
   Pro Val Thr Lys Ala Glu Met Leu Gly Ser Val Val Gly Asn Trp Gln
       130                 135                 140
   Tyr Phe Phe Pro Val Ile Phe Ser Lys Ala Ser Ser Ser Leu Gln Leu
   145                 150                 155                 160
   Val Phe Gly Ile Glu Leu
                   165

<210>  762
<211>  15
<212>  PRT
<213>  Homo sapiens

<400>  762

Lys Leu Leu Thr Gln His Phe Val Gln Glu Asn Tyr Leu Glu Tyr
1               5                   10                  15

<210>  763
<211>  354
<212>  PRT
<213>  Homo sapiens

<400>  763

Met Asp Asp Leu Met Leu Ser Pro Asp Asp Ile Glu Gln Trp Phe Thr
1               5                   10                  15

Glu Asp Pro Gly Pro Asp Glu Ala Pro Arg Met Pro Glu Ala Ala Pro
            20                  25                  30

Pro Val Ala Pro Ala Pro Ala Ala Pro Thr Pro Ala Ala Pro Ala Pro
        35                  40                  45

Ala Pro Ser Trp Pro Leu Ser Ser Ser Val Pro Ser Gln Lys Thr Tyr
    50                  55                  60

Gln Gly Ser Tyr Gly Phe Arg Leu Gly Phe Leu His Ser Gly Thr Ala
65                  70                  75                  80

Lys Ser Val Thr Cys Thr Tyr Ser Pro Ala Leu Asn Lys Met Phe Cys
                85                  90                  95

Gln Leu Ala Lys Thr Cys Pro Val Gln Leu Trp Val Asp Ser Thr Pro
            100                 105                 110

Pro Pro Gly Thr Arg Val Arg Ala Met Ala Ile Tyr Lys Gln Ser Gln
        115                 120                 125

His Met Thr Glu Val Val Arg Arg Cys Pro His His Glu Arg Cys Ser
    130                 135                 140

Asp Ser Asp Gly Leu Ala Pro Pro Gln His Leu Ile Arg Val Glu Gly
145                 150                 155                 160

Asn Leu Arg Val Glu Tyr Leu Asp Asp Arg Asn Thr Phe Arg His Ser
                165                 170                 175

Val Val Val Pro Tyr Glu Pro Pro Glu Val Gly Ser Asp Cys Thr Thr
            180                 185                 190

Ile His Tyr Asn Tyr Met Cys Asn Ser Ser Cys Met Gly Gly Met Asn
        195                 200                 205

Arg Arg Pro Ile Leu Thr Ile Ile Thr Leu Glu Asp Ser Ser Gly Asn
    210                 215                 220

Leu Leu Gly Arg Asn Ser Phe Glu Val Arg Val Cys Ala Cys Pro Gly
225                 230                 235                 240

Arg Asp Arg Arg Thr Glu Glu Glu Asn Leu Arg Lys Lys Gly Glu Pro
                245                 250                 255

His His Glu Leu Pro Pro Gly Ser Thr Lys Arg Ala Leu Pro Asn Asn
            260                 265                 270

Thr Ser Ser Ser Pro Gln Pro Lys Lys Lys Pro Leu Asp Gly Glu Tyr
        275                 280                 285

Phe Thr Leu Gln Ile Arg Gly Arg Glu Arg Phe Glu Met Phe Arg Glu
    290                 295                 300

Leu Asn Glu Ala Leu Glu Leu Lys Asp Ala Gln Ala Gly Lys Glu Pro
305                 310                 315                 320

Gly Gly Ser Arg Ala His Ser Ser His Leu Lys Ser Lys Lys Gly Gln
                325                 330                 335

Ser Thr Ser Arg His Lys Lys Leu Met Phe Lys Thr Glu Gly Pro Asp
            340                 345                 350

Ser Asp

<210> 764
<211> 19
<212> PRT
<213> Homo sapiens

<400> 764

His Ser Gly Thr Ala Lys Ser Val Thr Cys Thr Tyr Ser Pro Ala Leu
1               5                   10                  15

Asn Lys Met

<210> 765
<211> 102
<212> PRT
<213> Homo sapiens

<400> 765

Met Phe Cys Gln Leu Ala Lys Thr Cys Pro Val Gln Leu Trp Val Asp
1               5                   10                  15

Ser Thr Pro Pro Pro Gly Thr Arg Val Arg Ala Met Ala Ile Tyr Lys
            20                  25                  30

Gln Ser Gln His Met Thr Glu Val Val Arg Arg Cys Pro His His Glu
        35                  40                  45

Arg Cys Ser Asp Ser Asp Gly Leu Ala Pro Pro Gln His Leu Ile Arg
    50                  55                  60

Val Glu Gly Asn Leu Arg Val Glu Tyr Leu Asp Asp Arg Asn Thr Phe
65                  70                  75                  80

Arg His Ser Val Val Val Pro Tyr Glu Pro Pro Glu Val Gly Ser Asp
                85                  90                  95

Cys Thr Thr Ile His Tyr
            100

<210>  766
<211>  9
<212>  PRT
<213>  Unknown

<220>
<223>  DR7 preferred motif

<220>
<221>  misc_feature
<222>  (1)..(1)
<223>  Xaa may be Met, Phe, Leu, Ile, Val, Trp or Tyr

<220>
<221>  misc_feature
<222>  (5)..(5)
<223>  Xaa may be any naturally occurring amino acid

<220>
<221>  misc_feature
<222>  (6)..(6)
<223>  Xaa may be Ile, Val, Met, Ser, Ala, Cys, Thr, Pro or Leu

<220>
<221>  misc_feature
<222>  (8)..(8)
<223>  Xaa may be any naturally occurring amino acid

<220>
<221>  misc_feature
<222>  (9)..(9)
<223>  Xaa may be Ile or Val

<400>  766

Xaa Met Trp Ala Xaa Xaa Met Xaa Xaa
1               5

<210>  767
<211>  9
<212>  PRT
<213>  Unknown

<220>

<223>  DR7 deleterious motif

<220>
<221>  misc_feature
<222>  (1)..(1)
<223>  Xaa may be any naturally occurring amino acid

<220>
<221>  misc_feature
<222>  (3)..(3)
<223>  Xaa may be any naturally occurring amino acid

<220>
<221>  misc_feature
<222>  (5)..(6)
<223>  Xaa may be any naturally occurring amino acid

<220>
<221>  misc_feature
<222>  (7)..(7)
<223>  Xaa may be Gly, Arg or Asp

<400>  767

Xaa Cys Xaa Gly Xaa Xaa Xaa Asn Gly
1               5